(19) Europäisches Patentamt
European Patent Office
Office européen des brevets

(11) EP 4 480 943 A1

(12) EUROPEAN PATENT APPLICATION

(43) Date of publication:
**25.12.2024 Bulletin 2024/52**

(21) Application number: **23305999.7**

(22) Date of filing: **22.06.2023**

(51) International Patent Classification (IPC):
***C07C 233/49*** *(2006.01)* ***C07C 237/12*** *(2006.01)*
***C07C 237/22*** *(2006.01)* ***C07C 271/16*** *(2006.01)*
***C07D 233/64*** *(2006.01)* ***C07K 5/00*** *(2006.01)*
***C07D 295/13*** *(2006.01)* ***A61K 47/54*** *(2017.01)*

(52) Cooperative Patent Classification (CPC):
**C07C 233/49; A61K 47/542; A61K 47/6929; C07C 237/12; C07C 237/22; C07C 271/16; C07D 233/64; C07D 295/13; C07K 5/06086**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Oz Biosciences**
**13288 Marseille Cedex 09 (FR)**

(72) Inventors:
- **ZELPHATI, Olivier**
  **13013 MARSEILLE (FR)**
- **POULHES, Florent**
  **13013 MARSEILLE (FR)**

(74) Representative: **Santarelli**
**Tour Trinity**
**1 bis Esplanade de la Défense**
**92035 Paris La Défense Cedex (FR)**

# (54) NEW CLASS OF LIPIDS FOR DELIVERING ACTIVE INGREDIENTS INTO CELLS

(57) The present invention concerns a lipid of formula (I), a composition comprising said lipid of formula (I), a process for manufacturing said lipid of formula (I) and/or said composition, the lipid of formula (I) and/or the composition for its use as a medicament, the use of said lipid of formula (I) and/or of said composition as a vector for delivering an active ingredient to subject(s), organ(s), cell(s) and/or tissue(s). a method for transfecting cells with a nucleic acid and/or a protein and/or a peptide and/or a polysaccharide and/or a lipid and/or a small organic or inorganic molecule and/or any type of bioactive molecule using said lipid of formula (I) and/or said composition, transfected cells obtained by said method, and a kit comprising said lipid of formula (I) and/or said composition.

EP 4 480 943 A1

## Description

## FIELD OF THE INVENTION

The present invention concerns a lipid of formula **(I),** a composition comprising said lipid of formula (I), a process for manufacturing said lipid of formula (I) and/or said composition, the lipid of formula (I) and/or the composition for its use as a medicament, the use of said lipid of formula (I) and/or of said composition as a vector for delivering an active ingredient to subject(s), organ(s), cell(s) and/or tissue(s). a method for transfecting cells with a nucleic acid and/or a protein and/or a peptide and/or a polysaccharide and/or a lipid and/or a small organic or inorganic molecule and/or any type of bioactive molecule using said lipid of formula (I) and/or said composition, transfected cells obtained by said method, and a kit comprising said lipid of formula (I) and/or said composition.

## BACKGROUND AND PRIOR ART

Gene therapy, including nucleic acid-mediated therapy, represents today one of the most promising opportunities for curing diseases otherwise impossible to fight by traditional means. These innovative protocols imply the delivery *in cellulo* of biologically active ingredients in a safe way, using a broad variety of delivery vehicles. The intracellular delivery of biologically active agents into cells, organs and/or organisms finds applications in a large variety of field ranging from biology to medicine. In biology, the introduction of nucleic acids (transfection) such as DNA or RNA can be used in particular for studying the regulation of the expression of genes, clarifying, silencing or editing their function as well as overexpressing them. In the same way, the intracellular transport of peptides, proteins, polysaccharides, lipids, small organic and inorganic molecules and any other biologically active molecule makes it possible to study the fundamental biological mechanisms and to develop therapeutics. Nucleic acids therapeutics are susceptible to degradation by nucleases and cannot penetrate the cells due to their size and charge. It is also the case with various proteins, peptides or small molecules. Accordingly, development of drug delivery systems to target cells by clinically translatable systems is critical to provide opportunities to tackle a series of life-threatening diseases.

Among the large number of techniques used for introducing nucleic acids into cells, lipids and particularly cationic lipids in the form of systems which are organized or not (such as liposomes, uni-lamellar or multi-lamellar vesicles, hexagonal phases and/or micelles), and/or a mixture of lipids are probably the most widely used. They form complexes with nucleic acids such as lipoplexes or lipopolyplexes or lipid nanoparticles (LNPs) and allow the delivery of nucleic acids, proteins and peptides to pass through the cell membranes. Positively charged lipid formulation forms complexes with negatively charged nucleic acid. The resulting positively charged complexes can then bind to the negatively charged cell membrane. The exact mechanism of how nucleic acid/chemical complexes pass through cell membrane is unclear but it is believed that endocytosis is involved in the process of cellular uptake. The efficiency of chemical methods is largely dependent on factors such as pH, nucleic acid / reagent ratio, salt content or temperature. As a consequence, it is clearly difficult to predict how a chemical reagent will behave in transfection, especially in an *in vivo* application.

Among all the main known cationic lipid structures, we can cite in a non-exhaustive manner and by way of examples: monovalent cationic lipids in the form of quaternary ammonium salts, such as DOTMA (N-[1-(2,3-dioleyloxy) propyl]-N,N,N-trimethylammonium chloride), DOTAP (I ,2-dioleoy-3-tri-methylarnmonium propane) or DDAB (dioctadecyldimethylammonium bromide), monovalent cationic lipids in the form of pyridinium salts such as SAINT-2 (N-methyl-4-(dioleyl) methylpyridinium chloride), multivalent cationic lipids in the form of lipospermines such as DOGS (5-carboxyspermylglycine-dio-ctadecylamine), and DOSPA (2,3-dioleyloxy-N-[2( sperminecarboxamido)ethyl]-N,N-dimethyl- propanammonium trifluoroacetate), multivalent cationic lipids in the form of lipopolylysines and cholesterol cationic derivatives such as DC-Chol (3 ?[N-(N'-N',-dimethylaminomethane)-carbamoyl] cholesterol).

The use of cationic lipids (formulated or not with a neutral or helper lipid) for transfection has numerous advantages as they are not immunogenic, unlike viral agents, are simple to use, make it possible to deliver nucleic acids without a size limit, have high affinity with any kind of nucleic acids and can be produced in large quantities. Furthermore, lipofection (delivery of nucleic acids by lipid-based formulation) by formulated cationic lipids is the most used method in research laboratories for transfecting cells *in vitro* due to its ease of use, its efficacy in the presence or absence of serum for a large variety of cell types, in particular adherent cells and its versatility for the delivery of nucleic acids. These assets justify the large number of research program dealing with the development of both *in vitro* and *in vivo* cationic lipids as transfection vectors. However, these synthetic vectors are poorly or not at all effective for the transfection of certain cell types, in particular cells in suspension such as lymphocytes, stem cells, primary cells (non-dividing) etc. The reduced efficacy of the cationic lipids in transfecting certain cells is explained by a poor ability of the lipoplexes to attach to the surface of these cells.

In this way, various approaches using specific ligands such as transferrin, epidermal growth factor (EGP), monoclonal antibodies or peptides, coupled or attached with a cationic polymer or a co-lipid formulated with cationic lipids, have been successfully tested. However, quiescent cells are also very difficult to transfect using lipofection, as the absence

of means for targeting the nucleus, such as nuclear localization sequences prevent the complexes present in the cytosol to pass through the pores of the nuclear membrane and thus to be transcribed. Several approaches using ternary complexes (lipoplexes: cationic lipids and nucleic acids plus a nuclear targeting element) based on histones, peptide nuclear localization sequences, have been studied in order to get round this problem. However, the addition of these targeting elements to the cationic lipids makes the formulation and preparation of the complexes difficult and their use is thus very limited. Furthermore, and most importantly, the *in vivo* efficacy of lipofection remains too low, limited by the enzymatic degradation of the complexes, their pharmacology and by the presence of transfection inhibitors in body fluids and mucus, such as proteins and polysaccharides, which strongly inhibit transfection. The main parameters affecting lipoplex transfection efficacy have been widely studied, suggesting that the best levels of *in vivo* gene expression were obtained using high lipid/nucleic acid ratios which lead to significant toxicity. Thus, the *in vivo* cytotoxicity of the cationic lipids remains the major drawbacks of this strategy, mainly because of poor biodegradability of the cationic lipids which are not naturally present in cells as well as the use of permanent positive charge that is well-known for being detrimental to cell's survival.

Various efforts have been made to address those issues, such as the synthesis of lipids having a bond sensitive to the reducing medium in the fatty chains in order to facilitate their metabolism, or the incorporation of a vinyl ether group in the spacer arm of a cationic lipid. Vinyl group being sensitive to acidic pH, it allowed a rapid cleavage of the lipid in the acid medium of the endosomes, leading to a destabilization of the structure of the lipoplexes embedded in the endosome's membrane, allowing thus early release of the DNA in the cytosol. Similarly, a family of cationic lipids provided with a spacer arm containing a linear or cyclic orthoester group stable at physiological pH, that hydrolysed at acid pH has been described, while there are numerous examples of cationic lipids containing ester groups such as DMT-M(Gly) and DOTM(Gly)-tetraesters.

In the same way, to tackle biodegradability issue, lack of targeting and to add an additional biocompatibility feature, amino acid-based lipids with cleavable linkers have been designed and developed (WO 2009/106713 A3). These molecules displayed significant progress in transfection efficiency, safety and extended transfection applications to a variety of biomolecules, due to their innovative design. However, the presence of permanent positive charge(s) at physiological pH, minimizes their in *vivo* efficiency despite a structure-related better biodegradability in intracellular medium.

As recent reports demonstrated that the combined presence of permanent positive charge and of non-degradable backbones were the two main obstacles for safe and efficient *in vivo* gene delivery, new generations of lipids have been developed to reduce toxicity of permanently charged cationic lipid without compromising transfection efficacy. Based on the same structural model (a polar nitrogen-based head, a linker region, and two or multiple nonpolar hydrocarbon tails), they present an optimal pKa value of 6.2-7.0, which is designed to be positively charged at acidic pH to allow efficient encapsulation of nucleic acids such as DNAs or RNAs into lipid nanoparticles (LNPs) and effective DNA or RNA intracytoplasmic release upon protonation under acidic condition (endosomal pathway). On the other hand, they are neutral under physiologic conditions (pH=7.0 to 7.4) which prevent nonspecific interactions with fluids components and tissues, higher biocompatibility level and significant reduction of toxicity in comparison to the permanently charges cationic lipids. Lipids with an appropriate pKa would change their electrostatic charge to ensure proper vector formation, optimal *in vivo* circulation, and efficient cytosolic release of the therapeutic cargo. Specifically, such ionizable lipids should be designed to be positively charged at acidic pH during the production of lipid nanoparticles (LNPs) so that the electrostatic interactions with the Nucleic Acids (NAs) could be maximized (high NA condensation = high loading efficiency). Then, the lipids should turn into almost neutral under physiological conditions to prevent rapid sequestration by immune cells during systemic circulation. Also, the ionizable lipids should become positive again upon exposure to the typical acidic environment of the endosomes, thus, destabilizing the endosomal membrane and promoting the cytosolic delivery of the genetic cargo. Finally, in the cytosol, the neutral pH would favour the release of the NAs from the ionizable lipids, thus, enabling their free interaction with the cell machinery. The ionizable lipids is thus the critical component of the nanoparticle formulation since it is responsible to protect nucleic acids from degradation and to release the NAs into cells.

Ionizable lipids have been associated with specific lipidic components into supramolecular architectures known as lipid nanoparticles (LNP) or stable nucleic acid lipid particles (SNALP) or lipid polycations-DNA (LPD) or solid lipid nanoparticles (SLN) or nanostructured lipid carriers (NLC) when encapsulating a biomolecule such as a nucleic acid. These components provide the LNPs with additional features that potentialize the positive effect carried by ionizable components such as neutral phospholipids or sterol derivatives. Furthermore, when addressing *in vivo* injection, polyethylene glycol (PEG) modified-lipids (PEGylation) have been included in the LNPs formulation to increase its circulation stability, reduce nonspecific detrimental interactions, reduce their aggregation, and thus improve nucleic acid delivery efficiency. The versatile nature of lipids and their capacity to be easily modulated and modified make them ideal candidates for the transport of long and fragile nucleic acids such as DNA, mRNA or saRNA. In recent years, the number of research studies oriented towards the design and synthesis of such LNPs and especially on the design and synthesis of ionizable lipids has grown exponentially. Some recent examples may be found in comprehensive reviews (An ionizable lipid toolbox for RNA delivery, Han et al., Nature Communications 12, 7233 (2021); Lipid nanoparticles for mRNA delivery,

Hou et al., Nature reviews materials 6, pages 1078-1094 (2021)). The molecules described in these different studies highlighted the fact that ionizable lipids used in LNPs can be of different structures that may impact positively nucleic acid delivery, especially *in vivo.* Accordingly in recent years several ionizable lipid structure have been described:

- unsaturated ionizable lipids (such as DLin-MC3-DMA, A6, OF-02, A18-Iso5-2DC18),
- multi-tail ionizable lipids (such as SM102, ALC-0315, A9, Lipid 2,2 (8,8); 98N12-5, C12-200, cKK-E12 and 9A1P9),
- ionizable polymer-lipids (such as 7C1 and G0-C14),
- acid-sensitive ionizable lipids (such as L319, 30403, OF-Deg-Lin and 306-O12B)
- branched-tail ionizable lipids (such as 306Oi10 and FTT5).

To date, formulation in lipid nanoparticles (LNPs) represents the most advanced delivery platform for gene therapy, vaccines and reliable candidates to treat manifold diseases. LNPs were recently FDA approved for the treatment of amyloidosis disease by delivery of siRNA, and more recently for the widely distributed SARS-CoV-2 vaccines, based on mRNA-LNPs. Interestingly, ionizable lipids efficient for siRNA (such as DLin-MC3-DMA) did not share the same structural patterns than those used for efficient mRNA delivery (such as SM102 and ALC-0315). This demonstrates the need to develop a reliable platform for the synthesis of a wide range of ionizable lipids able to provide components perfectly tuned to the requirements of each possible application. One universal LNPs based synthetic platform will be more convenient, cost effective and somehow safest for nucleic acids therapeutics. Furthermore, and despite the current LNPs breakthrough, there are still some concerns about the inherent toxicity of some of these nanocarriers. Notably, the biodistribution and cellular uptake of LNPs which are affected by their surface composition as well as by their colloidal stability. Consequently, new generation of tunable lipids with higher biodegradability & biocompatibility, better capacity to form small and polydisperse nanoparticles, diverse and/or broader pKa range in order to target different tissues, diseases or harsh biological environment and higher compatibility towards multiple nucleic acids or other types of molecules such as proteins, antibodies, peptides are needed.

The present inventors have surprisingly found a new class of lipids, and in particular a new class of tunable lipids, which can be used for delivering active agents into cells without having the above-mentioned drawbacks.

In particular, the present inventors have surprisingly found a new versatile family of lipids, preferably tunable lipids, which are compatible with the presence of serum and are non-toxic, allowing an effective *in vitro, ex vivo* and *in vivo* delivery of all types of biomolecules such as nucleic acids, peptides, proteins, polysaccharides, and lipids into living cells.

In particular, the present inventors have surprisingly found new class of lipids that may comprise a wide variety of amine-based end-groups allowing a fine tuning of reversible positive charges thereby conferring to the new class of lipids a net pKa value consistent with the formulation of LNPs associated to efficient delivery of biomolecules into cells. The innovative structure of these new lipids, preferably new tunable lipids, combines:

- The amphiphilic properties of the lipids in order to form organized structures (such as liposomes, lipid nanoparticles and micelles) allowing the transport and vectorization of active molecules towards their target, their properties of forming non-covalent complexes with negatively charged nucleic acids, and their properties of destabilizing the cell membranes.

- The properties of the head group of the lipid, preferably of the tunable head group of the lipid, advantageously constituted by a chemical moiety centred on a Nitrogen atom, itself part of a chemical group chosen to become protonated only at acidic pH while remaining neutral otherwise. Moreover, these sequences of these groups, preferably of these tunable groups, interact with several types of biomolecules including nucleic acids, peptides and proteins and are completely non-toxic.

- The ability to degrade rapidly in intracellular environment due to the presence of a spacer arm between the lipophilic part and the head, preferably the tunable head, provided with a functional group incorporating a bond sensitive to its environment (such as pH, oxidation-reduction and/or enzymes) which can be cleaved in cytosolic medium and/or ester bond in the hydrophobic chain. The compounds originating from the resultant degradation are natural molecules (fatty acids. amino acids-based products) which are easily metabolized by the cell. The biodegradable character of these molecules makes them non-cytotoxic as a result.

The present invention thus offers an innovative solution to produce delivery system such as lipid nanoparticles or liposomes or lipid-based particles using a brand-new series of lipidic molecules able to efficiently complex, protect and deliver a wide array of various active ingredients due to their carefully chosen molecular design. The present invention describes with care an association of a lipophilic area mixing in adequate proportions several hydrophobic moieties such as alkyl chains, unsaturation, and optionally bioreducible groups that offer to these molecules an increased biocompatible and/or biodegradable character. This strategic choice is completed by the incorporation of a linker pattern that plays the

dual role of maintaining a right distance between hydrophobic and hydrophilic parts and of contributing to the biodegradability of the whole structure. Finally, the polar head is constituted by a chemical moiety centred on a Nitrogen atom, itself part of a chemical group chosen to become protonated only at acidic pH (*i.e.*, *pH=3.5-6.9*) while remaining not charged at higher pH.

Special care has been put to the incorporation of biodegradable or bioreducible moiety into the lipidic chemical structure. Indeed, such moiety provides the molecule a faster metabolization and its removal from the body following delivery of the active pharmaceutical ingredient to a target area.

Additionally, compounds claimed in this invention are all parts of pharmaceutical formulation, meaning that their efficiency can rely on the choice of the other components of the formulation, their association (self-assembly) using a carefully experimental procedure including microfluidic processing, high pressure flow or extrusion systems or any other controlled formulations procedure.

## SUMMARY OF THE INVENTION

The present invention relates to a lipid of formula (I), or a stereoisomer thereof, or a pharmaceutically acceptable salt thereof:

$$R\text{-}Sp\text{-}Z_s \qquad (I)$$

where:

- **R** comprises one or more branched or linear, unsaturated or saturated, optionally fluorinated, alkyl chains, comprising 6 to 24 carbon atoms, preferably between 10 and 18 carbon atoms, optionally comprising one or more heteroatoms other than fluorine; or one or more cyclic or polycyclic groups known to be lipophilic, optionally comprising one or more heteroatoms, such as a steroid group optionally comprising one or more heteroatoms, a polyaromatic group optionally comprising one or more heteroatoms, or an alkaloid derivative group optionally comprising one or more heteroatoms; or a natural or synthetic lipid optionally comprising one or more heteroatoms; or a combination thereof;
- **Sp** comprises one or more branched or linear, unsaturated or saturated, optionally fluorinated alkyl chains, comprising 6 to 24 carbon atoms, preferably between 10 and 18 carbon atoms, optionally comprising one or more heteroatoms and/or bioreducible bonds; and
- $\mathbf{Z_s}$ comprises one or more branched or linear, unsaturated or saturated, optionally fluorinated alkyl chains, comprising 2 to 36 carbon atoms, preferably comprising 2 to 24 carbon atoms, optionally comprising one or more heteroatoms other than fluorine, and/or bioreducible bonds, covalently linked to one or more linear or cyclic nitrogen-based chemical moieties;

characterized in that:

- **R** corresponds to formula **(II):**

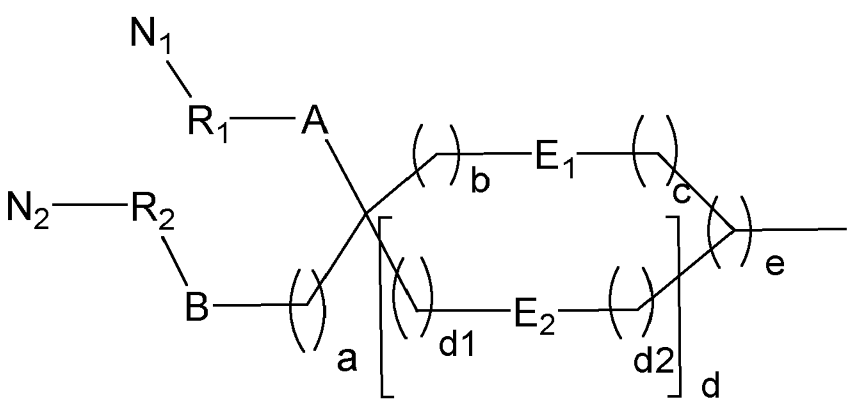

**(II)**

where:

- $\mathbf{N_1}$ **and** $\mathbf{N_2}$, identical or different represent a linear, branched saturated or unsaturated, hydrocarbon group comprising from 1 to 8 carbon atoms, preferably from 1 to 4, used to terminate carbon chains, preferably $\mathbf{N_1}$ **and** $\mathbf{N_2}$, identical or different, are either a methyl, ethyl, *n*-propyl, *i*-propyl, *n*-butyl, *s*-butyl, *i*-butyl or *t*-butyl group;

- **$R_1$ and $R_2$**, identical or different, represent a linear, branched and/or cyclic, saturated or unsaturated, hydrocarbon group comprising from 6 to 24 carbon atoms, preferably from 10 to 18 and optionally comprising one or more heteroatoms;
- **A and B,** identical or different, represent a O-C(O), C(O) NH-, -NHCO-, -NH- or O-group;
- **a** is an integer comprised between 1 and 4, preferably **a** is an integer equal to 1, 2 or 3;
- **b** is an integer comprised between 0 and 6, preferably **b** is an integer equal to 0 or 1;
- **$E_1$ and $E_2$**, identical or different, represent a -C(O)O-, -C(O)-NH-, -NH-, -O- or -S-group;
- **c** is an integer comprised between 0 and 2;
- **d** is an integer equal to 0 or 1;
- **$d_1$** is an integer comprised between 0 and 6, preferably **$d_1$** is an integer equal to 0 or 1;
- **$d_2$** is an integer comprised between 0 and 6, preferably **$d_2$** is an integer comprised between 0 and 2; and
- **e** is an integer comprised between 0 and 6, preferably between 0 and 2, and more preferably between 0 and 1;

- **Sp** corresponds to the general formula (III):

$$-\left(Gr\right)_m\left(RAc\right)_n- \quad \textbf{(III)}$$

where:

- **Gr** represents a linear or branched hydrocarbon group, comprising from 1 to 15 carbon atoms, preferably from 1 to 8, and optionally comprising one or more heteroatoms;
- **m** is an integer equal to 0 or 1;
- **RAc** represents an amino acid radical; and
- **n** is an integer equal to 0 or 1; and

- **Zs** corresponds to formula **(IV)**:

$$\left[\left(S_1-D-S_2\right)_p\left(Q\right)_q\left(Z\right)_r\right]_s \quad \textbf{(IV)}$$

where:

- **$S_1$ and $S_2$,** identical or different, represent a linear or branched hydrocarbon group comprising from 1 to 15 carbon atoms, preferably from 1 to 6 carbon atoms, optionally comprising one or more heteroatoms preferably chosen from nitrogen, oxygen, sulfur, bromine, iodine, chlorine and fluorine, more preferably from nitrogen, sulphur, bromine, iodine, chlorine and fluorine;
- **D** represents one or more vinyl ether groups, one or more acylhydrazone groups, a disulfide bond, an ester bond or one or more photosensitive groups;
- **p** is an integer equal to 0 or 1;
- **Q** is a branched hydrocarbon group comprising from 1 to 20 carbon atoms, preferably from 1 to 12 carbon atoms, and one or more heteroatoms, and which can optionally be covalently coupled with the **$S_2$** or **RAc** or **Gr** or **R** group on the one hand, and to at least two **Q** and/or **Z** groups on the other hand;
- **q** is an integer comprised between 0 and 8, preferably between 0 and 3;
- **Z** is a nitrogen (N) centred terminal ionizable moiety, preferably Z represents a tertiary aliphatic amine such as N,N-dialkylamine, or a cyclic amine moiety such as N-substituted Pyrrolidine, N-substituted piperidine, N-substituted morpholine, N-substituted piperazine or N-substituted pyridine;
- **r** is an integer comprised between 1 and 16, preferably between 1 and 8, it being preferably understood that if **q** is equal to 1 then **r** is at least equal to 2 and that if **r** is greater than 1, then the **Z** groups can be identical or different; and
- **s** is an integer equal to 1 or 2.

The present invention also relates to a composition comprising the lipid of formula (I) as previously defined.

The present invention also relates to a process for manufacturing the lipid of formula (I) as previously defined and/or the composition as previously defined.

The present invention also relates to a lipid of formula (I) as previously defined and/or composition as previously defined for its use as a medicament.

The present invention also relates to the use of the lipid of formula (I) as previously defined and/or of the composition as previously defined as a vector for delivering an active ingredient to subject(s), organ(s), cell(s) and/or tissue(s).

The present invention also relates a method for transfecting cells with a nucleic acid and/or a protein and/or a peptide and/or a polysaccharide and/or a lipid and/or a small organic or inorganic molecule and/or any type of bioactive molecule using the lipid of formula (I) as previously defined and/or the composition as previously defined.

The present invention also relates to transfected cells obtained by the method as previously defined.

The present invention also relates a kit comprising the lipid of formula (I) as previously defined and/or the composition as previously defined further comprising a nucleic acid and/or a protein and/or a peptide and/or a polysaccharide and/or a lipid and/or a small organic or inorganic molecule and/or any type of bioactive molecule, and optionally instructions of use.

## DEFINITIONS

By "lipid" it is preferably understood an organic amphiphilic molecule that includes in its structure an apolar hydrophobic carbonated region and a polar hydrophilic region, linked together by a chemical spacer. By "tunable lipid" it is preferably understood a lipid as previously defined having an hydrophilic area that may include one or several positive charges depending on the pH of its direct environment, enabling the possibility for the user to tune its chemical properties by choosing the right pH. Preferably, it does not present any permanent positive charges at physiological pH (7.0-7.4) while it will become cationic as the pH of its physiological environment decreases. Preferably, its amphiphilic nature makes it generally insoluble in water and soluble in some extent in organic solvent. Preferably, the term "tunable lipid" also implies that such lipid, when processed properly may form self-assembled structures in aqueous environment.

By "biodegradable lipid" it is preferably understood an organic amphiphilic molecule that includes in its structure an apolar hydrophobic carbonated region and a polar hydrophilic region, linked together by a chemical spacer, and that organic amphiphilic molecule chemical and physical characteristics may undergo deterioration and may completely degrade under biological conditions when exposed to microorganisms, aerobic, and anaerobic processes. In addition, the compounds or chemical entities resulting of the degradation if this molecule are biocompatible.

In the present invention, unless otherwise indicated, by "heteroatom" it is preferably understood an atom chosen from nitrogen, oxygen, sulfur, phosphorus and halogens.

In the present invention, unless otherwise indicated, by "halogen" it is preferably understood bromine, iodine, chlorine and fluorine.

By "fluorinated" it is preferably understood comprising one or more fluorine atoms.

By "comprising one or more heteroatoms other than fluorine" it is preferably undersood comprising one or more atoms chosen from nitrogen, oxygen, sulfur, phosphorus and halogens other than fluorine.

By "hydrocarbon group" is preferably understood any group comprising one or more carbon atoms, optionally bonded to one or more hydrogen atoms.

By "one or more" it is preferably understood one, two, three, four, five, six, seven, eight, nine or ten, more preferably one, two, three, four or five and even more preferably one, two or three.

By "transfection" it is preferably understood the process to introduce native, wild type or recombinant genetic material or proteins into the target cell via synthetic or non-viral delivery systems. The nature of the genetic material introduced, and the mechanisms involved in this step are dependent on the type of nucleic acids, proteins or small molecules used. For example, the genetic materials introduced can encode a large variety of gene of interest such as reporter genes, enzymes, endonucleases, nucleases or recombinase for genome editing, short harping RNA, antisense RNA for gene silencing, mRNA, tRNA, receptors such as engineering antigen receptor (including, but not limited to, T cell receptor, chimeric antigen receptor and chimeric cytokine receptor), growth factors, hormone, cell surface proteins, secreted proteins, signalling proteins or any polypeptides, and proteins or nucleic acids with therapeutic interest.

By "biologically active molecule", "active molecule", "biologically active agent" and/or "active ingredient", it is preferably understood any molecule or macromolecule having a specific activity in cells and used in numerous fields ranging from cell biology to medicine. Examples of such molecule or macromolecule include, but are not limited to, nucleic acid(s), polysaccharide(s), lipid(s), polynucleotide(s), morpholino oligonucleotide(s), aptamer(s), protein(s), peptide(s), polypeptide(s), peptoid(s), small organic or inorganic molecule(s), drug(s) or any compounds of pharmaceutical interest, antigen(s), and mixtures thereof.

By "small organic or inorganic molecule(s)" it is preferably understood a molecule of known structure and

molecular weight, that is not of polymeric nature, and that has preferentially a molecular weight below 1500 g/mol.

Drug(s) or any compounds of pharmaceutical interest may be any molecule or compound, including a salt or derivative thereof, capable of exerting a desired effect such as a biological, physiological, and/or cosmetic effect, on a cell, tissue, tumor, organ, or subject. Examples of drug(s) or any compounds of pharmaceutical interest include, but are not limited to, oncology drugs (including chemotherapy drugs, hormonal therapeutic agents, immunotherapeutic agents, and/or radiotherapeutic agents), lipid-lowering agents, anti- viral drugs, antiinflammatory compounds, antidepressants, stimulants, analgesics, antibiotics, birth control medication, antipyretics, vasodilators, anti-angiogenics, cytovascular agents, signal transduction inhibitors, cardiovascular drugs such as anti-arrhythmic agents, hormones, vasoconstrictors, and steroids. More particularly, examples of drug(s) or any compounds of pharmaceutical interest include, but are not limited to, folate or sodium valproate, antibodies, antigens, lymphokines, interleukins, necrosis and apoptosis factors, interferons, growth factors, tissue plasminogen activators, factor VIIIc, erythropoietin, insulin, calcitocin, thymidine kinase, and combination thereof.

Drug(s) or any compounds of pharmaceutical interest may be therapeutically active themselves or may be prodrugs, which become active upon further modification.

Examples of nucleic acids, include but are not limited to, deoxyribonucleic acid (DNA) such as a gene or plasmid DNA (pDNA), linear coding DNA, artificial chromosomes, antisense oligonucleotides (ASOs) or a ribonucleotide acid (RNA) such as messenger RNAs (mRNAs), small interfering RNAs (siRNA), self-amplifying RNAs (saRNAs), circular RNA (cirRNA), microRNAs (miRNAs), single-guide RNAs (sgRNAs)-mediated CR!SPR-Cas9 system, double-stranded RNA (dsRNA), short hairpin RNA (shRNAs) ribozymes, or a modified nucleic acid such as a peptide nucleic acid (PNA), locked nucleic acid (LNA), morpholino oligonucleotides or aptamers. Nucleic acids may be natural or artificial. Nucleic acids may be of animal, human, plant, bacterial or viral origin. Nucleic acids can be used to express, alter, downregulate or silence the translation (that is the expression) of a gene of interest. Their function as a therapeutic agent can be a gene or mRNA coding for a polypeptide and/or a protein of interest in a host cell or can be an antisense function controlling the expression of a gene, its transcription to RNA or its translation to protein. They can also act as a ribozyme or interfering RNA (such as sRNA, shRNA or miRNA) with the expression of a gene. They can also edit a gene by correcting or adding a mutation (such as CRISPR Cas9).

Non-limiting examples of genes of interest include, but are not limited to, genes associated with metabolic diseases and disorders (such as liver diseases and disorders), genes associated with cell proliferation, tumorigenesis, and/or cell transformation (including a cell proliferative disorder such as cancer), angiogenic genes, receptor ligand genes, immunomodulator genes (such as those associated with inflammatory and autoimmune responses), genes associated with viral infection and survival, and genes associated with neurodegenerative disorders.

The nucleic acid that is present in a lipid-nucleic acid particle can be in any form. The nucleic acid can, for example, be single-stranded DNA or RNA, or double- stranded DNA or RNA, or DNA-RNA hybrids. Non-limiting examples of double-stranded RNA include siRNA and miRNA. Non-limiting examples of single-stranded RNA include mRNA. Non-limiting examples of double-stranded DNA include plasmid and linearized DNA. The nucleic acid may optionally comprise one or more modified nucleotides including, but not limited to, 2'-O-methyl nucleotides, 2'-deoxy nucleotides, 2'-O-(2-methoxyethyl) nucleotides, locked nucleic acid (LNA) nucleotides, 5-C-methyl nucleotides, 4'-thio nucleotides, -amino nucleotides, 2'-C-allyl nucleotides, and mixtures thereof.

For use in the fields of gene therapy, vaccines and immunotherapy, the nucleic acid may advantageously be DNA and RNA and preferably comprises an expression cassette constituted by one or more sequences of DNA or RNA encoding the polypeptide of interest under the control of one or more promoters and a transcriptional terminator which are active in the target cells.

By "polypeptide" it is preferably understood any amino acid chain irrespective of its size. Thus, this term may cover peptides and proteins.

By "target cell" it is preferably understood any cell that can be targeted for transfection.

By "cell" is preferably understood a single and/or isolated cell, or a cell that is part of a multicellular entity such as a tissue, an organism or a cell culture. The cell can be a primary cell or established cell lines or a stem cell or a progenitor cell.

The term "primary cell" as used herein is well known in the art and may refer to a cell that has been isolated from a tissue and has been established for growth *in vitro.*

The cell can be an eukaryotic cell such as for example, but not limited to, endothelial, epithelial, fibroblastic, hepatic, hematopoietic (including but not limited to lymphocytes and/or monocytes macrophages natural killer dendritic cells), muscle, nerve cells (an in particular all types of neurons including but not limited to cortical, hippocampal, sensory neurons, motor neurons, dorsal route ganglion, oligodendrocytes, cerebellar granule, neural stem cells and basket cells, Betz cells, medium spiny neurons, Purkinje cells, pyramidal cells, Renshaw cells, granule cells or anterior horn cells and glial cells astrocytes), stem cells, embryonic stem cells, hematopoietic stem cells, germ cells, a tumor cells, a lymphoid cell lineage and somatic cells.

An eukaryotic cell as used herein, can refer to any cell of a multi-cellular eukaryotic organism, including cells from animals like vertebrates. Preferably, said target cell can be a mammalian cell. The term "mammalian cell" as used herein, is

well known in the art and may refer to any cell belonging to or derived from an animal that is grouped into the class of Mammalia.

The cells can be prokaryotic (such as bacteria), plant cells, insect, yeast, or parasite cells.

The cells can be differentiated or totipotent or omnipotent or multipotent or oligopotent or unipotent or pluripotent. The cells can be embryonic stem cells or induced pluripotent stem cells (iPS) or differentiated from embryonic or iPS. The cells to be transfected can be non-permissive cells, hard-to-infect cells, or easy to infect cells. Tumor cells and cell lines as known in the art can be for example, but not limited to, lymphoma cell lines (such as for example Jurkat, CEM, H9, Daudi, SUP-M2 and KARPAS-299), dendritic cell lines (such as DC2.4 and Mutu), natural killer cell lines (such as NK-92 and KHYG-1), epithelial cell lines (such as NIH-3T3, COS, CHO and HEK293), pancreatic tumor cells (such as PANC-1), a stem cell line (KG1a), breast cancer cells (such as MCF-7, T74D and MDA-MB361), a neuroblastoma (such as SH-SY5Y and N2a), a fibrosarcoma (such as HT1080), an astrocytoma (such as 1321N1), endothelial cells (such as HUVEC, HMEC and HCAEC).

By "signalling molecules and molecules that may be involved in the signalling pathways inside of the target cells" it is preferably understood activators or inhibitors of the protein kinase C or phospholipase signalling pathway, such as activators or inhibitors of the cellular metabolism pathways (such as for example AMPK signalling, insulin receptor signalling and glutamine metabolism pathway), activators or inhibitors of the TLR-pathway (such as Toll-Like Receptor), of the PRRs-pathway (Pattern Recognition Receptors), of the PAMPs-pathway (Pathogen-Associated Molecular Patterns), of the DAMPs-pathway (Damage-Associated Molecular Patterns), AhR ligands (aryl hydrocarbon receptor ligands), the NOD-Like receptors (NLRs), the RIG-I-Like receptors (RLRs), cytosolic DNA sensors (CDS), the C-type lectin receptors (CLRs), activators or inhibitors of inflammasomes or inflammation or autophagy pathway, activators or inhibitors of the JAK/STAT pathway, activators or inhibitors of the ubiquitin and ubiquitin-like/proteasome pathway, activators or inhibitors of the PI3K/AKT pathway, activators or inhibitors of the tyrosine kinase, activators or inhibitors of the MAP kinase pathways, activators or inhibitors of the GPCR, calcium, cAMP signalling pathway, activators or inhibitors of the cell cycle, checkpoint control and DNA repair mechanisms, activators or inhibitors of the apoptosis pathway, regulators of the reactive oxygen species (such as ROS and NOS), activators or inhibitors of immune checkpoints, regulators of protein synthesis and RNA degradation process, regulators of keys elements having a role at the chromatin or DNA level (such as activators or inhibitors of protein acetylation, histone lysine methylation, DNA methylation and nuclear receptor signalling), regulators of microtubule and actin dynamics pathways.

By "lipid of formula (I)" it is preferably understood the lipid of the present invention.

Preferably, the terms "conjugated" and "coupled" used herein have the same meaning.

## DETAILED DESCRIPTION

For the purpose of this application, unless otherwise stated, the ranges of values indicated are inclusive.

A first object of the present invention relates to a lipid, preferably a tunable biodegradable lipid of formula **(I),** or a stereoisomer thereof, or a pharmaceutically acceptable salt thereof:

$$R\text{-}Sp\text{-}Z_s \qquad (I)$$

wherein:

- **R** represents a lipophilic region, preferably R comprises one or more branched or linear, unsaturated or saturated, optionally fluorinated, alkyl chains, comprising 6 to 24 carbon atoms, preferably between 10 and 18 carbon atoms, optionally comprising one or more heteroatoms other than fluorine; or one or more cyclic or polycyclic groups known to be lipophilic, optionally comprising one or more heteroatoms, such as a steroid group optionally comprising one or more heteroatoms, a polyaromatic group optionally comprising one or more heteroatoms, or an alkaloid derivative group optionally comprising one or more heteroatoms; or a natural or synthetic lipid optionally comprising one or more heteroatoms; or a combination thereof.
- **Sp** represents a spacer region used to separate the lipophilic region from the hydrophilic polar region, preferably hydrophilic tunable polar region, more preferably **Sp** comprises one or more branched or linear, unsaturated or saturated, optionally fluorinated alkyl chains, comprising 6 to 24 carbon atoms, preferably between 10 and 18 carbon atoms, optionally comprising one or more heteroatoms and/or bioreducible bonds; and
- $\mathbf{Z_s}$ represents a hydrophilic polar region, preferably a hydrophilic tunable polar region, used to complex and efficiently deliver biologically active component into cells. More preferably, $\mathbf{Z_s}$ comprises one or more branched or linear, unsaturated or saturated, optionally fluorinated alkyl chains, comprising 2 to 36 carbon atoms, preferably comprising 2 to 24 carbon atoms, optionally comprising one or more heteroatoms other than fluorine, and/or bioreducible bonds, covalently linked to one or more linear or cyclic nitrogen-based chemical moieties, that preferably does not bear charges at physiological pH but become positively charged if the pH of the medium dropped below the pKa value of the

entity. This polar region might incorporate bioreducible molecular patterns enabling a faster release of the biologically active compound and enhanced biocompatibility of the whole molecule.

Preferably, due to the definition of $\mathbf{Z_s}$, the lipids of the present invention, preferably the tunable lipids of the present invention, do not include any positive charge at physiological pH (7.0-7.4), which implies that the pKa of the molecules covered by the formula (I) is always lower than 7.0.

Examples of nitrogen-based chemical moieties include, but are not limited to, amines, amides, oximes, hydroxylamines, carbamates, isocyanates, isothiocyanates or cyanhydrins, either aliphatic or aromatic.

Examples of cyclic or polycyclic groups known to be lipophilic, optionally comprising one or more heteroatoms, include, but are not limited to, steroid groups.

Examples of steroid group include, but are not limited to, cholesterol and its derivatives, ergosterol, estradiol, stigmasterol, testosterone, progesterone and androsterone.

By "cholesterol derivative", it is preferably understood, cholesterol, bile acids, Vitamin $D_2$, Progestins, glucocorticoids, estrogens, androgens, phytosterols or oxysterols.

Examples of polyaromatic group include, but are not limited to, naphthalene derivatives, dansyl derivatives or anthracene derivatives.

By "naphtalene derivative" it is preferably understood 1-Naphtoic acid, 1-Naphtol, 1-Nitronaphtalen or 1-halogenonaphtalene.

By "dansyl derivatives" it is preferably understood 5-(dimethylamino)naphthalene-1-sulfone.

By "anthracene derivative" it is preferably understood anthrancene, mono-hydroxyanthracene, or poly-hydroxyanthracene.

Examples of alkaloid derivative include, but are not limited to, hygrine, retronecine, indicine, N-acetylloline, atropine, morphine, mescaline, adrenaline, putrescine, spermine, theobromine, cathinone, sedamine, connine, matrine, swainsonine, pipecolic acid, iophorecine, amurensine, berberine, glaucine, imerubine, muscimol, serotonine, quinine, ergotamine, colchicine, solanidine, cyclopamine, batrachotoxin and isoquinoline.

Examples of natural or synthetic lipid include, but are not limited to, phosphatidylcholine or its derivatives, phosphatidylethanolamine or its derivatives, phosphatidylinositol or its derivatives, sphingomyelin or its derivatives, phosphatic acid or its derivatives, lyso-phosphatidylcholine or its derivatives, lyso-phosphatidylethanolamine or its derivatives, glycero-3-phosphocholine or its derivatives, and glycerol or its derivatives.

By "bioreducible bonds" it is preferably understood disulphide bond, ester bond, vinyl ether bond or acylhydrazone bond.

By "bioreducible molecular patterns" it is preferably understood a biocompatible or bioinspired groups of atoms that can undergo degradation or deterioration under biological conditions when exposed to microorganisms, aerobic, and anaerobic processes, increasing as a consequence biocompatibility of the whole molecule. Examples of such groups include but are not limited to bifunctional ribose groups, ethylene oxide groups or amino acids groups.

Preferably, the following compounds are excluded from general formula (I):

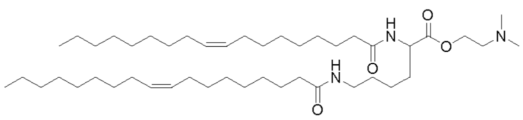
;
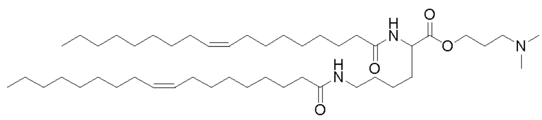
;
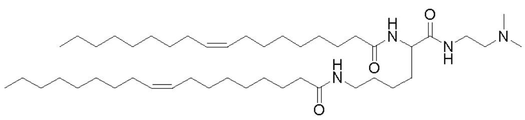
and
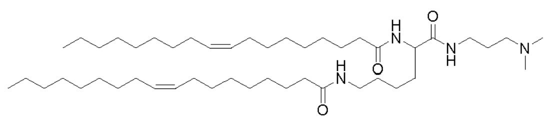
.

Preferably, in formula **(I)** as previously defined **R** corresponds to formula **(II):**

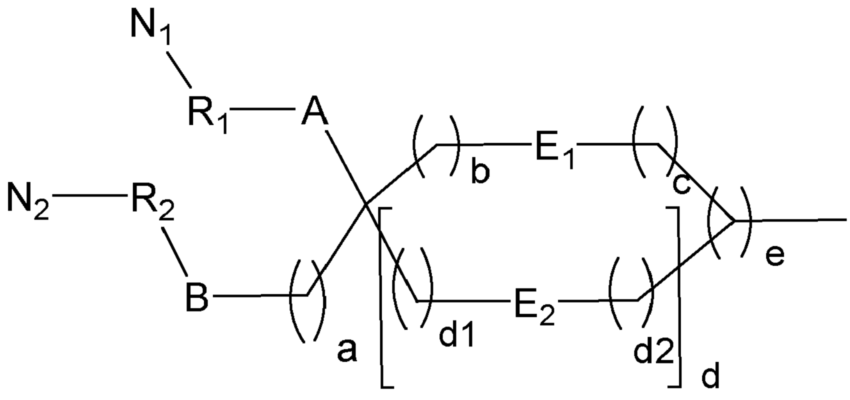

**(II)**

wherein:

- $\mathbf{N_1}$ **and** $\mathbf{N_2}$, identical or different represent a linear, branched saturated or unsaturated, hydrocarbon group comprising from 1 to 8 carbon atoms, preferably from 1 to 4, used to terminate carbon chains, preferably $\mathbf{N_1}$ **and** $\mathbf{N_2}$, identical or different, are either a methyl, ethyl, n-propyl, *i*-propyl, *n*-butyl, *s*-butyl, *i*-butyl or *t*-butyl group;
- $\mathbf{R_1}$ **and** $\mathbf{R_2}$, identical or different, represent a linear, branched and/or cyclic, saturated or unsaturated, hydrocarbon group comprising from 6 to 24 carbon atoms, preferably from 10 to 18 and optionally comprising one or more heteroatoms;
- **A and B,** identical or different, represent a O-C(O), C(O) NH-, -NHCO-, -NH- or O- group;
- **a** is an integer comprised between 1 and 4, preferably **a** is an integer equal to 1, 2 or 3;
- **b** is an integer comprised between 0 and 6, preferably **b** is an integer equal to 0 or 1;
- $\mathbf{E_1}$ **and** $\mathbf{E_2}$, identical or different, represent a -C(O)O-, -C(O)-NH-, -NH-, -O- or -S- group;
- c is an integer comprised between 0 and 2;
- **d** is an integer equal to 0 or 1;
- $\mathbf{d_1}$ is an integer comprised between 0 and 6, preferably $\mathbf{d_1}$ is an integer equal to 0 or 1;
- $\mathbf{d_2}$ is an integer comprised between 0 and 6, preferably $\mathbf{d_2}$ is an integer comprised between 0 and 2;
- **e** is an integer comprised between 0 and 6, preferably between 0 and 2, and more preferably between 0 and 1.

Preferably, in formula **(II)** as previously defined, when **a** is an integer equal to 4, the following compounds are excluded:

- 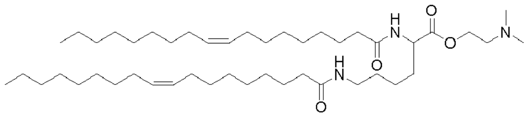

wherein according to formula I **Sp** is absent and **Zs** is $CH_2$-$CH_2$-$N(CH_3)_2$ and wherein according to formula II $\mathbf{N_1=N_2=}$ $CH_3$, $\mathbf{R_1=R_2=}$ $C_{17}H_{30}$, **A and B** identical representing -C(O)NH-, **a**=4, $\mathbf{E_1}$ representing a -C(O)O- group $\mathbf{E_2}$ being absent and **b=c=e=d1=d2=**0;

- 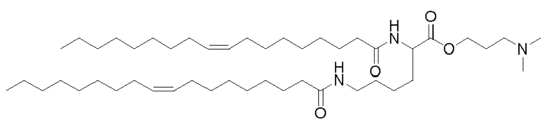

wherein according to formula I **Sp** is absent and **Zs** is $CH_2$-$CH_2$-$CH_2$-$N(CH_3)_2$ and wherein according to formula II $\mathbf{N_1=N_2=}$ $CH_3$, $\mathbf{R_1=R_2=}$ $C_{17}H_{30}$, **A and B** identical representing -C(O)NH-, **a**=4, $\mathbf{E_1}$ representing a -C(O)O- group $\mathbf{E_2}$ being absent and **b=c=e=d1=d2=**0;

- 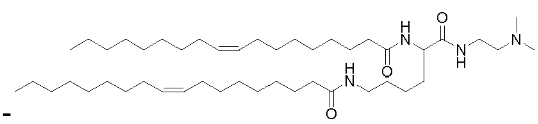

wherein according to formula I **Sp** is absent and **Zs** is $CH_2$-$CH_2$-$N(CH_3)_2$ and wherein according to formula II **$N_1$=$N_2$=** $CH_3$, **$R_1$=$R_2$=** $C_{17}H_{30}$, **A and B** identical representing -C(O)NH-, **a=**4, **$E_1$** representing a -C(O)NH- group **$E_2$** being absent and **b=c=e=d1=d2=**0;

- 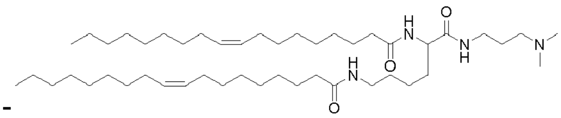

wherein according to formula I **Sp** is absent and **Zs** is $CH_2$-$CH_2$-$CH_2$-$N(CH_3)_2$ and wherein according to formula II **$N_1$=$N_2$=** $CH_3$, **$R_1$=$R_2$=** $C_{17}H_{30}$, **A and B** identical representing -C(O)NH-, **a=**4, **$E_1$** representing a -C(O)NH- group **$E_2$** being absent and **b=c=e=d1=d2=**0.

Preferably, in the above formula **(II)**, it is likely understood that if either or both **$R_1$ and $R_2$** are branched, each alkyl chain of the branched molecular pattern would be terminated by a **$N_1$ and/or $N_2$** moiety as previously defined.

Preferably, in the above formula **(II)**, it is likely understood that if **d** equals 0, **$d_1$ and $d_2$** would automatically equals 0 and **$E_2$** would be absent.

Preferably, in formula (I) as previously defined **Sp** corresponds to the general formula **(III)**:

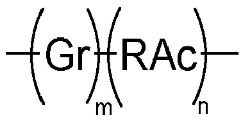

**(III)**

wherein:

- **Gr** represents a linear or branched hydrocarbon group, comprising from 1 to 15 carbon atoms, preferably from 1 to 8, and optionally comprising one or more heteroatoms;
- **m** is an integer equal to 0 or 1;
- **RAc** represents an amino acid radical; and
- **n** is an integer equal to 0 or 1.

By "amino acid radical" it is preferably understood, the group of atoms which consists of this amino acid when the latter is covalently bonded, on the one hand to **Gr** or **R** group, and on the other hand to one or more **Zs** groups.

Thus, in formula (III) as previously defined **Gr** and **RAc** can be absent when m and/or n is equal to 0.

Preferably, in formula (I) as previously defined **$Z_s$** corresponds to **formula (IV)**:

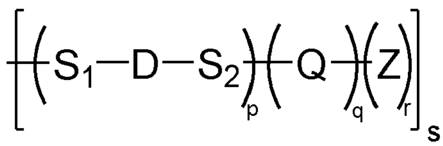

**(IV)**

wherein:

- **$S_1$ and $S_2$**, identical or different, represent a linear or branched hydrocarbon group comprising from 1 to 15 carbon atoms, preferably from 1 to 6 carbon atoms, optionally comprising one or more heteroatoms preferably chosen from nitrogen, oxygen, sulfur, bromine, iodine, chlorine and fluorine, more preferably from nitrogen, sulphur, bromine, iodine, chlorine and fluorine;
- **D** represents a functional group which can incorporate at least one bond which is sensitive to its environment, which is stable in extracellular medium and rapidly cleaved in the intracellular medium as it is sensitive to stimuli such as pH reduction (for example vinyl ether or acylhydrazone groups sensitive to acid medium) or a change in the oxidation-reduction potential (for example a disulfide bond, cleaved in reducing medium), to enzymes (for example an ester bond, cleaved by endogenous esterases); or also to light radiation (bearing photosensitive groups for example); preferably **D** represents one or more vinyl ether groups, one or more acylhydrazone groups, a disulfide bond, an ester bond or one or more photosensitive groups;
- **p** is an integer equal to 0 or 1;
- **Q** is a branched hydrocarbon group comprising from 1 to 20 carbon atoms, preferably from 1 to 12 carbon atoms, and

one or more heteroatoms, and which can optionally be covalently coupled with the **$S_2$** or **RAc** or **Gr** or **R** group on the one hand, and to at least two **Q** and/or **Z** groups on the other hand;

- **q** is an integer comprised between 0 and 8, preferably between 0 and 3;
- **Z** is a nitrogen (N) centred terminal ionizable moiety preferably incorporating chemical functions bearing no charges at physiological pH that can go positively charged if the pH of the medium dropped below the pKa value of the entity; More preferably Z represents a tertiary aliphatic amine such as N,N-dialkylamine, or a cyclic amine moiety such as N-substituted Pyrrolidine, N-substituted piperidine, N-substituted morpholine, N-substituted piperazine or N-substituted pyridine. Substituants include, but are not limited to, alkyl groups such as one or more methyl, ethyl, n-propyl, i-propyl, n-butyl, s-butyl, i-butyl or t-butyl group(s), aromatic rings based on benzene or naphthalene groups. Substituants may include one or more heteroatoms;
- **r** is an integer comprised between 1 and 16, preferably between 1 and 8, it being preferably understood that if **q** is equal to 1 then r is at least equal to 2 and that if r is greater than 1, then the **Z** groups can be identical or different; and
- **s** is an integer equal to 1 or 2.

Examples of photosensitive groups include, but are not limited to, diazo, nitrobenzyl and phenacyl groups.

By "amino acid radical" is preferably understood, the group of atoms which consists of this amino acid when the latter is covalently bonded, on the one hand to the **Gr** or **R** group, and on the other hand to one or more **$S_1$** or **Q** or **Z** groups.

Preferably, in formula (IV) as previously defined **Z** can be described according to formula **(V):**

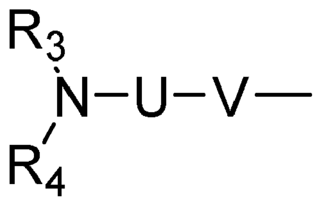

**(V)**

wherein:

- **$R_3$ and $R_4$** are either the same or different and selected from $C_1$-$C_6$ alkyl, $C_2$-$C_6$ alkenyl, or $C_2$-$C_6$ alkynyl, optionally substituted by one or more methyl, ethyl, n-propyl, i-propyl, n-butyl, s-butyl, i-butyl or t-butyl group(s), or **$R_3$ and $R_4$** may join to form an optionally substituted by one or more methyl, ethyl, n-propyl, i-propyl, s-butyl, i-butyl or t-butyl group(s) heterocyclic ring such as pyrrol, pyrrolidine, pyridine, piperazine, morpholine, piperidine or indoline optionally substituted by one or more methyl, ethyl, n-propyl, i-propyl, s-butyl, i-butyl or t-butyl group(s).
- N is a Nitrogen;
- V is O, S, N(**$R_6$**), C(O), C(O)O, OC(O), C(O)N(**$R_6$**), N(**$R_6$**)C(O), OC(O)N(**$R_6$**), N(**$R_6$**)C(O)O, C(O)S, C(S)O, S(O), S(O)(O), C(S), or an optionally substituted heterocyclic ring such as a pyrrole, pyrrolidine, pyridine, piperazine, morpholine, piperidine or indoline, optionally substituted by one or more methyl, ethyl, n-propyl, i-propyl, n-butyl, s-butyl, i-butyl or t-butyl group(s), wherein **$R_6$** is either an hydrogen (H) or a $C_1$-$C_{10}$ alkyl, $C_2$-$C_{10}$ alkenyl, or $C_2$-$C_{10}$ alkynyl, optionally substituted by one or more methyl, ethyl, n-propyl, i-propyl, n-butyl, s-butyl, i-butyl or t-butyl group(s); preferably $R_6$ is selected from the group consisting of hydrogen (H), a $C_1$ alkyl (methyl) group, and a **$C_2$**, **$C_3$**, **$C_4$**, **$C_5$**, **$C_6$**, **$C_7$**, **$C_8$**, **$C_9$**, and **$C_{10}$** alkyl, alkenyl, and alkynyl group, incorporating or not one or more heteroatoms, such as -O, -NH, -S and mixtures of thereof;
- **U** is either absent or is a $C_1$-$C_{12}$ alkyl, $C_2$-$C_{12}$ alkenyl, or $C_2$-$C_{12}$ alkynyl, optionally substituted by one or more methyl, ethyl, n-propyl, i-propyl, n-butyl, s-butyl, i-butyl or t-butyl group(s).

Preferably, **$R_3$ and $R_4$** are each independently a $C_1$-$C_2$ alkyl, $C_1$-$C_3$ alkyl, $C_1$-$C_4$ alkyl, $C_1$-$C_5$ alkyl,$C_2$-$C_3$ alkyl,$C_2$-$C_4$ alkyl, $C_2$-$C_5$ alkyl, $C_2$-$C_6$ alkyl, $C_3$-$C_4$ alkyl, $C_3$-$C_5$ alkyl, $C_3$-$C_6$ alkyl, $C_4$-$C_5$ alkyl, $C_4$-$C_6$ alkyl, $C_5$-$C_6$ alkyl, $C_2$-$C_3$ alkenyl, $C_2$-$C_4$ alkenyl, $C_2$-$C_5$ alkenyl, $C_2$-$C_6$ alkenyl, $C_3$-$C_4$ alkenyl, $C_3$-$C_5$ alkenyl, $C_3$-$C_6$ alkenyl, $C_4$-$C_5$ alkenyl, $C_4$-$C_6$ alkenyl, $C_5$-$C_6$ alkenyl, $C_2$-$C_3$ alkynyl, $C_2$-$C_4$ alkynyl, $C_2$-$C_5$ alkynyl, $C_2$-$C_6$ alkynyl, $C_3$-$C_4$ alkynyl, $C_3$-$C_5$ alkynyl, $C_3$-$C_6$ alkynyl, $C_4$-$C_5$ alkynyl, $C_4$-$C_6$ alkynyl, or $C_5$-$C_6$ alkynyl, optionally substituted by one or more methyl, ethyl, n-propyl, i-propyl, n-butyl, s-butyl, i-butyl or t-butyl group(s).

Preferably, **$R_3$ and $R_4$** are both methyl groups, either ethyl groups, or a combination of one methyl group and one ethyl group.

Preferably, **$R_3$** and **$R_4$** are joined to form an optionally substituted heterocyclic ring comprising 1, 2, 3, 4, 5, 6, or more carbon atoms and 1, 2, 3, 4, or more heteroatoms such as nitrogen (N), oxygen (O), sulfur (S), and mixtures thereof. The optionally substituted heterocyclic ring may comprise:

- from 2 to 10 carbon atoms and from 1 to 3 heteroatoms such as nitrogen (N), oxygen (O), and/or sulfur (S); or
- an imidazole, triazole (such as 1,2,3-triazole, 1,2,4-triazole), pyrazole, thiazole, pyrrole, furan, oxazole, isoxazole, oxazoline, oxazolidine, oxadiazole, tetrazole, pyrrole, pyrrolidine, pyridine, piperazine, morpholine, piperidine or indoline, optionally substituted by alkyl groups such as one or more methyl, ethyl, n-propyl, i-propyl, n-butyl, s-butyl, i-butyl or t-butyl group(s), and/or by aromatic rings based on benzene or naphthalene groups, that may include one or more heteroatoms, or
- 5 carbon atoms and 1 nitrogen atom, wherein the heterocyclic ring can be substituted with a substituent such as a hydroxyl (-OH) group at the ortho, meta, and/or para positions; or
- an imidazole group, optionnaly substituted by alkyl groups such as one or more methyl, ethyl, n-propyl, i-propyl, n-butyl, s-butyl, i-butyl or t-butyl group(s), and/or by aromatic rings based on benzene or naphthalene groups, that may include one or more heteroatoms.

In the above mentioned formula **(II)**, when $\mathbf{R_1}$ and $\mathbf{R_2}$ are different, their structure can be described according to formulae **(VI a)** and **(VI b)**:

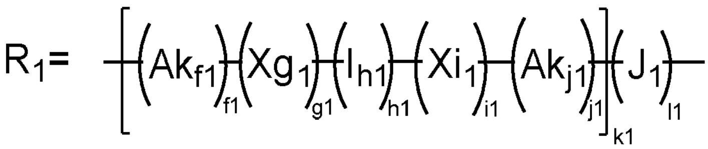

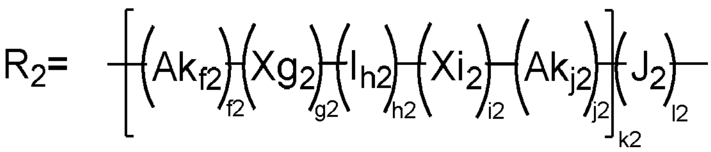

**(VI a) and (VI b)**

wherein:

- $\mathbf{Ak_{f1}}$, $\mathbf{Ak_{j1}}$, $\mathbf{Ak_{f2}}$, $\mathbf{Ak_{j2}}$ identical or different, represent a linear or branched hydrocarbon group comprising from 1 to 22 carbon atoms, preferably from 1 to 12 carbon atoms, optionally comprising one or more heteroatoms;
- **f1** and **f2** are integers equal to 0 or 1;
- **j1** and **j2** are integers equal to 0 or 1;
- $\mathbf{Xg_1}$ ,$\mathbf{Xi_1}$, $\mathbf{Xg_2}$ **and** $\mathbf{Xi_2}$, identical or different can be O, S, N($\mathbf{R_7}$), C(O), C(O)O, OC(O), C(O)N($\mathbf{R_7}$), N($\mathbf{R_7}$)C(O), OC(O)N($\mathbf{R_7}$), N($\mathbf{R_7}$)C(O)O, C(O)S, C(S)O, S(O), S(O)(O), C(S), wherein $\mathbf{R_7}$ is either an hydrogen (H) or a $C_1$-$C_{10}$ alkyl, $C_2$-$C_{10}$ alkenyl, or $C_2$-$C_{10}$ alkynyl, optionally substituted by one or more methyl, ethyl, n-propyl, i-propyl, n-butyl, s-butyl, i-butyl or t-butyl group(s); more preferably $\mathbf{R_7}$ is selected from the group consisting of hydrogen (H), a $C_1$ alkyl (methyl) group, and a $C_2$, $C_3$, $C_4$, $C_5$, $C_6$, $C_7$, $C_8$, $C_9$, and $C_{10}$ alkyl, alkenyl, and alkynyl group, optionally comprising one or more heteroatoms, such as -O-, NH-, S and mixtures of thereof. Preferably, it is likely understood that if $\mathbf{Xg_1}$, $\mathbf{Xi_1}$, $\mathbf{Xg_2}$ and $\mathbf{Xi_2}$ happened to be different in nature while being independently represented by N($\mathbf{R_7}$), C(O)N($\mathbf{R_7}$), N($\mathbf{R_7}$)C(O), OC(O)N($\mathbf{R_7}$), N($\mathbf{R_7}$)C(O)O, then $\mathbf{R_7}$ might be of several different nature among the possibilities depicted above within the same molecule;
- **g1** and **g2** are integers equal to 0 or 1;
- **i1** and **i2** are integers equal to 0 or 1;
- $I_{h1}$ and $I_{h2}$, identical or different, both represent a linear or branched non-saturated hydrocarbon chains comprising from 2 to 16 carbon atoms, preferably from 2 to 8 carbon atoms and at least one unsaturation, more preferably $I_{h1}$ and $I_{h2}$, identical or different are independently chosen from:

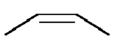 ; 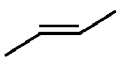 ; 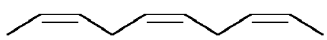 ;  ;  ;

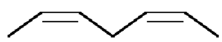 and  .

- **h1** and **h2** are integers equal to 0 or 1;
- $\mathbf{J_1}$ and $\mathbf{J_2}$ are branched hydrocarbon groups comprising from 1 to 20 carbon atoms, preferably from 1 to 12, and/or one or more heteroatoms. $\mathbf{J_1}$ can optionally be covalently coupled with either $\mathbf{Ak_{f1}}$, $\mathbf{Ak_{j1}}$, or $\mathbf{Xg_1}$, $\mathbf{Xi_1}$, or $\mathbf{I_{h1}}$ group on the one

hand, and to **A** on the other hand, whereas $J_2$ can optionally be covalently coupled with either $Ak_{f2}$, $Ak_{j2}$ $Xg_2$ and $Xi_2$, or $I_{h2}$ on the one hand, and to **B** on the other hand;

- **l1** and **l2** are integers equal to 0 or 1; it being preferably understood that if **l1** is equal to 0, then either $Ak_{f1}$, $Ak_{j1}$, or $Xg_1$, $Xi_1$, or $I_{h1}$ group is covalently attached to **A**. Similarly, if **l2** is equal to 0, then either $Ak_{f2}$ $Ak_{j2}$ $Xg_2$ and $Xi_2$, or $I_{h2}$ group is covalently attached to **B**;
- **k1** and **k2** are integers comprised between 1 or 3, it being preferably understood that if **l1** is equal to 1 then **k1** is at least equal to 2. Similarly, if **l2** is equal to 1 then **k2** is at least equal to 2.

In the above mentioned formula **(IV)**, when $R_1$ and $R_2$ are identical, their structure can be described according to formula **(VII)**:

$$R_1=R_2= \left[ \left(Ak_f\right)_f \left(Xg\right)_g \left(I_h\right)_h \left(Xi\right)_i \left(Ak_j\right)_j \right]_k \left(J\right)_l$$

**(VII)**

wherein:

- $Ak_f$ and $Ak_j$, identical or different, represent a linear or branched hydrocarbon group comprising from 1 to 22 carbon atoms, preferably from 1 to 12 carbon atoms, optionally comprising one or more heteroatoms;
- **f** is an integer equal to 0 or 1;
- **j** is an integer equal to 0 or 1;
- **Xg** and **Xi**, identical or different can be O, S, N($R_7$), C(O), C(O)O, OC(O), C(O)N($R_7$), N($R_7$)C(O), OC(O)N($R_7$), N($R_7$) C(O)O, C(O)S, C(S)O, S(O), S(O)(O), C(S), wherein $R_7$ is either an hydrogen (H) or a $C_1$-$C_{10}$ alkyl, $C_2$-$C_{10}$ alkenyl, or $C_2$-$C_{10}$ alkynyl, optionally substituted by one or more methyl, ethyl, n-propyl, i-propyl, n-butyl, s-butyl, i-butyl or t-butyl group(s); more preferably $R_7$ is selected from the group consisting of hydrogen (H), a $C_1$ alkyl (methyl) group, and a $C_2$, $C_3$, $C_4$, $C_5$, $C_6$, $C_7$, $C_8$, $C_9$, and $C_{10}$ alkyl, alkenyl, and alkynyl group, optionally comprising one or more heteroatoms, such as -O-, NH-, S and mixtures of thereof;
- **g** is an integer equal to 0 or 1;
- i is an integer equal to 0 or 1;
- $I_h$ represent a linear or branched non-saturated hydrocarbon chain comprising from 2 to 16 carbon atoms, preferably from 2 to 8 carbon atoms and at least one unsaturation, more preferably $I_h$ is selected from:

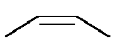 ; 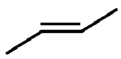 ; 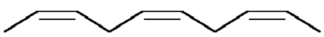 ; 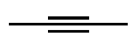 ;

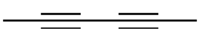 ; 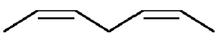 and 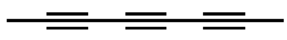.

- **h** is an integer equal to 0 or 1;
- **J** is a branched hydrocarbon groups comprising from 1 to 20 carbon atoms, preferably from 1 to 12, and/or one or more heteroatoms. **J** can optionally be covalently coupled with the $Ak_j$ or **Xi** or $I_h$ or **Xg** or $Ak_f$ group on the one hand, and to **A** or **B** on the other hand;
- **l** is an integer equal to 0 or 1; it being preferably understood that if **l** is equal to 0, then either $Ak_j$ or **Xi** or $I_h$ or **Xg** or $Ak_f$ is covalently attached to **A** on the one hand and to **B** on the other hand;
- **k** is an integer comprised between 1 or 3; it being preferably understood that if **l** is equal to 1 then **k** is at least equal to 2.

Preferably, in formula (I) as previously defined **R** incorporates an amino-acid acting as "biocompatible template".

Examples of amino-acid acting as "biocompatible template" include, but are not limited to, glutamic acid, aspartic acid and ornithine.

By "biocompatible template" it is preferably understood a multivalent amino-acid acting as a basis for the structural backbone of these new lipids, preferably new tunable lipids, such as glutamic acid, aspartic acid, lysine and ornithine that each present three chemical functions able to react with a broad variety of components following the basic principles of the peptide coupling chemistry. In the case of glutamic acid and aspartic acid, two carboxylic functions and one primary amine are free to be used for coupling, while in the case of lysine and ornithine, it is two amine functions for a

carboxylic acid function that are available.

Preferably, in formula (**I**) as previously defined **R** is selected from:

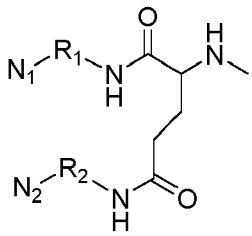
;
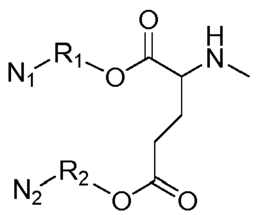
;
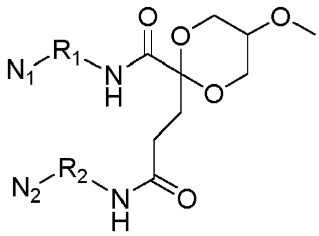
;
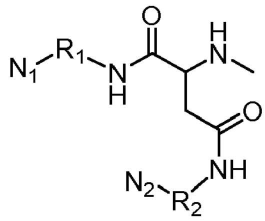
;
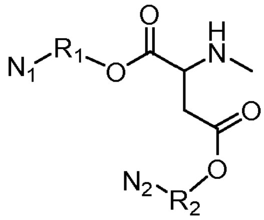
;
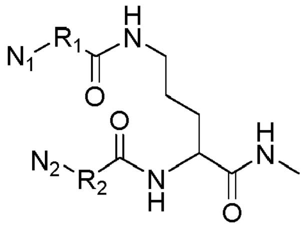
;
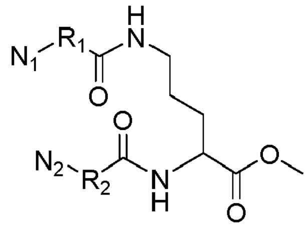
;
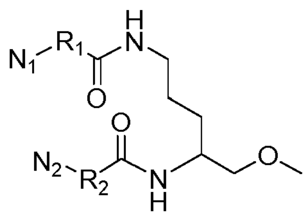
;
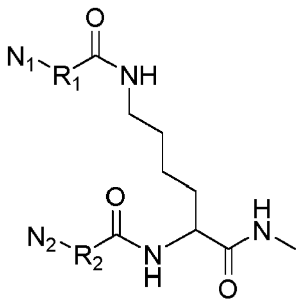
;
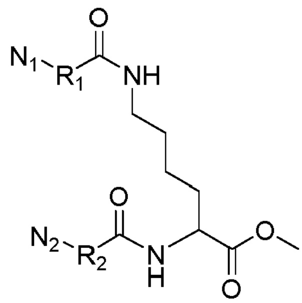
and
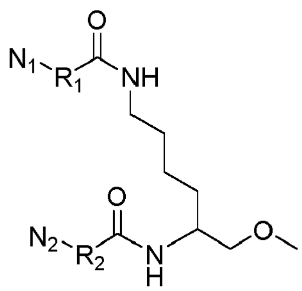
.

**(Formula VIII)**

wherein, $\mathbf{R_1}$, $\mathbf{R_2}$, $\mathbf{N_1}$ and $\mathbf{N_2}$ are as previously defined.

Preferably, in formulae **(VI a) and (VI b)** as previously defined, $\mathbf{J_1}$ and $\mathbf{J_2}$, identical or different correspond to formula $-CO-Y_1-NH-Y_2-N-[Y_3-NH]_2-$ or $-NH-Y_2-N-[Y_3-NH]_2-$,
wherein $\mathbf{Y_1}$, $\mathbf{Y_2}$ and $\mathbf{Y_3}$, identical or different, represent a bridging alkylene group comprising from 1 to 8 carbon atoms, preferentially from 1 to 4.

Preferably, $\mathbf{Y_1}$ represents a methylene, whilst $\mathbf{Y_2}$ and $\mathbf{Y_3}$ represent a bridging alkylene group comprising from 1 to 4 carbon atoms and more preferably 2 carbon atoms.

Preferably, $\mathbf{J_1}$ and $\mathbf{J_2}$ therefore possess three functional groups making it possible for $\mathbf{J_1}$ to form a covalent bond optionally with at least two iterations of either $\mathbf{Ak_{f1}}$, $\mathbf{Ak_{j1}}$, or $\mathbf{Xg_1}$, $\mathbf{Xi_1}$, or $\mathbf{I_{h1}}$ group on the one hand, and with **A** on the other hand, whereas $\mathbf{J_2}$ can optionally be covalently coupled with at least two iterations of either $\mathbf{Ak_{f2}}$, $\mathbf{Ak_{j2}}$ $\mathbf{Xg_2}$ and $\mathbf{Xi_2}$, or $\mathbf{I_{h2}}$ on the one hand, and with **B** on the other hand.

Preferably, in the case where $\mathbf{R_1}$ and $\mathbf{R_2}$ are identical, then $\mathbf{J_1}$ and $\mathbf{J_2}$ are identical and can be named **J**, which defined the same way as $\mathbf{J_1}$ and $\mathbf{J_2}$ Thus, in this case, **J** possesses three functional groups making it possible for **J** to form a covalent bond optionally with at least two iterations of either $\mathbf{Ak_f}$, $\mathbf{Ak_j}$, or **Xg**, $\mathbf{Xi_1}$ or $\mathbf{I_h}$ group on the one hand, and with **A**&**B** on the other hand.

Preferably, in ***formula (III)*** as previously defined, **Gr** can be absent or **Gr** can serve as a spacer arm and thus corresponds to the molecular pattern noted as $\mathbf{-W_4 -Y_4 -W_4 -}$,

wherein $\mathbf{Y_4}$ represents a bridging alkylene group comprising from 1 to 8 carbon atoms, preferentially from 1 to 4, whilst $\mathbf{W_4}$ represents a -O-, -CO- or -NH- group.

Preferably, in ***formula (III)*** as previously defined, **Gr** corresponds to the formula CO-$\mathbf{Y_4}$-CO, wherein $\mathbf{Y_4}$ has the same meaning as previously defined and is connected by an amide bond to the lipophilic region **R** on the one hand, and either to the **RAc** radical, or directly to the $S_1$ or **Q** or **Z** group on the other hand.

Preferably, the amino acid, the radical of which is denoted **RAc** in ***formula (III)*** as previously defined, is chosen from aspartic acid, glutamic acid, alanine, arginine, asparagine, cysteine, glutamine, glycine, histidine, isoleucine, leucine, lysine, methionine, ornithine, phenylalanine, praline, serine, threonine, tyrosine, tryptophan and valine.

Preferably, in formula **(III)** as previously defined, the amino acid, the radical of which being denoted **RAc** is chosen from aspartic acid, glutamic acid, isoleucine, leucine, lysine and phenylalanine, and more preferably from aspartic acid, glutamic acid, and lysine. However, this amino acid can also be chosen from rarer amino acids such as for example, β-alanine, y-aminobutyric acid, α-aminoadipic acid, hydroxyproline, hydroxylysine, phenylserine, α,β-diaminopimelic acid, ornithine and any other modified amino acids, any amino acid being suitable since it comprises, by definition, two functional groups, one being carboxylic acid, the other amine, allowing it to bind covalently, on the one hand to the spacer arm **Gr** or to **R** and, on the other hand, to at least one $\mathbf{S_1}$ or **Q** or **Z** group. Preferably, the choice of the amino acid depends in particular on the value that it is desired to give to **s** in ***formula (IV),*** to the extent that it must comprise at least three functional groups to allow **s** to be equal to 2, whilst it is sufficient for it to comprise only two functional groups to have **s** equal to 1. Preferably, it is preferred that **RAc** is an amino acid radical belonging to the L series. However, it is also possible that **RAc** is an amino acid radical of the D series.

Preferably, in ***formula (IV),*** $\mathbf{S_1}$ can correspond to the molecular pattern noted as: **-$\mathbf{W_5}$-$\mathbf{Y_5}$-** and $\mathbf{S_2}$ can correspond to the pattern noted as: **-$\mathbf{Y_6}$-$\mathbf{W_6}$-** in which $\mathbf{Y_5}$ and $\mathbf{Y_6}$, identical or different, represent a bridging alkylene group comprising from 1 to 8 carbon atoms, preferentially from 1 to 4, whilst $\mathbf{W_5}$ and $\mathbf{W_6}$, identical or different, represent a -CO-, -NH-or-O- group. In this case, preferably, $\mathbf{S_1}$ forms a covalent bond with an **RAc**, **Gr** or **R** group on the one hand, and with the **D** group on the other hand, whilst $\mathbf{S_2}$ forms a covalent bond with a **D** group on the one hand, and with a **Q** or **Z** group on the other hand.

Preferably, in ***formula (IV)*** as previously defined, **D** represents an ester (-CO-O-), disulfide (-S-S-), vinyl ether (-O--C=C-), acylhydrazone (CO-NR-N=CR'R") group, the ester and disulfide groups being particularly preferred. In this case, preferably, these groups form a covalent bond with the $\mathbf{S_1}$ group on the one hand and the $\mathbf{S_2}$ group on the other hand.

Preferably, **Q** corresponds to the molecular pattern noted as -CO-$\mathbf{Y_7}$-NH-$\mathbf{Y_8}$-N-[$\mathbf{Y_9}$-NH]$_2$- or as -NH-$\mathbf{Y_8}$-N-[$\mathbf{Y_9}$-NH]$_2$-, in which $\mathbf{Y_7}$, $\mathbf{Y_8}$ and $\mathbf{Y_9}$, identical or different, represent a bridging alkylene group comprising from 1 to 8 carbon atoms, preferentially from 1 to 4. Additionally, $\mathbf{Y_7}$ preferably represents a methylene, whilst $\mathbf{Y_8}$ and $\mathbf{Y_9}$ preferably represent a bridging alkylene group comprising from 1 to 4 carbon atoms and, better still, 2 carbon atoms. In this case, preferably, **Q** therefore possesses three functional groups making it possible for it to form a covalent bond with optionally $\mathbf{S_2}$ or **RAc** or **Gr** or **R** on the one hand, and at least two **Q** and/or **Z** groups on the other hand.

Preferably, in **formula (IV)** as previously defined, **Z** represents ionizable molecular pattern able to form a covalent bond optionally with **Q** or $\mathbf{S_2}$ or **RAc** or **Gr** or **R** on the one hand, and one or two basic reactive functions (such as for example amine and alcohol) making it possible to form a covalent bond optionally with one or two other **Z** groups on the other hand.

By "ionizable molecular pattern" it is preferably understood a pattern of chemical functions incorporating at least one nitrogen atom and bearing no charges at physiological pH, that can go positively charged if the pH of the medium dropped below the pKa value of the entity

Preferably, the compounds of ***formula (I)*** are selected from:

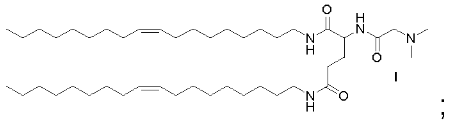
I;

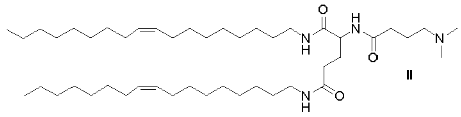
II;

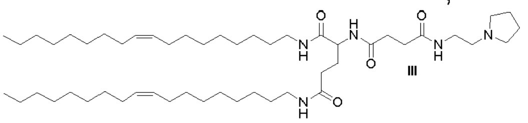
III;

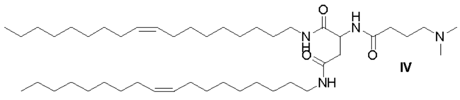
IV;

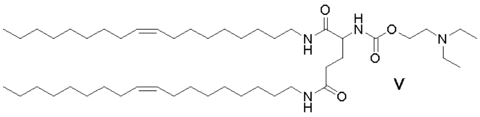
V;

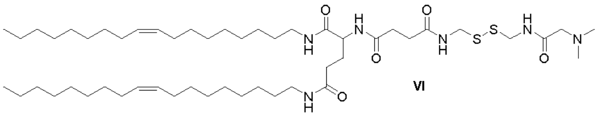
VI;

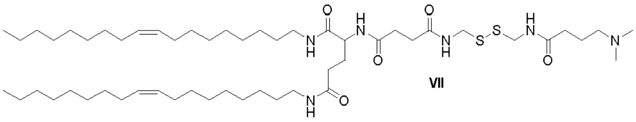
VII;

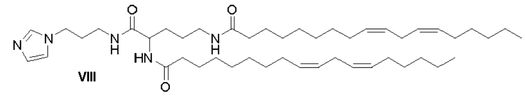
VIII;

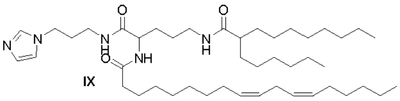
IX;

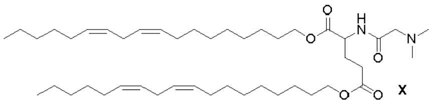
X;

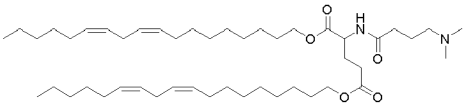
XI;

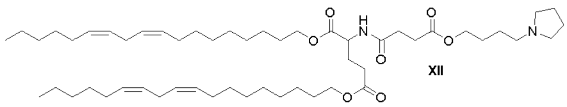
XII;

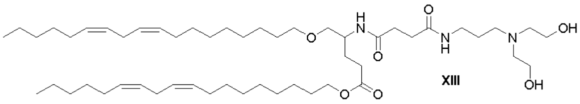
XIII;

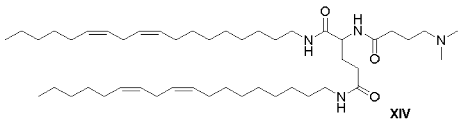
XIV;

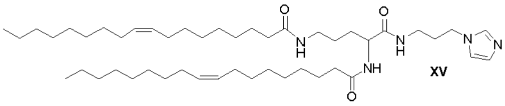
XV;

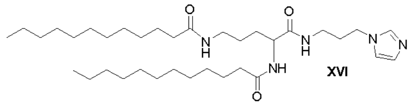
XVI;

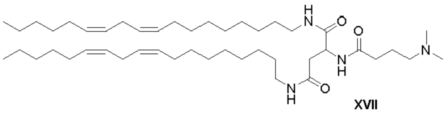
XVII;

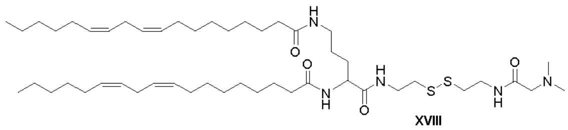
XVIII;

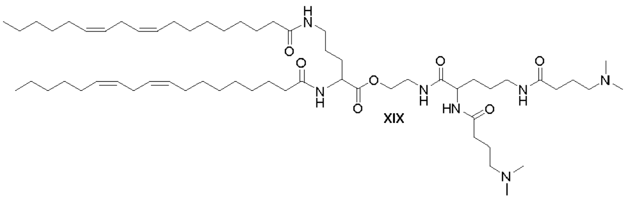
XIX;

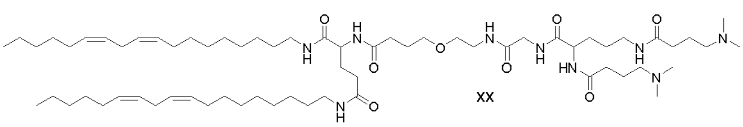
XX;

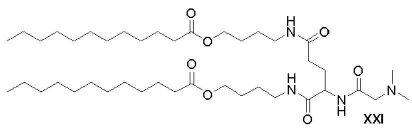
XXI;

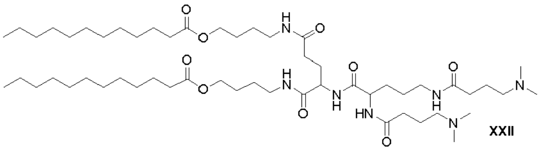
XXII;

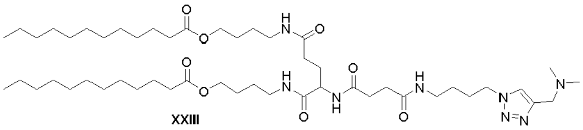
XXIII;

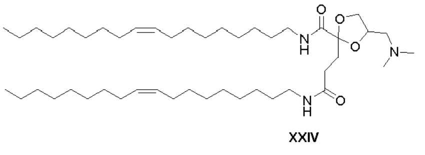
XXIV;

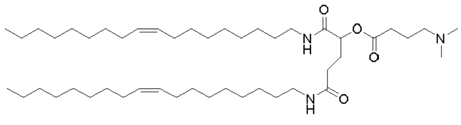
XXV;

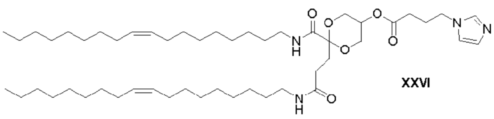
XXVI ;

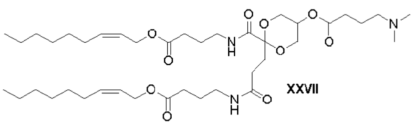
XXVII ;

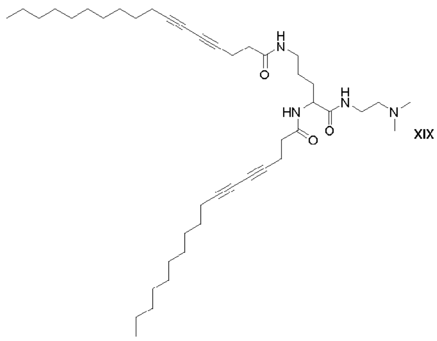
XIX ;

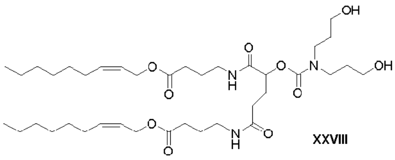
XXVIII ;

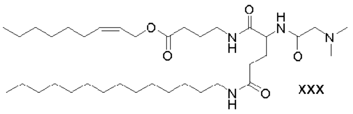
XXX ;

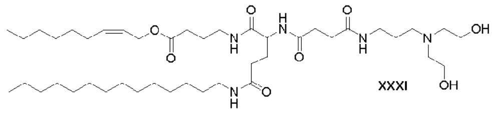
XXXI ;

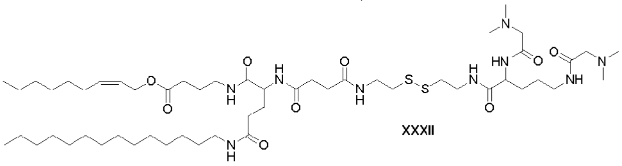
XXXII ;

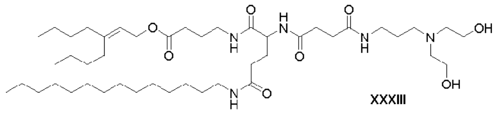
XXXIII ;

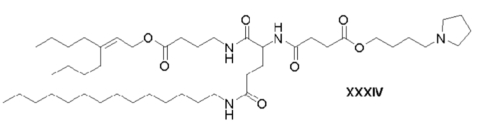
XXXIV ;

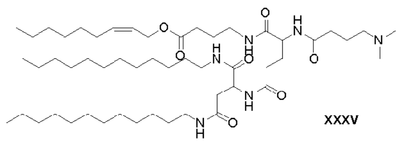
XXXV ;

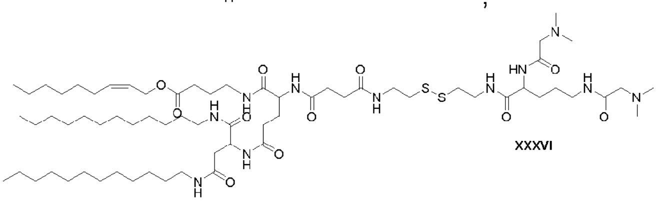
XXXVI ;

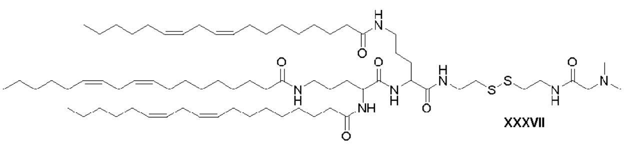
XXXVII ;

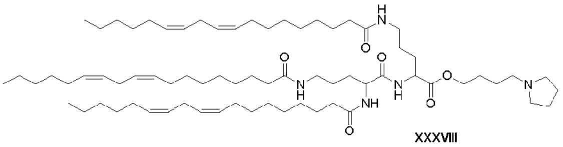
XXXVIII ;

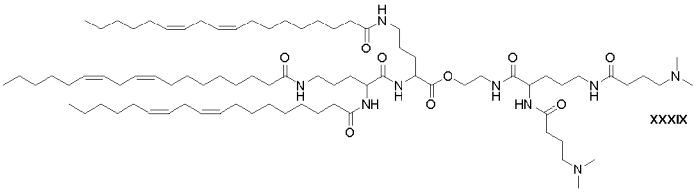
XXXIX ;

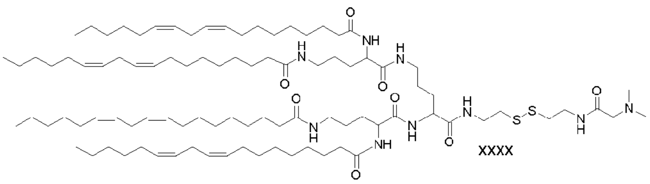
XXXX ;

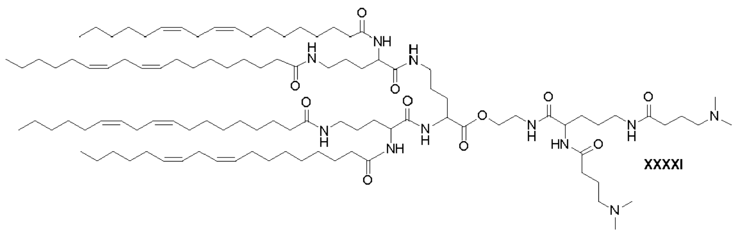

XXXXI

;

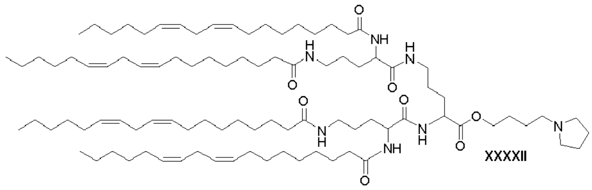

XXXXII

and

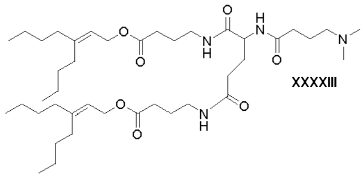

XXXXIII

.

Another object of the present invention relates to a mixture comprising one or more lipid(s) of formula (I) as previously defined.

The lipid of formula (I) as previously defined may be present under the form of a racemic mixture.

The mixture comprising the lipid of formula (I) as previously defined may be enriched in one diastereomer of the lipid of formula (I) as previously defined. In particular, the mixture of the lipid of formula (I) as previously defined may have at least 95%, at least 90%, at least 80% or at least 70% diastereomeric excess.

The mixture comprising the lipid of formula (I) as previously defined may be enriched in one enantiomer of the lipid of formula (I) as previously defined. In particular, the mixture of the lipid of formula (I) as previously defined may have at least 95%, at least 90%, at least 80% or at least 70% enantiomer excess.

The mixture comprising the lipid of formula (I) as previously defined may be chirally pure. In particular, the lipid of formula (I) as previously defined may be in the form of a single optical isomer.

The mixture comprising the lipid of formula (I) as previously defined may be enriched in one optical isomer of the lipid of formula (I) as previously defined.

Where a double bond is present (such as a carbon-carbon double bond or carbon-nitrogen double bond), there can be isomerism in the configuration of the double bond (such as cis/trans or E/Z isomerism). Where the configuration of a double bond is illustrated in a chemical structure, it is preferably understood that the corresponding isomer can also be present. The amount of isomer present can vary, depending on the relative stabilities of the isomers and the energy required to switch between the isomers. Accordingly, some double bonds may be, for practical purposes, present in only a single configuration, whereas others (for example where the relative stabilities are similar and the energy of conversion low) may be present as inseparable equilibrium mixture of configurations.

The lipid of formula (I) as previously defined may include one or more biodegradable groups. The biodegradable group(s) may include one or more bonds that may undergo bond breaking reactions in a biological environment (such as for example in an organism, organ, tissue, cell, or organelle). Examples of functional groups that contain a biodegradable bond include, but are not limited to, esters, dithiols and oximes.

The present invention also relates to a process for preparing the lipid of formula (**I**) as previously defined, and to a process for prepraring the mixture comprising the lipid of formula (**I**) as previously defined

Thus, the present invention also relates to a process for preparing the lipid of formula (**I**) as previously defined or the mixture comprising the lipid of formula (**I**) as previously defined.

For example, the process for preparing the lipid of formula (**I**) as previously defined or the mixture comprising the lipid of formula (**I**) as previously defined may comprise the steps of:

- attaching one or two chemical functions of the chosen biocompatible template to an hydrophobic (or lipophilic) group of atoms as defined in formula (I) and/or (II);
- optionally introducing a spacer region using the remaining chemical functions of the biocompatible template that now also contains the hydrophobic (or lipophilic) area. This spacer region can be defined according formula (I) and/or (III), and may include one or more chemical bonds or entities sensitive to their environment, able to enhance the lipid degradability, preferably the tunable lipid degradability. This spacer can also include a chemical functions able to be attached to the polar region as previously defined in Formula (I);
- finally introducing a polar region, preferably a tunable polar region, that will act as the hydrophilic part of the whole chemical entity. This polar region can be constructed according to the formula (IV) as previously defined. In the case of a spacer region previously attached to the biocompatible template, this polar region can be chemically attached to it. If the spacer region is absent, the polar region can be attached directly to the above-described biocompatible template, using one or two of its remaining chemical functions.

This process can be modified, with its steps switched, depending on the global synthetic process chosen to obtain the targeted lipid, preferably tunable lipid. It means that in some case for instance, the polar region might be first attached to the spacer region before this new entity might be attached to the biocompatible template. The person skilled in the art is able to select the appropriate process to obtain the lipid of formula (I) as previously defined or the mixture comprising the lipid of formula (I) as previously defined.

The lipid of formula (I) as previously defined or the mixture comprising the lipid of formula (I) as previously defined may also be prepared by well-known organic synthesis methods, including the methods described in the examples of the present invention.

Examples of synthesis methods that may be used to synthesize the lipid of formula (I) as previously defined or the mixture comprising the lipid of formula (I) as previously defined, include, but are not limited to, coupling methods involving carbodiimide such as diisopropylcarbodiimide or uronium salts-based coupling agents such as HBTU, or nitrogen-based protecting group chemistry (as described for example in Greene's Protective Groups in Organic Synthesis, T.W. Greene et. al., Wiley-Interscience New York City, 1999).

In particular, the lipid of formula (I) as previously defined may comprise at least one protonatable or deprotonatable group, such that the whole lipid may be positively charged at a first pH (such a pH ranging from 3.0 to 6.9) below physiological pH (such a pH ranging from 7.0 to 7.4), and may be neutral at a second pH, higher than the first pH and preferably at or above physiological pH. It will be understood by one having ordinary skill in the art that the addition or removal of protons as a function of pH is an equilibrium process, and that the reference to a charged or a neutral lipid refers to the nature of the predominant species and does not require that all of the lipid be present in the charged or neutral form.

The lipid of formula (I) as previously defined may have more than one protonatable or deprotonatable group or may be zwitterionic.

The pKa's values of the lipid of formula (I) as previously defined may be lower than the pH observed in physiological medium (such as a pH ranging from 7.0 to 7.4). Preferably, the lipid of formula (I) as previously defined has a global pKa value ranging from 4 to 6.90.

In the present invention, the pKa value preferably corresponds to a dimensionless number related to the acid dissociation constant of the corresponding acid specie and is thus a quantitative measure of the acidic strength in solution of a chemical specie.

Preferably, the pKa value of the lipid of formula (I) as previously defined is finely tuned to stay under the value of the observed in physiological medium (such as a pH ranging from 7.0 to 7.4). Thus, at this pH, all the lipid of formula (I) as previously defined can be present in solution mostly under their non-protonated form (such as basic form). It will be understood by one having ordinary skill in the art that the value of the pKa constant as defined above may cover the acidic strength of the lipid of formula (I) as previously defined. Thus, the present invention may also cover a lipid of formula (I) bearing several protonable moieties (protonatable lipid) and present at physiological pH mainly under its neutral form (such as non-protonated form).

Preferably, protonatable lipids according to the invention have a pKa for the protonatable group in the range of about 4 to about 7. Accordingly, such lipids will be cationic at a lower pH, whereas formulation including the tunable lipids, preferably the tunable lipids, will be largely (though not completely) surface neutralized at physiological pH of around pH 7.4. One of the benefits of choosing this value for pKa is that at least some nucleic acid associated with the outside surface will lose its electrostatic interaction at physiological pH and be removed by a simple dialysis, thus greatly reducing the particle's susceptibility to clearance.

Advantageously, the lipid of formula (I) as previously defined may incorporate a neutral nitrogen-centred chemical function ables to get protonated at highly acidic pH while remaining neutral at physiological pH.

Thus, the lipids of formula (I) as previously defined may be under free form, as well as pharmaceutically acceptable salts and stereoisomers thereof. Thus, the lipid, preferably the tunable lipid, of the present invention can be under its protonated form, that is cationic form.

By "lipid under free form" it is preferably understood the lipid in a non-salt form. The free form may be regenerated by treating the lipid salt with a suitable dilute aqueous base solution such as aqueous NaOH, potassium carbonate, ammonia and/or sodium bicarbonate solution, preferably dilute aqueous NaOH, potassium carbonate, ammonia and/or sodium bicarbonate solution.

The pharmaceutically acceptable salts of the lipids of formula (I) as previously defined can be synthesized from the lipids of formula (I) as previously defined which contain a basic or acidic moiety by conventional chemical methods. Generally, the salts of the basic lipids may be prepared either by ion exchange chromatography or by reacting the free base with stoichiometric amounts or with an excess of the desired salt-forming inorganic or organic acid in a suitable solvent or various combinations of solvents. Similarly, the salts of the acidic lipids may be formed by reactions with the appropriate inorganic or organic base.

Examples of pharmaceutically acceptable salts of the lipids of formula (I) as previously defined include, but are not limited to, non-toxic salts of the lipids of formula (I) as previously defined as those formed by reacting a basic lipid with an inorganic or organic acid.

Examples of non-toxic salts include, but are not limited to, those derived from inorganic acids such as hydrochloric acid, hydrobromic acid, sulfuric acid, sulfamic acid, phosphoric acid and nitric acid, as well as salts prepared from organic acids such as formic acid, acetic acid, propionic acid, succinic acid, glycolic acid, stearic acid, lactic acid, malic acid, tartaric acid, citric acid, ascorbic acid, palmoic acid, maleic acid, hydroxymaleic acid, phenylacetic acid, glutamic acid, benzoic acid, salicylic acid, sulfanilic acid, 2-acetoxy-benzoic acid, fumaric acid, toluenesulfonic acid, methanesulfonic acid, ethane disulfonic acid, oxalic acid, isethionic acid, and trifluoroacetic (TFA) acid.

When the lipids of formula (I) as previously defined are acidic lipids, suitable "pharmaceutically acceptable salts" refers to salts prepared from pharmaceutically acceptable non-toxic bases including inorganic bases and organic bases. Salts prepared from pharmaceutically acceptable inorganic non-toxic bases include, but are not limited to, salts of aluminum, ammonium, calcium, copper, ferric, ferrous, lithium, magnesium, manganic salts, manganous, potassium, sodium, zinc and mixtures thereof. Preferably, the base is selected from ammonium, calcium, magnesium, potassium and/or sodium. Salts prepared from pharmaceutically acceptable organic non-toxic bases include, but are not limited to, salts of primary amines, secondary amines, tertiary amines, substituted amines including naturally occurring substituted amines, cyclic amines and basic ion exchange resins, such as arginine, betaine, caffeine, choline, N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and mixtures thereof.

The lipids of formula (I) as previously defined may potentially be internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety.

The lipid of formula (I) as previously defined may be formulated, optionally with one or more active ingredients, into an active supra-molecular architecture called a "lipid particle".

Examples of lipid particles include, but are not limited to, liposomes, lipoplexes, micelles and lipid picoparticles, microparticles or nanoparticles.

By "liposome" it is preferably understood a structure having lipid-containing membranes enclosing an aqueous interior.

By "lipoplexe" is preferably understood a system obtained by complexation between a nucleic acid and a lipidic composition containing one or several lipids. Those complexes are usually obtained by adding a nucleic acid solution to a lipid dispersion followed by gentle mixing and short incubation.

Thus, another object of the present invention relates to a lipid particle comprising one or more lipid of formula (I) as previously defined or to a composition comprising the lipid particle as previously defined.

Thus, the lipid particle and the composition comprising the lipid particle as previously defined may include one or more lipids of formula (I) as previously defined.

The present invention also relates to a composition comprising the lipid of formula (I) as previously defined.

The composition comprising the lipid of formula (I) as previously defined may further include one or more active ingredients, and optionally:

- one or more neutral lipids, and/or
- one or more components capable of reducing aggregation of said composition, and/or
- one or more sterols or sterol derivatives, and/or
- one or more lipids bearing a targeting ligand.

Neutral lipids can be, when present, any lipid species which exist either in an uncharged or neutral zwitterionic form at physiological pH. Preferably, the neutral lipid contains saturated fatty acids with carbon chain, potentially mono or di-unsaturated, which carbon chain's lengths stand in the range of $C_8$ to $C_{22}$. More preferably, the neutral lipid includes mono or di-unsaturated fatty acids with carbon chain lengths in the range of $C_{10}$ to $C_{20}$. More preferably, examples neutral lipids include, but are not limited to, phospholipids such as diacylphosphatidylethanolamine, diacylphosphatidylcholine, dihydrosphingomyelin, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyolphosphatidylglycerol (POPG), dioleoylphosphatidylethanolamine,4-(N-maleimidomethyl)-cyclohexane-1-carboxylate(DOPE-mal), dipalmitoylphosphatidylethanolamine(DPPE), dimyristoyl phosphatidylethanolamine (DMPE), distearoylphosphatidylethanolamine(DSPE), monomethylphosphatidylethanolamine, dimethylphosphatidylethanolamine, dielaidoylphosphatidylethanolamine(DEPE), stearoyloleoylphosphatidylethanolamine(SOPE), lysophosphatidylcholine and dilinoleoylphosphatidylcholine, nonphosphorous containing lipids such as for example stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl aryl sulfate polyethyloxylated fatty acid amides, dioctadecyldimethyl ammonium bromide, ceramide, sphingomyelin, cerebrosides, cephalin, and mixtures thereof.

The neutral lipid that may be present in the composition of the present invention may comprise or may consist of one or more phospholipids (such as a cholesterol-free lipid composition).

The sterol that may be present in the composition of the present invention may comprise or consist of cholesterol or a derivative thereof (such as a phospholipid-free lipid composition).

Examples of components capable of reducing aggregation of said composition include, but are not limited to, polyethylene glycol (PEG)-modified lipids (PEG-modified lipids), polyethylene glycol (PEG) optionally substituted, conjugated and/or coupled, hydrophilic polymers other than PEG, poloxamers, monosialoganglioside (Gml), polyamide oligomers (PAO), and mixture thereof.

Examples of PEG-modified lipids include, but are not limited to, PEG-modified phosphatidylethanolamine and phosphatidic acid, PEG-ceramide conjugates (such as PEG-CerC14 or PEG-CerC20), PEG-modified dialkylamines, PEG-modified 1,2-diacyloxypropan-3-amines, PEG-modified diacylglycerols and dialkylglycerols, mPEG (MW=2000)-diastearoylphosphatidylethanolamine (PEG-DSPE), mPEG (MW=2000)-dimyristoylphosphatidylethanolamine (PEG-DMPE), mPEG (MW=2000)-dipalmitoylphosphatidylethanolamine (PEG-DPPE), mPEG (MW=2000)-dioleoyl-phosphatidylethanolamine (PEG-DOPE), 1-(monomethoxy-polyethyleneglycol)-2,3-dimyristoylglycerol (PEG-DMG), N-[(methoxypoly(ethylene glycol) 2000 carbamyl]-1,2-dimyristyloxlpropyl-3-amine (PEG-c-DMA), a pegylated phosphatidylethanoloamine (PEG-PE),4-O-(2',3'-dictetradecanoyloxy)propyl-1-O-(w-methoxy(polyethoxy)ethyl)butanedioate(PEG-S-DMG), a pegylated ceramide (PEG-cer), or a PEG dialkoxypropyl carbamate such as ()-methoxy(polyethoxy)ethyl-N-(2,3-di(tetradecanoxy)propyl)carbamate or 2,3-di(tetradecanoxy) propyl-N-(c)-methoxy(polyethoxy) ethyl)carbamate, and mixtures thereof.

By "polyethylene glycol (PEG)" it is preferably understood a linear, water-soluble polymer of ethylene glycol repeating units with two terminal hydroxyl groups. PEGs are classified by their molecular weights and include, but are not limited to, monomethoxypolyethylene glycol (MePEG-OH), monomethoxypolyethylene glycol-succinate (MePEG-S), monomethoxypolyethylene glycol-succinimidyl succinate (MePEG-SNHS), monomethoxypolyethylene glycolamine (MePEG-NH), monomethoxypolyethylene glycol tresylate (MePEG-TRES), monomethoxypolyethylene glycol-imidazolyl-carbonyl (MePEG-IM), as well as such compounds containing a terminal hydroxyl group instead of a terminal methoxy group (such as HO-PEG-S, HO-PEG-S NHS, HO-PEG-NH).

The PEG moiety of the PEG-conjugated lipid of the present invention may have an average molecular weight ranging from 550 daltons to 10,000 daltons, preferably ranging from 750 daltons to 5,000 daltons (such as from 1,000 daltons to 5,000 daltons, from 1,500 daltons to 3,000 daltons, from 750 daltons to 3,000 daltons, from 750 daltons to 2,000 daltons), and even more preferably of 2,000 daltons or 750 daltons.

The PEG may be optionally substituted by an alkyl, alkoxy, acyl, or aryl group.

The PEG can be conjugated directly to the lipid or may be linked to the lipid *via* a linker moiety. Any linker moiety suitable for coupling the PEG to a lipid can be used including, but not limited to, non-ester containing linker moieties and ester-containing linker moieties. Preferably, the linker moiety is a non-ester-containing linker moiety.

Suitable non-ester-containing linker moieties include, but are not limited to, amido (-C(O)NH-), amino (-NR-), carbonyl (-C(O)-), carbamate (-NHC(O)O), urea (-NHC(O)NH), disulfide (-S-S-), ether (-O-), succinyl (-(O)CCHCHC(O-), succinamidyl (-NHC(O)CHCHC(O)NH-), as well as combinations thereof (such as a linker containing both a carbamate linker moiety and an amido linker moiety). Preferably, a carbamate linker is used to couple the PEG to the lipid.

Suitable ester-containing linker moieties include, but are not limited to, carbonate (-OC(O)O-), succinoyl phosphate esters (-O-(O)POHO-), sulfonate esters, and combinations thereof.

Phosphatidylethanolamines having a variety of acyl chain groups of varying chain lengths and degrees of saturation can be conjugated to PEG.

Such phosphatidylethanolamines are commercially available, or can be isolated or synthesized using conventional techniques known by one having ordinary skill in the art. Phosphatidylethanolamines containing saturated or unsaturated fatty acids with carbon chain lengths in the range of $C_{10}$ to $C_{20}$ or phosphatidylethanolamines with mono- or di-unsaturated fatty acids and mixtures of saturated and unsaturated fatty acids can be used. Preferably, phosphatidylethanolamines containing saturated or unsaturated fatty acids with carbon chain lengths in the range of $C_{10}$ to $C_{20}$ are used. More preferably, suitable phosphatidylethanolamines include, but are not limited to, dimyristoyl-phosphatidylethanolamine (DMPE), dipalmitoyl-phosphatidylethanolamine (DPPE), dioleoyl-phosphatidylethanolamine (DOPE), distearoyl-phosphatidylethanolamine (DSPE), and mixtures thereof.

The PEG may be coupled together with a diacylglycerol (DAG) moiety. By "diacylglycerol" or "DAG" it is preferably understood a compound having 2 fatty acyl chains having both independently between 2 and 30 carbons bonded to the 1- and 2-position of glycerol by ester linkages. The acyl chains can be saturated or may have varying degrees of unsaturation. Suitable acyl groups include, but are not limited to, lauroyl ($C_{12}$), myristoyl ($C_{14}$), palmitoyl ($C_{16}$), stearoyl ($C_{18}$), and icosoyl ($C_{20}$). Preferably, the two acyl chains are identical, more preferably the two acyl chains are both

myristoyl (such as dimyristoyl), or both stearoyl (such as distearoyl).

The PEG may be coupled together with a dialkyloxypropyl (DAA) moiety. By "dialkyloxypropyl" or "DAA" it is preferably understood a compound having 2 alkyl chains which have independently between 2 and 30 carbons. The alkyl chains can be saturated or may have varying degrees of unsaturation. Preferably, the PEG-DAA conjugate is a PEG-didecyloxypropyl ($C_{10}$) conjugate, a PEG-dilauryloxypropyl ($C_{12}$) conjugate, a PEG-dimyristyloxypropyl ($C_{14}$) conjugate, a PEG-dipalmityloxypropyl ($C_{16}$) conjugate, or a PEG-distearyloxypropyl ($C_{18}$) conjugate.

When the PEG is coupled, it preferably has an average molecular weight of 750 or 2,000 daltons.

Preferably, the terminal hydroxyl group of the PEG is substituted with a methyl group, thereby creating a terminal methoxy end group.

The component capable of reducing aggregation of the composition may also be chosen between other hydrophilic polymers other than PEG. Examples of suitable hydrophilic polymers other than PEG include, but are not limited to, polyglycerol, polyvinylpyrrolidone, polymethyloxazoline, polyethyloxazoline, polyhydroxypropylmethacrylamide, polymethacrylamide and polydimethylacrylamide, polylactic acid, polyglycolic acid, and derivatized celluloses such as hydroxymethylcellulose or hydroxyethylcellulose, and mixtures thereof.

The component capable of reducing aggregation of the composition may also be chosen in the poloxamer family. By "poloxamer" it is preferably understood well-known class of non-ionic triblock copolymers composed by a central hydrophobic chain of polyoxypropylene (PPO) surrounded by two hydrophilic chains belonging to the polyethylene oxide (PEO) family [PEO-PPO-PEO]. These poloxamers can be linear or X-shaped, those latest being known as poloxamines. Examples of poloxamers include, but are not limited to, the meroxapols, also known as "reverse poloxamers" in the literature, which present a hydrophilic block based on a polyethylene oxide polymer, surrounded by two hydrophobic blocks, based on polypropylene oxide polymers. Poloxamers can be named after their manufacturer's commercial name, such as "Pluronic" "Symperonic" or "Tetronic". For example, poloxamers suitable to be used as component able to reduce aggregation of the composition might be chosen between F127, F108, F68, F87, P123, L61, L64, F88 or F98.

The poloxamer may be associated with another component capable of reducing aggregation such as polyethylene glycol (PEG)-modified lipids (PEG-modified lipids) and/or polyethylene glycol (PEG) optionally substituted, conjugated and/or coupled as previously defined.

Examples of monosialoganglioside (Gml) include, but are not limited to, ganglioside GD1b, GQ1b, GT1b, GD1a, GM4 or GD3.

Examples of polyamide oligomers (PAO) include, but are not limited to, polyamide 6 dimer, polyamide 6 trimer, polyamide 6 tetramer, polyamide 6 cyclic dimer, polyamide 6 cyclic trimer, polyamide 6 cyclic tetramer.

The composition may also include one or more sterols or sterol derivatives, such as cholesterol and/or derivatives thereof. Examples of cholesterol derivatives include, but are not limited to, polar analogues such as 5C-cholestanol, 5C-coprostanol, cholesteryl-(2-hydroxy)-ethyl ether, cholesteryl-(4'-hydroxy)-butyl ether, and 6-ketocholestanol, non-polar analogues such as 5C-cholestane, cholestenone, 5C-cholestanone, 5C-cholestanone, and cholesteryl decanoate, and mixtures thereof. Preferably, the cholesterol derivative is a polar analogue such as cholesteryl-(4'-hydroxy)-butyl ether.

The composition may further include one or more lipids bearing a targeting ligand.

Examples of targeting ligand conjugatable with lipids include, but are not limited to, any peptides, proteins, antibodies, sugar, aptamers, small organic and inorganic molecules for examples RGDF peptides, A54 peptides, cRGD, Lyp-1 peptide, hEGF ligands, Anti-CD44 antibody, HIV trans-activating transcriptional activator peptide, Ega1 nanobodies, hyaluronic acid, galactose, mannose, transferrin, folate, and mixtures thereof.

Examples of lipids able to conjugate with targeting ligands include but are not limited to phospholipids such as dimyristoyl-phosphatidylethanolamine (DMPE), dipalmitoyl-phosphatidylethanolamine (DPPE), dioleoyl-phosphatidylethanolamine (DOPE), distearoyl-phosphatidylethanolamine (DSPE), and mixtures thereof or sterols such as cholesterol, 5C-cholestanol, 5C-coprostanol, cholesteryl-(2-hydroxy)-ethyl ether, cholesteryl-(4'-hydroxy)-butyl ether, and 6-ketocholestanol, and mixtures thereof.

The composition may further include one or more active ingredients such as nucleic acid(s), polysaccharide(s), lipid(s), polynucleotide(s), morpholino oligonucleotide(s), aptamer(s), protein(s), peptide(s), polypeptide(s), peptoid(s), small organic or inorganic molecule(s), drug(s) or any compounds of pharmaceutical interest, antigen(s), or mixtures thereof, to form picoparticles, microparticles, nanoparticles, liposomes, lipoplexes or micelles with the lipid of formula (I) as previously defined.

More particularly, examples of drug(s) or any compounds of pharmaceutical interest include, but are not limited to, folate or sodium valproate, antibodies, antigens, lymphokines, interleukins, necrosis and apoptosis factors, interferons, growth factors, tissue plasminogen activators, factor VIIIc, erythropoietin, insulin, calcitocin, thymidine kinase, and combination thereof.

The composition may further include adjuvants capable of specifically targeting a determinant at the surface and/or inside the cells. These adjuvants can be covalently or non-covalently attached to the lipid of formula (I) or to any other component contained in the composition comprising the lipid of formula (I).

Examples of adjuvant capable of specifically targeting a determinant at the surface and/or inside the cells include, but are not limited to, ligands of receptors expressed at the surface of the target cells, for example a sugar, folate, transferrin, insulin, hormone, prostaglandins, peptide, antibody, metabolite, vitamins or any other molecule which can recognize an extracellular receptor, or an element of intracellular vectorization for targeting specific compartments such as the mitochondria, nucleus or cytoplasm, such as for example a nuclear or cytoplasm or mitochondrial localization signal such as for example a sugar, a ligand or a ligand fragment. a hormone, a peptide or polypeptide, an antibody (such as polyclonal antibodies, monoclonal antibodies, antibody fragments, humanized antibodies, recombinant antibodies and recombinant human antibodies), a protein, cytokines, growth factors, apoptotic factors, a metabolite, vitamins, an aptamer, differentiation- inducing factors, cell-surface receptors and their ligands, hormones, or any other molecule which can recognize an extracellular receptor and mixtures thereof. The adjuvant can also be a fluorophore such as rhodamine, fluorescein or biotin.

The composition may further include signalling molecules and molecules that may be involved in the signalling pathways inside of the target cells.

The lipids of formula (I) as previously defined may be combined with other lipid compounds, polymers (synthetic or natural), surfactants, cholesterol, carbohydrates, saccharides, glycerol, cyclodextrins, magnetic crystal or particles, particles based on organic or inorganic compounds, histones, deoxycholic acid, proteins or combination thereof, to form the lipid particles as previously defined. Thus, the composition as previously defined may also comprise other lipid compounds, polymers (synthetic or natural), surfactants, cholesterol, carbohydrates, saccharides, glycerol, cyclodextrins, magnetic crystal or particles, particles based on organic or inorganic compounds, histones, deoxycholic acid, proteins or combination thereof.

The present invention also relates to process for preparing the composition as previously defined.

For example, the process for preparing the composition as previously defined may comprise the steps of:

- preparing an organic or aqueous phase by dissolving one or more lipids of the present invention, preferably tunable lipids of the present invention, one or more neutral neutral lipids as previously defined, one or more components capable of reducing aggregation of said composition as previously defined, and optionally one or more sterol derivatives as previously defined, and optionally one or more lipids bearing a targeting ligand as previously defined, and optionally additional components as previously defined. When an organic phase is prepared, the chosen organic solvent must be miscible with water and may include, but not limited to, ethanol, tetrahydrofuran and acetonitrile;
- preparing an organic or aqueous phase comprising one or more active ingredients as previously defined. Preferably, the pH of this organic or aqueous phase is controlled to be inferior to the pKa of the lipid of formula (I) as previously defined, preferably of the tunable lipid of formula (I) as previously defined;
- mixing both phases to form of the composition comprising lipid particles encapsulating one or more active ingredients to be delivered in a lipidic architecture.

Suitable physical methods enabling a correct mixture phases include, but are not limited to, microfluidic mixing, microinjection, extrusion, T-mixing flow focusing, pressure-driven flow focusing, sonication, and combination thereof.

Advantageously, the nitrogen-containing portion of the lipids of formula (I) as previously defined, preferably of the tunable lipids of the present invention, may be used to complex the active ingredient, thereby enhancing their delivery and preventing their degradation.

In particular, the lipids of formula (I) as previously defined, preferably the tunable lipids of formula (I) as previously defined, are particularly attractive for delivering active ingredients for several reasons: they contain nitrogen-based groups for interacting with the active ingredient to be delivered such as DNA, RNA, or other polynucleotides, and other negatively charged active ingredients, such as negatively-charged drugs of interest like folate or sodium valproate. They are also extremely useful for buffering the pH, for causing endo-osmolysis, and for protecting the agent to be delivered. Furthermore, they can be synthesized from commercially available starting materials, and/or they are pH responsive and can be engineered with a desired pKa in the correct broad range (pKa varying from 4 to 6.9).

Thus, the lipid of formula (I) and/or the lipid particles as previously defined may be used to protect and deliver an active ingredient to a target site of interest (such as cell, tissue and/or organ).

The active ingredient to be delivered by the picoparticles, microparticles, nanoparticles, liposomes, lipoplexes or micelles may be in the form of a gas, liquid, or solid.

Preferably, the lipids of formula (I) as previously defined are biocompatible and biodegradable, and the formed picoparticles, microparticles, nanoparticles, liposomes, lipoplexes or micelles as previously defined are also biodegradable and biocompatible and may be used to provide controlled, sustained release of the active ingredient to be delivered.

The composition as previously defined may optionally be combined with a pharmaceutical excipient to form a pharmaceutical composition.

Thus, another object of the present relates to a pharmaceutical composition comprising the composition as previously defined and a pharmaceutical excipient.

The present invention also relate to the first therapeutic use of the lipid of formula (I) as previously defined, of the lipid particles as previously defined and/or of the composition as previously defined.

In particular, the lipid of formula (I) as previously defined, the lipid particles as previously defined and/or the composition as previously defined can be used for treating and/or preventing and/or diagnosing a disease and/or a disorder.

Thus, the present invention also relate to the use of the lipid of formula (I) as previously defined, of the lipid particles as previously defined and/or of the composition as previously defined for the manufacture of a medicament.

The present invention also relate to the lipid of formula (I) as previously defined, the lipid particles as previously defined and/or the composition as previously defined for its use as a medicament.

The present invention also relate to a method for treating and/or preventing and/or diagnosing a disease and/or a disorder comprising the use of the lipid of formula (I) as previously defined, of the lipid particles as previously defined and/or of the composition as previously defined.

Examples of diseases and disorders include, but are not limited to, cancers, bacterial or viral infections, inflammatory and genetic disorders, and/or metabolic deficiencies such as diabetes.

The lipids of formula (I) as previously defined, the lipid particles as previously defined and/or the compositions as previously defined are particularly suitable for delivering an active ingredient such as nucleic acid(s), polysaccharide(s), lipid(s), polynucleotide(s), morpholino oligonucleotide(s), aptamer(s), protein(s), peptide(s), polypeptide(s), peptoid(s), small organic or inorganic molecule(s), drug(s) or any compounds of pharmaceutical interest, antigen(s), or mixtures thereof, to subject(s), organ(s), cell(s) and/or tissue(s).

The subject(s) may be human(s), animal(s) and/or organism(s) such as plant(s).

The tissues can be for example, but not limited to, muscle, connective, epithelial or nervous tissues. Methods to obtain samples from various tissues and methods to establish primary cell lines and immortalized cell lines are well-known in the art (see for example Freshney, R.I. Culture of Animal Cells: A Manual of Basic Technique, Fifth Edition, 2005 Ed: John Wiley & Sons, Inc.).

Thus, another object of the present invention relates to the use of the lipids of formula (I) as previously defined, the lipid particles as previously defined and/or the compositions as previously defined as a vector for delivering an active ingredient such as nucleic acid(s), polysaccharide(s), lipid(s), polynucleotide(s), morpholino oligonucleotide(s), aptamer(s), protein(s), peptide(s), polypeptide(s), peptoid(s), small organic or inorganic molecule(s), drug(s) or any compounds of pharmaceutical interest, antigen(s), or mixtures thereof, to subject(s), organ(s), cell(s) and/or tissue(s).

The present invention relates to the lipids of formula (I) as previously defined, the lipid particles as previously defined and/or the compositions as previously defined for their use as a vector for delivering an active ingredient such as nucleic acid(s), polysaccharide(s), lipid(s), polynucleotide(s), morpholino oligonucleotide(s), aptamer(s), protein(s), peptide(s), polypeptide(s), peptoid(s), small organic or inorganic molecule(s), drug(s) or any compounds of pharmaceutical interest, antigen(s), or mixtures thereof, to subject(s), organ(s), cell(s) and/or tissue(s).

The lipids of formula (I) as previously defined, the lipid particles as previously defined and/or the compositions as previously defined can be used *in vitro, ex vivo* and *in vivo.*

The present invention also relate to a method for delivering an active ingredient such as nucleic acid(s), polysaccharide(s), lipid(s), polynucleotide(s), morpholino oligonucleotide(s), aptamer(s), protein(s), peptide(s), polypeptide(s), peptoid(s), small organic or inorganic molecule(s), drug(s) or any compounds of pharmaceutical interest, antigen(s), or mixtures thereof, to subject(s), organism(s), organ(s), cell(s) and/or tissue(s) comprising a step of contacting the lipids of formula (I) as previously defined, the lipid particles as previously defined and/or the compositions as previously defined with the active ingredient.

The method can be performed *in vivo, ex vivo* or *in vitro.*

The lipids of formula (I) as previously defined and/or the lipid particles as previously defined may be included in any pharmaceutical, cosmetic, veterinarian, diagnostic and/or research product compositions.

Thus, the composition as previously defined may be a pharmaceutical, cosmetic, veterinarian, diagnostic and/or research product composition.

Thus, the present invention also relate to the pharmaceutical, cosmetic, veterinarian, diagnostic and/or research product use of the composition as previously defined.

The lipids of formula (I) as previously defined and/or the lipid particles as previously defined may also be used for other purposes as well as, for example, for coating, additives, excipients and/or materials for bioengineering purposes.

Thus, another object of the present invention relates to the use of the lipids of formula (I) as previously defined and/or the lipid particles as previously defined as materials for coating, additives, excipients and/or materials for bioengineering purposes.

Examples of materials for bioengineering purposes include, but are not limited to, filtration membrane, synthetic biotissues, or hydrogels.

The present invention also relate to a method for transfecting cells with a nucleic acid and/or a protein and/or a peptide and/or a polysaccharide and/or a lipid and/or a small organic or inorganic molecule and/or any type of bioactive

molecule using the lipid of formula (I) as previously defined, the lipid particle as previously defined and/or the composition as previously defined.

The method may comprise the step of contacting said cells with the lipid of formula (I) as previously defined, the lipid particle as previously defined and/or the composition as previously defined and a nucleic acid and/or a protein and/or a peptide and/or a polysaccharide and/or a lipid and/or a small organic or inorganic molecule and/or any type of bioactive molecule.

The present invention also relate to transfected cells obtained by the method.

The transfected cells may be used in cell therapy, preferably in cell gene therapy or CAR T-cell therapy.

The present invention also relate to the use of the lipid of formula (I) as previously defined, the lipid particle as previously defined and/or the composition as previously defined with a nucleic acid and/or a protein and/or a peptide and/or a polysaccharide and/or a lipid and/or a small organic or inorganic molecule and/or any type of bioactive molecule for transfecting cells.

According to the present invention, any kind of cell culture material or cell culture methods can be used respectively in 2 dimensions (2D) or onto 3 dimensional (3D) supports to transfect said cells called "target cells". Preferably, 2D devices are defined as support that allow cells to be cultured in an adherent way (cells adhere at the bottom of the well), in suspension way (cells remains floating into the culture medium), or in co-culture (2 or more different kinds of cell types separated or not with an insert) such as treated or non-treated cell culture plates and dishes (from 1536-wells to 4-well cell culture plates, 35 mm, 60 mm, 90 mm, 100 mm petri dishes) or cell flasks (such as 25cm$^2$, 75cm$^2$ or 150cm$^2$). The present invention may also be applied to 3D matrices such as hydrogel or solid 3D scaffolds of various compositions such as collagen, atelocollagen, glycosan, polystyrene, hyaluronic acid, polycaprolactone and/or polyethylene glycol used as cell culture material or in cell culture methods.

The lipids of formula (I) as previously defined, the lipid particles as previously defined and/or the compositions as previously defined can also be used in combination with molecules known to influence a transfection procedure. Examples of molecules known to influence a transfection procedure include, but are not limited to, polypeptides recombinant or not, liposomes, cationic lipids, cationic polymers such as polyethylenimine, protamine sulphate, polybrene, poly-L-lysine, DEAE Dextran, polylactic-co-glycolic acid, chitosans, amphiphilic polymers such as poloxamer, chemically modified or not, polyethylene glycol and derivatives, anionic polymers such as starch and hyaluronic acid, and combination thereof. These molecules known to influence a transfection procedure can be used before, during and/or after the transfection. Thus, these molecules known to influence a transfection procedure can be added to the cells from 1 week before to 4 weeks before and/or after the transfection, more preferentially from 1 day before to 7 days before and/or after the transfection.

The present invention also relates to the use of magnetic-based particles preferably magnetic-based nanoparticles such as iron-based nanoparticles, with the lipids of formula (I) as previously defined, the lipid particles as previously defined and/or the compositions as previously defined in *in cellulo* delivery of active ingredients as previously defined.

The present invention also relates to a method for *in cellulo* delivery of active ingredients as previously defined with magnetic-based particles, preferably magnetic-based nanoparticles such as iron-based nanoparticles, with the lipids of formula (I) as previously defined, the lipid particles as previously defined and/or the compositions as previously defined.

In case, where the lipids of formula (I) as previously defined, the lipid particles as previously defined and/or the compositions as previously defined are used in combination with magnetic-based particles, preferably magnetic-based nanoparticles, a permanent or oscillating or electro-mediated magnetic field can be applied to the experiment after the cells, the lipids of formula (I) as previously defined, the lipid particles as previously defined and/or the compositions as previously defined, and the magnetic-based nanoparticles have been put in contact. This step can last from 10 seconds to 96 h, preferentially from 1 minute to 48 h, and more preferentially from 5 min to 4 h.

Preferably, the composition as previously defined further include a nucleic acid as active ingredient to form a nucleic acid-lipid particle, preferably nucleic acid-lipid nanoparticle, with the lipid of formula (I) as previously defined.

The nucleic acid-lipid nanoparticle can also comprise other active ingredient as previously defined such as drug(s) or any compounds of pharmaceutical interest as previously defined.

The active ingredient of the nucleic acid-lipid particle can be used as a template for producing one or several proteins of interest.

The nucleic acid can encode in an effective manner for a polypeptide of pharmaceutical interest which, during its expression in the host cell, makes it possible to remedy a malfunction of the receiving organism.

As a result, the composition of the present invention can be used in *in vitro* and *in vivo* research and development or in *in vivo* and *ex vivo* gene therapy and/or nucleic acid-based therapy and/or cell therapy.

The nucleic acid can also encode in an effective manner for a polypeptide capable of producing an immune response against it in humans or animals or of inducing an immune response.

As a result, the composition of the present invention finds a particular application in gene therapy and/or cell therapy, for example in the field of vaccines, and of immunotherapy, in particular for treating or preventing cancers or

bacterial or viral infections.

Thus, the present invention also relates to the composition as previously defined for its use in gene therapy and/or nucleic acid-based therapy and/or cell therapy, for example in the field of vaccines and of immunotherapy.

Examples of cell therapy include, but are not limited to, cell gene therapy or CAR T-cell therapy.

In particular, the present invention relates to the composition as previously defined for its use for treating and/or preventing cancers, bacterial or viral infections, inflammatory and genetic disorders, and metabolic deficiencies such as diabetes.

The present invention also relates to the use of the composition as previously defined for treating or preventing cancers, bacterial or viral infections, inflammatory and genetic disorders, and metabolic deficiencies such as diabetes.

The present invention also relates to a method for treating or preventing cancers, bacterial or viral infections, inflammatory and genetic disorders, and metabolic deficiencies such as diabetes comprising administering the composition as previously defined.

The lipids of formula (I) as previously defined, the lipid particles as previously defined and/or the compositions as previously defined may be used for systemic applications, as they can exhibit extended circulation lifetimes following intravenous (i.v.) injection, they can accumulate at distal sites (including sites physically separated from the administration site).

The lipids of formula (I) as previously defined, the lipid particles as previously defined and/or the compositions as previously defined may be used for topical, cutaneous, oral, rectal, vaginal, parenteral, intranasal, intravenous, intramuscular, sub-cutaneous, intraocular, transdermal, intratracheal and/or intraperitoneal administration.

The present invention also relates to a kit comprising the lipid of formula (I) as previously defined, the lipid particles as previously defined and/or the composition as previously defined further comprising a nucleic acid and/or a protein and/or a peptide and/or a polysaccharide and/or a lipid and/or a small organic or inorganic molecule and/or any type of bioactive molecule, and optionally instructions of use.

## BRIEF DESCRIPTION OF FIGURES

**Figure 1** Reaction scheme for the synthesis of molecule I: i) Oleylamine, DIC, HOBt, DMF/$CHCl_3$; ii) Piperidine, DMF; iii) Dimethylglycine (DMG), pyBOP, DIPEA, DMF/DCM.

**Figure 2:** Reaction scheme for the synthesis of molecule II i) N,N-dimethylaminobutyric acid, TCFH, Methylimidazole, MeCN/DCM.

**Figure 3:** Reaction scheme for the synthesis of molecule III: i) Succinic anhydride, $Et_3N$, DCM; ii) DIC, HOBt, $Et_3N$, DCM/DMF.

**Figure 4:** Reaction scheme for the synthesis of molecule IV: i) Oleylamine, DIC, HOBt, DMF/$CHCl_3$ ; ii) Piperidine, DMF; iii) Dimethylaminobutyric acid (DMABA), DIC, HOBt, DMF/$CHCl_3$.

**Figure 5:** Reaction scheme for the synthesis of molecule V: i) Diethylaminoethanol, Discuccinimidyl carbonate, $Et_3N$, MeCN; ii) 1b, $Et_3N$, DCM.

**Figure 6:** Reaction scheme for the synthesis of molecule 6b: i) Succinic anhydride, $Et_3N$, DMF/DCM; ii) 1b, DIC, HOBt, $Et_3N$, DMF/DCM; iii) TFA, DCM.

**Figure 7:** Reaction scheme for the synthesis of molecule VI & VII: i) DMG, HBTU, DIPEA, DMF/DCM; ii) DMABA, HBTU, DIPEA, DMF/DCM.

**Figure 8:** Reaction scheme for the synthesis of molecule VIII: i) $Boc_2O$, NaOH, $H_2O$/t-BuOH; ii) 3-aminopropylimidazole, HBTU, DIPEA, DMF/DCM; iii) TFA, DCM iv) linoleic acid, HBTU, DIPEA, DMF/DCM.

**Figure 9:** Reaction scheme for the synthesis of molecule IX: i) 3-aminopropylimidazole, COMU, DIPEA, DMF; ii) TFA, DCM; iii) 2-hexyldecanoic acid, COMU, DIPEA, DMF; iv) Piperidine, DMF; v) linoleic acid, HBTU, DIPEA, DMF.

**Figure 10:** Reaction scheme for the synthesis of molecule X: i) p-TsOH, Toluene reflux, Dean-Stark; ii) N,N-dimethylglycine, TCFH, Methyl-imidazole, MeCN/DCM.

**Figure 11:** Reaction scheme for the synthesis of molecule XI: i) N,N-dimethylaminobutyric acid, TCFH, Methyl-imidazole, MeCN/DCM.

**Figure 12:** Reaction scheme for the synthesis of molecule XII: i) Succinic anhydride, Et3N, DCM/DMF; ii) 4-(1-pyrrolidinyl)-1-butanol, DCC, DMAP, DCM.

**Figure 13:** Reaction scheme for the synthesis of molecule XIII: i) N-(3-Aminopropyl)diethanolamine, HBTU, DIPEA, DMF.

**Figure 14:** Reaction scheme for the synthesis of molecule 14a: i) Phtalimide, $PPh_3$, DIAD, THF; ii) $NH_2NH_2$, 70°C; iii) HCl conc., 70°C.

**Figure 15:** Reaction scheme for the synthesis of molecule XIV: i) 14a, DIC, HOBt, $Et_3N$, DMF/$CHCl_3$; ii) Piperidine; iii) DMABA, pyBOP, DIPEA, DMF/DCM.

**Figure 16:** Reaction scheme for the synthesis of molecule XV: i) Oleic acid, HBTU, DIPEA, HOBt, DCM/DMF.

**Figure 17:** Reaction scheme for the synthesis of molecule XVI: i) Lauric acid, HBTU, DIPEA, HOBt, DMF.

**Figure 18:** Reaction scheme for the synthesis of molecule XVII i) 14a, DIC, HOBt, $Et_3N$, DMF/$CHCl_3$; ii) Piperidine; iii) DMABA, pyBOP, DIPEA, DMF/DCM.

**Figure 19:** Reaction scheme for the synthesis of molecule XVIII: i) DMG, HBTU, DIPEA, HOBt, DCM/DMF; ii)TFA, DCM; iii) DIC, HOBt, $Et_3N$, DCM/DMF; iv) TFA, DCM, RT; v) Linoleyl acid, HBTU, HOBt DIPEA, DMF/DCM.

**Figure 20:** Reaction scheme for the synthesis of molecule 19d: i) DIC, HOBt, $Et_3N$, DCM/DMF; ii) TFA, DCM; iii) N,N-dimethylaminobutyric acid, TCFH, Methyl-imidazole, MeCN/DCM; iv) TBAF, THF 0°C; v) DIC, HOBt, $Et_3N$, DCM/DMF. vi) TFA, DCM.

**Figure 21:** Reaction scheme for the synthesis of molecule XIX: i) Linoleic acid, HBTU, DIPEA, HOBt, DMF/DCM.

**Figure 22:** Reaction scheme for the synthesis of molecule XX: i) 2-amino-N-(2-hydroxyethyl)acetamide HBTU, DIPEA, DMF; ii) Succinic acid, $Et_3N$, DCM; iii) 20b, DCC, DMAP, DCM; iv) TFA, DCM; v) DMABA, HBTU, DIPEA, DMF/DCM.

**Figure 23:** Reaction scheme for the synthesis of molecule 21d: i) $Boc_2O$, EtOH, 97%; ii) Lauric Acid, DIC, HOBt, $Et_3N$, DCM/DMF; iii) Trifluoroacetic acid (TFA), DCM; iv) Fmoc-Glu(OH), DIC, HOBt, $Et_3N$, DCM/DMF; Piperidine, RT.

**Figure 24:** Reaction scheme for the synthesis of molecule XXI: i) N,N-dimethylglycine, HBTU, DIPEA, DMF.

**Figure 25:** Reaction scheme for the synthesis of molecule XXII: i) (Boc)-Orn(Boc)OH, DIC, HOBt, $Et_3N$, DCM/DMF; ii) TFA, DCM; iii) DMABA, HBTU, DIPEA, DMF.

**Figure 26:** Reaction scheme for the synthesis of molecule XXIII: i) Succinic anhydride, $Et_3N$, DCM; ii), 4-Azido-butylamine, DIC, HOBt, $Et_3N$, DCM/DMF; iii) N,N-dimethyl-propargylamine, CuI,$PPh_3$, Na ascorbate.

**Figure 27:** Reaction scheme for the synthesis of molecule XXIV: i) PyBOP, DIPEA, DCM/DMF; ii) 3-(dimethylamino) propane-1,2-diol, ppTs, Toluene reflux.

**Figure 28:** Reaction scheme for the synthesis of molecule XXV: i) $NaBH_4$ EtOH; ii) N,N-dimethylaminobutyric acid, TCFH, Methyl-imidazole, MeCN/DCM.

**Figure 29:** Reaction scheme for the synthesis of molecule XXVI: i) 2-{[Dimethyl(2-methyl-2-propanyl)silyl]oxy}-1,3-propanediol, p-TsOH, Toluene/THF; ii) TBAF, THF, 0°C; iii) Imidazole, MeCN; iv) TCFH, Methyl-imidazole, MeCN/DCM.

**Figure 30:** Reaction scheme for the synthesis of molecule 27b: i) $Boc_2O$, NaOH, t-BuOH, $H_2O$; ii) (Z)-2-nonen-1-ol; DIC, HOBt, $Et_3N$, DCM/DMF; iii) TFA, DCM; iv) $\alpha$-ketoglutamic acid, PyBOP, DIPEA.

**Figure 31:** Reaction scheme for the synthesis of molecule XXVII: i) 2-{[Dimethyl(2-methyl-2-propanyl)silyl]oxy}-1,3-propanediol; ii) TBAF, THF, 0°C; iii) N,N-dimethylaminobutyric acid, TCFH, Methyl-imidazole, MeCN/DCM.

**Figure 32:** Reaction scheme for the synthesis of molecule XXVIII: i) $NaBH_4$ EtOH; ii) DSC, $Et_3N$, DCM; iii) TBDMSCl, Imidazole, DMF; iv) $Et_3N$, DCM; v) TBAF, THF.

**Figure 33:** Reaction scheme for the synthesis of molecule XIX: i)N,N-dimethylethylenediamine, TCFH, Methyl-imidazole, MeCN/DCM; ii) TFA, DCM; iii) 4,6-Heptadecadiynoic acid, HBTU, HOBt, DIPEA, DMF.

**Figure 34:** Reaction scheme for the synthesis of molecule XXX: i) 30b, COMU, DIPEA, DMF; ii) TFA, DCM; iii) Tetradecylamine, DIC, HOBt, $Et_3N$, DMF; iv) Piperidine, DMF; v) DMG, TCFH, 1-methylimidazole, MeCN/DCM.

**Figure 35:** Reaction scheme for the synthesis of molecule XXXI: i) Succinic anhydride, $Et_3N$, DCM; ii) N-(3-Aminopropyl)diethanolamine, TCFH, 1-methylimidazole, MeCN/DCM.

**Figure 36:** Reaction scheme for the synthesis of molecule XXXII: i) 6a, HBTU, DIPEA, DMF; ii) TFA, DCM; iii) Boc-Orn(Boc)-OH, HBTU, DIPEA, DMF; iv) TFA, DCM; v) DMG, pyBOP, DIPEA, DMF.

**Figure 37:** Reaction scheme for the synthesis of molecule 33a: i) 3-Butyl-2-hepten-1-ol, DCC, DMAP, DCM; ii) TFA, DCM.

**Figure 38:** Reaction scheme for the synthesis of molecule XXXIII & XXXIV: i) Fmoc-Glu(OtBu)-OH, COMU, DIPEA, DMF; ii) TFA, DCM; iii) Tetradecylamine, DIC, HOBt, $Et_3N$, DMF/$CHCl_3$; iv) Piperidine, DMF; v) Succinic anhydride, $Et_3N$, DCM; vi) N-(3-Aminopropyl)diethanolamine, TCFH, 1-methylimidazole, MeCN; vii) 4-(1-pyrrolidinyl)-1-butanol, DCC, DMAP, DCM.

**Figure 39:** Reaction scheme for the synthesis of molecule XXXV: i) Dodecylamine, HOBt, DIC, DMF/$CHCl_3$. ii) Piperidine; iii) 30a, pyBOP, DIPEA, DMF; iv) Piperidine; v) DMABA, 1-methylimidazole, TCFH, MeCN.

**Figure 40:** Reaction scheme for the synthesis of molecule XXXVI: i) 6a, DIC, HOBt, $Et_3N$, DMF/DCM; ii) TFA, DCM; iii) BocOrn(Boc)OH, HBTU, DIPEA, DMF; iv) TFA, DCM; v) DMG, 1-methylimidazole, TCFH, MeCN.

**Figure 41:** Reaction scheme for the synthesis of molecule XXXVII: i) Fmoc-Orn(Boc)-OH, HOBT, DIC, $Et_3N$, DMF/DCM; ii) Piperidine; iii) BocOrn(Boc)OH, HBTU, DIPEA, DMF; iv) TFA, DCM; v) Linoleic acid, pyBOP, DIPEA, DMF/DCM.

**Figure 42:** Reaction scheme for the synthesis of molecule XXXVIII: i) 4-(1-pyrrolidinyl)-1-butanol, DCC, DMAP, DCM; ii) Piperidine, DMF; iii) BocOrn(Boc)OH, HBTU, DIPEA, DMF/DCM; iv) TFA, DCM; v) Linoleic acid, pyBOP, DIPEA, DMF.

**Figure 43:** Reaction scheme for the synthesis of molecule XXXIX: i) Ethanolamine, EDC·HCl, HOBt, DMF; ii) TFA, DCM; iii) DMABA, HBTU, DIPEA, DMF; iv) Fmoc-Orn(Boc)-OH, DCC, DMAP, $Et_3N$; v) Piperidine, DMF; vi) Boc-Orn(Boc)-OH, HBTU, DIPEA, DMF; vii) TFA, DCM; viii) Linoleic acid, pyBop, DIPEA, DMF/DCM.

**Figure 44:** Reaction scheme for the synthesis of molecule XXXX; i) DIC, HOBt, $Et_3N$, DCM/DMF. ii) TFA, DCM; iii) Linoleic acid, HBTU, DIPEA, HOBt, DMF/DCM.

**Figure 45:** Reaction scheme for the synthesis of molecule XXXXI: i) DIC, HOBt, $Et_3N$, DCM/DMF; ii) TFA, DCM; iii) Linoleic acid, HBTU, DIPEA, HOBt DMF/DCM.

**Figure 46:** Reaction scheme for the synthesis of molecule XXXXII: i) DIC, HOBt, $Et_3N$, DCM/DMF; ii) TFA, DCM; iii) BocOrn(Boc)OH, DIC, HOBt, $Et_3N$, DCM/DMF; iv) TFA, DCM; v) Linoleic acid, PyBOP, DIPEA, DMF.

**Figure 47:** Reaction scheme for the synthesis of molecule XXXX!!!; i) 33a, DIC, HOBt, DMF/$CHCl_3$; ii) Piperidine, DMF; iii) DMABA, HBTU, DIPEA, DMF.

**Figure 48 A, B:** Evaluation of LNPs mRNA formulation in a transfection procedure using a mRNA coding for GFP protein: HeLa cell line was transfected with a growing dose of mRNA (0.1-20 $\mu$g) formulated in lipid nanoparticles

based of tunable lipids I and XIV, or complexed in the same range using a commercially available transfection reagent (Tr Reag). After 24 h incubation, % of GFP positive cells (A) and % alive and not permeabilized cells (B) were evaluated by flow cytometry.

**Figure 49 A, B:** Evaluation of LNPs mRNA formulation in a transfection procedure using a mRNA coding for GFP protein: Jurkat T-cells cell line was transfected with a growing dose of mRNA (0.1-20 μg) formulated in lipid nanoparticles based of tunable lipids I and XIV, or complexed in the same range using a commercially available transfection reagent (Tr Reag). After 24 h incubation, % of GFP positive cells (A) and % alive and not permeabilized cells (C) were evaluated by flow cytometry.

**Figure 50 A, B:** Bioluminescence signal 3h after i.p. administration of 3 mRNA-LNPs formulation: F-Luc mRNA-LNP I, F-Luc mRNA LNP XIV and F-Luc mRNA LNP DOTAP in nude mice ; 3B : Kinetics of bioluminescence signal over 25h after i.p. administration 2 mRNA-LNPs formulation: : F-Luc mRNA-LNP I and F-Luc mRNA LNP XIV in nude mice (dose equivalent to 10μg mRNA).

**Figure 51 A, B, C, D:** Relative gene expression from firefly luciferase in different organs using tunable lipids. A: relative gene expression in liver's tissues from mouse with F-Luc-mRNA I, F-Luc-mRNA VI and F-Luc-mRNA XIV IP 10μg Luc-mRNA. B: relative gene expression in lungs' tissues from mouse with F-Luc-mRNA I, F-Luc-mRNA VI and F-Luc-mRNA XIV IP 10μg Luc-mRNA. C: relative gene expression in kidneys' tissues from mouse with F-Luc-mRNA I, F-Luc-mRNA VI and F-Luc-mRNA XIV IP 10μg Luc-mRNA. D: relative gene expression in spleen's tissues from mouse with F-Luc-mRNA I, F-Luc-mRNA VI and F-Luc-mRNA XIV IP 10μg Luc-mRNA.

**Figure 52 A, B, C:** Humoral response to OVA subunit vaccine (10μg) in absence or presence of vaccine adjuvant (Alum 1X) and to mRNA(OVA)-LNPs (mRNA dose equals to 10μg) upon sub-cutaneous prime boost injection (DO-14) in C57BL6J mice. A: monitoring of the total Anti-OVA IgG production over time; B: monitoring of the Anti-OVA IgG1 production over time; C monitoring of the Anti-OVA IgG2c production over time.

**Figure 53 A, B:** Th1 Humoral biomarker (IgG2c/IgG1) and B: CD8+ T cell response to OVA subunit vaccine (10μg) in absence or presence of vaccine adjuvant (Alum 1X) and to mRNA(OVA)-LNPs (mRNA dose equals to 10μg) upon sub-cutaneous prime boost injection (D0-14) in C57BL6J mice.

**Figure 54:** Evaluation of LNPs DNA formulation in a transfection procedure using a DNA coding for F-Luc protein: Jurkat T cell line was transfected with a growing dose of DNA (0.1-10 μg) formulated in lipid nanoparticles based on tunable lipids XI, or complexed in the same range using a commercially available transfection reagent (MTX reagent). After 48 h incubation, luminescence of lysed transfected cells has been assessed using the Luciferase Assay Kit (OZ Biosciences).

**Figure 55:** Confocal microscopy pictures of fluorescently labelled LNPs based on tunable lipid XI encapsulating Cy5-labelled NTsiRNA, after 4h of contact with HEK-293 cells. Cell nucleus has been labelled using Hoechst dye, siRNA with cyanine 5, while LNPs have been labelled using DiO.

Picture on the left displays internalization of the LNPs after 4h exposure. Picture on the right displays siRNA-Cy5 uptake.

## EXAMPLES

### I- MATERIAL AND METHODS:

#### a) Materials

Most of the solvents and reagents have been obtained from VWR Prolabo (Briare, France), Sigma-Aldrich SA (St Quentin-Fallavier, France), TCI Europe N V (Zwijndrecht, Belgium), and Bachem Biochimie SARL (Voisin-le-Bretonneux, France). All reagents have been purchased with reagent grade have been used without any further purification procedure, unless otherwise stated. Amino-acids used in this work all derived from natural one (*i.e.*, *L-isomer),* unless otherwise stated.

**b) Methods**

Thin layer chromatographies (TLC) are carried out on aluminium plates covered with silica gel 60 Fasa (Merck). The compounds are developed under UV light (254 nm), with iodine, by immersion in a ninhydrin developer (0.2% in butanol) followed by a stage of heating at 150° C. in the case of the compounds possessing nitrogen-based functions, or by immersion in a cerium/concentrated molybdate developer: 90/10/15/1) followed by a stage of heating at 110° C. in the case of the Sulfur-containing compounds. The synthesized products are purified on chromatography columns on silica. The flash chromatography separations are carried out on silica gel 60 (230-400 mesh ASTM) (Merck). Particle size and zeta potential measurements: The mean hydrodynamic particle size and charge measurements were performed using Dynamic Light Scattering (DLS) and Laser Doppler Velocimetry (LDV), respectively, using Malvern Nano ZS instrument and DTS software (Malvern Instruments, UK) in a water of Grade 2. Various amounts of cationic poloxamers were diluted in 100 μl of water and mixed with an equal volume of the same water containing DNA. The measurements were carried out in automatic mode and the results are presented as mean +/- SEM, n= 3. Each mean represents the average value of 30 measurements.

**1 - Synthesis Molecule I**

**2-amino-N,N'-bis[(Z)-octadec-9-enyl]pentanediamide (1b)**

Fmoc-glutamic acid (10 mmol ; 3.69 g) is dissolved in 80 mL of anhydrous DMF under stirring and under an inert atmosphere. DIC (32 mmol ; 4.96 mL), then HOBt (32 mmol ; 4.32 g), in solution in 20 mL anhydrous DMF, are successively added to the reaction medium. The reaction is then stirred for 3 hours before oleylamine (30 mmol ; 11.6 mL), in solution in 100 mL of anhydrous $CHCl_3$, is added dropwise to the reaction medium. After 36 hours, 30 mL of piperidine are added and the reaction medium is stirred for another 4 hours. The reaction is stopped and the reaction medium is evaporated to dryness under a rough vacuum. The residue is taken back in 100 mL of EtOAc and heated until dissolution. The solution is put in falcons at -20°C. The formed precipitate is washed several times by centrifugation with cold EtOAc and Et2O and dried under high vacuum. A white solid is obtained with a yield of 93 % (9.3 mmol; 6.0 g). TLC : Rf=0.4 (DCM:MeOH 95/5 (v/v)).

**2-[[2-(dimethylamino)acetyl]amino]-N,N'-bis[(Z)-octadec-9-enyl]pentanediamide (I)**

N,N'-dimethylglycine (0.28 mmol ; 29 mg) is placed in a 25 mL flask under an inert atmosphere then dissolved in 2 mL of anhydrous DMF. DIPEA (0.63 mmol; 109 μL) and pyBOP (0.3 mmol ; 156 mg) are added to the solution successively. The reaction medium is stirred under an inert atmosphere at ambient temperature for 15 minutes before Xx (0.25 mmol ; 162 mg), in solution in 2 mL of anhydrous DMF and 2 mL of anhydrous DCM, is added to the reaction medium. After 24 hours, the reaction is stopped and the reaction medium is evaporated to dryness under a high vacuum. The coupling product is then purified by flash chromatography on silica gel (elution gradient from 4 to 10 % MeOH in DCM). In this way, the product is isolated in a yield of 34 % (0.171 mmol ; 125 mg). TLC : Rf = 0.2 (DCM:MeOH 95/5 (v/v))

**2 - Synthesis Molecule II**

**2-[4-(dimethylamino)butanoylamino]-N'-[(Z)-heptadec-9-enyl]-N-[(Z)-octadec-9-enyl]pentanediamide (II)**

N,N-dimethylaminobutyric acid (DMABA, 40 mg, 0.23 mmoles) is dissolved using strong stirring under argon, in 4 mL of dry 2/1 (v/v) mixture solution of MeCN and DCM that already contains methylimidazole (74 μL, 0.92 mmoles). After complete dissolution, a solution of 2 mL of DCM containing compound 1b (0.25 mmoles, 161 mg) is added under agitation. The mixture is cooled down to zero degrees, and Chloro-N,N,N',N'-tetramethylformamidinium hexafluorophosphate (TCFH) (98 mg, 0.35 mmoles) is then introduced in one portion. After addition, the reaction is let warmed to RT during 12h before being concentrated. The obtained residue is then resuspended in 15 mL of ethyl acetate and washed twice with water and once with brine, dried on sodium sulphate, filtered and concentrated. The product is collected by flash chromatography on silica gel using a DCM /methanol mixture as eluent. A gradient has been used ranging from 98/2 to 90/10 (DCM/MeOH, v/v). 149 mg (0.2 mmoles, 85% yield) of a gluey solid are collected. TLC (DCM/MeOH: 95/5) rf: 0.3.

**3 - Synthesis Molecule III**

**4-oxo-4-(2-pyrrolidin-1-ylethylamino)butanoic acid (3a)**

2-pyrrolidin-1-ylethanamine (251 μL, 2 mmoles) is dissolved under argon in 20 mL of a 1/1 (v/v) mixture of dry

DCM and DMF. 2 equivalents (4 mmoles, 560 μL) of triethylamine ($Et_3N$) are then added slowly and the reaction is stirred for 5 minutes. Then, succinic anhydride (4 mmoles, 400 mg) is added and the mixture is stirred for 12h at room temperature. The reaction is then concentrated, and redissolved in 80 mL of ethyl acetate. The organic phase is then washed with a saturated aqueous ammonium chloride solution, twice with MilliQ water, once with brine, dried on MgSO4, filtered and concentrated. This work-up lead to the clean expected product with 79% yield (339 mg, 1.58 mmoles), after high vacuum drying TLC (DCM/MeOH: 95/5) rf: 0.7.

**N,N'-bis[(Z)-octadec-9-enyl]-2-[[4-oxo-4-(2-pyrrolidin-1-ylethylamino)butanoyl]amino]pentanediamide (III)**

Molecule **III** has been isolated from the coupling of compound **3a** (54 mg, 0.25 mmoles) and compound **1b** (0.25 mmoles, 161 mg) using methylimidazole (74 μL, 0.92 mmoles), and TCFH (98 mg, 0.35 mmoles) in 4 mL of 2/1 (v/v) mixture solution of MeCN and DCM according to the procedure described for the compound **II**. Reaction time: 12h room temperature; Purification: Silica gel chromatography gradient 98/2 to 90/10 (DCM/MeOH, v/v). Yield: 65% (135 mg,0.16 mmoles, white solid TLC (DCM/MeOH: 90/10) rf: 0.4.)

## 4 - Synthesis Molecule IV

**2-amino-N,N'-bis[(Z)-octadec-9-enyl]butanediamide (4a)**

Compound **4a** has been isolated from the coupling of Fmoc-Aspartic acid (2.8 mmol; 1 g) and oleylamine (8.4 mmol ; 3.26 mL) using DIC (8.1 mmol ; 1.26 mL), HOBt (9.25 mmol ; 1.25 g) and then 8 mL of piperidine in DMF (30 mL) and $CHCl_3$ (30 mL) according to the procedure described for compound **1b.** Reaction time: 96h room temperature; Yield: 68% (1.9 mmol; 1.2 g, white solid; TLC: Rf=0.4 (DCM:MeOH 95/5 (v/v))

**2-[4-(dimethylamino)butanoylamino]-N,N'-bis[(Z)-octadec-9-enyl]butanediamide (IV)**

DMABA (0.25 mmol ; 162 mg) is placed in a dry 25 mL flask under an inert atmosphere then dissolved in 2.5 mL of anhydrous DMF under stirring. DIC (0.75 mmol; 116 μL) and HOBt (0.75 mmol; 101 mg) are successively added to the reaction medium. The reaction medium is stirred under an inert atmosphere at ambient temperature for 3 hours before **4a** (0.25 mmol ; 158 mg), in solution in 2.5 mL of anhydrous DCM, is added to the reaction medium with triethylamine (0.88 mmol; 122 μL). After 48 hours, the reaction is stopped and the reaction medium is evaporated to dryness under a high vacuum. The coupling product is then purified by flash chromatography on silica gel (elution gradient from 4 to 10 % MeOH in DCM). In this way the product is isolated in a yield of 12 % (0.03 mmol ; 22 mg). TLC : Rf = 0.3 (DCM:MeOH 95/5 (v/v))

## 5 - Synthesis Molecule V

**2-(diethylamino)ethyl-N-[4-[[(Z)-heptadec-9-enyl]amino]-1-[[(Z)-octadec-9-enyl]carbamoyl]-4-oxo-butyl]carbamate (V)**

N,N'-Disuccinimidyl carbonate (1.5 mmol ; 384 mg) is added to a solution of diethylamino ethanol (1 mmol; 132 μL) in 5 mL of anhydrous MECN under stirring. After 5 minutes triethylamine (3 mmol ; 420 μL) is added and the reaction medium is stirred under an inert atmosphere and at ambient temperature until total alcohol consumption. When the reaction is over, the solvent is evaporated and the product dried under high vacuum. The residue is taken back in 5 mL of anhydrous DCM while **1b** (0.5 mmol ; 323 mg) and triethylamine (2 mmol ; 140 μL) are added to the reaction medium. After stirring at ambient temperature under an inert atmosphere for 12 hours, 10 mL of DCM are added and the organic layer is washed with an aqueous solution of NH2Cl and brine. The organic layer is dried on MgSO4 and evaporated to dryness under a high vacuum. The residue is purified by flash chromatography on silica gel (elution gradient from 2 to 8 % MeOH in DCM). In this way, the product is isolated in a yield of 15 % (0.08 mmol ; 59 mg) . TLC : Rf = 0.3 (DCM:MeOH 95/5 (v/v))

## 6 - Synthesis Molecule VI

**4-[2-[2-(tert-butoxycarbonylamino)ethyldisulfanyl]ethylamino]-4-oxo-butanoic acid (6a)**

Compound **6a** is obtained by reacting Boc-Cystamine (5.3 mmol ; 1.34 g), succinic anhydride (10.6 mmol ; 1.06 g) and triethylamine (7.95 mmol ; 1.1 mL) 20 mL of DCM and 5 mL of DMF according to the method described for compound **3a.** Reaction time: 16h room temperature; Purification: Silica gel chromatography gradient 98/2 to 95/5 (DCM/MeOH, v/v). Yield: 93% (1.6 g, 4.92 mmol, TLC (DCM/MeOH: 90/10) Rf: 0.6.)

**2-[2-[[4-[[4-[[(Z)-octadec-9-enyl]amino]-1-[[(Z)-octadec-9-enyl]carbamoyl]-4-oxobutyl]amino]-4-oxo-butanoyl] amino]ethyldisulfanyl]ethylammonium trifluoroacetate (6b)**

6a (1.6 mmol ; 500 mg) is placed beforehand in a dry 100 mL flask under an inert atmosphere then dissolved in 40 mL of DCM under stirring. DIC (2.3 mmol ; 360 μL) and HOBt (2.3 mmol ; 314 mg), dissolved in 5 mL of anhydrous DMF, are successively added to the reaction medium. The reaction is then maintained under an inert atmosphere at ambient temperature for 2 and a half hours. **1b** (2.2 mmol; 1.4 g) in solution in 10 mL of anhydrous DCM and 5 mL of anhydrous DMF with triethylamine (2.9 mmol; 400 μL) is added to the reaction medium under stirring. After 48 hours, the reaction is stopped and the reaction medium is evaporated to dryness under a rough vacuum. The intermediate product is first purified by flash chromatography on silica gel (elution gradient from 2 to 8 % MeOH in DCM), then dissolved in 33 mL of DCM in a 50 mL flask, before 2.5 mL of trifluoroacetic acid is added to the reaction medium. The reaction is then maintained for one night under stirring at ambient temperature. The reaction medium is evaporated to dryness under a rough vacuum. In order to drive off the trifluoroacetic acid excess, the final residue is taken back four times, in succession, in DCM and four times in $Et_2O$ then evaporated to dryness. In this way, the pure product is isolated, in a yield of 42 % (0.68 mmol ; 657 mg, 2 steps).

## 7 - Synthesis Molecule VI & VII

**2-[[4-[2-[2-[[2-(dimethylamino)acetyl]amino]ethyldisulfanyl]ethylamino]-4-oxo-butanoyl]amino]-N,N'-bis [(Z)-octadec-9-enyl]pentanediamide (VI).**

DMG (0.4 mmol, 41 mg) is placed in a dry 25 mL flask under an inert atmosphere then dissolved in 2 mL of anhydrous DMF under stirring. 2-(1*H*-benzotriazol-1-yl)-1,1,3,3-tetramethyluronium hexafluorophosphate (HBTU, 0.4 mmol ; 152 mg) and DIPEA (0.8 mmol ; 136 μL)) are successively added to the reaction medium. The reaction is then maintained under an inert atmosphere at ambient temperature for 15 minutes before Xx (0.2 mmol; 198 mg), in solution in 1 mL of anhydrous DCM and 3 mL of DMF, is added to the reaction medium. After stirring at ambient temperature under an inert atmosphere for 48 hours, the reaction is stopped and the reaction medium is evaporated to dryness under a rough vacuum. The product is purified by flash chromatography on silica gel (elution gradient from 2 to 8 % MeOH in DCM). In this way the product is isolated, in a yield of 32 % (0.064 mmol ; 60 mg). TLC : Rf =0.4 (DCM:MeOH 95/5 (v/v))

**2-[[4-[2-[2-[4-(dimethylamino)butanoylamino]ethyldisulfanyl]ethylamino]-4-oxo-butanoyl]amino]-N,N'-bis [(Z)-octadec-9-enyl]pentanediamide (VII).**

Molecule **VII** has been isolated from the coupling of DMABA (0.6 mmol, 101 mg) and **6b** (0.3 mmol; 298 mg) using HBTU (0.6 mmol ; 227 mg) and DIPEA (1.2 mmol; 204 μL) in 6 mL DMF and 2 mL DCM, according to the procedure described for the molecule **VI.** Reaction time: 48h room temperature; Purification: Silica gel chromatography gradient 98/2 to 92/8 (DCM/MeOH, v/v). Yield: 35% (110 mg, 0.10 mmol, TLC (DCM/MeOH: 95/5) Rf: 0.4.)

## 8 - Synthesis Molecule VIII

**5-amino-2-(tert-butoxycarbonylamino)pentanoic acid/(Boc-Orn(Boc)OH (8a))**

NaOH (24.7 mmoles ; 987 mg) is dissolved in 25 mL of water in a 100 mL flask. A solution of ornithine hydrochloride (11 mmol ; 1.9 g) in 8 mL *t*-BuOH is added to the reaction medium followed by $Boc_2O$ (23.7 mmol ; 5.2 g) also dissolved in 8 mL of *t*-BuOH. $Boc_2O$ is added dropwise to the previous solution. After stirring at ambient temperature for 16 hours, 50 mL of petroleum ether is added and the aqueous layer is extracted 3 times with 50 mL ether. The organic layer is washed two times with $NaHCO_3$. The pH of the aqueous phases is acidified to 2 with concentrated HCl and extracted again 5 times with 100 mL of $Et_2O$. The organic extracts are washed with water, brine, dried on $MgSO_4$ and evaporated to dryness under high vacuum. The obtained oil is crystallized with petroleum ether and filtered, the filtrate is evaporated and crystallized also to give a white powder in a yield of 85 % (20.1 mmol ; 6.7 g).

**5-((3-(1H-imidazol-1-yl)propyl)amino)-5-oxopentane-1,4-diaminium (8b)**

Molecule **8b** has been isolated from the coupling of compound **8a** (1.5 mmol, 500 mg) and 3-aminopropylimidazole (2 mmol ; 238 μL) using HBTU (1.5 mmol ; 569 mg) and DIPEA (2 mmol ; 340 μL) dissolved in 20 mL DCM and 10 mL of DMF according to the procedure described for the molecule **VI.** Reaction time: 48h room temperature. The crude is taken back in 50 mL of EtOAc and washed 3 times with water and brine. The organic layer is dried on MgSO4 and evaporated. The product is crystallized in petroleum ether, dried and is then dissolved in 60 mL DCM and 3 mL of trifluoroacetic acid is added to the reaction medium. The reaction is then maintained for one night under stirring at ambient temperature. The

reaction medium is evaporated to dryness under a rough vacuum. The final residue is taken back four times, in succession, in DCM and four times in $Et_2O$ then evaporated to dryness. The reaction is quantitative (1.5 mmoles, 701 mg).

**(9Z,12Z)-N-[5-(3-imidazol-1-ylpropylamino)-4-[[(9Z,12Z)-octadeca-9,12-dienoyl]amino]-5-oxo-pentyl]octadeca-9,12-dienamide (VIII).**

Molecule **VIII** has been isolated from the coupling of Linoleic acid (1.5 mmol, 468 µL) and compound **8b** (0.6 mmol ; 280 mg) using HBTU (1.5 mmol ; 569 mg) and DIPEA (3 mmol; 509 µL) dissolved in 6 mL DCM and 4 mL of DMF according to the procedure described for the molecule VI. Reaction time: 48h room temperature; Purification: Silica gel chromatography gradient 98/2 to 94/6 (DCM/MeOH, v/v). Yield: 45% (206 mg, 0.27 mmol, TLC (DCM/MeOH: 90/10) Rf: 0.3.)

## 9 - Synthesis Molecule IX

**5-((3-(1H-imidazol-1-yl)propyl)amino)-4-((((9H-fluoren-9-yl)methoxy)carbonyl)amino)-5-oxopentan-1-aminium (9a)**

Fmoc-Ornithine(Boc)-OH (1 mmol, 454 mg) is placed beforehand in a dry 50 mL flask under an inert atmosphere then dissolved in 10 mL of anhydrous DMF under stirring. 1-Cyano-2-ethoxy-2-oxoethylidenaminooxy)dimethylamino-morpholino-carbenium hexafluorophosphate (COMU 1.1 mmol ; 471 mg) and DIPEA (2 mmol ; 346 µL) are successively added to the reaction medium. The reaction is then maintained under an inert atmosphere and 0°C for 15 minutes before 3-aminopropylimidazole (1.1 mmol; 131 µL) is added to the reaction medium under stirring. The solution is allowed to warm to ambient temperature under an inert atmosphere overnight. The reaction medium is evaporated to dryness under a rough vacuum. The crude is taken back in 50 mL of EtOAc and washed with 1M HCl, $NaHCO_3$ and brine. The organic layer is dried on $MgSO_4$ and evaporated. The product is used without further purification. TLC : Rf =0.4 (DCM:MeOH 90/10 (v/v)).

The product is taken back in 50 mL DCM and 2 mL of TFA are added. The reaction is then maintained for one night under stirring at ambient temperature. The reaction medium is evaporated to dryness under a rough vacuum. The final residue is taken back four times, in succession, in DCM and four times in $Et_2O$ then evaporated to dryness. The reaction is quantitative; Yield 66% (2steps, 380 mg, 0.66 mmoles).

**N-[4-amino-5-(3-imidazol-1-ylpropylamino)-5-oxo-pentyl]-2-hexyl-decanamide (9b)**

2-hexyldecanoic acid (1.5 mmol, 440 µL) is placed beforehand in a dry 50 mL flask under an inert atmosphere then dissolved in 15 mL of anhydrous DMF under stirring. COMU (1.5 mmol ; 642 mg) and DIPEA (3 mmol ; 519 µL) are successively added to the reaction medium. The reaction is then maintained under an inert atmosphere and 0°C for 15 minutes before Xx (570 mg), dissolved in 5 mL of anhydrous DMF, is added to the reaction medium under stirring. The solution is allowed to warm to ambient temperature under an inert atmosphere overnight. The reaction medium is evaporated to dryness under a rough vacuum. The crude is taken back in 50 mL of EtOAc and washed with 1M HCl, NaHCO3 and brine. The organic layer is dried on MgSO4 and purified by flash chromatography on silica gel (elution gradient from 2 to 5 % MeOH in DCM). In this way, the intermediary product is isolated, in a yield of 80 % (0.8 mmol ; 560 mg). TLC : Rf =0.5 (DCM:MeOH 90/10 (v/v))

The product is taken back in 20 mL DMF and 5 mL of piperidine are added. The reaction is then maintained for one night under stirring at ambient temperature. The reaction medium is evaporated to dryness under a rough vacuum. The residue is triturated several times in ether, filtrated and used as it is.

**(9Z,12Z)-N-[4-(2-hexyldecanoylamino)-1-(3-imidazol-1-ylpropylcarbamoyl)butyl]octadeca-9,12-dienamide (IX)**

Molecule **IX** has been isolated from the coupling of Linoleic acid (4 mmol, 1.1 g) and crude compound **9b** (0.8 mmol; 382 mg) using HBTU (4 mmol ; 1.5 mg) and DIPEA (6 mmol; 1 mL) dissolved in 20 mL of DMF according to the procedure described for the molecule VI. Reaction time: 48h room temperature; Purification: Silica gel chromatography gradient 96/4 to 90/10 (DCM/MeOH, v/v). Yield: 22% (130 mg, 0.18 mmol, TLC (DCM/MeOH: 95/5) Rf: 0.4.)

## 10 - Synthesis Molecule X

**Bis[(9Z,12Z)-octadeca-9,12-dienyl] 2-aminopentanedioate (10a)**

L-Glutamic acid (220 mg, 1.5 mmoles), Linoleyl alcohol (1.16 mL, 3.75 mmoles) and *para*toluenesulfonic acid

hydrate (715 mg, 3.75 mmoles) are dissolved under stirring in dry toluene (30 mL). The flask is equipped with a Dean-Stark trap and a condenser. The reaction is then stirred under reflux for 18h before being cooled-down and concentrated. The residue is then taken back in DCM, washed twice with a saturated sodium bicarbonate solution, twice with water, and once with brine. Organic extracts are the dried-on magnesium sulfate, filtered and concentrated under vacuum. The product is collected by flash chromatography on silica gel using a gradient of a petrol spirit/ethyl acetate (from 100/0 v/v to 70/30 v/v) mixture as eluent. 322 mg (0.5 mmol, 30% yield) of a colourless oil is collected. TLC (PS/EA: 80/20) rf: 0.4.

**Bis[(9Z,12Z)-octadeca-9,12-dienyl]-2-[[2-(dimethylamino)acetyl]amino]pentanedioate (X)**

Molecule **X** has been isolated from the coupling of DMG (24 mg, 0.23 mmoles) and compound **10a** (0.25 mmoles, 161 mg) using methylimidazole (74 µL, 0.92 mmoles) and TCFH (98 mg, 0.35 mmoles) dissolved in 4 mL of 2/1 (v/v) solution of MeCN and DCM, according to the procedure described for the molecule **II**. Reaction time: 12h 0°C to RT; Purification: Silica gel chromatography gradient 98/2 to 90/10 (DCM/MeOH, v/v). Yield: 72% (124 mg of gluey solid, 0.17 mmol, TLC (DCM/MeOH: 95/5) Rf: 0.2.).

## 11 - Synthesis Molecule XI

**bis[(9Z,12Z)-octadeca-9,12-dienyl] 2-[4-(dimethylamino)butanoylamino]pentanedioate (XI)**

Molecule **XI** has been isolated from the coupling of DMABA (40 mg, 0.23 mmoles) and compound **10a** (0.25 mmoles, 161 mg) using methylimidazole (74 µL, 0.92 mmoles) and TCFH (98 mg, 0.35 mmoles) dissolved in 4 mL of 2/1 (v/v) solution of MeCN and DCM, according to the procedure described for the molecule **II**. Reaction time: 12h 0°C to RT; Purification: Silica gel chromatography gradient 98/2 to 90/10 (DCM/MeOH, v/v). Yield: 72% (136 mg of gluey solid, 0.180 mmol, TLC (DCM/MeOH: 95/5) Rf: 0.2.).

## 12 - Synthesis Molecule XII

**4-[[4-[(9Z,12Z)-octadeca-9,12-dienoxy]-1-[(9Z,12Z)-octadeca-9,12-dienoxy]carbonyl-4-oxo-butyl]amino]-4-oxo-butanoic acid (12a)**

Compound **10a** (1 mmol ; 644 mg) is placed beforehand in a dry 50 mL flask under an inert atmosphere then dissolved in 15 mL of DCM and 5 mL of anhydrous DMF under stirring. Succinic anhydride (1.5 mmol ; 150 mg) and triethylamine (2 mmol ; 280 µL) are added to the reaction medium. After stirring at ambient temperature under an inert atmosphere for 16 hours, the reaction is stopped and the reaction medium is evaporated to dryness under a high vacuum. The crude is purified by flash chromatography on silica gel (elution gradient from 0 to 6 % MeOH in DCM). The product is obtained in a yield of 91 % (0.91 mmol ; 677 mg). TLC : Rf =0.5 (DCM:MeOH 95/5 (v/v))

**bis[(9Z,12Z)-octadeca-9,12-dienyl]2-[[4-oxo-4-(4-pyrrolidin-1-ylbutoxy)butanoyl]amino]pentanedioate (XII)**

4-(1-pyrrolidinyl)-1-butanol (0.7 mmol ; 100 mg) is added under stirring to a solution of **12a** (0.47 mmol ; 350 mg), N,N'-Dicyclohexylcarbodiimide (DCC,0.7 mmol ; 145 mg) and 4-(Dimethylamino)pyridine (DMAP,0.094 mmol ; 11 mg) in 18 mL of DCM. After stirring at ambient temperature under an inert atmosphere for 15 hours, the reaction is stopped and the reaction medium is evaporated to dryness under high vacuum The crude is taken back in 20 mL of DCM and washed with 1M HCl, $NaHCO_3$ and brine. The organic layer is dried on $MgSO_4$ and purified by flash chromatography on silica gel (elution gradient from 2 to 10 % MeOH in DCM). In this way, the product is isolated, in a yield of 73 % (0.34 mmol ; 298 mg). TLC : Rf =0.3 (DCM:MeOH 95/5 (v/v)).

## 13 - Synthesis Molecule XIII

**bis[(9Z,12Z)-octadeca-9,12-dienyl]2-[[4-[3-[bis(2-hydroxyethyl)amino]propylamino]-4-oxo-butanoyl]amino] pentanedioate (XIII)**

Molecule **XIII** has been isolated from the coupling of compound **12a** (0.4 mmol, 298 mg) and N-(3-aminopropyl) diethanolamine (0.5 mmol; 76 µL) using HBTU (0.4 mmol ; 152 mg) and DIPEA (0.8 mmol ; 136 µL) dissolved in 8 mL of DMF according to the procedure described for the molecule **VI** () Reaction time: 16h RT; Purification: Silica gel chromatography gradient 98/2 to 90/10 (DCM/MeOH, v/v). Yield: 66% (0.26 mmol ; 234 mg), TLC (DCM/MeOH: 95/5) Rf: 0.2.).

## 14 - Synthesis Molecule XIV

### [(9Z,12Z)-octadeca-9,12-dienyl]ammonium, chloride (14a)

A solution of linoleyl alcohol (7.6 mmol ; 2.02 g) and diisopropyl azodicarboxylate (12 mmol ; 6.4 mL) in 14 mL of THF was added at 0°C to a solution of $PPh_3$ (9 mmol, 2.36 g) and phthalimide (9 mmol ; 1.32 g) in 10 mL of THF over 20 min. The reaction medium is allowed to warm to ambient temperature overnight. After the total conversion of the alcohol, hydrazine hydrate (2 mL, 50 wt %) is added, and the resulting solution is heated at reflux for 6 h. The solution is allowed to cool to ambient temperature, and 10 mL concentrated HCl was slowly added. The reaction medium is then refluxed for 2 h, then allowed to cool to ambient temperature, and stirred overnight. The precipitate was removed by filtration, and the solvent was removed in vacuo. The residual solid is dissolved in water and extracted 3 times with 40 mL DCM. The aqueous layer was concentrated to provide the ammonium in a yield of 53 % (4.02 mmol; 1.21 g).

### 2-amino-N,N'-bis[(9Z,12Z)-octadeca-9,12-dienyl]pentanediamide (14b)

Following the procedure described for compound **1b,** compound **14b** is obtained in two steps by first reacting Fmoc-glutamic acid (0.6 mmol ; 222 mg) and compound **14a** (1.5 mmol ; 453 mg) in presence of DIC (1.92 mmol ; 297 μL) HOBt (1.92 mmol ; 259 mg), and $Et_3N$ (3 mmol ; 408 μL) dissolved in 6 mL DMF and 6 mL $CHCl_3$. Piperidine (3 mL) is then added after 36h. Reaction time: 40h RT; Yield: 84% (white solid, 0.5 mmol ; 380 mg), TLC (DCM/MeOH: 95/5) Rf: 0.2.).

### 2-[4-(dimethylamino)butanoylamino]-N,N'-bis[(9Z,12Z)-octadeca-9,12-dienyl]pentanediamide (XIV)

Following the procedure described for compound **I,** molecule **XIV** is obtained by coupling DMABA (0.3 mmol ; 50 mg) and compound **14b** (0.2 mmol ; 129 mg) using DIPEA (0.6 mmol ; 100 μL) and pyBOP (0.3 mmol ; 156 mg) dissolved in 4 mL of DMF. Reaction time: 24h RT; Purification: Silica gel chromatography gradient 98/2 to 92/8 (DCM/MeOH, v/v). Yield: 45% (0.09 mmol ; 68 mg), TLC (DCM/MeOH: 95/5) Rf: 0.3.).

## 15 - Synthesis Molecule XV

### (Z)-N-[5-(3-imidazol-1-ylpropylamino)-4-[[(Z)-octadec-9-enoyl]amino]-5-oxo-pentyl]octadec-9-enamide (XV)

Oleic acid (251 μL, 0.8 mmoles) is dissolved under argon in 4 mL of a DMF solution that already contains DIPEA (175 μL, 1 mmoles) and HOBt (122 mg, 0.9 mmoles). After dissolution, a solution of 4 mL of DCM containing compound **9a** (0.25 mmoles, 117 mg) is added under agitation. The mixture is cooled down to zero degrees, and HBTU (341 mg, 0.9 mmoles) is then introduced portion wise over 30 minutes. After addition, the reaction is let warmed to RT during 12h, and stirred for 36 additional hours before being concentrated. The obtained residue is then resuspended in 30 mL of ethyl acetate and put 3 h at -20°C. The resulting liquid and solid are then separated by centrifugation at 3000 rpm, and this procedure is repeated two times. All the liquid are collected and concentrated under vacuum while the solids are discarded. The product is collected by flash chromatography on silica gel using a DCM /methanol mixture as eluent. A gradient has been used ranging from 99/1 to 90/10 (DCM/MeOH, v/v). 102 mg (0.133 mmol, 53% yield) of a white creamy solid are collected. TLC (DCM/MeOH: 95/5) rf: 0.2.

## 16 - Synthesis Molecule XVI

### N-[4-(dodecanoylamino)-5-(3-imidazol-1-ylpropylamino)-5-oxo-pentyl]dodecanamide (XVI)

Following a similar protocol than for compound **XV** but using Lauric acid (0.8 mmoles, 160 mg) lead to the obtention of compound **XVI** with 68 % yield (0.17 mmoles, 103 mg) as a white powder. TLC (DCM/MeOH: 95/5) rf: 0.25.

## 17 - Synthesis Molecule XVII

### 2-amino-N,N'-bis[(9Z,12Z)-octadeca-9,12-dienyl]butanediamide (17a)

Following the procedure described for compound **1b,** compound **17a** is obtained in two steps by first reacting Fmoc-aspartic acid (0.6 mmol ; 213 mg) and compound **14a** (2 mmol ; 604 mg) in presence of DIC (1.92 mmol ; 297 μL) HOBt (1.92 mmol ; 259 mg), and $Et_3N$ (3 mmol ; 408 μL) dissolved in 6 mL DMF and 6 mL $CHCl_3$. Piperidine (3 mL) is then added after 36h. Reaction time: 40h RT; Yield: 69% (white solid, 0.41 mmol; 260 mg), TLC (DCM/MeOH: 90/10) Rf: 0.4).

**2-[4-(dimethylamino)butanoylamino]-N,N'-bis[(9Z,12Z)-octadeca-9,12-dienyl]butanediamide (XVII)**

Following the procedure described for compound I, molecule **XVII** is obtained by coupling DMABA (0.6 mmol ; 101 mg) and compound **17a** (0.4 mmol ; 260 mg) using DIPEA (1.2 mmol ; 207 μL) and pyBOP (0.6 mmol ; 312 mg) dissolved in 8 mL of DMF. Reaction time: 24h RT; Purification: Silica gel chromatography gradient 98/2 to 92/8 (DCM/MeOH, v/v). Yield: 33% (0.13 mmol ; 97 mg), TLC (DCM/MeOH: 95/5) Rf: 0.3.).

**18 - Synthesis Molecule XVIII**

**2-[2-[[2-(dimethylamino)acetyl]amino]ethyldisulfanyl]ethylammonium (18a)**

DMG (1 mmol, 103 mg) is placed in a 50 mL flask under an argon then dissolved in 10 mL of DMF under stirring. HBTU (1 mmol ; 379 mg) and DIPEA (2 mmol ; 346 μL) are successively added to the reaction medium. The reaction is then maintained under an inert atmosphere at RT for 15 minutes before Boc-Cystamine (0.83 mmol; 186 mg), in solution in 4 mL of DCM and 2 mL of DMF, is added to the reaction medium. After stirring at RT under argon for 16 hours, the reaction is stopped and the reaction medium is evaporated to dryness under a rough vacuum. The crude is taken back in 50 mL of EtOAc and washed 3 times with water and brine. The organic layer is dried on $MgSO_4$ and evaporated. The product, used without further purification (TLC : Rf =0.2 (DCM:MeOH 90/10 (v/v)),
is then dissolved in 40 mL of DCM, before 2 mL of trifluoroacetic acid are introduced into the reaction medium. The reaction is then maintained for 12 hours under stirring at RT. The reaction medium is evaporated to dryness under a vacuum. The final residue is taken back four times, in succession, in DCM and four times in $Et_2O$ then evaporated to dryness. Yield 83% (2 steps, 0.83 mmoles, 291 mg) .

**[5-[2-[2-[[2-(dimethylamino)acetyl]amino]ethyldisulfanyl]ethylamino]-5-oxo-4-[(2,2,2-trifluoroacetyl)ammonio] pentyl]-(2,2,2-trifluoroacetyl)ammonium (18b)**

Boc-L-Orn(Boc)-OH, 365 mg, 1.1 mmoles) is dissolved under Argon in 10 mL of a 9/1 v/v mixture of dry DCM/DMF, in a three-necks round-bottom flask equipped with a dropping funnel. HOBt (162 mg, 1.2 mmoles) and DIC (201 μL, 1.3 mmoles) are subsequently added and the reaction is stirred at RT for 3h, increasing the turbidity of the medium. Then compound **18a** (351 mg, 1 mmoles) and $Et_3N$ (210 μL, 1.5 mmoles) dissolved in 10 mL of a 9/1 v/v mixture of dry DCM/DMF are added over 30 min dropwise via the dropping funnel. After the completion of the addition, the reaction is stirred 36h at room temperature and concentrated to dryness. The crude mixture is then resuspended in 20 mL of ethyl acetate and put 3 h at -20°C. The resulting liquid and solid are then separated by centrifugation at 3000 rpm, and this procedure is repeated three times. The solid residues are collected and washed subsequently three times using diethyl ether, before being dried overnight under vacuum.

The crude compound is dissolved under argon in 25 mL of dry DCM. 190 μL (2.5 mmoles) of trifluoracetic acid (TFA) are then added slowly and the reaction is pursued until complete conversion evidenced by TLC (2h). The mixture is then concentrated, co-evaporated thrice with 20 mL of DCM, thrice with 20 mL of diethyl ether ($Et_2O$), and dried under high vacuum overnight. The product is then used as it without further purification (0.63 mmoles, 345 mg, 63%).

**(9Z,12Z)-N-[5-[2-[2-[[2-(dimethylamino)acetyl]amino]ethyldisulfanyl]ethylamino]-4-[[(9Z,12Z)-octadeca-9,12-dienoyl]amino]-5-oxo-pentyl]octadeca-9,12-dienamide (XVIII)**

Following the procedure described for compound **XV**, molecule **XVIII** is obtained by coupling Linoleic acid (404 μL, 1.3 mmoles) with compound **18b** (0.63 mmoles, 345 mg) with DIPEA (263 μL, 1.5 mmoles), HOBt (183 mg, 1.35 mmoles) and HBTU (530 mg, 1.4 mmoles) dissolved in 6 mL DMF and 6 mL DCM. Reaction time: 18h 0°C to RT; Purification: Silica gel chromatography gradient 99/1 to 90/10 (DCM/MeOH, v/v). Yield: 78% (429 mg of white solid, 0.49 mmol, TLC (DCM/MeOH: 90/10) Rf: 0.4.).

**19- Synthesis Molecule XIX**

**19a. Synthesis molecule 19d**

**2,5-diamino-N-[2-[tert-butyl(dimethyl)silyl]oxyethyl]pentanamidebis-trifluoroacetate. (19a)**

Following the procedure described for compound **18b**, molecule **19a** is obtained in two steps by coupling first Boc-L-Orn(Boc)-OH, (3.65 g, 11 mmoles) with 2-[[*tert*-Butyl(dimethyl)silyl]oxy]ethylamine (2.04 mL, 10 mmoles) with HOBt (1.62 g, 12 mmoles), DIC (2.01 mL, 13 mmoles) $Et_3N$ (2.10 mL, 15 mmoles) dissolved in 10 mL DMF and 90 mL DCM.

Deprotection is carried out using 1.9 mL (25 mmoles) TFA in 250 mL DCM. Reaction time 72h RT then 6h RT. Yield: 71% (3.45 g of white solid, 7.1 mmoles).

**N-[2-[tert-butyl(dimethyl)silyl]oxyethyl]-2,5-bis[4-(dimethylamino)butanoylamino]pentanamide (19b)**

Molecule **19b** has been isolated from the coupling of DMABA (400 mg, 2.4 mmoles) and compound 1**9a** (485 mg, 1 mmole) using methylimidazole (650 µL, 8 mmoles) and TCFH (1.13 g, 4 mmoles) dissolved in 50 mL of 2/1 (v/v) solution of MeCN and DCM, according to the procedure described for the molecule **II**. Reaction time: 18h 0°C to RT; Purification: Silica gel chromatography gradient 98/2 to 90/10 (DCM/MeOH, v/v). Yield: 82% (423 mg of white solid, 0.82 mmol, TLC (DCM/MeOH: 95/5) Rf: 0.5.).

**2,5-bis[4-(dimethylamino)butanoylamino]-N-(2-hydroxyethyl)pentanamide (19c)**

Compound **19b** (258 mg, 0.5 mmoles) is dissolved in 12 mL of dry THF. Tetrabuylammonium fluoride (TBAF, 1M in THF, 2 mmoles, 2 mL) is then introduced and the reaction is stirred for 2 h at 0°C. After concentration of the medium, the obtained residue is resuspended in 15 mL of ethyl acetate and washed twice with water and once with brine, dried on sodium sulphate, filtered and concentrate, yielding the pure compound with 95% yield (0.48 mmoles, 193 mg).

**[1-[2-[2,5-bis[4-(dimethylamino)butanoylamino]pentanoylamino]ethoxycarbonyl]-4-[(2,2,2-trifluoroacetyl)ammonio]butyl]-(2,2,2-trifluoroacetyl)ammonium (19d)**

Following the procedure described for compound **18b,** molecule **19d** is obtained in a two steps sequence by coupling first Boc-L-Orn(Boc)-OH, (92 mg, 0.275 mmoles) with compound **19c** (100 mg, 0.25 mmoles) with HOBt (41 mg, 0.3 mmoles), DIC (50.1 µL, 0.33 mmoles) and $Et_3N$ (53 µL, 0.375 mmoles) dissolved in 3 mL of a 9/1 v/v mixture of dry DCM/DMF. Deprotection is carried out after intermediary purification, by using 76 µL (1 mmoles) TFA in 2.5 mL DCM. Reaction time 36h RT then 2h RT. Yield: 79% (140 mg of colourless syrup, 0.197 mmoles).

<u>**19b. Synthesis molecule XIX**</u>

**2-[2,5-bis[4-(dimethylamino)butanoylamino]pentanoylamino]ethyl-2,5-bis[[(9Z,12Z)-octadeca-9,12-dienoyl] amino]pentanoate (XIX).**

Following the procedure described for compound **XV,** molecule **XIX** is obtained by coupling linoleic acid (81 µL, 0.33 mmoles) with compound **19d** (0.125 mmoles, 88.8 mg) with DIPEA (70 µL, 0.3 mmoles), HOBt (45 mg, 0.33 mmoles) and HBTU (106 mg, 0.28 mmoles) dissolved in 2 mL DMF and 2 mL DCM. Reaction time: 36h 0°C to RT; Purification: Silica gel chromatography gradient 98/2 to 90/10 (DCM/MeOH, v/v). Yield: 83% (108 mg of white solid, 0.104 mmol, TLC (DCM/MeOH: 95/5) Rf: 0.2).

<u>**20 - Synthesis Molecule XX**</u>

***tert*-butyl-N-[4-(tert-butoxycarbonylamino)-5-[[2-(2-hydroxyethylamino)-2-oxo-ethyl]amino]-5-oxo-pentyl]carbamate (20a)**

Molecule 20a has been isolated from the coupling of Boc-L-Orn(Boc)-OH (1.5 mmol, 500 mg) and 2-amino-N-(2-hydroxyethyl)acetamide (2 mmol ; 236 mg) using HBTU (1.5 mmol ; 569 mg) and DIPEA (2 mmol; 340 µL) dissolved in 20 mL of DMF according to the procedure described for the molecule VI. Reaction time: 48h room temperature; Purification: Silica gel chromatography gradient 96/4 to 90/10 (DCM/MeOH, v/v). Yield: 62% (402 mg, 0.93 mmol, TLC (DCM/MeOH: 95/5) Rf: 0.3.).

**4-[[4-[[(9Z, 12Z)-octadeca-9,12-dienyl]am ino]-1 -[[(9Z, 12Z)-octadeca-9,12-dienyl]carbamoyl]-4-oxo-butyl]amino]-4-oxo-butanoic acid (20b)**

Molecule **20b** is obtained following the procedure described for the synthesis of molecule **3a** using **14b** (1.28 g, 2 mmoles), $Et_3N$ (4 mmoles, 560 µL), succinic anhydride (4 mmoles, 400 mg) dissolved under argon in 20 mL of a 1/1 (v/v) mixture of dry DCM and DMF. Reaction time: 18h, 78% yield (1.16g, 1.56 mmoles), TLC (DCM/MeOH: 95/5) rf: 0.5.

**[4-azaniumyl-5-[[2-[2-[4-[[4-[[(9Z,12Z)-octadeca-9,12-dienyl]amino]-1-[[(9Z,12Z)-octadeca-9,12-dienyl]carbamoyl]-4-oxo-butyl]amino]-4-oxo-butanoyl]oxyethylamino]-2-oxo-ethyl]amino]-5-oxo-pentyl]ammonium (20c)**

Compound **20b** (0.3 mmol, 224 mg) is placed in a dry 25 mL flask under an argon then dissolved in 5 mL of DCM under stirring. DCC (0.6 mmol ; 124 mg) and DMAP (0.1 mmol; 12 mg) are added to the reaction medium. The reaction is then stirred for 10 minutes before **20b** (0.4 mmol ; 173 mg) is introduced. After stirring at RT under argon for 16 hours, the reaction is stopped and the medium is evaporated under vacuum. The crude is taken back in 20 mL of DCM and washed with 1M HCl, $NaHCO_3$ and brine. The organic extract is dried on $MgSO_4$ and purified by flash chromatography on silica gel (elution gradient from 2 to 5 % MeOH in DCM, TLC : Rf = 0.5 (DCM:MeOH 90/10 (v/v)) before being dissolved in 20 mL DCM while 1 mL of trifluoroacetic acid is added. The reaction is then maintained for one night under stirring at RT. The reaction medium is evaporated and is coevaporated, four times in DCM and four times in $Et_2O$ before drying. Yield 84% (2 steps, 0.252 mmoles, 299 mg).

**2-[[2-[2,5-bis[4-(dimethylamino)butanoylamino]pentanoylamino]acetyl]amino]ethyl 4-[[4-[[(9Z,12Z)-octadeca-9,12-dienyl]amino]-1-[[(9Z,12Z)-octadeca-9,12-dienyl]carbamoyl]-4-oxo-butyl]amino]-4-oxo-butanoate (XX)**

Molecule **XX** has been isolated from the coupling of compound **20c** (0.25 mmol ; 290 mg) and 2 equivalents of DMABA (0.6 mmol ; 168 mg) using HBTU (0.6 mmol ; 227 mg) and DIPEA (1.2 mmol ; 204 μL) dissolved in 6 mL of DMF and 2 mL of DCM according to the procedure described for the molecule VI. Reaction time: 12h RT; Purification: Silica gel chromatography gradient 98/2 to 90/10 (DCM/MeOH, v/v). Yield: 58% (0.15 mmol ; 117mg), TLC (DCM/MeOH: 95/5) Rf: 0.2).

**<u>21 - Synthesis Molecule XXI</u>**

**<u>21a. Synthesis of molecule 21d</u>**

**tert-butyl N-(4-hydroxybutyl)carbamate (21a)**

4-aminobutan-1-ol (10 mmoles, 922μL) is dissolved in 20 mL of dry absolute ethanol. The mixture is then cooled down to zero degree. Then 2.3 mL of BoczO (10 mmoles) are added dropwise over 30 minutes while stirring at 0°C. The reaction is then pursued for 48h before being concentrated under vacuum. The oily residue is the redissolved in 20 mL of a 1/1 (v/v) mixture of DCM and water. The phase is decanted in a funnel, and the aqueous layer is extracted thrice with 5 mL of DCM. Combined organic extracts are then washed with brine, dried on magnesium sulfate, filtered and concentrated to dryness. The oil is used without additional purification. (97% yield, 1.84g, 9.7 mmoles). TLC (DCM/MeOH: 95/5) rf: 0.7.

**4-(*tert*-butoxycarbonylamino)butyl dodecanoate (21b)**

Compound **21b** is obtained according to the procedure describing molecule **IV** coupling Lauric acid (3.3 mmoles, 661 mg) and compound **21a** (3 mmoles, 567 mg) using 557 μL of DIC (3.6 mmoles), 486 mg of HOBt (3.6 mmoles) and triethylamine (546 μL, 3.9 mmoles) in solution of 56 mL DCM and 4 mL DMF. The crude mixture is concentrated then resuspended in 40 mL of ethyl acetate and put 3 h at -20°C. The resulting liquid and solid are then separated by centrifugation at 3000 rpm, and this procedure is repeated three times. All the liquid are collected and concentrated under vacuum. The product is collected by flash chromatography on silica gel using a petrol spirit/ethyl acetate (80/20 v/v) mixture as eluent. 781 mg (2.1 mmol, 71% yield) of a colourless oil is collected. TLC (PS/EA: 80/20) rf: 0.5.

**4-dodecanoyloxybutylammonium trifluoroacetate (21c)**

Compound **21c** (781 mg, 2.1 mmoles) is dissolved under argon in 40 mL of dry DCM. 2 equivalents (4.2 mmoles, 321 μL) of trifluoroacetic acid (TFA) are then added slowly and the reaction is pursued until complete conversion evidenced by TLC (2h). The mixture is then concentrated, co-evaporated thrice with 20 mL of DCM, thrice with 20 mL of diethyl ether ($Et_2O$), and dried under high vacuum overnight. The product is then used as it without further purification (2.08 mmoles, 768 mg, 99%).

**4-[[4-amino-5-(4-dodecanoyloxybutylamino)-5-oxo-pentanoyl]amino]butyl dodecanoate (21d)**

Compound **21d** is obtained following the 2-steps procedure described for molecule **1b,** by first coupling N-[(9H-Fluoren-9-ylmethoxy)carbonyl]-L-glutamic Acid (Fmoc-Glu-OH, 259 mg, 0.7 mmoles) with compound **21c** (768 mg, 2.08 mmoles) using HOBt (331 mg, 2.45 mmoles), DIC (325 μL, 2.1 mmoles) and $Et_3N$ (394 μL, 2.8 mmoles) dissolved in

chloroform (7 mL) and DMF (7 mL), then by removing the Fmoc group using piperidine (2 mL). The crude mixture is then concentrated then resuspended in 40 mL of ethyl acetate and placed 3 h at -20°C. The resulting liquid and solid are then separated by centrifugation at 3000 rpm, and this procedure is repeated three times. All the liquid are collected and concentrated under vacuum while the solids are discarded. The product is collected by flash chromatography on silica gel using a DCM /methanol mixture as eluent. A gradient has been used ranging from 98/2 to 94/6 (DCM/MeOH, v/v). 163 mg (0.25 mmol, 36% yield) of a white viscous solid are collected. TLC (DCM/MeOH: 95/5) rf: 0.2.

**21b. Synthesis of molecule XXI**

**4-[[4-[[2-(dimethylamino)acetyl]amino]-5-(4-dodecanoyloxybutylamino)-5-oxo-pentanoyl]amino]butyl dodecanoate (XXI)**

Following the procedure described for compound **XV**, molecule **XXI** is obtained by coupling DMG (34 mg, 0.325 mmoles) with compound **21d** (0.25 mmoles, 163 mg) with DIPEA (90 µL, 0.5 mmoles), HOBt (50 mg, 0.375 mmoles) and HBTU (142 mg, 0.375 mmoles) dissolved in 2 mL DMF and 2 mL DCM. Reaction time: 36h 0°C to RT; Purification: Silica gel chromatography gradient 99/1 to 92/8 (DCM/MeOH, v/v). Yield: 48% (89 mg of white solid, 0.12 mmol, TLC (DCM/MeOH: 95/5) Rf: 0.4).

**22 - Synthesis Molecule XXII**

**4-[[4-[2,5-bis(tert-butoxycarbonylamino)pentanoylamino]-5-(4-dodecanoyloxybutylamino)-5-oxo-pentanoyl]amino]butyl dodecanoate (22a)**

Boc-L-Orn(Boc)-OH Boc-L-Orn(Boc)-OH, (365 mg, 1.1 mmoles) is dissolved under Argon in 10 mL of a 9/1 v/v mixture of dry DCM/DMF, in a three-necks round-bottom flask equipped with a dropping funnel. HOBt (162 mg, 1.2 mmoles) and DIC (201 µL, 1.3 mmoles) are subsequently added and the reaction is stirred at RT for 3h. Then compound **21d** (654 mg, 1 mmoles) and $Et_3N$ (210 µL, 1.5 mmoles) dissolved in 10 mL of a 9/1 v/v mixture of dry DCM/DMF are added over 30 min dropwise via the dropping funnel. After the completion of the addition, the reaction is stirred 36h at room temperature and concentrated to dryness. The crude mixture is then resuspended in 20 mL of ethyl acetate and put 3 h at -20°C. The resulting liquid and solid are then separated by centrifugation at 3000 rpm, and this procedure is repeated three times. The solid residues are collected and washed subsequently three times using diethyl ether, before being dried overnight under vacuum, yielding the desired compound pure as white solid with 85% yield (823 mg, 0.85 mmoles). TLC (DCM/MeOH: 95/5) rf: 0.4.

**4-[[5-(4-dodecanoyloxybutylamino)-4-(2-methylhexanoylamino)-5-oxo-pentanoyl]amino]butyl dodecanoate bis-trifluoroacetate (22b)**

Boc protecting groups have been removed following the procedure described for compound **21c** using compound **22a** (823 mg, 0.85 mmoles) and 2 equivalents ( 1.7 mmoles, 130 µ)L of TFA dissolved under argon in 18 mL of DCM for 3h at RT. The product is isolated with 98 % yield (0.833 mmoles, 803 mg)

**4-[[4-[2,5-bis[4-(dimethylamino)butanoylamino]pentanoylamino]-5-(4-dodecanoyloxybutylamino)-5-oxo-pentanoyl]amino]butyl dodecanoate (XXII)**

Molecule **XXII** has been isolated following the procedure described for molecule **VI,** by coupling DMABA (109 mg, 0.65 mmoles) with compound 22b (0.5 mmoles, 482 mg) using DIPEA (305 µL, 1.75 mmoles) and HBTU (663 mg, 1.75 mmoles) in 8 mL DMF. Reaction time: 48h 0°C to RT; Purification: Silica gel chromatography gradient 99/1 to 90/10 (DCM/MeOH, v/v). Yield: 56% (278 mg of white solid, 0.28 mmol, TLC (DCM/MeOH: 95/5) Rf: 0.25).

**23 - Synthesis Molecule XXIII**

**4-[[4-(4-dodecanoyloxybutylamino)-1-(4-dodecanoyloxybutylcarbamoyl)-4-oxobutyl]amino]-4-oxo-butanoic acid (23a)**

Molecule **23a** has been isolated following the procedure described for compound **3a,** using compound **21d** (491 mg, 0.75 mmoles), $Et_3N$ (1.5 mmoles, 210 µL) and succinic anhydride (1.5 mmoles, 150 mg) dissolved under argon in 4 mL of a 1/1 (v/v) mixture of DCM and DMF. Reaction time: 12h RT. Yield 82% (463 mg, 0.62 mmoles TLC (DCM/MeOH: 95/5) rf: 0.6.

**4-[[4-[[4-(4-azidobutylamino)-4-oxo-butanoyl]amino]-5-(4-dodecanoyloxybutylamino)-5-oxo-pentanoyl]amino] butyl dodecanoate (23b)**

Compound **23a,** (231 mg, 0.31 mmoles) is dissolved under Argon in 3 mL of a 9/1 v/v mixture of dry DCM/DMF, in a three-necks round-bottom flask equipped with a dropping funnel. HOBt (55 mg, 0.4 mmoles) and DIC (62 μL, 0.4 mmoles) are subsequently added and the reaction is stirred at RT for 2h30, increasing the turbidity of the medium. Then 4-Azidobutylamine (1M in DCM, 470 μL, 0.47 mmoles) and $Et_3N$ (87 μL, 0.62 mmoles) dissolved in 3 mL of a 9/1 v/v mixture of dry DCM/DMF are added over 30 min dropwise via the dropping funnel. After the completion of the addition, the reaction is stirred 33h at room temperature and concentrated to dryness. The crude mixture is then resuspended in 20 mL of ethyl acetate and put 3 h at - 20°C. The resulting liquid and solid are then separated by centrifugation at 3000 rpm, and this procedure is repeated three times. All the liquid are collected and concentrated under vacuum while the solids are discarded. The remaining oil is then dried overnight under vacuum, yielding the desired compound (182 mg, 0.21 mmoles). TLC (DCM/MeOH: 98/2) rf: 0.5.

**4-[[4-[[4-[4-[4-[(dimethylamino)methyl]triazol-1-yl]butylamino]-4-oxo-butanoyl]amino]-5-(4-dodecanoyloxybutylamino)-5-oxo-pentanoyl]amino]butyl dodecanoate (XXIII)**

Copper sulphate heptahydrate (0.012 mmoles, 3 mg), ascorbic acid (Sigma, 0.06 mmoles, 12 mg) and triphenylphosphine (0.012 mmoles, 3mg) are mixed together under stirring in 1 mL of dry DMSO. While this solution is stirring (15 minutes), compound **23b** (87 mg 0.1 mmoles) and N,N-Dimethylpropargylamine (108 μL, 1 mmoles) are dissolved in 4 mL of a THF. After complete dissolution of the starting materials, the copper-based yellowish solution is added to the reaction flask and stirred with the starting material for 12h at room temperature. The reaction is then diluted in 10 mL of water and extracted with 4X20 mL of DCM. The organic extracts are then combined, dried on magnesium sulphate, filtered and concentrated under vacuum. The product is collected by flash chromatography on silica gel using a DCM /methanol mixture as eluent. A gradient has been used ranging from 99/1 to 90/10 (DCM/MeOH, v/v). 135 mg (0.145 mmol, 49% yield) of a white solid are collected. TLC (DCM/MeOH: 95/5) rf: 0.3.

**24 - Synthesis Molecule XXIV**

**N,N'-bis[(Z)-octadec-9-enyl]-2-oxo-pentanediamide (24a)**

α-ketoglutaric acid (146 mg, 1 mmole) is dissolved in a 4/1 (v/v) mixture of DCM/DMF under argon. DIPEA (558 μL, 3.2 mmoles) is then added and the reaction is stirred for 5 minutes. PyBOP (1.56 g, 3 mmoles) is introduced and the mixture is stirred for another 5 minutes, turning orange in colour. Oleylamine (1.16 mL 3 mmoles) is finally added dropwise over 15 minutes, and the reaction is maintained at room temperature for 48h, before being concentrated. The oily residue is taken back in 30 mL of EtOAc, placed 3 h at -20°C, and the resulting solid is eliminated by centrifugation at 3000 rpm. The remaining liquid is concentrated, and the product is obtained by purification via by flash chromatography on silica gel using a DCM /methanol mixture as eluent. A gradient has been used ranging from 99/1 to 95/5 (DCM/MeOH, v/v). 310 mg (0.48 mmol, 48% yield) of a light-yellow solid is collected. TLC (DCM/MeOH: 95/5) rf: 0.45.

**4-[(dimethylamino)methyl]-N-[(Z)-octadec-9-enyl]-2-[3-[[(Z)-octadec-9-enyl]amino]-3-oxo-propyl]-1,3-dioxolane-2-carboxamide (XXIV)**

Compound **24a** (161 mg, 0.25 mmoles), 3-(dimethylamino)propane-1,2-diol (59 μL, 0.5 mmoles) and pyridinium-para-toluenesulfonate (PPTS, 11 mg, 0.05 mmoles) are dissolved under stirring in dry toluene (20 mL). The round-bottom flask is equipped with a Dean-Stark trap and a condenser. The reaction is then stirred under reflux for 18h before being cooled-down and concentrated. The residue is then taken back in DCM, washed twice with a saturated sodium bicarbonate solution, twice with water, and once with brine. Organic extracts are the dried on magnesium sulfate, filtered and concentrated under vacuum. The product is collected by flash chromatography on silica gel using a gradient of a DCM /methanol (from 100/0 v/v to 95/5 v/v) mixture as eluent. 63 mg (0.085 mmol, 34% yield) of a colourless solid is collected. TLC (DCM/MeOH: 97/3) rf: 0.3.

**25 - Synthesis Molecule XXV**

**2-hydroxy-N,N'-bis[(Z)-octadec-9-enyl]pentanediamide (25a)**

Compound **24a** (0.25 mmoles, 161 mg) is dissolved in 2.5 mL of absolute ethanol. The reaction is then cooled down to 0°C and sodium borohydride (19 mg, 0.5 mmoles) is introduced in 2 equal portions. The reaction then takes place

during 3 h at 0°C, before being quenched at 0°C with 5 mL of saturated aqueous ammonium chloride. The aqueous phase is then extracted with ethyl acetate (3*10mL). Combined organic extracts are washed with water a brine, dried on magnesium sulfate, filtered and concentrated. Pure product (149 mg, 0.23 mmoles) is collected as a colourless oil and used as it in the next step (91% yield).

**[4-[[(Z)-octadec-9-enyl]amino]-1-[[(Z)-octadec-9-enyl]carbamoyl]-4-oxo-butyl]4-(dimethylamino)butanoate (XXV)**

Esterification leading to molecule **XXV** has been accomplished adapting the procedure described for compound **II,** linking DMABA (40 mg, 0.24 mmoles) with compound **25a** (129 mg, 0.2 mmoles) using methylimidazole (65 μL, 0.8 mmoles) and TCFH (113 mg, 0.4 mmoles dissolved in 3 5 mL of dry 2/1 (v/v) mixture solution of MeCN and DCM). Reaction time: 18h 0°C to RT; Purification: Silica gel chromatography gradient 98/2 to 90/10 (DCM/MeOH, v/v). Yield: 61% (93 mg of white solid, 0.122 mmol, TLC (DCM/MeOH: 90/10) Rf: 0.4).

**26 - Synthesis Molecule XXVI**

**5-hydroxy-N-[(Z)-octadec-9-enyl]-2-[3-[[(Z)-octadec-9-enyl]amino]-3-oxo-propyl]-1,3-dioxane-2-carboxamide (26a)**

Ketalization of molecule **24a** (161 mg, 0.25 mmoles) using 2-{[Dimethyl(2-methyl-2-propanyl)silyl]oxy}-1,3-propanediol (103 mg, 0.5 mmoles) and PPTS(11 mg, 0.05 mmoles) dissolved toluene (20 mL) has been accomplished following the procedure described for compound **XXIV** during 18h.

Hydroxyl functions are then released following the procedure describing **19c**, using TBAF (1M in THF, 1 mmoles, 1 mL) in 5 mL of dry THF. After concentration of the medium, the targeted product is recovered by flash chromatography on silica gel using a DCM /methanol mixture as eluent. A gradient has been used ranging from 98/2 to 90/10 (DCM/MeOH, v/v). 49 mg (0.068 mmoles, 27% yield, two steps) of a white solid are collected. TLC (DCM/MeOH: 90/10) rf: 0.5.

**4-imidazol-1-ylbutanoic acid (26b)**

4-bromobutyric acid (10 mmoles, 1.67g) is dissolved in dry MeCN (100 mL). Imidazole (10.5 mmoles, 714 mg) is then introduced and the reaction is stirred overnight at 60°C before being concentrated. The resulting solid is then precipitated in diethyl ether, filtered and washed thrice with cold diethyl ether. Product (9.6 mmoles, 1.48g, 96%) is used as it without further purification.

**[2-[3-[[(Z)-octadec-9-enyl]amino]-3-oxo-propyl]-2-[[(Z)-octadec-9-enyl]carbamoyl]-1,3-dioxan-5-yl] 4-imidazol-1-ylbutanoate (XXVI)**

Compound **XXVI** has been isolated adapting the procedure described for compound **II,** linking compound **26b** (15,4 mg, 0.1 mmoles) with compound **26a** (72 mg, 0.1 mmoles) using methylimidazole (33 μL, 0.4 mmoles) and TCFH (113 mg, 0.4 mmoles) dissolved in 2 mL of a 2/1 (v/v) mixture solution of MeCN and DCM. Reaction time: 18h 0°C to RT; Purification: Silica gel chromatography gradient 98/2 to 90/10 (DCM/MeOH, v/v). Yield: 52% (44 mg of white solid, 0.122 mmol, TLC (DCM/MeOH: 90/10) Rf: 0.3).

**27 - Synthesis Molecule XXVII**

**27a. Synthesis molecule 27b**

**[(Z)-non-2-enyl] 4-aminobutanoate trifluoroacetate (27a)**

Boc protecting groups have first been introduced according to the procedure described for compound **8a,** using 4-aminobutyric acid (1.03g, 10 mmoles), NaOH (440 mg, 11 mmoles), and $Boc_2O$ (10.5 mmoles 2.41 mL) in a 1/1 mixture of water and *tert*-BuOH. Reaction time is 12h.

Following the procedure described for compound **18b,** the vacuum-dried crystallized compound (2.03g), is then coupled with (Z)-2-nonen-1-ol (1.77 mL, 10.5 mmoles), using DIC (1.7 mL, 11 mmoles), HOBt (1.49 g, 11 mmoles), and $Et_3N$ (2.1 mL, 15 mmoles) dissolved in 100 mL of a 9/1 v/v mixture of dry DCM/DMF during 36h. Accordingly after initial workup, the amine functions are then released using 1.3 mL of TFA in 60 mL of DCM for 6h. The product is then used pure as it after workup without further purification (8.8 mmoles, 4.97g, 88% 3 steps).

**[(Z)-non-2-enyl]4-[[5-[[4-[(Z)-non-2-enoxy]-4-oxo-butyl]amino]-4,5-dioxo-pentanoyl]amino]butanoate (27b)**

Molecule **27b** has been isolated following the procedure describing compound **24a**, in which α-ketoglutaric acid (146 mg, 1 mmole) and Compound **27a** (813mg, 2.5 mmoles) are linked using DIPEA (558 μL, 3.2 mmoles) and PyBOP (1.56 g, 3 mmoles) dissolved in 20 mL of a 4/1 (v/v) mixture of DCM/DMF under argon. Reaction time: 48h RT; Purification: Silica gel chromatography gradient 98/2 to 92/8 (DCM/MeOH, v/v). Yield: 42% (237 mg of light-yellow solid, 0.42 mmol, TLC (DCM/MeOH: 95/5) Rf: 0.25).

**27b. Synthesis Molecule XXVII**

**[(Z)-non-2-enyl]4-[3-[5-hydroxy-2-[[4-[(Z)-non-2-enoxy]-4-oxo-butyl]carbamoyl]-1,3-dioxan-2-yl]propanoylamino]butanoate (27c)**

Ketalization of molecule **27b** (565 mg, 1 mmoles) using 2-{[Dimethyl(2-methyl-2-propanyl)silyl]oxy}-1,3-propanediol (412 mg, 2 mmoles) and PPTS(44 mg, 0.2 mmoles) dissolved toluene (100 mL) has been accomplished following the procedure described for compound **XXIV** during 18h.

Hydroxyl functions are then released following the procedure describing **19c**, using TBAF (1M in THF, 4 mmoles, 4 mL) in 20 mL of dry THF, 2h at 0°C. After concentration of the medium, the targeted product is recovered by flash chromatography on silica gel using a DCM /methanol mixture as eluent. A gradient has been used ranging from 98/2 to 90/10 (DCM/MeOH, v/v). 83 mg (0.13 mmoles, 13%, two steps) of a white solid are collected. TLC (DCM/MeOH: 95/5) rf: 0.35.

**[(Z)-non-2-enyl]4-[3-[5-[4-(dimethylamino)butanoyloxy]-2-[[4-[(Z)-non-2-enoxy]-4-oxobutyl]carbamoyl]-1,3-dioxan-2-yl]propanoylamino]butanoate (XXVII)**

Compound **XXVII** has been isolated adapting the procedure described for compound **II,** by linking compound DMABA (40 mg, 0.24 mmoles) with compound **27c** (83 mg, 0.13 mmoles) using methylimidazole ((65 μL, 0.8 mmoles) and TCFH (113 mg, 0.4 mmoles) dissolved in 5 mL of a 2/1 (v/v) mixture solution of MeCN and DCM. Reaction time: 18h 0°C to RT; Purification: Silica gel chromatography gradient 98/2 to 90/10 (DCM/MeOH, v/v). Yield: 64% (63 mg of white solid, 0.083 mmol, TLC (DCM/MeOH: 90/10) Rf: 0.25).

**28 - Synthesis Molecule XXVIII**

**3-[tert-butyl(dimethyl)silyl]oxy-N-[3-[tert-butyl(dimethyl)silyl]oxypropyl]propan-1-amine (28a)**

3-(3-Hydroxy-propylamino)-propan-1-ol (10 mmoles, 1.33g) is dissolved in dry DMF (100 mL). The solution is cooled down to 0°C before Imidazole (21 mmoles, 1.43 g) and *tert*Butyldimethylsilyl Chloride (25 mmoles, 3.75g) are then introduced over 1 h in 4 equal portions. The reaction is stirred overnight at room temperature before being concentrated. The obtained residue is then redissolved in 100 mL of DCM and washed twice saturated sodium carbonate solution, twice with water and once with brine, dried on sodium sulphate, filtered and concentrated. The product is collected by flash chromatography on silica gel using a DCM /$E_{t2}O$ (50/50 v/v) mixture as eluent). 3.4g (9.4 mmoles, 94% yield) of an oily solid are collected. TLC (DCM /$Et_2O$ (50/50 v/v)) rf: 0.5.

**[(Z)-non-2-enyl]4-[[4-(2,5-dioxopyrrolidin-1-yl)oxycarbonyloxy-5-[[4-[(Z)-non-2-enoxy]-4-oxo-butyl]amino]-5-oxo-pentanoyl]amino]butanoate (28b)**

Compound **27a** (565 mg, 1 mmoles) is first reduced as described for molecule **25a** by using sodium borohydride (76 mg, 2 mmoles) reacted in 10 mL of absolute ethanol at 0°C for 3h. The crude compound is then dissolved in 10 mL of dry DCM. Triethylamine (1.3 mmoles, 182 μL) is added, followed 5 minutes after by N,N-disuccinimidyl carbonate (307 mg, 1.2 mmoles). The solution is stirred at room temperature for 12h before washing the organics with saturated ammonium chloride solution, water and brine. The organic phase is then dried on magnesium sulfate, filtered and concentrated. The product is collected by flash chromatography on silica gel using a 95/5 v/v DCM /methanol mixture as eluent. 580 mg (0.82 mmoles, 82% yield 2 steps) of a white solid are collected. TLC (DCM/MeOH: 95/5) rf: 0.4.

**[(Z)-non-2-enyl]4-[[4-[bis(3-hydroxypropyl)carbamoyloxy]-5-[[4-[(Z)-non-2-enoxy]-4-oxo-butyl]amino]-5-oxo-pentanoyl]amino]butanoate (XXVIII)**

Compound **28a** (181 mg, 0.5 mmoles) is dissolved under argon in 5 mL of a 1/1 (v/v) mixture of dry DCM and DMF.

2 equivalents (1 mmoles, 140 μL) of t $Et_3N$ are then added slowly and the reaction is stirred for 5 minutes. Then, compound **28b** (0.5 mmoles, 353 mg) is added and the mixture is stirred for 24h at room temperature. The mixture is then concentrated, and redissolved in 80 mL of ethyl acetate. The organic phase is then washed with a saturated aqueous ammonium chloride solution, twice with MilliQ water, once with brine, dried on $MgSO_4$, filtered and concentrated. The obtained crude product is then redissolved in 25 mL of dry THF. TBAF (1M in THF, 4 mmoles, 4 mL) is then introduced and the reaction is stirred for 4 h at 0°C. After concentration of the medium, the targeted product is recovered by flash chromatography on silica gel using a DCM /methanol mixture as eluent. A gradient has been used ranging from 98/2 to 94/6 (DCM/MeOH, v/v). 400 mg (0.55 mmoles, 55% yield, two steps) of a colourless oil is collected. TLC (DCM/MeOH: 95/5) rf: 0.4.

**<u>29 - Synthesis Molecule XIX</u>**

**[5-[2-(dimethylamino)ethylamino]-5-oxo-4-[(2,2,2-trifluoroacetyl)ammonio]pentyl]-(2,2,2-trifluoroacetyl)ammonium (29a)**

Compound **29a** has been isolated in two steps by first adapting the procedure described for compound **II,** by linking compound Boc-L-Orn(Boc)-OH (365 mg, 1.1 mmoles) with N,N-dimethylethylenediamine (66 μL, 0.6 mmoles) using methylimidazole (299 μL, 3.7 mmoles) and TCFH (520 mg, 1.85 mmoles) dissolved in 28 mL of a 2/1 (v/v) mixture solution of MeCN and DCM. Reaction time: 18h 0°C to RT; Purification: Silica gel chromatography gradient 98/2 to 95/5 (DCM/MeOH, v/v). Yield: 77% (185 mg 0.46 mmoles, MW=402.5 g/mol, 77%). Hydroxyl functions are then released adapting the procedure described for compound **21c** using 76 μL (1 mmoles) of TFA in 10 mL of DCM until completion (2h). Usual workup provides compound as colourless solid (0.245 mmoles, 97 mg, 98%).

**N-[5-[2-(dimethylamino)ethylamino]-4-(heptadeca-4,6-diynoylamino)-5-oxo-pentyl]heptadeca-4,6-diynamide (XXIX)**

Molecule **XXIX** has been isolated following the procedure described for molecule **XV,** by coupling heptadeca-4,6-diynoic acid (193 mg, 0.74 mmoles) with compound **29b** (0.245 mmoles, 97 mg) using DIPEA (175 μL, 1 mmoles) and HBTU (341 mg, 0.9 mmoles) in 4 mL DMF and 4 mL DCM. Reaction time: 36h 0°C to RT; Purification: Silica gel chromatography gradient 95/5 to 90/10 (DCM/MeOH, v/v). Yield: 49% (83 mg of white solid, 0.120 mmol, TLC (DCM/MeOH: 95/5) Rf: 0.15).

**<u>30 - Synthesis Molecule XXX</u>**

**4-(9H-fluoren-9-ylmethoxycarbonylamino)-5-[[4-[(Z)-non-2-enoxy]-4-oxo-butyl]amino]-5-oxo-pentanoic acid (30a)**

Fmoc-Glu(OtBu)-OH (2.5 mmol ; 1.06 g) is placed in a 100 mL flask under an argon then dissolved in 40 mL of anhydrous DMF under stirring. COMU (2.5 mmol ; 1.07 g) and DIPEA (5 mmol ; 865 μL) are successively added to the reaction medium. The reaction is then maintained under an argon and 0°C for 15 minutes before **30a** (4.9 mmol ; 1.67 g), dissolved in 10 mL of anhydrous DMF, is added to the reaction medium under stirring. The solution is allowed to warm to RT overnight. before being stopped evaporated to dryness under vacuum. The crude is taken back in 50 mL of EtOAc and washed with 1M HCl, $NaHCO_3$ and brine. The organic layer is dried on $MgSO_4$ and purified by flash chromatography on silica gel (elution gradient from 30 to 60 % EtOAc in petroleum ether). In this way, the intermediate is isolated, in a yield of 74 % (1.85 mmol ; 1.2 g). TLC : Rf =0.3 (EtOAc:Petroleum ether 40/60 (v/v))and then dissolved in 90 mL of DCM containing 10 mL of trifluoroacetic acid. The reaction is then stirred for one night at RT. The reaction medium is evaporated to dryness. The mixture is then concentrated, co-evaporated thrice with 50 mL of DCM, thrice with 50 mL of diethyl ether (Et2O), and dried under high vacuum overnight (74% 2 steps, 1.85 mmoles 1.07g)

**[(Z)-non-2-enyl] 4-[[2-amino-5-oxo-5-(tetradecylamino)pentanoyl]amino]butanoate (30b)**

Molecule **30b** has been isolated in two consecutive steps following the procedure described for molecule **1b,** by coupling tetradecylamine (1.5 mmol ; 320 mg) with compound **30a** (1 mmol ; 579 mg) using DIC (2 mmol ; 313 μL), HOBt (2 mmol ; 270 mg) and $Et_3N$ (2 mmol ; 272 μL) in 18 mL DMF and 30 mL $CHCl_3$. Piperidine (10mL) is added after 30h and stirred 6h more. Reaction time: 36h RT; Yield: 61% (0.61 mmol ; 337 mg) TLC : Rf=0.4 (DCM:MeOH 90/10 (v/v)).

**[(Z)-non-2-enyl]4-[[2-[[2-(dimethylamino)acetyl]amino]-5-oxo-5-(tetradecylamino)pentanoyl]amino]butanoate (XXX)**

Compound **XXX** has been isolated adapting the procedure described for compound **II,** by linking compound DMG (0.23 mmol; 24 mg) with compound **30b** (0.3 mmol ; 170 mg) using methylimidazole (0.81 mmol ; 65 μL) and TCFH (0.28 mmol ; 79 mg) dissolved in 5 mL of a 2/1 (v/v) mixture solution of MeCN and DCM. Reaction time: 12h 0°C to RT; Purification: Silica gel chromatography gradient 98/2 to 95/5 (DCM/MeOH, v/v). Yield: 83% (121mg of white solid, 0.19 mmol, TLC (DCM/MeOH: 90/10) Rf: 0.4).

**31** - **Synthesis Molecule XXXI**

**4-[[1-[[4-[(Z)-non-2-enoxy]-4-oxo-butyl]carbamoyl]-4-oxo-4-(tetradecylamino)butyl]amino]-4-oxo-butanoic acid (31a)**

Molecule **31a** has been isolated following the procedure described for compound **3a**, using compound **30b** (83 mg, 0.15 mmoles), $Et_3N$ (0.4 mmoles, 56 μL) and succinic anhydride (0.3 mmoles, 30 mg) dissolved under argon in 3mL of DCM. Reaction time: 16h RT. Purification: Silica gel chromatography gradient 100/0 to 95/5 (DCM/MeOH, v/v). Yield: 91% (89 mg of white solid, 0.14 mmol, TLC (DCM/MeOH: 90/10) Rf: 0.4).

**[(Z)-non-2-enyl]-4-[[2-[[4-[3-[bis(2-hydroxyethyl)amino]propylamino]-4-oxo-butanoyl]amino]-5-oxo-5-(tetradecylamino)pentanoyl]amino]butanoate (XXXI)**

Compound **XXXI** has been isolated adapting the procedure described for compound **II,** by linking compound **31a** 0.14 mmol ; 89 mg) with N-(3-Aminopropyl)diethanolamine (0.12 mmol ; 18 μL) using methylimidazole (0.4 mmol ; 32 μL) and TCFH (0.14 mmol ; 40 mg) dissolved in 4 mL of a 2/1 (v/v) mixture solution of MeCN and DCM. Reaction time: 12h 0°C to RT; Purification: Silica gel chromatography gradient 98/2 to 90/10 (DCM/MeOH, v/v). Yield: 64% (61 mg of white solid, 0.08 mmol, TLC (DCM/MeOH: 95/5) Rf: 0.3).

**32** - **Synthesis Molecule XXXII**

**2-[2-[[4-[[1-[[4-[(Z)-non-2-enoxy]-4-oxo-butyl]carbamoyl]-4-oxo-4-(tetradecylamino)butyl]amino]-4-oxo-butanoyl]amino]ethyldisulfanyl]ethylammonium (32a)**

Boc-cystamine (0.28 mmol ; 91 mg) is placed in a 25 mL flask under argon then dissolved in 2 mL of anhydrous DMF under stirring. HBTU (0.28 mmol ; 106 mg) and DIPEA (0.56 mmol ; 97 μL) are added to the solution successively. The reaction medium is stirred under argon at RT for 15 minutes before compound **30b** (0.14 mmol ; 89 mg), in solution in 1 mL of anhydrous DMF and 1 mL of anhydrous DCM, is added to the reaction medium. After 12 hours, the reaction is stopped and the reaction medium is evaporated to dryness under a high vacuum. The crude is taken back in 10 mL of EtOAc and washed with 1M HCl, $NaHCO_3$ and brine. The organic layer is dried on $MgSO_4$ and purified by flash chromatography on silica gel (elution gradient from 2 to 5 % MeOH in DCM). The isolated intermediate (0.1 mmol ; 79 mg) is dissolved in 5 mL of DCM, then 250 μL of trifluoroacetic acid is added to the reaction medium. The reaction is then maintained for 4 hours under stirring at RT. The mixture is then concentrated, co-evaporated thrice with 50 mL of DCM, thrice with 50 mL of diethyl ether ($Et_2O$), and dried under high vacuum overnight Yield: 67% (2 steps, 84 mg, 0.094 mmol).

**[4-azaniumyl-5-[2-[2-[[4-[[1-[[4-[(Z)-non-2-enoxy]-4-oxo-butyl]carbamoyl]-4-oxo-4-(tetradecylamino)butyl]amino]-4-oxo-butanoyl]amino]ethyldisulfanyl]ethylamino]-5-oxo-pentyl]ammonium (32b)**

Compound **32b** is obtained following the two steps procedure experienced for the synthesis of compound **32a.** First Boc-L-Orn(Boc)-OH (0.2 mmol ; 66 mg) and compound **32a** (0.1 mmol ; 90 mg) are linked together using HBTU (0.2 mmol ; 76 mg) and DIPEA (0.4 mmol ; 69 μL) dissolved in 3 mL of DMF for 16h at RT. After isolation and purification of the intermediate, amine functions are released by reacting TFA (250μL) in 5 mL DCM during 1h. Yield: 61% (2 steps, 69 mg, 0.061 mmol).

**[(Z)-non-2-enyl]4-[[2-[[4-[2-[2-[2,5-bis[[2-(dimethylamino)acetyl]amino]pentanoylamino]ethyldisulfanyl]ethylamino]-4-oxo-butanoyl]amino]-5-oxo-5-(tetradecylamino)pentanoyl]amino]butanoate (XXXII)**

Following the procedure described for compound **I**, molecule **XXXII** is obtained by coupling DMG (0.18 mmol ; 19 mg) and compound **32b** (0.06 mmol ; 68 mg) using DIPEA (0.4 mmol ; 69 μL) and pyBOP (0.2 mmol ; 104 mg) dissolved in 3

mL of DMF. Reaction time: 48h RT; Purification: Silica gel chromatography gradient 98/2 to 90/10 (DCM/MeOH, v/v). Yield: 32% (0.02 mmol ; 21 mg), TLC (DCM/MeOH: 95/5) Rf: 0.2.).

## 33 and 34 - Synthesis Molecule XXXIII

### 33a. Synthesis of molecule 33a

**[4-(3-butylhept-2-enoxy)-4-oxo-butyl]ammonium (33a)**

3-Butyl-2-hepten-1-ol (5.5 mmol ; 1.11 mL) is added under stirring to a solution of N-Boc-gamma-aminobutyric acid (5 mmol, 1.02 g), DCC (5.5 mmol; 1.13 g) and DMAP (1 mmol ; 122 mg) dissolved in 50 mL of anhydrous DCM. The reaction is then maintained under argon at RT for 16 hours. After total consumption of the acid the reaction medium is evaporated to dryness under vacuum. The crude is taken back in 50 mL of DCM and washed with 1M HCl, NaHCOs and brine. The organic layer is dried on $MgSO_4$ et then purified by flash chromatography on silica gel (elution gradient from 0 to 50 % EtOAc in petroleum ether Leading to an intermediate (3.95 mmol ; 1.4 g) that).

is dissolved in 100 mL of DCM in presence of 5 mL of TFA. The reaction is then maintained for one night under stirring at ambient temperature. The mixture is then concentrated, co-evaporated thrice with 50 mL of DCM, thrice with 50 mL of diethyl ether ($Et_2O$), and dried under high vacuum overnight Yield: 79% (2 steps, 1.46g, 3.95 mmol).

### 33b. 34. Synthesis of molecules XXXIII & XXXIV

**5-[[4-(3-butylhept-2-enoxy)-4-oxo-butyl]amino]-4-(9H-fluoren-9-ylmethoxycarbonylamino)-5-oxo-pentanoic acid (33b)**

Molecule **33b** is obtained by adapting a two steps method described for the synthesis of molecule **30a,** in which Fmoc-Glu(OtBu)-OH (2.5 mmol ; 1.06 g) and compound **33a** 4.9 mmol ; 1.67 g are coupled in presence of COMU (2.5 mmol ; 1.07 g) and DIPEA (5 mmol ; 865 μL) dissolved in 40 mL of DMF under stirring. Reaction time: 18h 0°C to RT; Purification: Silica gel chromatography gradient 70/30 to 40/60 (EP/EA, v/v). The ester function is then successfully cleaved using TFA (20 mL) in 20 mL DCM while stirring 24h at RT. The mixture is then concentrated, co-evaporated thrice with 50 mL of DCM, thrice with 50 mL of diethyl ether ($Et_2O$), and dried under high vacuum overnight Yield: 74% (2 steps, 1.12g, 1.85 mmol).

**3-butylhept-2-enyl-4-[[2-amino-5-oxo-5-(tetradecylamino)pentanoyl]amino]butanoate (33c)**

Following the procedure described for compound **1b,** molecule **33c** is obtained in two steps by coupling Tetradecylamine (2.8 mmol; 592 mg) and compound **33b** (0.06 mmol; 68 mg) using DIC (3.7 mmol ; 579 μL) and HOBt (3.7 mmol ; 500 mg) dissolved in 30 mL of DMF and 30 mL of $CHCl_3$ containing $Et_3N$ (3 mmol ; 408 μL). After 36h stirring at RT, Fmoc deprotection is carried out by introducing 10 mL of piperidine and stirring 4h at RT. Reaction time: 36h+4h RT; Yield: 77 % (1.42 mmol ; 826 mg); TLC : Rf=0.4 (DCM:MeOH 90/10 (v/v)).).

**4-[[1-[[4-(3-butylhept-2-enoxy)-4-oxo-butyl]carbamoyl]-4-oxo-4-(tetradecylamino)butyl]amino]-4-oxo-butanoic acid (33d)**

Molecule **33d** has been isolated following the procedure described for compound **3a**, using compound **33c** (1.42 mmol ; 826 mg), $Et_3N$ (2.1 mmol ; 295 μL) and succinic anhydride (2.1 mmol ; 210 mg) dissolved under argon in 20mL of DCM and 10 mL of DMF. Reaction time: 16h RT. Purification: Silica gel chromatography gradient 98/2 to 92/8 (DCM/MeOH, v/v). Yield: 88% (850 mg of white solid, 1.25 mmol, TLC (DCM/MeOH: 95/5) Rf: 0.4).

**3-butylhept-2-enyl-4-[[2-[[4-[3-[bis(2-hydroxyethyl)amino]propylamino]-4-oxo-butanoyl]amino]-5-oxo-5-(tetradecylamino)pentanoyl]amino]butanoate (XXXIII)**

Compound **XXXIII** has been isolated adapting the procedure described for compound **II,** by linking compound **33d** (0.5 mmol ; 340 mg) with N-(3-Aminopropyl)diethanolamine (0.6 mmol ; 91 μL) using methylimidazole (1.5 mmol ; 120 μL) and TCFH (0.6 mmol ; 170 mg) dissolved in 15 mL of a 2/1 (v/v) mixture solution of MeCN and DCM. Reaction time: 12h 0°C to RT; Purification: Silica gel chromatography gradient 98/2 to 94/6 (DCM/MeOH, v/v). Yield: 61% (256 mg of white solid, 0.31 mmol, TLC (DCM/MeOH: 90/10) Rf: 0.3).

**4-pyrrolidin-1-ylbutyl-4-[[1-[[4-(3-butylhept-2-enoxy)-4-oxo-butyl]carbamoyl]-4-oxo-4-(tetradecylamino)butyl] amino]-4-oxo-butanoate (XXXIV)**

Compound **XXXIV** has been isolated adapting the procedure described for compound **20c,** by coupling compound **33d** (0.5 mmol ; 340 mg) with 4-(1-pyrrolidinyl)-1-butanol (0.7 mmol ; 100 mg) using DCC (0.7 mmol ; 145 mg) and DMAP (0.1 mmol ; 12 mg) dissolved in 20 mL of a DCM. Reaction time: 15h RT; Purification: Silica gel chromatography gradient 98/2 to 90/10 (DCM/MeOH, v/v). Yield: 58% (234 mg of white solid, 0.29 mmol, TLC (DCM/MeOH: 95/5) Rf: 0.3).

## 35 - Synthesis Molecule XXXV

**2-amino-N,N'-didodecyl-butanediamide (35a)**

Following the procedure described for compound **1b**, molecule **35a** is obtained in two steps by coupling Fmoc-aspartic acid (3 mmol ; 1.07 g) and Dodecylamine (9 mmol ; 2.07 mL) using DIC (10.5 mmol ; 1.66 mL) and HOBt (10.5 mmol ; 1.42 g) dissolved in 40 mL of DMF and 40 mL of $CHCl_3$. After 24h stirring at RT, Fmoc deprotection is carried out by introducing 9mL of piperidine and stirring 4h at RT. Reaction time: 24h+4h RT; Yield: 82 % (2.46 mmol ; 1.12 g); TLC : Rf=0.4 (DCM:MeOH 95/5 (v/v)).).

**[(Z)-non-2-enyl]-4-[[2-amino-5-[[3-(dodecylamino)-1-(dodecylcarbamoyl)-3-oxopropyl]amino]-5-oxo-pentanoyl] amino]butanoate (35b)**

**30a** (1.89 mmol ; 1.09 g) is placed in a dry 100 mL flask under argon then dissolved in 20 mL of anhydrous DMF under stirring. DIPEA (5.7 mmol ; 987 µL) and pyBOP (2.46 mmol ; 1.28 g) are added to the solution successively. The reaction medium is stirred under argon at RT for 15 minutes before 35a (2.46 mmol ; 1.12 g), in solution in 10 mL of anhydrous DMF and 5 mL of anhydrous DCM, is added to the reaction medium. After stirring at RT for 36 hours, 7 mL of piperidine are added and the reaction medium is stirred for another 4 hours. The reaction is stopped and the reaction medium is evaporated to dryness. The coupling product is then purified by flash chromatography on silica gel (elution gradient from 0 to 5 % MeOH in DCM). In this way, the product is isolated in a yield of 42 % (0.79 mmol ; 636 mg). TLC : Rf = 0.5 (DCM:MeOH 90/10 (v/v)).

**[(Z)-non-2-enyl]-4-[[2-[4-(dimethylamino)butanoylamino]-5-[[3-(dodecylamino)-1-(dodecylcarbamoyl)-3-oxo-propyl]amino]-5-oxo-pentanoyl]amino]butanoate (XXXV)**

Compound **XXXV** has been isolated adapting the procedure described for compound **II,** by linking compound **35b** (0.26 mmol ; 210 mg) with DMABA (0.2 mmol ; 34 mg) using methylimidazole (0.7 mmol ; 120 µL) and TCFH (0.6 mmol ; 170 mg) dissolved in 5 mL of a 2/1 (v/v) mixture solution of MeCN and DCM. Reaction time: 12h 0°C to RT; Purification: Silica gel chromatography gradient 98/2 to 90/10 (DCM/MeOH, v/v). Yield: 61% (147 mg of white solid, 0.16 mmol, TLC (DCM/MeOH: 90/10) Rf: 0.3).

## 36 - Synthesis Molecule XXXVI

**2-[2-[[4-[[4-[[3-(dodecylamino)-1-(dodecylcarbamoyl)-3-oxo-propyl]amino]-1-[[4-[(Z)-non-2-enoxy]-4-oxo-butyl] carbamoyl]-4-oxo-butyl]amino]-4-oxo-butanoyl]amino]ethyldisulfanyl]ethylammonium (36a)**

Compound **36a** is obtained following the two steps procedure experienced for the synthesis of compound **6b**. First, compound **6a** (0.9 mmol ; 292 mg) and compound **35b** (0.6 mmol; 484 mg) are linked together using DIC (0.9 mmol; 141 µL) and HOBt (0.9 mmol; 122 mg) dissolved in 12mL of DMF and 4 mL of DCM containing $Et_3N$ (0.8 mmol; 112 µL); the reaction is then stirred for 48h at RT. After isolation and purification of the intermediate (flash chromatography on silica gel, elution gradient from 0 to 5% MeOH in DCM), amine functions are released by reacting TFA (550µL) in 11 mL DCM during 12h. Yield: 38% (2 steps, 265 mg, 0.23 mmol).

**[4-azaniumyl-5-[2-[2-[[4-[[4-[[3-(dodecylamino)-1-(dodecylcarbamoyl)-3-oxo-propyl]amino]-1-[[4-[(Z)-non-2-enoxy]-4-oxo-butyl]carbamoyl]-4-oxo-butyl]amino]-4-oxo-butanoyl]amino]ethyldisulfanyl]ethylamino]-5-oxo-pentyl]ammonium (36b)**

Compound **36a** is obtained following the two steps procedure experienced for the synthesis of compound **32a.** First, Boc-L-Orn(Boc)-OH (0.4 mmol ; 132 mg) and compound **36a** (0.22 mmol ; 254) are linked together using HBTU (0.4

mmol ; 152 mg) and DIPEA (0.8 mmol ; 138 μL) dissolved in 6mL of DMF; the reaction is then stirred for 16h at RT. After isolation and purification of the intermediate (flash chromatography on silica gel, elution gradient from 2 to 10% MeOH in DCM), amine functions are released by reacting TFA (350μL) in 5 mL DCM during 12h. Yield: 63% (2 steps, 176 mg, 0.14 mmol).

**[(Z)-non-2-enyl]-4-[[2-[[4-[2-[2-[2,5-bis[[2-(dimethylamino)acetyl]amino]pentanoylamino]ethyldisulfanyl]ethylamino]-4-oxo-butanoyl]amino]-5-[[3-(dodecylamino)-1-(dodecylcarbamoyl)-3-oxo-propyl]amino]-5-oxo-pentanoyl]amino]butanoate (XXXVI)**

Compound **XXXVI** has been isolated adapting the procedure described for compound **II,** by linking compound **36b** (0.14 mmol ; 178 mg) with DMG (0.35 mmol ; 36 mg) using methylimidazole (1.05 mmol ; 84 μL) and TCFH 0.42 mmol; 119 mg) dissolved in 15 mL of a 2/1 (v/v) mixture solution of MeCN and DCM. Reaction time: 12h 0°C to RT; Purification: Silica gel chromatography gradient 98/2 to 90/10 (DCM/MeOH, v/v). Yield: 44% (82 mg of white solid, 0.06 mmol, TLC (DCM/MeOH: 95/5) Rf: 0.2).

## 37 - Synthesis Molecule XXXVII

***tert*-butyl-N-[4-amino-5-[2-[2-[[2-(dimethylamino)acetyl]amino]ethyldisulfanyl]ethylamino]-5-oxo-pentyl]carbamate**

**(37a)**Fmoc-Orn(Boc)-OH (1 mmol ; 1.07 g) is placed beforehand in a dry 50 mL flask under an inert atmosphere then dissolved in 10 mL of anhydrous DMF under stirring. DIC (1.2 mmol ; 188 μL), then HOBt (1.2 mmol ; 162 mg), in solution in 1 mL anhydrous DMF, are successively added to the reaction medium. The reaction is then maintained under an inert atmosphere at ambient temperature for 2 and a half hours. **18a** (0.83 mmol ; 292 mg) and triethylamine (2 mmol ; 281 μL) in solution in 6 mL of anhydrous DCM is added dropwise to the reaction medium. After stirring at ambient temperature under an inert atmosphere for 24 hours, 3 mL of piperidine are added and the reaction medium is stirred for another 4 hours. The reaction is stopped and the reaction medium is evaporated to dryness under a rough vacuum. The residue is taken back in 20 mL of EtOAc and washed 3 times with water and brine. The organic layer is dried on MgSO4 and purified by flash chromatography on silica gel (elution gradient from 2 to 6 % MeOH in DCM). In this way, the product is isolated, in a yield of 87 % (0.72 mmol ; 485 mg). TLC : Rf =0.3 (DCM:MeOH 90/10 (v/v))

The product is taken back in 20 mL of DMF and 2 mL of piperidine is added and the reaction medium is stirred for 4 hours. The reaction is stopped and the reaction medium is evaporated to dryness under a rough vacuum. The residue is triturated several times in petroleum ether, filtrated and used as it is.

**[4-azaniumyl-5-[[4-azaniumyl-1-[2-[2-[[2-(dimethylamino)acetyl]amino]ethyldisulfanyl]ethylcarbamoyl]butyl]amino]-5-oxo-pentyl]ammonium (37b)**

Compound **37b** is obtained following the two steps procedure experienced for the synthesis of compound **32a.** First, Boc-L-Orn(Boc)-OH (1.4 mmol ; 465 mg) and compound **37a** (0.72 mmol ; 325 mg) are linked together using HBTU (1.4 mmol ; 531 mg) and DIPEA (2.8 mmol ; 485 μL) dissolved in 10mL of DMF; the reaction is then stirred for 16h at RT. After isolation and purification of the intermediate (flash chromatography on silica gel, elution gradient from 2 to 5% MeOH in DCM), amine functions are released by reacting TFA (2 mL) in 25 mL DCM during 12h. Yield: 63% (2 steps, 392 mg, 0.51 mmol).

**(9Z,12Z)-N-[5-[[1-[2-[2-[[2-(dimethylamino)acetyl]amino]ethyldisulfanyl]ethylcarbamoyl]-4-[[(9Z,12Z)-octadeca-9,12-dienoyl]amino]butyl]amino]-4-[[(9Z,12Z)-octadeca-9,12-dienoyl]amino]-5-oxo-pentyl]octadeca-9,12-dienamide (XXXVII)**

Compound **XXXVII** has been isolated adapting the procedure described for compound **1b**, by linking compound Linoleic acid (1.8 mmol ; 505 mg) with compound **37b** (0.51 mmol ; 411 mg) using DIPEA (2 mmol ; 353 μL) and PyBOP (1.8 mmol ; 936 mg) dissolved in 15 mL of DMF. Reaction time: 24h RT; Purification: Silica gel chromatography gradient 98/2 to 94/6 (DCM/MeOH, v/v). Yield: 26% (166 mg of white solid, 0.13 mmol, TLC (DCM/MeOH: 95/5) Rf: 0.4).

## 38 - Synthesis Molecule XXXVIII

**4-pyrrolidin-1-ylbutyl 2-amino-5-(tert-butoxycarbonylamino)pentanoate (38a)**

Fmoc-Orn(Boc)-OH (0.5 mmol ; 227 mg) is placed in a 50 mL flask under an argon and is then dissolved in 20 mL

of DCM. DCC (0.6 mmol ; 124 mg) and DMAP (0.1 mmol ; 11 mg) are added to the reaction medium followed by 4-(1-pyrrolidinyl)-1-butanol (0.7 mmol ; 100 mg). After stirring at RT under argon, for 15 hours, the reaction is stopped and the reaction medium is evaporated to dryness. The crude is taken back in 20 mL of DCM and washed with 1M HCl, NaHCOs and brine. The organic layer is dried on $MgSO_4$ and purified by flash chromatography on silica gel (elution gradient from 4 to 10 % MeOH in DCM). In this way, the intermediate product is isolated, and then taken back in 20 mL of DMF and 2 mL of piperidine is added and the reaction medium is stirred for 4 hours. The reaction is stopped and the reaction medium is evaporated to dryness under a rough vacuum. The residue is triturated several times in petroleum ether, filtrated and used as it is (82% yield, two steps, 0.41 mmoles, 147 mg).

**[4-azaniumyl-5-[[4-azaniumyl-1-(4-pyrrolidin-1-ylbutoxycarbonyl)butyl]amino]-5-oxo-pentyl]ammonium (38b)**

Compound **38b** is obtained following the two steps procedure experienced for the synthesis of compound **32a.** First, Boc-L-Orn(Boc)-OH (0.7 mmol ; 233 mg) and compound **38a** (0.41 mmol ; 147 mg) are linked together using HBTU (0.7 mmol ; 265 mg) and DIPEA (1.4 mmol ; 243 μL) dissolved in 9mL of DMF; the reaction is then stirred for 16h at RT. After isolation and purification of the intermediate (flash chromatography on silica gel, elution gradient from 2 to 5% MeOH in DCM), amine functions are released by reacting TFA (1 mL) in 10 mL DCM during 12h. Yield: 51% (2 steps, 149 mg, 0.21 mmol).

**4-pyrrolidin-1-ylbutyl-2-[2,5-bis[[(9Z,12Z)-octadeca-9,12-dienoyl]amino]pentanoylamino]-5-[[(9Z,12Z)-octadeca-9,12-dienoyl]amino]pentanoate (XXXVIII)**

Compound **XXXVIII** has been isolated adapting the procedure described for compound **1b**, by linking Linoleic acid (0.74 mmol ; 206 mg) with compound **38b** (0.21 mmol ; 149 mg) using DIPEA (1.4 mmol ; 247 μL) and PyBOP (0.74 mmol ; 385 mg) dissolved in 8mL of DMF. Reaction time: 24h RT; Purification: Silica gel chromatography gradient 98/2 to 94/6 (DCM/MeOH, v/v). Yield: 32% (78 mg of white solid, 0.07 mmol, TLC (DCM/MeOH: 95/5) Rf: 0.3).

**39 - Synthesis Molecule XXXIX**

**[4-azaniumyl-5-(2-hydroxyethylamino)-5-oxo-pentyl]ammonium (39a)**

A solution of , Boc-L-Orn(Boc)-OH (2 mmol ; 665 mg) in DMF is stirred at room temperature and EDC·HCl (2.4 mmol ; 460 mg), HOBt (2.4 mmol ; 324 mg) are successively added to the reaction medium. The reaction is stirred under Argon at RT for one hour before ethanolamine (10 mmol ; 604 μL) is added. After stirring at RT for 24 hours more, the reaction is stopped and the reaction medium is evaporated to dryness. The residue is taken back in 50 mL of water and the amide is extracted 3 times with $CHCl_3$, the organic layers are dried on $MgSO_4$ and purified by flash chromatography on silica gel (elution gradient from 2 to 6 % MeOH in DCM). The isolated intermediary product (1.82 mmol ; 683 mg) is then dissolved in 90 mL of DCM, then 5 mL of trifluoroacetic acid are added to the reaction medium. The reaction is then maintained for 16 hours under stirring at ambient temperature. The mixture is then concentrated, co-evaporated thrice with 50 mL of DCM, thrice with 50 mL of diethyl ether ($Et_2O$), and dried under high vacuum overnight Yield: 91% (2 steps, 734 mg, 1.82 mmol).

**2,5-bis[4-(dimethylamino)butanoylamino]-N-(2-hydroxyethyl)pentanamide (39b)**

Compound **39b** has been isolated adapting the procedure described for compound **VI**, by coupling DMABA (4.55 mmol, 763 mg) with compound **39a** (1.82 mmol; 734 mg) using DIPEA (9 mmol ; 1.55 mL) and HBTU (4.55 mmol; 1.72 g) dissolved in 30mL of DCM and 5 mL DMF. Reaction time: 48h RT; Purification: Silica gel chromatography gradient 98/2 to 95/5 (DCM/MeOH, v/v). Yield: 51% (373 mg of white solid, 0.93 mmol, TLC (DCM/MeOH: 95/5) Rf: 0.4).

**2-[2,5-bis[4-(dimethylamino)butanoylamino]pentanoylamino]ethyl2-amino-5-(tert-butoxycarbonylamino)pentanoate (39c)**

Compound **39c** is obtained following the two steps procedure experienced for the synthesis of compound **38a.** First, Fmoc-L-Orn(Boc)-OH (1 mmol ; 454 mg) and compound **39b** (0.93 mmol ; 373 mg) are coupled together using DCC (1.2 mmol ; 248 mg) and DMAP (0.2 mmol ; 22 mg) dissolved in 40mL of DCM; the reaction is then stirred for 16h at RT. After isolation and purification of the intermediate (flash chromatography on silica gel, elution gradient from 2 to 5% MeOH in DCM), Fmoc group is released by reacting piperidine (2 mL) in 20 mL DMF during 4h. Yield: 76% (2 steps, 437 mg, 0.71mmol).

**[4-azaniumyl-5-[[4-azaniumyl-1-[2-[2,5-bis[4-(dimethylamino)butanoylamino]pentanoylamino]ethoxycarbonyl] butyl]amino]-5-oxo-pentyl]ammonium (39d)**

Compound **39d** is obtained following the two steps procedure experienced for the synthesis of compound **32a.** First, Boc-L-Orn(Boc)-OH (1.4 mmol ; 465 mg) and compound **39c** ((0.71 mmol ; 437 mg) are linked together using HBTU (1.4 mmol ; 531 mg) and DIPEA (2.8 mmol ; 485 μL) dissolved in 15 mL of DMF; the reaction is then stirred for 16h at RT. After isolation and purification of the intermediate (flash chromatography on silica gel, elution gradient from 2 to 5% MeOH in DCM), amine functions are released by reacting TFA (2 mL) in 30 mL DCM during 12h. Yield: 59% (2 steps, 408 mg, 0.42 mmol).

**2-[2,5-bis[4-(dimethylamino)butanoylamino]pentanoylamino]ethyl 2-[2,5-bis[[(9Z,12Z)-octadeca-9,12-dienoyl] amino]pentanoylamino]-5-[[(9Z,12Z)-octadeca-9,12-dienoyl]amino]pentanoate (XXXIX)**

Compound **XXXIX** has been isolated adapting the procedure described for compound **1b**, by linking Linoleic acid (1.5 mmol ; 421 mg) with compound **38b** (0.42 mmol ; 408 mg) using DIPEA (2 mmol ; 353 μL) and PyBOP (1.5 mmol ; 780 mg) dissolved in 16 mL of DMF and 2 mL DCM. Reaction time: 24h RT; Purification: Silica gel chromatography gradient 98/2 to 94/6 (DCM/MeOH, v/v). Yield: 26% (156 mg of white solid, 0.11 mmol, TLC (DCM/MeOH: 95/5) Rf: 0.3).

**40 - Synthesis Molecule XXXX**

**[4-methyl-5-[[4-[[2-methyl-5-[(2,2,2-trifluoroacetyl)ammonio]pentanoyl]amino]-5-oxo-5-(4-pyrrolidin-1-ylbutoxy)pentyl]amino]-5-oxo-pentyl]-(2,2,2-trifluoroacetyl)ammonium-bis-trifluoroacetate (40a)**

Compound **40a** is obtained following the two steps procedure experienced for the synthesis of compound **18b**. First, Boc-L-Orn(Boc)-OH (365 mg, 1.1 mmoles) and compound **18a** (274 mg, 0.5 mmoles) are linked together using HOBt (162 mg, 1.2 mmoles) and DIC (201 μL, 1.3 mmoles) dissolved in 20 mL of a 9/1 v/v mixture of dry DCM/DMF that contains also $Et_3N$ (210 μL, 1.5 mmoles); the reaction is then stirred for 72h at RT. After isolation and purification of the intermediate compound, amine functions are released by reacting TFA (190 μL) in 25 mL DCM during 2h. Yield: 48% (2 steps, 0.24 mmoles, 234 mg).

**(9Z,12Z)-N-[5-[[4-[2,5-bis[[(9Z,12Z)-octadeca-9,12-dienoyl]amino]pentanoylamino]-5-[2-[2-[[2-(dimethylamino) acetyl]amino]ethyldisulfanyl]ethylamino]-5-oxo-pentyl]amino]-4-[[(9Z,12Z)-octadeca-9,12-dienoyl]amino]-5-oxo-pentyl]octadeca-9,12-dienamide (XXXX)**

Compound **XXXX** has been isolated adapting the procedure described for compound XV, by linking Linoleic acid (404 μL, 1.3 mmoles) with compound **40a** (0.21 mmoles, 204 mg) using DIPEA (263 μL, 1.5 mmoles), HBTU (530 mg, 1.4 mmoles) and HOBt (183 mg, 1.35 mmoles) dissolved in 6 mL of DMF and 6 mL DCM. Reaction time: 36h 0°C to RT; Purification: Silica gel chromatography gradient 95/5 to 90/10 (DCM/MeOH, v/v). Yield: 54% (179 mg of white solid, 0.11 mmol, TLC (DCM/MeOH: 95/5) Rf: 0.3).

**41 - Synthesis Molecule XXXXI**

**[5-[[1-[2-[2,5-bis[4-(dimethylamino)butanoylamino]pentanoylamino]ethoxycarbonyl]-4-[[2-methyl-5-[(2,2,2-trifluoroacetyl)ammonio]pentanoyl]amino]butyl]amino]-4-methyl-5-oxo-pentyl]-(2,2,2-trifluoroacetyl)ammonium (41a)**

Compound **41a** is obtained following the two steps procedure experienced for the synthesis of compound **18b**. First, Boc-L-Orn(Boc)-OH (183 mg, 0.55 mmoles) and compound **19d** (178 mg, 0.25 mmoles) are linked together using HOBt (81 mg, 0.6 mmoles) and DIC (100 μL, 0.65 mmoles) dissolved in 10 mL of a 9/1 v/v mixture of dry DCM/DMF that contains also $Et_3N$ 105 μL, 0.75 mmoles); the reaction is then stirred for 72h at RT. After isolation and purification of the intermediate compound, amine functions are released by reacting TFA (95 μL) in 15 mL DCM during 2h. Yield: 51% (2 steps; 0.127 mmoles, 145 mg).

**2-[2,5-bis[4-(dimethylamino)butanoylamino]pentanoylamino]ethyl2,5-bis[2,5-bis[[(9Z,12Z)-octadeca-9,12-dienoyl]amino]pentanoylamino]pentanoate (XXXXI)**

Compound **XXXXI** has been isolated adapting the procedure described for compound **XV,** by linking Linoleic acid ((202 μL, 0.65 mmoles) with compound **41a** (0.1 mmoles, 113 mg) using DIPEA (132 μL, 0.75 mmoles), HBTU (265 mg,

0.7 mmoles) and HOBt (92 mg, 0.68 mmoles) dissolved in 6 mL of DMF and 6 mL DCM. Reaction time: 36h 0°C to RT; Purification: Silica gel chromatography gradient 93/7 to 90/10 (DCM/MeOH, v/v). Yield: 54% (179 mg of white solid, 0.11 mmol, TLC (DCM/MeOH: 95/5) Rf: 0.2).

## 42 - Synthesis Molecule XXXXII

**[5-oxo-5-(4-pyrrolidin-1-ylbutoxy)-4-[(2,2,2-trifluoroacetyl)ammonio]pentyl]-(2,2,2-trifluoroacetyl)ammonium (42a)**

Compound **42a** is obtained following the two steps procedure experienced for the synthesis of compound **18b.** First, Boc-L-Orn(Boc)-OH (10 mmoles, 3.32g) and 4-(1-Pyrrolidinyl)-1-butanol (1.43g, 10 mmoles) are linked together using HOBt (1.49 g, 11 mmoles) and DIC (1.7 mL, 11 mmoles) dissolved in 100 mL of a 9/1 v/v mixture of dry DCM/DMF that contains also $Et_3N$ (2.1 mL, 15 mmoles); the reaction is then stirred for 36h at RT. After isolation and purification of the intermediate compound, amine functions are released by reacting TFA (1.3 mL) in 60 mL DCM during 2h. Yield: 83% (2 steps; 8.3 mmoles, 3.76g).

**[4-methyl-5-[[4-[[2-methyl-5-[(2,2,2-trifluoroacetyl)ammonio]pentanoyl]amino]-5-oxo-5-(4-pyrrolidin-1-ylbutoxy)pentyl]amino]-5-oxo-pentyl]-(2,2,2-trifluoroacetyl)ammonium (42b)**

Compound **42b** is obtained following the two steps procedure experienced for the synthesis of compound **18b**. First, Boc-L-Orn(Boc)-OH (1.46 g, 4.4 mmoles) and **42a** (906 mg, 2 mmoles) are linked together using HOBt (648 mg, 4.8 mmoles) and DIC (800 μL, 5.2 mmoles) dissolved in 40 mL of a 9/1 v/v mixture of dry DCM/DMF that contains also $Et_3N$ (2.1 mL, 15 mmoles); the reaction is then stirred for 72h at RT. After isolation and purification of the intermediate compound, amine functions are released by reacting TFA (760 μL) in 30 mL DCM during 6h. Yield: 44% (2 steps; 8.3 mmoles, 772 mg).

**4-pyrrolidin-1-ylbutyl-2,5-bis[2,5-bis[[(9Z,12Z)-octadeca-9,12-dienoyl]amino]pentanoylamino]pentanoate (XXXXII)**

Compound **XXXXII** has been isolated adapting the procedure described for compound **XV**, by linking Linoleic acid (404 μL, 1.3 mmoles) with compound **42b** (0.25 mmoles, 219 mg) using DIPEA (263 μL, 1.5 mmoles), HBTU (530 mg, 1.4 mmoles) and HOBt (183 mg, 1.35 mmoles) dissolved in 6 mL of DMF and 6 mL DCM. Reaction time: 36h 0°C to RT; Purification: Silica gel chromatography gradient 95/5 to 90/10 (DCM/MeOH, v/v). Yield: 59% (227 mg of white solid, 0.148 mmol, TLC (DCM/MeOH: 95/5) Rf: 0.25).

## 43 - Synthesis Molecule XXXXIII

**3-butylhept-2-enyl 4-[[4-amino-5-[[4-(3-butylhept-2-enoxy)-4-oxo-butyl]amino]-5-oxo-pentanoyl]amino]butanoate (43a)**

Following the procedure described for compound **21d,** molecule **43a** is obtained in two steps by coupling (Fmoc-Glu-OH, 259 mg, 0.7 mmoles) and compound **33a** (1.82 mmol ; 672 mg) using HOBt (331 mg, 2.45 mmoles), DIC (325 μL, 2.1 mmoles) and $Et_3N$ (394 μL, 2.8 mmoles) dissolved in chloroform (7 mL) and DMF (7 mL), and stirring 48h. Then, the Fmoc group is removed by using piperidine (2 mL) and stirring 4h RT. Reaction time: 48h+4h RT; Purification: Silica gel chromatography gradient 95/5 to 90/10 (DCM/MeOH, v/v).Yield: 41 % (0.287 mmol ; 179 mg); TLC : Rf=0.4 (DCM:MeOH 90/10 (v/v)).).

**3-butylhept-2-enyl-4-[[5-[[4-(3-butylhept-2-enoxy)-4-oxo-butyl]amino]-4-[4-(dimethylamino)butanoylamino]-5-oxo-pentanoyl]amino]butanoate (XXXXIII)**

Compound **XXXXIII** has been isolated adapting the procedure described for compound VI, by coupling DMABA (0.72 mmol, 121 mg) with compound **43a** (0.287 mmol ; 179 mg) using DIPEA (1.42 mmol ; 245 μL) and HBTU (0.72 mmol ; 271 mg) dissolved in 2.5 mL of DCM and 2.5 mL DMF. Reaction time: 24h RT; Purification: Silica gel chromatography gradient 98/2 to 90/10 (DCM/MeOH, v/v). Yield: 38% (81 mg of white solid, 0.11 mmol, TLC (DCM/MeOH: 95/5) Rf: 0.2).

## II- RESULTS

*Encapsulation of mRAM in lipid nanoparticles using microfluidic formulation*

The lipids described in the present invention were then used to encapsulate mRNA *via* microfluidic formulation of lipid nanoparticles. First all lipidic components (*i.e.*, tunable lipid, helper neutral lipid, sterol derivative and PEG-lipid) were dissolved separately in glass vial at 25 mg/mL in a 99/1 (v/v) solution of chloroform in methanol as different molar ratios (Table 1). Then, the necessary amount of each component is introduced in a flask, that is evaporated 2h at 45°C under gentle vacuum. This step leads to the obtention of dry lipidic film that is then dried under high vacuum for one night. The lipidic film is then dissolved in sterile-filtered EtOH, and sonicated during 10 minutes. The volume of ethanol is chosen according the desired final formulation volume needed. Meanwhile mRNA is dissolved in a 1 mM citrate buffer at pH=4, so that the final concentration of mRNA solution is 0.125 mg/mL.

Formulation into lipid nanoparticles is realized using the *μ-encapsulator* system from Dolomite Microfluidics into the MicroMixer chip. Briefly the ethanolic lipid solution and mRNA solution are injected into the system and formed LNPs are collected at the output. The pump parameters have been optimized for each formulation depending on the lipid used, the targeted final concentration of lipids required and the size of chosen nucleic acid. As a basis, a Flow Rate Ratio (FRR) equals to 3 and a total flow rate of 1000 μL/min could be set up. Collected LNPs are subsequently dialyzed against PBS+5% w/v sucrose overnight, filtered on a sterile 0.45 μm filter and stored at -80°C.

**Table 1: Molar compositions used for the formation of LNPs based on tunable lipids**

| Lipid components | mol (%) N=1 | mol (%) N=2 |
|---|---|---|
| **Tunable Lipid** | 50 | 25 |
| **Helper neutral lipid** | 10 | 15 |
| **Sterol derivative** | 38.5 | 57.75 |
| **PEG-lipid (stealth lipid)** | 1.5 | 2.25 |
| **Global** | 100 | 100 |

*Characterization of mRAM LNPs*

The size and charge of the collected LNPs are determined using DLS (Malvern Instruments NanoZS) by dissolving 50 μL of the collected solution in 950 μL of MilliQ water. Then, formed LNPs are destabilized by adding 1% v/v of Triton X-100 and incubate at 30°C for 1h and vortexing. After disruption of the formulation, a 1/100 solution of SyBr™ Gold in water is prepared, and 0.5μL of it added to 49.5μL of the resulting mixture that includes Triton X-100. The SyBr™ Gold is also added the same way to 49.5μL of a solution that contains non-disrupted LNPs, and to 49.5μL of the mRNA mother solution. Fluorescence of each sample is then read after 30 min incubation at RT using a Victor (Perkin Elmer) fluorimeter (excitation: 495nm ; emission: 537nm ;). The encapsulation efficiency (ee(%)) is easily obtained from these fluorescence measurements using the formula below:

$$ee\,(\%) = \frac{(Fluo_LNP_{1v\%triton} - Fluo_LNP)}{Fluo_RNA_{stock\ solution}}\,x\,100$$

This methodology has been applied to several tunable lipids depicted in this invention (Table 2). For this first step of screening, 5-methoxyuridine-modified mRNA encoding for GFP (OZ Biosciences, Cat: MRNA15-1000) has been encapsulated.

**Table 2: Main physical properties of mRNA(GFP) LNPs based on tunable lipids I, II, V, VI, XIV, XV**

| LNPs Characterization | I | II | V | VI | X | XIV | XV |
|---|---|---|---|---|---|---|---|
| **Total Lipid concentration (mM)** | 3.75 | 3.75 | 3.75 | 3.75 | 3.75 | 3.75 | 3.75 |
| **Size (PDI)** | 83 nm (0.06) | 113 nm (0.2) | 95 nm (0.113) | 117 nm (0.344) | 141 nm (0.109) | 115 nm (0.186) | 79 nm (0.11) |

(continued)

| LNPs Characterization | I | II | V | VI | X | XIV | XV |
|---|---|---|---|---|---|---|---|
| Zeta | -9.3 mV | 5.57 mV | -17 mV | +0.3 mV | +11.6 mV | +15.6 mV | - 11.4mV |
| Loaded RNA (ng/μL) | 94.6 | 98.8 | 89 | 84 | 83.6 | 91 | 78.3 |
| N/P Ratio | 6 | 6 | 6 | 6 | 6 | 6 | 6 |
| Encapsulation Efficiency | 94.6% | 98.8% | 89% | 84% | 83.6% | 91% | 78.3% |

All the tested lipids proved to be able to form nanoparticles when associated with a neutral phospholipid, a sterol derivative and a PEG-lipid under classical microfluidic handling and high encapsulation efficiency was achieved. Regarding physico-chemical properties, a majority of the lipid allowed the obtention of small size LNPs, with DLS measurements showing values around 100 nm. Especially in the case of lipids **I, V** and **XV,** LNPs with size ranging below 100 nm could be obtained with polydispersity index around 0.1. Interestingly, value of Zeta potential varied from one lipid to another, even if the N/P ratio was maintained constant. Indeed, the surface charge of the LNP can vary from a quite negative value if lipid **V** is issued to a positive one, such in the case of lipid **XIV.** This implies that the geometry of the tunable lipid used has a clear influence on the future physical properties of the corresponding LNPs and probably on its biological behaviour. Consequently, a skilled scientist would have to test several lipids corresponding to the formula depicted in this invention and select the most suitable one depending on the properties he would want to reach for his LNPs.

*Cells*

Human cervical carcinoma (HeLa) cell lines were cultured in Dulbecco's Modified Eagle Medium (DMEM, Lonza, Walkersville, MD, USA) supplemented with 10 % Foetal Bovine Serum (FBD, St. Louis, MO, USA), 2mM final L-Glutamine, 100 units/ml penicillin and 100 μ/ml streptomycin (Lonza, Walkersville, MD, USA). Human immortalized Jurkat T-cells were cultured in Roswell Park Memorial Institute medium (RPMI, Lonza, Walkersville, MD, USA) supplemented with 10 % Foetal Bovine Serum (FBD, St. Louis, MO, USA), 2mM final L-Glutamine, 100 units/ml penicillin and 100 μ/ml streptomycin (Lonza, Walkersville, MD, USA). All cell lines were cultured in 75 $cm^2$ flasks (Sarstedt AG & Co., Germany) at 37°C in a humidified incubator (Sanyo, Tokyo, Japan) with 5% $CO_2$ atmosphere, and trypsinized at 80% confluency in order to maintain an exponential division rate before transduction assays. Transfection experiments were performed in 24 well-plates (Sarstedt AG & Co., Germany).

*In vitro transfection of GFP mRAM using LNPs*

Two LNPs formulations based respectively on lipids **I** and **XIV** have then been tested in an *in vitro* transfection experiment to demonstrate their potency to efficiently deliver mRNA, while enabling mRNA gene expression. Transfection efficiency has been monitored by flow cytometry by evaluating the percent of transfected cells, and the percent of living cells using flow cytometry. A quantity of mRNA ranging from 0.1 μg to 20 μg has been tested and RmesFect, a commercially available transfection reagent (a cationic lipid with permanent positive charges) devoted to mRNA delivery, has been used as a positive control.

This result (Figure 48) displays the potency of LNPs based on tunable lipids to transfect a cell line using a wide range of mRNA quantities, with a maximum of 90% of positive GFP cells Reached values proved to be better than those observed with a more classical transfection reagent, while maintaining cell viability. Indeed, regarding % of living cells, tunable lipids formulated as LNPs remained harmless in comparison to transfection reagent which led to a rapid decline in cell survival, with only 30% of cells still alive when 1 μg of mRNA is used (Figure 48).

These results clearly show the potency of LNPs based on tunable lipids to be efficient in an *in vitro* transfection context, at level equivalent to standard transfection reagent, without compromising cell survival especially at high nucleic acid doses.

These results were confirmed in another cellular model known for being difficult to transfect in a laboratory setup using non-viral transfection reagent (Figure 49). Jurkat T-cells, a suspension cell line was transfected as described above and . results using LNPs based on tunable lipids **I** and **XIV** are depicted in Figure 49. Observed results are in accordance with those observed in HeLa cells. The transfection efficiency was able to reach 40% while in the same time the commercially available reagent provides only 10% of positive cells at the maximum mRNA quantity used. Furthermore, regarding % of living cells after transfection, tunable lipids formulated as LNPs remained the safest in the whole range of

mRNA used quantities, whereas the transfection reagent used as control lead to a decline in cell survival with increasing mRNA quantities, with only 40% of cells still alive when 5 μg of mRNA is used.

*In vivo transfection of F-Luc mRNA using LNPs; Efficiency and biodistribution*

LNPs have been described as a really powerful gene delivery vehicle for *in vivo* application, due to their harmless nature and their potency to efficiently protect and deliver a wide array of nucleic acid. The tunable lipids described in this invention pushed the safe character even further as their whole design and synthesis has been developed to meet these important criteria, and to spread even more their use *in vivo.*

Lipids **I, VI** and **XIV** have been formulated into LNPs following the molar composition depicted before (Table 1). 5-methoxyuridine-modified mRNA encoding for *Firefly* Luciferase (OZ Biosciences, Cat: MRNA16-1000) has been entrapped into the LNP. To demonstrate the innovative nature of the tunable lipids, an additional LNP that includes a well-known cationic lipid has been chosen for comparison purposes. DOTAP has been selected for its widely documented *in vivo* behaviour and its permanent positive charge at physiological pH The main physical properties of the resulting LNPs are displayed in Table 3.

**Table 3: Main physical properties of mRNA(F-Luc) LNPs based on tunable lipids I, VI, XIV, and cationic lipid DOTAP**

| LNPs Characterizat ion | **I** | **VI** | **XIV** | **DOTAP** |
|---|---|---|---|---|
| Total Lipid concentration | 5 mM | 5 mM | 5 mM | 5 mM |
| Size (PDI) | 88 nm (0.09) | 115 nm (0.13) | 99 nm (0.13) | 96 nm (0.23) |
| Zeta | -7.3 mV | -2.25 mV | +4.85 mV | +17 mV |
| Loaded RNA (ng/μL) | 195 | 188 | 175 | 183 |
| N/P Ratio | 6 | 6 | 6 | 6 |
| Encapsulatio n Efficiency | 97.5% | 94% | 87.5% | 91.5% |

Overall, all LNPs formulations display consistent physico-chemical properties, controlled and reproducible size, charge, polydispersity and encapsulation rate to be used in small animals. A dose equivalent to 10 μg of mRNA has been injected i.p. (intra-peritoneally) in nude mice using three different LNPs. Tunable lipids **I** and **XIV** and DOTAP-based LNPs have been selected. Bioluminescence has been monitored over time using IVIS imaging system. Bioluminescence evaluation demonstrates that all three LNPs succeeded in delivering F-Luc mRNA into mice. Indeed 3h post injection, luminescence could be observed in all animals at different proportions (Figure 50). Specifically, F-Luc mRNA expression was higher in mice that received LNPs based on tunable lipids, whereas expression was much lower when DOTAP is used. Interestingly, the figure 50B that monitors the kinetics of the bioluminescence signal over time presents meaningful differences between the two LNP formulations. Indeed, LNPs based on tunable lipid **I** gave the highest overall luminescence signal, with a maximum after 6 hours post injection whereas LNPs based on lipid **XIV** allowed the maximum bioluminescence signal only after 3 h, earlier than with the other formulation. This result demonstrates the efficiency *in vivo* of the tunable lipids claimed in this invention, but also the complementary behaviour that can results from their structural diversity, providing the skilled scientist with formulations able to deliver efficiently nucleic acids with different kinetics in this case.

Biodistribution study involving tunable lipids-based LNPs (lipids **I**, **VI,** and **XIV**) has also been conducted in nude mice that were injected i.p. with Luciferase mRNA formulated into 3 different LNPs formulations at 10μg RNA dose to evaluate the nucleic acid delivery into various organs. The liver, lung, kidney and spleen were collected and analysed through RT-PCR 6 hours after injection. As observed previously, tunable lipids-based LNPs are appropriately efficient to allow the observation and quantitation of Luciferase protein in quantity 6 hours after i.p. injection. LNPs based on lipid **I** provides high protein expression rate, observed in lung and spleen and to lower extend also in the kidney. LNPs based on lipid **VI** showed a high delivery in the spleen after 6 hours. Interestingly this formulation is also able to deliver mRNA in the liver at the chosen N/P ratio, with a rate comparable to those observed in the lungs. Meanwhile LNPs based on lipid **XIV** offers the highest rate of gene expression in lung, with a rate as high as those observed in spleen. It is also the most efficient between the three tested formulation in expressing the Luciferase in kidneys. This biodistribution study, displayed the efficiency of the LNPs based on tunable lipids to transport nucleic acid in different organs *in vivo.* Furthermore, the complementary nature of the results, allowing different expression rate in organs depending on the chosen tunable lipid, is a clear demonstration of the importance of the structural variety offered by this invention, which permits organ targeting through the choice of the tunable lipid's structure.

*In vivo immunization experiment; Delivery of antigen OVA mRAM using LNPs*

Recent studies clearly demonstrated the need for the community to develop new generation of vaccines, easy to handle, safe to the patient while remaining efficient in immunization. In this context, the emergence of vaccine-based LNPs represents a clear opportunity to reach pathologies impossible to fight with more classical vaccine approach. The tunable lipid presented in this document could envisioned as potent pillar for future nucleic acid-based vaccine, due to their ground-breaking features in term of efficiency and safe character. Thus, LNPs based on tunable lipids **I** and **XIV** have been formulated to encapsulate unmodified mRNA coding for the ovalbumin (OVA) antigen (OZ Biosciences, Cat: MRNA42-1000) and their physico-chemical properties determined (Table 4).

**Table 4: Main physical properties of mRNA(OVA) LNPs based on tunable lipids I and XIV.**

| LNPs Characterization | I | XIV |
|---|---|---|
| Total Lipid concentration | 5 mM | 5 mM |
| Size (PDI) | 111.4 nm (0.153) | 131.7 nm (0.149) |
| Zeta | -9.01 mV | -7.9 mV |
| Loaded RNA (ng/μL) | 98.1 | 94 |
| N/P Ratio | 6 | 6 |
| Encapsulation Efficiency | 98.1% | 94% |

The two LNPs formulations showed consistent, and reproducible size, polydispersity charge and encapsulation efficiency. The two LNPs have been used in an immunization study to demonstrate their efficacy and potential in comparison to a classical recombinant subunit vaccine. C57BL6J mice received a prime boost (D0-14) immunizations of OVA antigen delivered by mRNA-based LNPs or by recombinant protein (alone or adjuvanted with aluminium-based adjuvant). To characterize the immuno-phenotyping of the mice, the sera and splenocytes were collected at different time (D0, D14, D21, D28) and analysed by ELISA and Elispot. The mice were immunized by s.c (sub-cutaneous) route with 100μL per injection at 10μg OVA subunit and 10μg mRNA doses. Results are depicted in figures 51 and 52, mRNA-OVA LNP formulations are efficient at priming a strong humoral response upon sub-cutaneous prime boost injection (D0-14). Indeed, a strong antibody response was measured after prime boost immunization. In addition, no significative signal was observed when the dose of 10μg of OVA antigen is directly administrated alone. It is noteworthy, the importance of the boost (D14) to obtain a long-lasting immune response. Both mRNA-OVA LNP based on lipid **I and** lipid **XIV** are efficient in generating an immune response following the administration of mRNA s.c., especially regarding the initial response of IgG2c. mRNA-OVA LNP formulations are efficient at priming a strong TH1 humoral response equivalent to the best TH1 adjuvants upon sub-cutaneous prime boost injection (D0-14). mRNA-OVA LNP formulations are also efficient at priming a strong anti-OVA CD8 T cell response equivalent to one of the most efficient adjuvants upon sub-cutaneous prime boost injection (D0-14) as shown by IFN□ Elispot results and also IL-18 production (not shown).

*Encapsulation of DNA in lipid nanoparticles using microfluidic formulation;*

The potency of the tunable lipids was further assessed for DNA delivery, several lipids belonging to this invention have been selected to encapsulate plasmid DNA into LNPs. After a initial screening, lipids **I** and **XI** have been selected as basis tunable compound for the formulation. The same microfluidic procedure used for encapsulation of mRNA has been translated to DNA applications. Ratio between all lipidic components (*i.e.*, tunable lipid, helper neutral lipid, sterol derivative and PEG-lipid) used with mRNA has been kept (see table 1) with DNA, and the microfluidic formulation procedure remained unchanged. As a basis, a Flow Rate Ratio (FRR) equals to 3 and a total flow rate of 1000 μL/min has been set up. Collected LNPs are subsequently dialyzed against PBS+5% w/v sucrose overnight, filtered on a sterile 0.45 μm filter and stored at -20°C. Plasmids DNA (pVectOZ) were designed and produced by OZ Biosciences. Three plasmids coding for Ppt, eGFP and F-Luc have been tested. Thus, LNPs based on tunable lipids **I** and **XI** have been formulated physico-chemical properties determined accordingly (Table 4).

**Table 5: Main physical properties of pVectOZ plasmids-based LNPs based on tunable lipids I and XI.**

| Tunable Lipid | I | I | XI |
|---|---|---|---|
| Total Lipid concentration | 4 mM | 3.91 mM | 4.6 mM |
| DNA | pVectOZ(Ppt) | pVectOZ (eGFP) | pVectOZ (F-Luc) |

(continued)

| Tunable Lipid | I | I | XI |
|---|---|---|---|
| Size (PDI) | 173 nm (0.079) | 158.9 nm (0.044) | 103 nm (0.079) |
| Zeta | +14.3 mV | +4.61 mV | +16.5 mV |
| Loaded DNA (ng/μL) | 0.125 | 0.125 | 94 |
| N/P Ratio | 10 | 6.6 | 12 |
| Encapsulation Efficiency | 82% | 83% | 92% |

Results displayed on the table clearly highlights the propensity of tunable lipids **I** and **XI** to efficiently encapsulate different DNA plasmids into LNPs. Due to higher size of DNA versus mRNA, N/P ratio have been adjusted, so that the size of the resulting nanostructures is maintained below 200 nm with polydispersity indexes having values lower than 0.1, that qualifies these structures for further testing and gives good insights about their mid-term stability.

*In vitro transfection of F-Luc DNA using LNPs*

The LNP formulation based on lipids **XI** has then been tested in an *in vitro* transfection experiment to demonstrate their potency to efficiently deliver DNA, while enabling DNA gene expression. Jurkat T-cells were used. Transfection efficiency has been assessed after 48h incubation using the Luciferase Assay Kit (OZ Biosciences) by evaluating the luminescence intensity (Perkin Elmer Victor NOVO luminometer) obtained after lysis of the transfected cells, in presence of luciferin, magnesium and ATP. A quantity of DNA ranging from 0.1 μg to 10 μg has been tested while MTX reagent (OZ Biosciences SAS), a commercially available transfection reagent (a cationic lipid with permanent positive charges) devoted to DNA delivery, has been used as a positive control.

This result (Figure 54) displays again the potency of LNPs based on tunable lipids to transfect a hard to transfect suspension cell line using a wide range of DNA quantities, with a growing of quantity of measured emitted light directly correlated with the amount of introduced DNA. Reached luminescence values proved to be similar than those observed with a more classical transfection reagent, while maintaining cell viability. These results clearly show the potency of LNPs based on tunable lipids to be efficient in an *in vitro* DNA transfection, at level equivalent to standard transfection reagent, without compromising cell survival especially at high nucleic acid doses.

*Encapsulation* of *siRNA in lipid nanoparticles using microfluidic formulation;*

Since tunable lipids proved to encapsulate efficiently large polynucleotides such as mRNA or DNA, their potency to efficiently protect and deliver smaller polynucleotides such as siRNA has also been investigated. Thus, several tunable lipids of to this invention have been selected to encapsulate NTsiRNA into lipid nanoparticles. After a selection round, ipids **XI** and **XIV** have been chosen as basis compound for the formulation. The same microfluidic used for encapsulation of mRNA has been translated to siRNA applications.

Chosen NTsiRNA have been labelled using Cyanine 5 (Cy5), to be able to monitor their uptake after LNPs exposure onto cells. Similarly, selected formulated LNPs have been labelled using DiO (Benzoxazolium, 3-octadecyl-2-[3-(3-octadecyl-2(3H)-benzoxazolylidene)-1-propenyl]-, perchlorate), a lipophilic green fluorescent dye widely used to label lipophilic regions. Ratio between all lipidic components (i.e., tunable lipid, helper neutral lipid, sterol derivative and PEG-lipid) used with siRNA have been kept (see table 1) with siRNA, the microfluidic formulation procedure remained unchanged. As a basis, a Flow Rate Ratio (FRR) equals to 3 and a total flow rate of 1000 μL/min has been set up. Collected LNPs are subsequently dialyzed against PBS+5% w/v sucrose overnight, filtered on a sterile 0.45 μm filter and stored at -20°C.

Thus, LNPs based on tunable lipids **XI** and **XIV** have been formulated physico-chemical properties determined accordingly (Table 5).

**Table 6: Main physical properties of siRNA-based LNPs based on tunable lipids XI and XIV**

| Tunable Lipid | XI | XI | XIV | XIV |
|---|---|---|---|---|
| Total Lipid concentration | 4.74 mM | 4.74 mM | 4.74 mM | 4.74 mM |
| siRNA | NTsiRNA-Cy5 | NTsiRNA-Cy5 | NTsiRNA-Cy5 | NTsiRNA-Cy5 |
| Size (PDI) | 116 nm (0.091) | 99 nm (0.073) | 114 nm (0.222) | 106 nm (0.198) |
| Zeta | +18.2 mV | +19.9 mV | +27 mV | -2.46 mV |

(continued)

| Tunable Lipid | XI | XI | XIV | XIV |
|---|---|---|---|---|
| Loaded siRNA (ng/μL) | 0.125 | 0.125 | 0.125 | 0.125 |
| DiO Label (Y/N) | N | Y | N | Y |
| N/P Ratio | 6 | 6 | 6 | 6 |
| Encapsulation Efficiency | >90% | >90% | >90% | >90% |

Results displayed on the table 6 clearly highlights the propensity of tunable lipids **XI** and **XIV** to efficiently encapsulate siRNA into LNPs. Encapsulation rates of siRNA proved to be higher than 90% whatever the lipid used, whereas the size of the LNP is in all cases is maintained around 100 nm. However, polydispersity indexes observed when using lipid **XI** are better than with lipid **XIV,** with impressive values stable below 0.1. Insertion of DiO dye does not affect the morphology neither the physical properties nor integrity of the LNPs.

*In-vitro uptake of fluorescently labelled siRNA formulated in lipid nanoparticles.*

The siRNA-based LNPs formulated above, have been used in an uptake study using confocal fluorescence microscopy. As already described before, the nanoparticules benefit from a double fluorescent-labelling on both the lipids used in the LNPs, and on the siRNA; that allow us to monitor uptake of the polynucleotide after exposure onto cells, as well as the potential internalization of the particles. Indeed, internalization is a crucial factor to control when envisioning extending the use of LNPs into a pre-clinical or clinical setting.

According, to the results displayed on table 6, LNPs based on tunable lipid **XI** have been selected for their improved physical properties. Five microliters of LNP have been put in contact of HEK-293 cells, before 4 hours of incubation. Pictures have then taken using a confocal microscope after nucleus coloration using Hoechst dye. These pictures are presented in figure 55.

On figure 55 (picture left), DiO-labelled particles are easily observed inside of the cytoplasm of cells, proving efficient internalization of the LNPs. The figure 55 (picture right) on the other hand, reveals the presence of Cy5-siRNA inside the cytoplasm that demonstrates efficient siRNA uptake after 4h exposure of the formulated LNPs onto cells. These observations univocally prove the potency of tunable-based LNPs to act as efficient siRNA delivery platform able to protect and deliver efficiently siRNA inside of the cell's cytoplasm.

*Encapsulation of olligonucleotides in lipid nanoparticles using microfluidic formulation;*

Oligonucleotides (ODN) are short DNA or RNA molecules, oligomers that have a wide range of applications in genetic testing, research, and forensics. For instance, aptamers, and antisense olligonucleotides are at the basis of many innovative therapeutic strategies due to their easy availability and their ability to target and modify very specific sequences of nucleic acid with high selectivity. A straightforward uptake, like for most small-molecule drugs, is hindered by the polyanionic backbone and the molecular size of ONs. Conversely, LNPs represent an interesting possibility as delivery platform able to protect and deliver efficiently single or double stranded olligonucleotides.

Thus, several lipids of to this invention have been selected to encapsulate a pool of four RNA ODNs into lipid nanoparticles. After a screening, lipid **XI** has been selected as basis tunable compound for formulations. The same microfluidic used for encapsulation of mRNA has been translated to ODN applications. 4 different RNA-ODN have been used for this encapsulation study, each formulated with lipid **XI.** The microfluidic formulation procedure remained unchanged. As a basis, a Flow Rate Ratio (FRR) equals to 3 and a total flow rate of 1000 μL/min has been set up. Collected LNPs are subsequently dialyzed against PBS+5% w/v sucrose overnight, filtered on a sterile 0.45 μm filter and stored at -80°C. Results of physico-chemical properties of the different LNPs are displayed on the table 7 below.

**Table 7: Main physical properties of ODNs-based LNPs based on tunable lipids XI**

| LNPs Characterization | ODN1-LNP-XI | ODN2-LNP-XI | ODN3-LNP-XI | ODN4-LNP-XI |
|---|---|---|---|---|
| Total Lipid concentration | 3 mM | 3 mM | 3 mM | 3 mM |
| Size (PDI) | 87.8 nm (0.043) | 89.7 nm (0.054) | 96.2 nm (0.135) | 106.7 nm (0.119) |
| Zeta | -1.39 mV | -5.67 mV | 3.56 mV | -3.74 mV |
| Loaded RNA (ng/μL) | 0.125 | 0.125 | 0.125 | 0.125 |
| N/P Ratio | 4 | 4 | 4 | 4 |

(continued)

| LNPs Characterization | ODN1-LNP-XI | ODN2-LNP-XI | ODN3-LNP-XI | ODN4-LNP-XI |
|---|---|---|---|---|
| **Encapsulation Efficiency** | 95% | 97% | 96% | 94% |

Results presented in table 7 highlight the potency of lipid **XI** to encapsulate various ODNs with superior efficiency. At a N/P of 4, all LNPs present a average size around or below 100 nm with controlled polydispersity close form 0.1 in all cases. Meanwhile, encapsulation rates are clearly in the upper range, with efficiencies in encapsulation close in all cases from 95%. Taken together these results validated the choice of lipid **XI** as basis for LNPs able to encapsulate efficiently ODNs. This invention offers thus to researchers to use an efficient encapsulation of ODNs of therapeutic interest.

## Claims

**1.** Lipid of formula **(I),** or a stereoisomer thereof, or a pharmaceutically acceptable salt thereof:

$$R\text{-}Sp\text{-}Z_s \qquad (I)$$

where:

- **R** comprises one or more branched or linear, unsaturated or saturated, optionally fluorinated, alkyl chains, comprising 6 to 24 carbon atoms, preferably between 10 and 18 carbon atoms, optionally comprising one or more heteroatoms other than fluorine; or one or more cyclic or polycyclic groups known to be lipophilic, optionally comprising one or more heteroatoms, such as a steroid group optionally comprising one or more heteroatoms, a polyaromatic group optionally comprising one or more heteroatoms, or an alkaloid derivative group optionally comprising one or more heteroatoms; or a natural or synthetic lipid optionally comprising one or more heteroatoms; or a combination thereof;
- **Sp** comprises one or more branched or linear, unsaturated or saturated, optionally fluorinated alkyl chains, comprising 6 to 24 carbon atoms, preferably between 10 and 18 carbon atoms, optionally comprising one or more heteroatoms and/or bioreducible bonds; and
- **$Z_s$** comprises one or more branched or linear, unsaturated or saturated, optionally fluorinated alkyl chains, comprising 2 to 36 carbon atoms, preferably comprising 2 to 24 carbon atoms, optionally comprising one or more heteroatoms other than fluorine, and/or bioreducible bonds, covalently linked to one or more linear or cyclic nitrogen-based chemical moieties;

**characterized in that**:

• **R** corresponds to formula **(II)**:

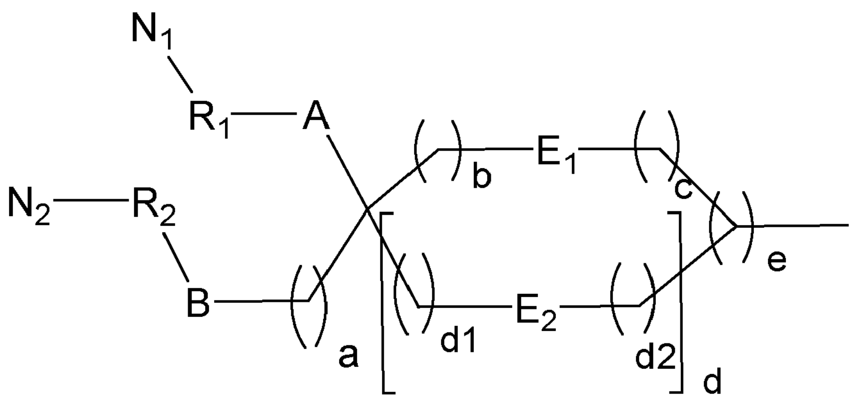

**(II)**

where:

- **$N_1$ and $N_2$**, identical or different represent a linear, branched saturated or unsaturated, hydrocarbon group comprising from 1 to 8 carbon atoms, preferably from 1 to 4, used to terminate carbon chains, preferably **$N_1$ and**

**$N_2$**, identical or different, are either a methyl, ethyl, *n*-propyl, *i*-propyl, *n*-butyl, *s*-butyl, *i*-butyl or *t*-butyl group;
- **$R_1$ and $R_2$**, identical or different, represent a linear, branched and/or cyclic, saturated or unsaturated, hydrocarbon group comprising from 6 to 24 carbon atoms, preferably from 10 to 18 and optionally comprising one or more heteroatoms;
- **A and B**, identical or different, represent a O-C(O), C(O) NH-, -NHCO-, -NH- or O-group;
- **a** is an integer comprised between 1 and 4, preferably **a** is an integer equal to 1, 2 or 3;
- **b** is an integer comprised between 0 and 6, preferably **b** is an integer equal to 0 or 1;
- **$E_1$ and $E_2$**, identical or different, represent a -C(O)O-, -C(O)-NH-, -NH-, -O- or -S-group;
- **c** is an integer comprised between 0 and 2;
- **d** is an integer equal to 0 or 1;
- **$d_1$** is an integer comprised between 0 and 6, preferably **$d_1$** is an integer equal to 0 or 1;
- **$d_2$** is an integer comprised between 0 and 6, preferably **$d_2$** is an integer comprised between 0 and 2; and
- **e** is an integer comprised between 0 and 6, preferably between 0 and 2, and more preferably between 0 and 1;

• **Sp** corresponds to the general formula (III):

$$-\left(Gr\right)_m\left(RAc\right)_n- \quad \textbf{(III)}$$

where:

- **Gr** represents a linear or branched hydrocarbon group, comprising from 1 to 15 carbon atoms, preferably from 1 to 8, and optionally comprising one or more heteroatoms;
- **m** is an integer equal to 0 or 1;
- **RAc** represents an amino acid radical; and
- **n** is an integer equal to 0 or 1; and

• **Zs** corresponds to formula **(IV)**:

$$\left[\left(S_1-D-S_2\right)_p\left(Q\right)_q\left(Z\right)_r\right]_s \quad \textbf{(IV)}$$

where:

- **$S_1$ and $S_2$**, identical or different, represent a linear or branched hydrocarbon group comprising from 1 to 15 carbon atoms, preferably from 1 to 6 carbon atoms, optionally comprising one or more heteroatoms preferably chosen from nitrogen, oxygen, sulfur, bromine, iodine, chlorine and fluorine, more preferably from nitrogen, sulphur, bromine, iodine, chlorine and fluorine;
- **D** represents one or more vinyl ether groups, one or more acylhydrazone groups, a disulfide bond, an ester bond or one or more photosensitive groups;
- **p** is an integer equal to 0 or 1;
- **Q** is a branched hydrocarbon group comprising from 1 to 20 carbon atoms, preferably from 1 to 12 carbon atoms, and one or more heteroatoms, and which can optionally be covalently coupled with the **$S_2$** or **RAc** or **Gr** or **R** group on the one hand, and to at least two **Q** and/or **Z** groups on the other hand;
- **q** is an integer comprised between 0 and 8, preferably between 0 and 3;
- **Z** is a nitrogen (N) centred terminal ionizable moiety, preferably Z represents a tertiary aliphatic amine such as N,N-dialkylamine, or a cyclic amine moiety such as N-substituted Pyrrolidine, N-substituted piperidine, N-substituted morpholine, N-substituted piperazine or N-substituted pyridine;
- **r** is an integer comprised between 1 and 16, preferably between 1 and 8, it being preferably understood that if **q** is equal to 1 then **r** is at least equal to 2 and that if **r** is greater than **1,** then the **Z** groups can be identical or different; and
- **s** is an integer equal to 1 or 2.

**2.** The lipid of formula (I) according to claim 1, wherein the following compounds are excluded from general formula (I):

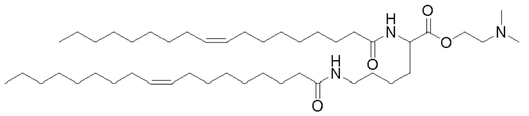 ; 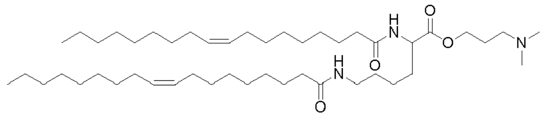 ;

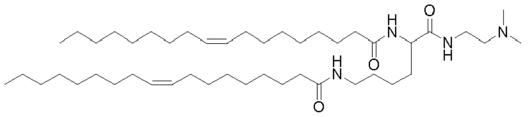 and 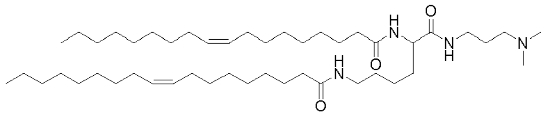 .

**3.** The lipid of formula (I) according to claim 1 or 2, wherein Z corresponds to formula **(V)**:

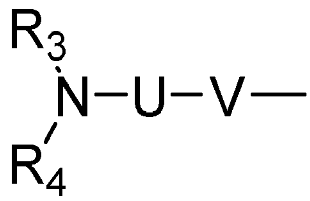

**(V)**

wherein:

- **$R_3$ and $R_4$** are either the same or different and selected from $C_1$-$C_6$ alkyl, $C_2$-$C_6$ alkenyl, or $C_2$-$C_6$ alkynyl, optionally substituted by one or more methyl, ethyl, n-propyl, i-propyl, n-butyl, s-butyl, i-butyl or t-butyl group(s), or **$R_3$ and $R_4$** may join to form an optionally substituted by one or more methyl, ethyl, n-propyl, i-propyl, s-butyl, i-butyl or t-butyl group(s) heterocyclic ring such as pyrrol, pyrrolidine, pyridine, piperazine, morpholine, piperidine or indoline optionally substituted by one or more methyl, ethyl, n-propyl, i-propyl, s-butyl, i-butyl or t-butyl group(s);
- **N** is a Nitrogen;
- **V** is O, S, N(**$R_6$**), C(O), C(O)O, OC(O), C(O)N(**$R_6$**), N(**$R_6$**)C(O), OC(O)N(**$R_6$**), N(**$R_6$**)C(O)O, C(O)S, C(S)O, S(O), S(O)(O), C(S), or an optionally substituted heterocyclic ring such as a pyrrole, pyrrolidine, pyridine, piperazine, morpholine, piperidine or indoline, optionally substituted by one or more methyl, ethyl, n-propyl, i-propyl, n-butyl, s-butyl, i-butyl or t-butyl group(s), wherein **$R_6$** is either an hydrogen (H) or a $C_1$-$C_{10}$ alkyl, $C_2$-$C_{10}$ alkenyl, or $C_2$-$C_{10}$ alkynyl, optionally substituted by one or more methyl, ethyl, n-propyl, i-propyl, n-butyl, s-butyl, i-butyl or t-butyl group(s); preferably **$R_6$** is selected from the group consisting of hydrogen (H), a $C_1$ alkyl (methyl) group, and a **$C_2$**, **$C_3$**, **$C_4$**, **$C_5$**, **$C_6$**, **$C_7$**, **$C_8$**, **$C_9$**, and **$C_{10}$** alkyl, alkenyl, and alkynyl group, incorporating or not one or more heteroatoms, such as -O, -NH,-S and mixtures of thereof; and
- **U** is either absent or is a $C_1$-$C_{12}$ alkyl, $C_2$-$C_{12}$ alkenyl, or $C_2$-$C_{12}$ alkynyl, optionally substituted by one or more methyl, ethyl, n-propyl, i-propyl, n-butyl, s-butyl, i-butyl or t-butyl group(s).

**4.** The lipid of formula (I) according to anyone of claims 1 to 3, wherein:

• **$R_1$** and **$R_2$** are different and their structures are represented by formulae **(VI a)** and **(VI b)** respectively:

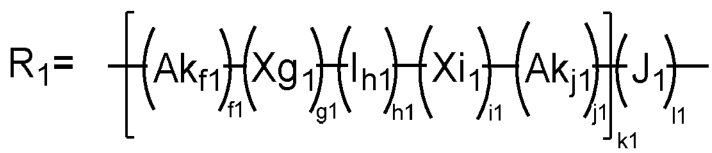

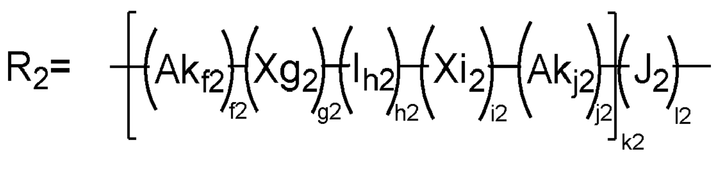

**(VI a) and (VI b)**

where:

- **$Ak_{f1}$**, **$Ak_{j1}$**, **$Ak_{f2}$**, **$Ak_{j2}$** identical or different, represent a linear or branched hydrocarbon group comprising from 1 to 22 carbon atoms, preferably from 1 to 12 carbon atoms, optionally comprising one or more heteroatoms;
- **f1** and **f2** are integers equal to 0 or 1;
- **j1** and **j2** are integers equal to 0 or 1;
- **$Xg_1$ ,$Xi_1$, $Xg_2$ and $Xi_2$**, identical or different are selected from O, S, N(**$R_7$**), C(O), C(O)O, OC(O), C(O)N(**$R_7$**), N(**$R_7$**)C(O), OC(O)N(**$R_7$**), N(**$R_7$**)C(O)O, C(O)S, C(S)O, S(O), S(O)(O) and C(S), wherein **$R_7$** is either an hydrogen (H) or a $C_1$-$C_{10}$ alkyl, $C_2$-$C_{10}$ alkenyl, or $C_2$-$C_{10}$ alkynyl, optionally substituted by one or more methyl, ethyl, n-propyl, i-propyl, n-butyl, s-butyl, i-butyl or t-butyl group(s); more preferably **$R_7$** is selected from the group consisting of hydrogen (H), a $C_1$ alkyl (methyl) group, and a $C_2$, $C_3$, $C_4$, $C_5$, $C_6$, $C_7$, $C_8$, $C_9$, and $C_{10}$ alkyl, alkenyl, and alkynyl group, optionally comprising one or more heteroatoms, such as -O-, NH-, S and mixtures of thereof; preferably, it is likely understood that if **$Xg_1$**, **$Xi_1$**, **$Xg_2$** and **$Xi_2$** happened to be different in nature while being independently represented by N(**$R_7$**), C(O)N(**$R_7$**), N(**$R_7$**)C(O), OC(O)N(**$R_7$**), N(**$R_7$**)C(O)O, then **$R_7$** might be of several different nature among the possibilities depicted above within the same molecule;
- **g1** and **g2** are integers equal to 0 or 1;
- **i1** and **i2** are integers equal to 0 or 1;
- **$I_{h1}$** and **$I_{h2}$**, identical or different, both represent a linear or branched non-saturated hydrocarbon chains comprising from 2 to 16 carbon atoms, preferably from 2 to 8 carbon atoms and at least one unsaturation, more preferably $I_{h1}$ and $I_{h2}$, identical or different are independently chosen from:

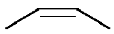 ; 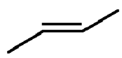 ; 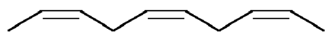 ; 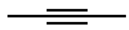 ; 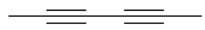 ;

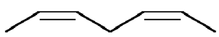 and 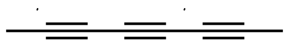 .

- **h1** and **h2** are integers equal to 0 or 1;
- **$J_1$** and **$J_2$** are branched hydrocarbon groups comprising from 1 to 20 carbon atoms, preferably from 1 to 12, and/or one or more heteroatoms; **$J_1$** can optionally be covalently coupled with either **$Ak_{f1}$**, **$Ak_{j1}$**, or **$Xg_1$**, **$Xi_1$**, or **$I_{h1}$** group on the one hand, and to **A** on the other hand, whereas **$J_2$** can optionally be covalently coupled with either **$Ak_{f2}$**, **$Ak_{j2}$** **$Xg_2$** and **$Xi_2$**, or **$I_{h2}$** on the one hand, and to **B** on the other hand;
- **I1** and **I2** are integers equal to 0 or 1; it being preferably understood that if **I1** is equal to 0, then either **$Ak_{f1}$**, **$Ak_{j1}$**, or**$Xg_1$**, **$Xi_i$**, or **$I_{h1}$** group is covalently attached to **A**; similarly, if **I2** is equal to 0, then either **$Ak_{f2}$** **$Ak_{j2}$** **$Xg_2$** and **$Xi_2$**, or **$I_{h2}$** group is covalently attached to **B**; and
- **k1** and **k2** are integers comprised between 1 or 3, it being preferably understood that if **I1** is equal to 1 then **k1** is at least equal to 2; similarly, if **I2** is equal to 1 then **k2** is at least equal to 2; or

• **$R_1$** and **$R_2$** are identical and their structure is represented by formula (VII):

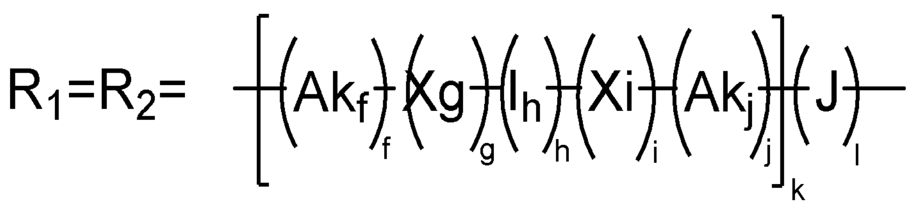

**(VII)**

where:

- **$Ak_f$** and **$Ak_j$**, identical or different, represent a linear or branched hydrocarbon group comprising from 1 to 22 carbon atoms, preferably from 1 to 12 carbon atoms, optionally comprising one or more heteroatoms;
- **f** is an integer equal to 0 or 1;
- **j** is an integer equal to 0 or 1;
- **Xg** and **Xi**, identical or different are selected from O, S, N(**$R_7$**), C(O), C(O)O, OC(O), C(O)N(**$R_7$**), N(**$R_7$**)C(O), OC(O)N(**$R_7$**), N(**$R_7$**)C(O)O, C(O)S, C(S)O, S(O), S(O)(O) and C(S), wherein **$R_7$** is either an hydrogen (H) or a $C_1$-$C_{10}$ alkyl, $C_2$-$C_{10}$ alkenyl, or $C_2$-$C_{10}$ alkynyl, optionally substituted by one or more methyl, ethyl, n-propyl, i-propyl, n-butyl, s-butyl, i-butyl or t-butyl group(s); more preferably **$R_7$** is selected from the group consisting of hydrogen (H), a **$C_1$** alkyl (methyl) group, and a $C_2$, $C_3$, $C_4$, $C_5$, $C_6$, $C_7$, $C_8$, $C_9$, and $C_{10}$ alkyl, alkenyl, and

alkynyl group, optionally comprising one or more heteroatoms, such as -O-, NH-, S and mixtures of thereof;
- **g** is an integer equal to 0 or 1;
- **i** is an integer equal to 0 or 1;
- $\mathbf{I_h}$ represent a linear or branched non-saturated hydrocarbon chain comprising from 2 to 16 carbon atoms, preferably from 2 to 8 carbon atoms and at least one unsaturation, more preferably $\mathbf{I_h}$ is selected from:

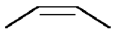 ; 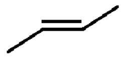 ; 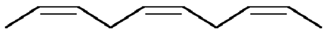 ; 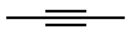 ;

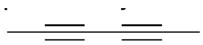 ; 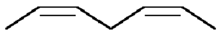 and 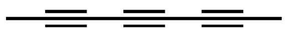 .

- **h** is an integer equal to 0 or 1;
- **J** is a branched hydrocarbon groups comprising from 1 to 20 carbon atoms, preferably from 1 to 12, and/or one or more heteroatoms; **J** can optionally be covalently coupled with the $\mathbf{Ak_j}$ or **Xi** or $\mathbf{I_h}$ or **Xg** or $\mathbf{Ak_f}$ group on the one hand, and to **A** or **B** on the other hand;
- **l** is an integer equal to 0 or 1; it being preferably understood that if **l** is equal to 0, then either $\mathbf{Ak_j}$ or **Xi** or $\mathbf{I_h}$ or **Xg** or $\mathbf{Ak_f}$ is covalently attached to **A** on the one hand and to **B** on the other hand; and
- k is an integer comprised between 1 or 3; it being preferably understood that if **l** is equal to 1 then **k** is at least equal to 2.

**5.** The lipid of formula (I) according to anyone of claims 1 to 4, wherein **R** is selected from:

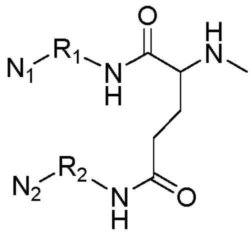 ; 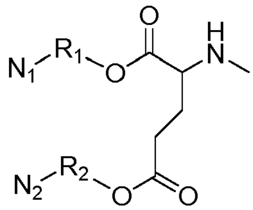 ; 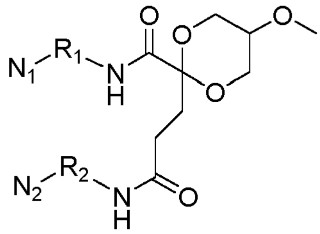 ; 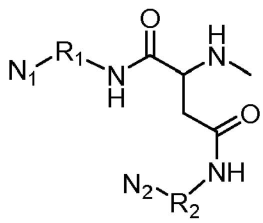 ;

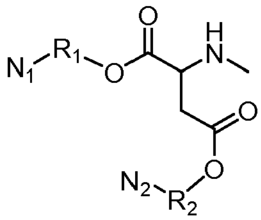 ; 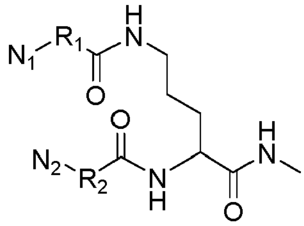 ; 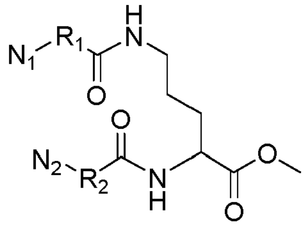 ;

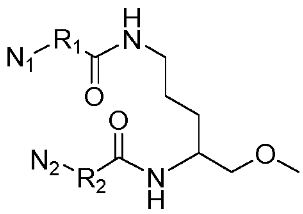 ; 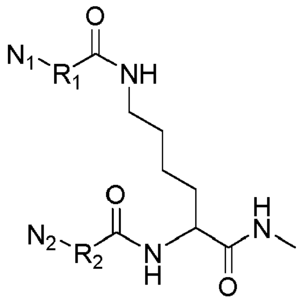 ; 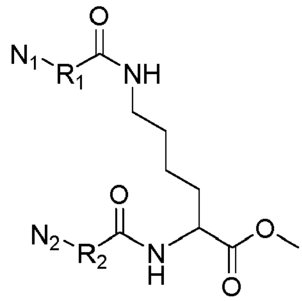 and

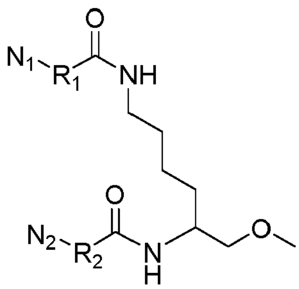 .

**(Formula VIII)**

wherein, $\mathbf{R_1}$, $\mathbf{R_2}$, $\mathbf{N_1}$ and $\mathbf{N_2}$ are defined in the preceding claims.

**6.** The lipid of formula (I) according to anyone of claims 1 to 6, wherein in ***formula (III)***:

- **Gr** is absent or **Gr** serves as a spacer arm and corresponds to the molecular pattern noted as $\mathbf{-W_4\ -Y_4\ -W_4\ -}$, where $\mathbf{Y_4}$ represents a bridging alkylene group comprising from 1 to 8 carbon atoms, preferentially from 1 to 4, whilst $\mathbf{W_4}$ represents a -O-, -CO- or -NH- group; or
- **Gr** corresponds to the formula CO-$\mathbf{Y_4}$-CO, where $\mathbf{Y_4}$ represents a bridging alkylene group comprising from 1 to 8 carbon atoms, preferentially from 1 to 4, and is connected by an amide bond to the lipophilic region **R** on the one hand, and either to the **RAc** radical, or directly to the $\mathbf{S_1}$ or **Q** or **Z** group on the other hand; and

in formula (III) the amino acid radical **RAc** is chosen from aspartic acid, glutamic acid, alanine, arginine, asparagine, cysteine, glutamine, glycine, histidine, isoleucine, leucine, lysine, methionine, ornithine, phenylalanine, praline, serine, threonine, tyrosine, tryptophan and valine.

**7.** The lipid of formula (I) according to anyone of claims 1 to 6, wherein the compounds of ***formula (I)*** are selected from:

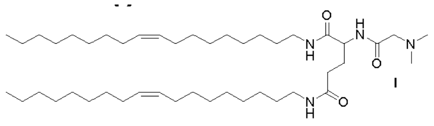
I;

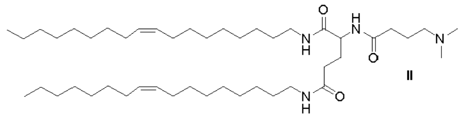
II;

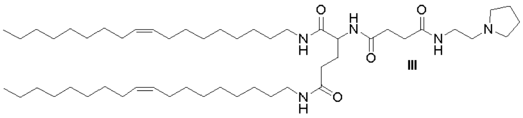
III;

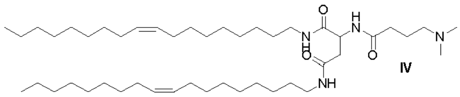
IV;

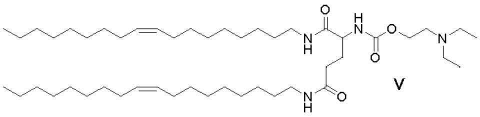
V;

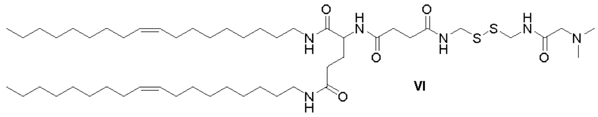
VI;

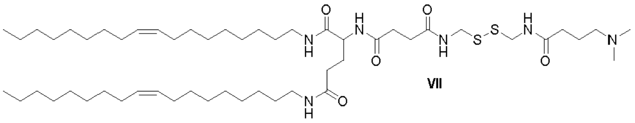
VII;

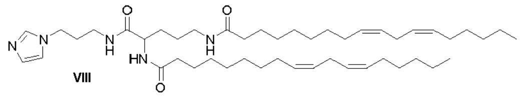
VIII;

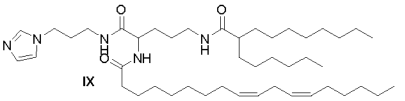
IX;

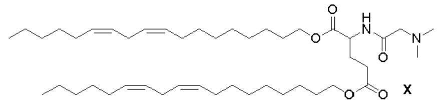
X;

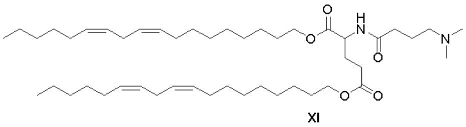
XI;

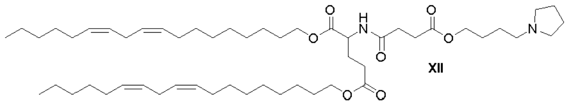
XII;

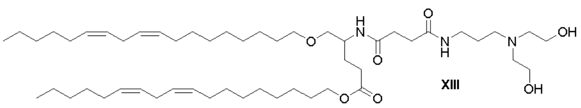
XIII;

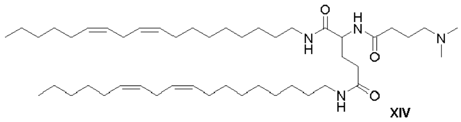
XIV;

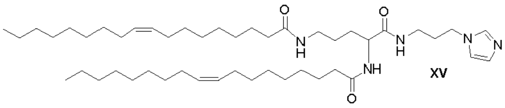
XV;

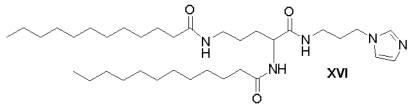
XVI;

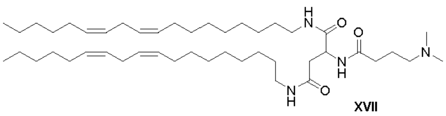
XVII;

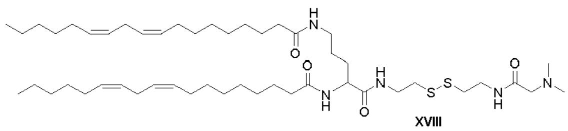
XVIII;

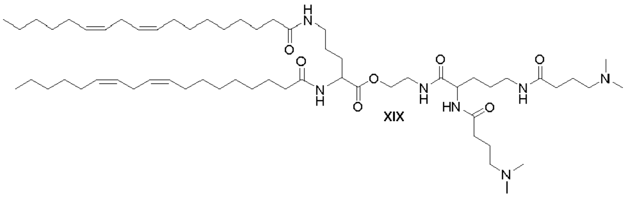
XIX;

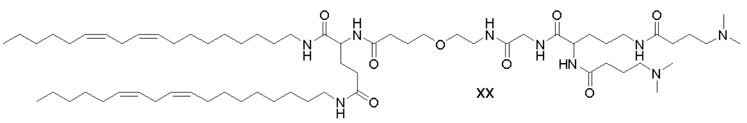
XX;

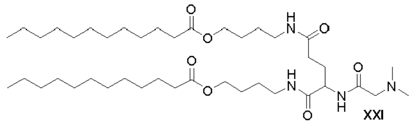
XXI;

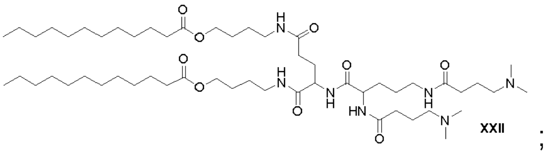
XXII ;

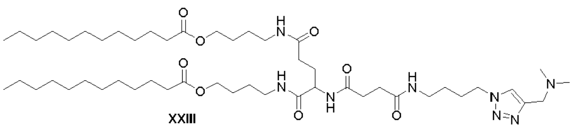
XXIII ;

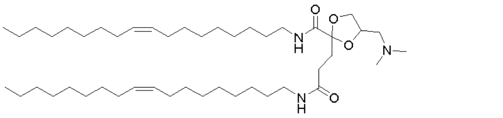
XXIV ;

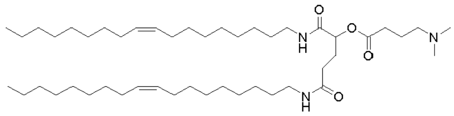
XXV ;

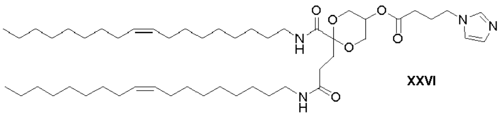
XXVI ;

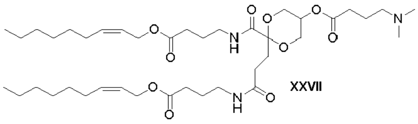
XXVII ;

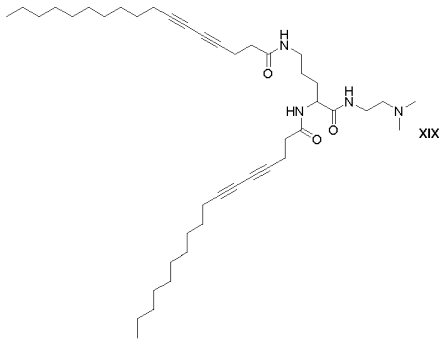
XIX ;

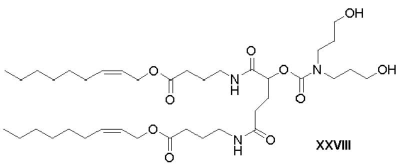
XXVIII ;

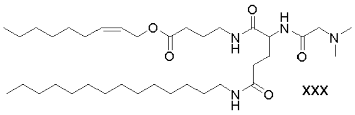
XXX ;

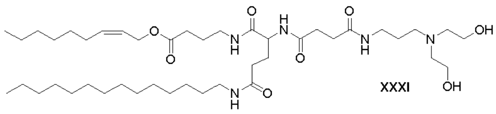
XXXI ;

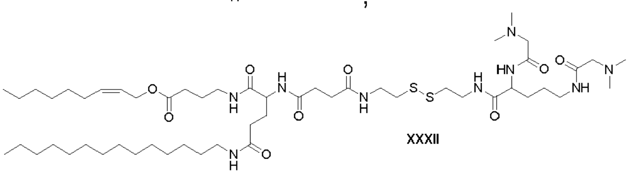
XXXII ;

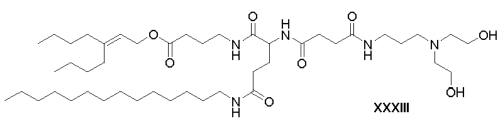
XXXIII ;

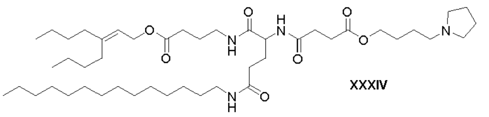
XXXIV ;

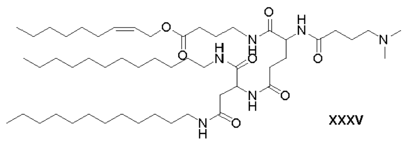
XXXV ;

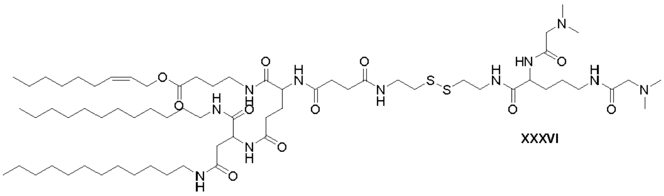
XXXVI ;

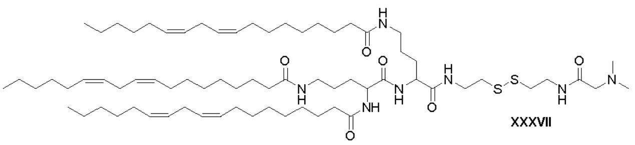
XXXVII ;

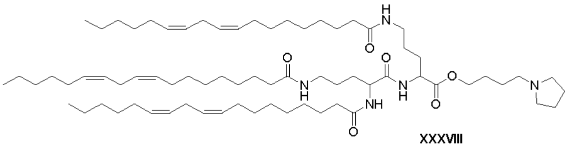
XXXVIII ;

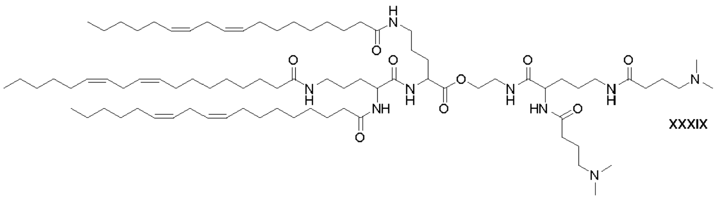

XXXIX

;

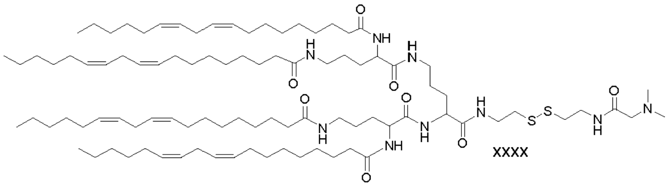

XXXX

;

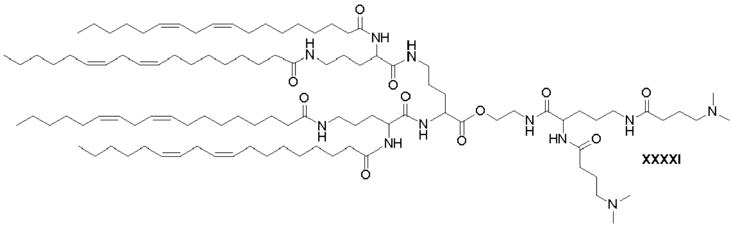

XXXXI

;

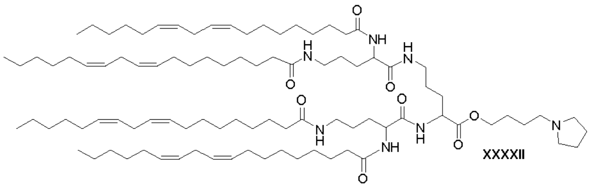

XXXXII

and

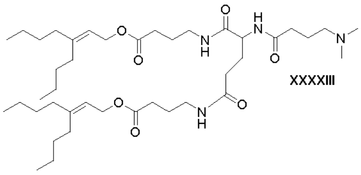

XXXXIII

.

**8.** Composition comprising the lipid of formula (I) as defined in anyone of claims 1 to 7.

**9.** Composition according to claim 8, wherein said composition further comprises one or more active ingredients and optionally:

- one or more neutral lipids, and/or
- one or more components capable of reducing aggregation of said composition, and/or
- one or more sterols or sterol derivatives, and/or
- one or more lipids bearing a targeting ligand.

**10.** Process for manufacturing the lipid of formula (I) as defined in anyone of claims 1 to 7 and/or the composition as defined in claims 8 or 9.

**11.** Lipid of formula (I) as defined in anyone of claims 1 to 7 and/or composition as defined in claims 8 or 9 for its use as a medicament.

**12.** Use of the lipid of formula (I) as defined in anyone of claims 1 to 7 and/or of the composition as defined in claims 8 or 9 as a vector for delivering an active ingredient to subject(s), organ(s), cell(s) and/or tissue(s).

**13.** Method for transfecting cells with a nucleic acid and/or a protein and/or a peptide and/or a polysaccharide and/or a lipid and/or a small organic or inorganic molecule and/or any type of bioactive molecule using the lipid of formula (I) as defined in anyone of claims 1 to 7 and/or the composition as defined in claims 8 or 9.

**14.** Transfected cells obtained by the method as defined in claim 13.

**15.** Kit comprising the lipid of formula (I) as defined in anyone of claims 1 to 7 and/or the composition as defined in claims 8 or 9 further comprising a nucleic acid and/or a protein and/or a peptide and/or a polysaccharide and/or a lipid and/or a small organic or inorganic molecule and/or any type of bioactive molecule, and optionally instructions of use.

## [Figure 1]

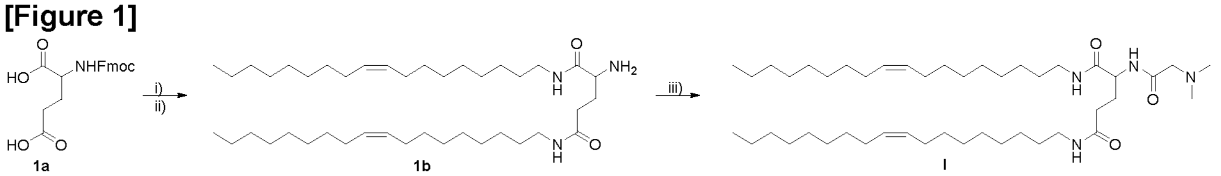


1a 1b I

$C_{41}H_{79}N_3O_2$
MW 646,10 g/mol

$C_{45}H_{86}N_4O_3$
MW 731,21 g/mol

## [Figure 2]

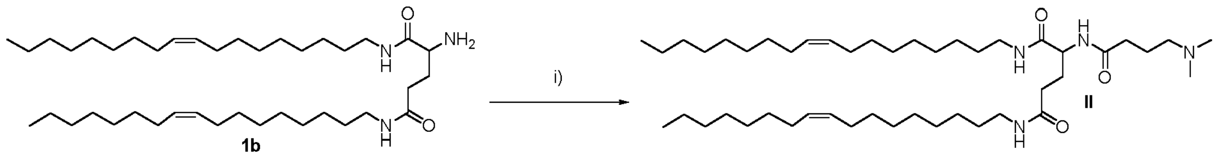


1b II

$C_{46}H_{88}N_4O_3$
745,24 g/mol

## [Figure 3]

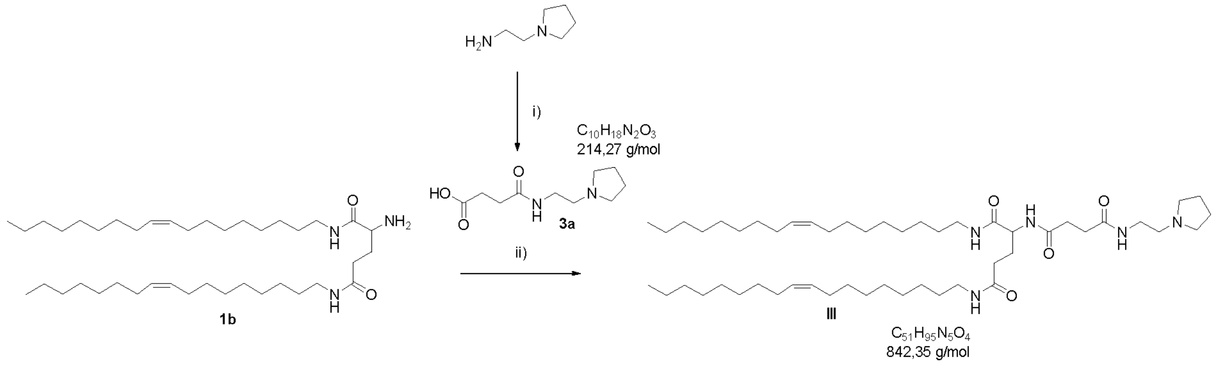


$C_{10}H_{18}N_2O_3$
214,27 g/mol

3a 1b III

$C_{51}H_{95}N_5O_4$
842,35 g/mol

## [Figure 4]

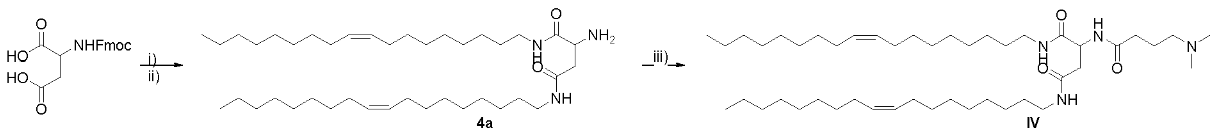


4a IV

$C_{40}H_{77}N_3O_2$
MW 632,08 g/mol

$C_{46}H_{88}N_4O_3$
MW 745,24 g/mol

## [Figure 5]

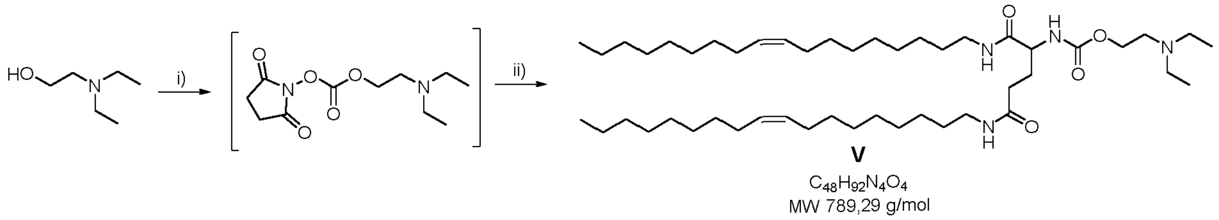


V
$C_{48}H_{92}N_4O_4$
MW 789,29 g/mol

## [Figure 6]

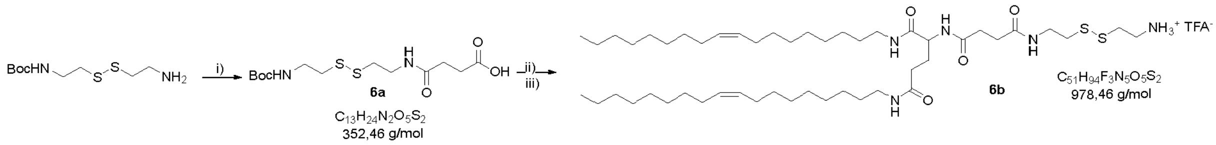


6a
$C_{13}H_{24}N_2O_5S_2$
352,46 g/mol

6b
$C_{51}H_{94}F_3N_5O_5S_2$
978,46 g/mol

## [Figure 7]

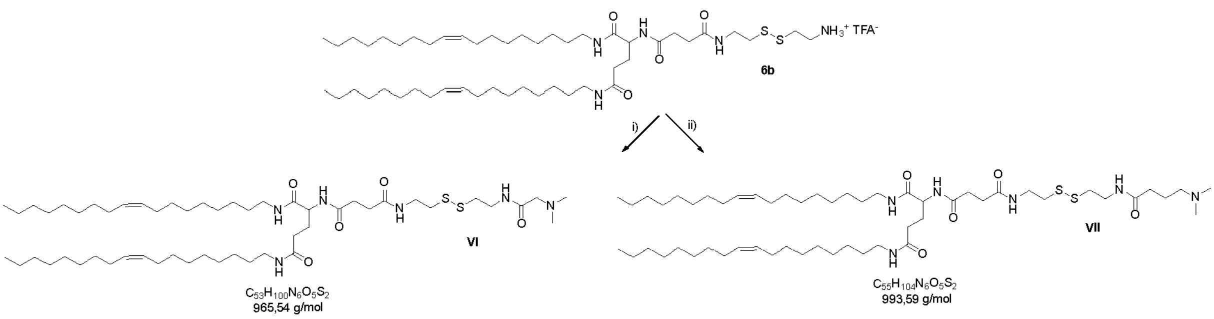


6b

VI
$C_{53}H_{100}N_6O_5S_2$
965,54 g/mol

VII
$C_{55}H_{104}N_6O_5S_2$
993,59 g/mol

## [Figure 8]

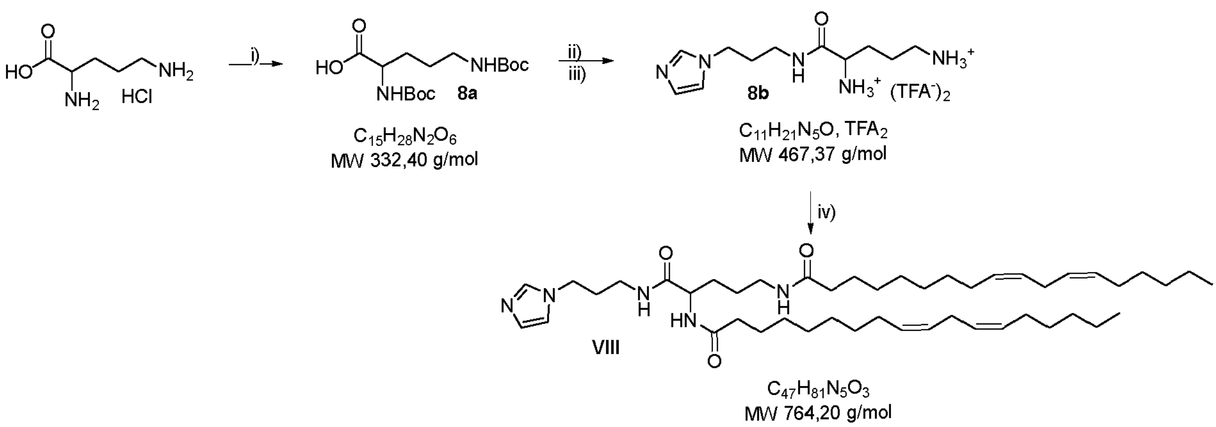


8a
$C_{15}H_{28}N_2O_6$
MW 332,40 g/mol

8b
$C_{11}H_{21}N_5O$, $TFA_2$
MW 467,37 g/mol

VIII
$C_{47}H_{81}N_5O_3$
MW 764,20 g/mol

## [Figure 9]

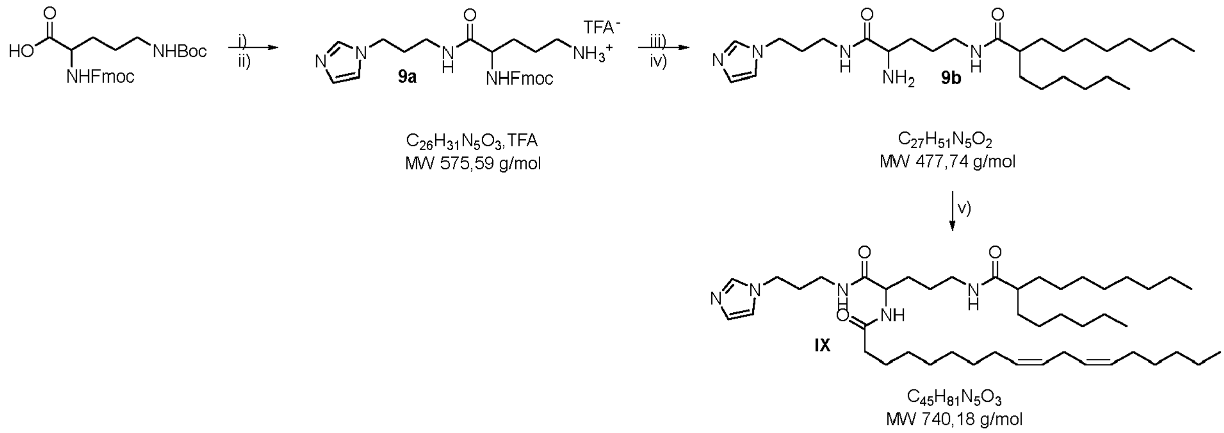


## [Figure 10]

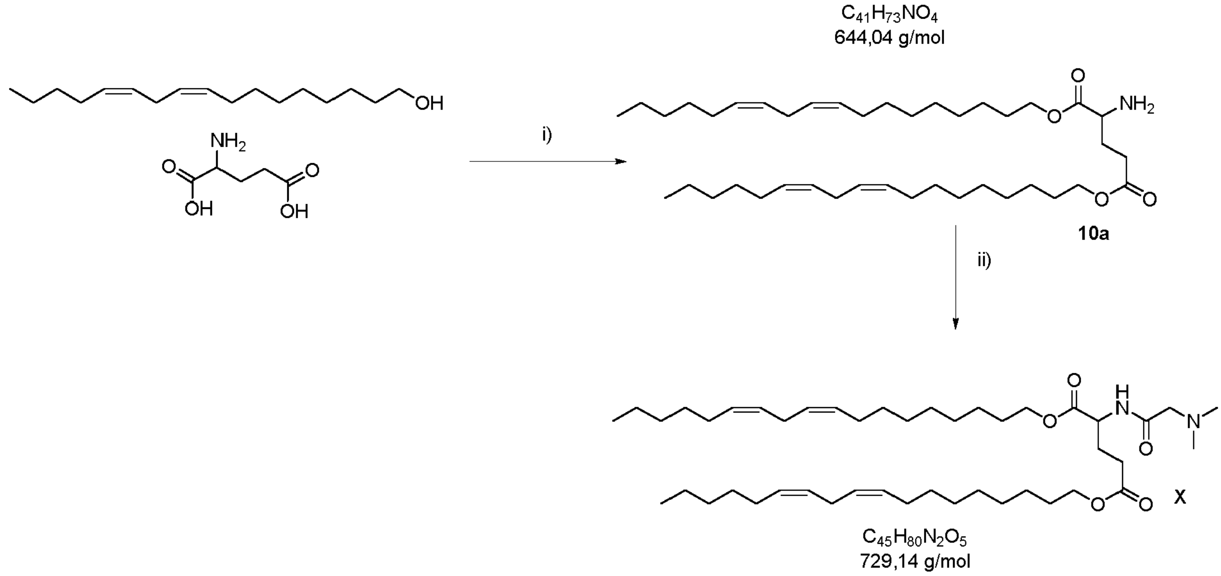


## [Figure 11]

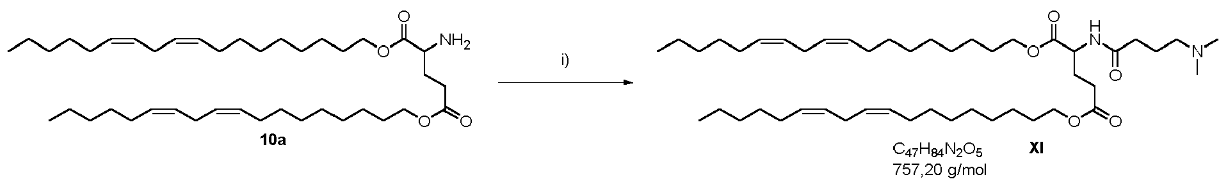

## [Figure 12]

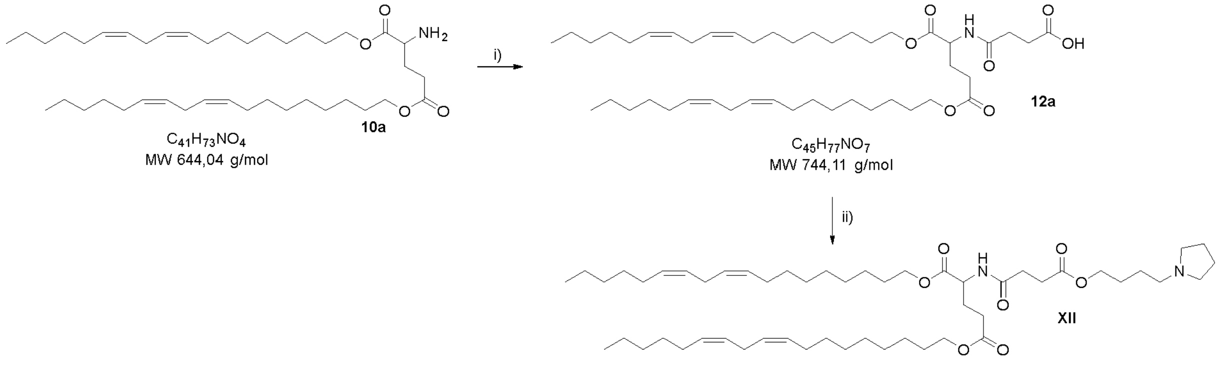


10a
$C_{41}H_{73}NO_4$
MW 644,04 g/mol

12a
$C_{45}H_{77}NO_7$
MW 744,11 g/mol

XII
$C_{53}H_{92}N_2O_7$
MW 869,33 g/mol

## [Figure 13]

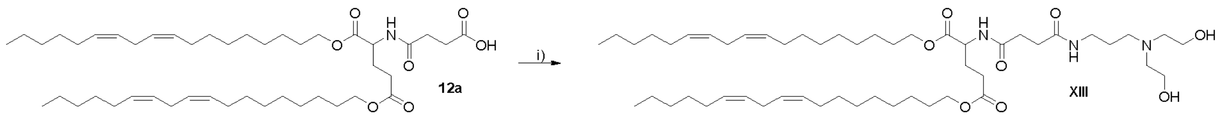


12a
$C_{45}H_{77}NO_7$
MW 744,11 g/mol

XIII
$C_{52}H_{93}N_3O_8$
MW 888,33 g/mol

## *[Figure 14]*

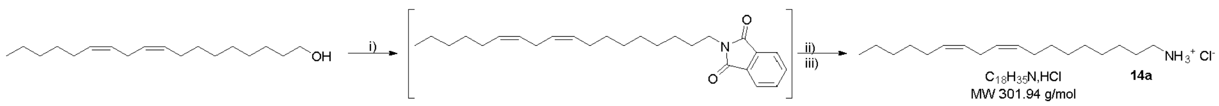


$C_{18}H_{35}N$,HCl
MW 301.94 g/mol
14a

## [Figure 15]

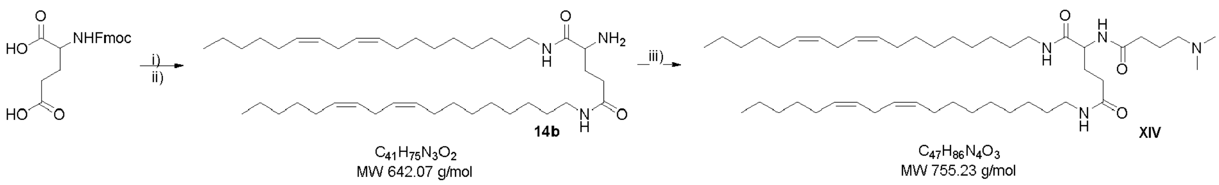


14b
$C_{41}H_{75}N_3O_2$
MW 642.07 g/mol

XIV
$C_{47}H_{86}N_4O_3$
MW 755.23 g/mol

## [Figure 16]

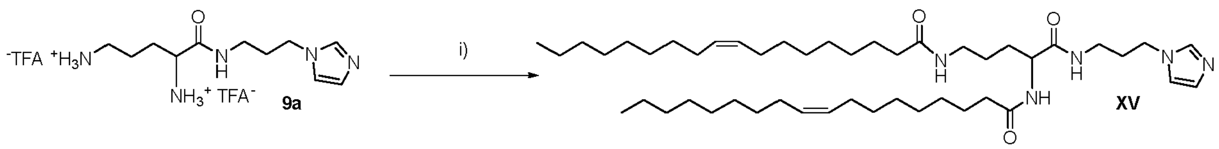


9a

XV
$C_{47}H_{85}N_5O_3$
768,23 g/mol

## [Figure 17]

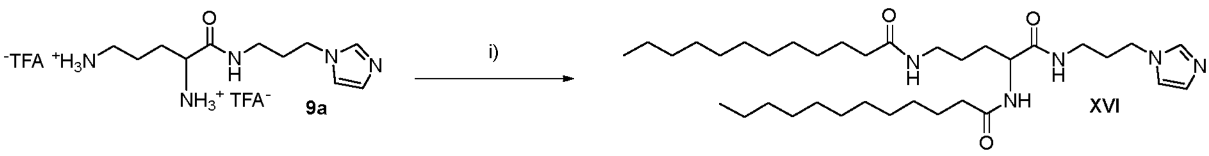


9a

XVI

$C_{35}H_{65}N_5O_3$
603,94 g/mol

## [Figure 18]

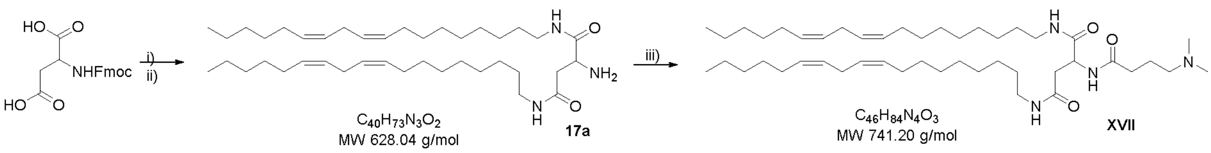


$C_{40}H_{73}N_3O_2$
MW 628.04 g/mol
17a

$C_{46}H_{84}N_4O_3$
MW 741.20 g/mol
XVII

## [Figure 19]

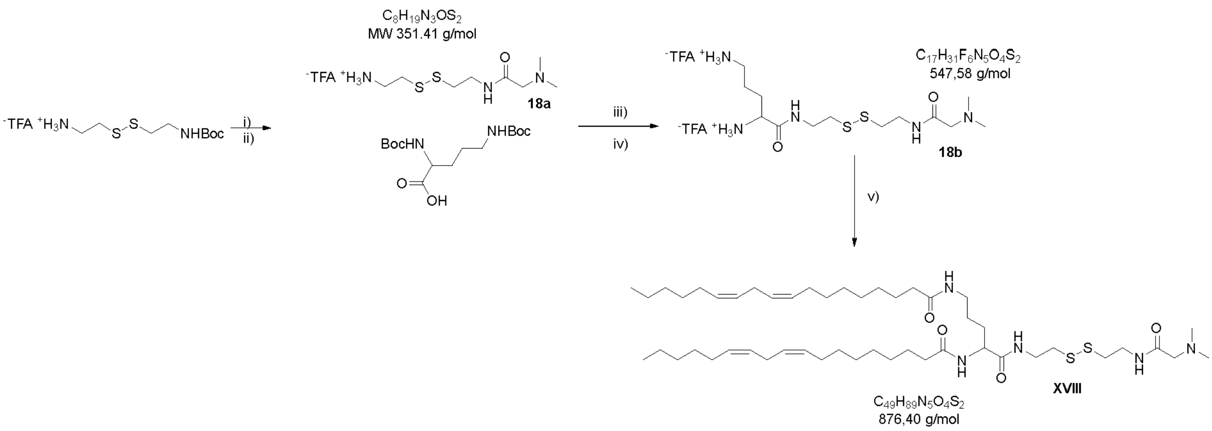


$C_8H_{19}N_3OS_2$
MW 351.41 g/mol
18a

$C_{17}H_{31}F_6N_5O_4S_2$
547,58 g/mol
18b

XVIII
$C_{49}H_{89}N_5O_4S_2$
876,40 g/mol

## [Figure 20]

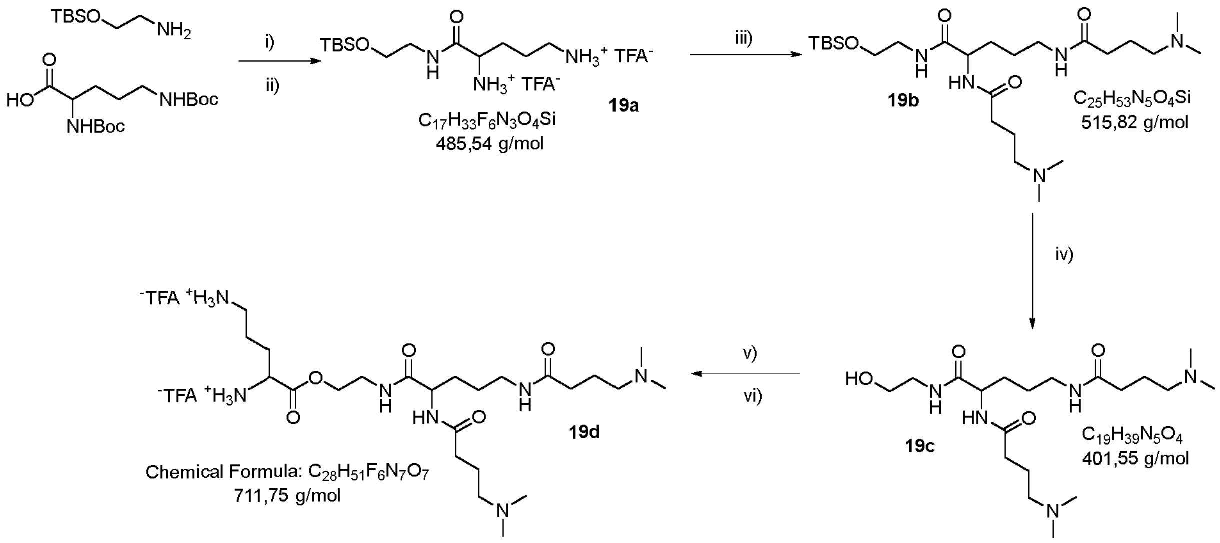


## [Figure 21]

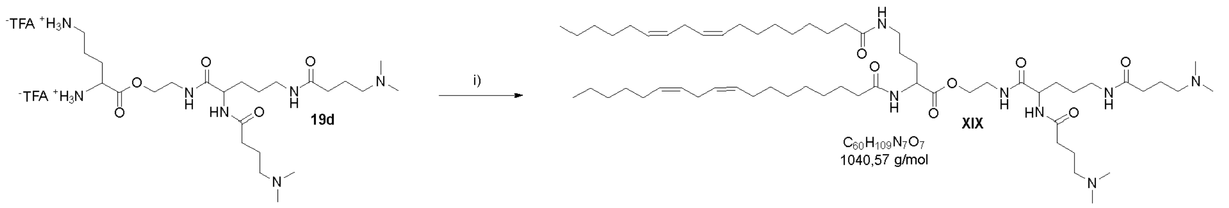


## [Figure 22]

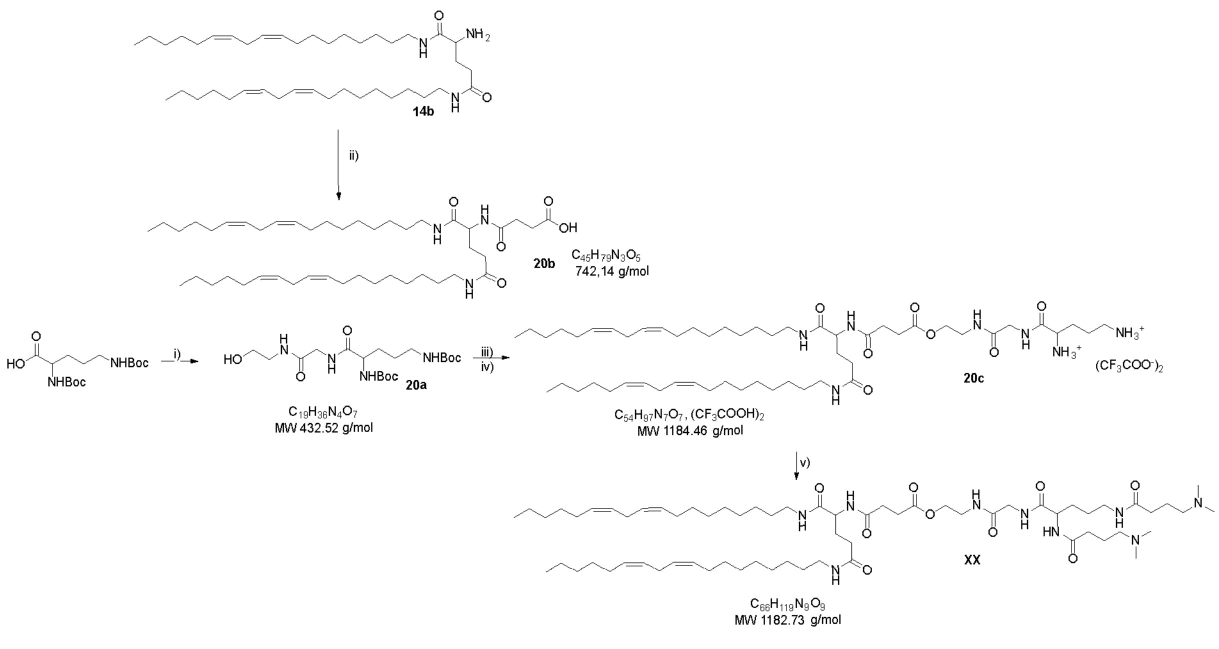

**[Figure 23]**

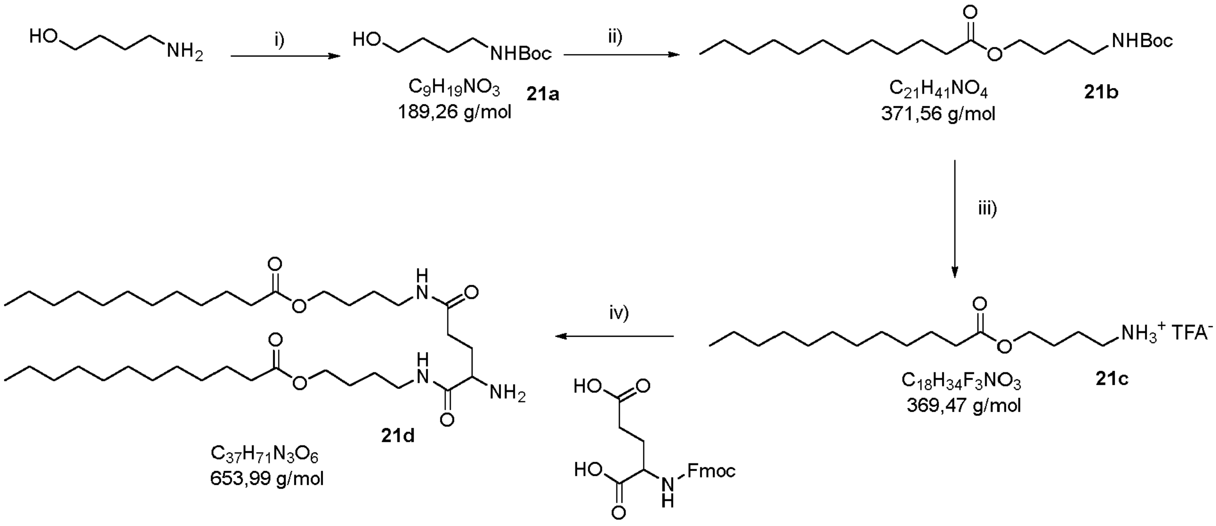


**[Figure 24]**

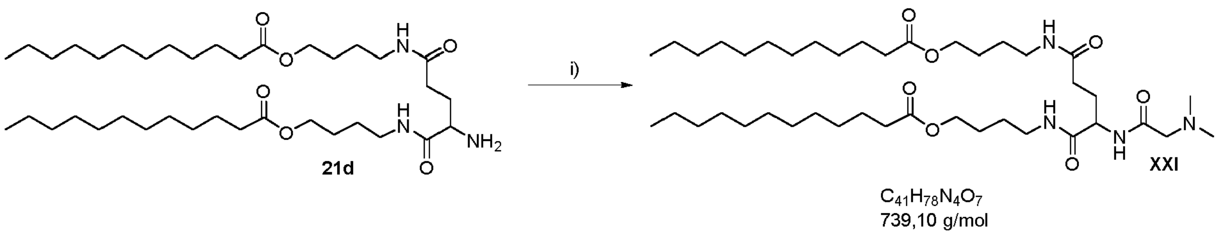


***[Figure 25]***

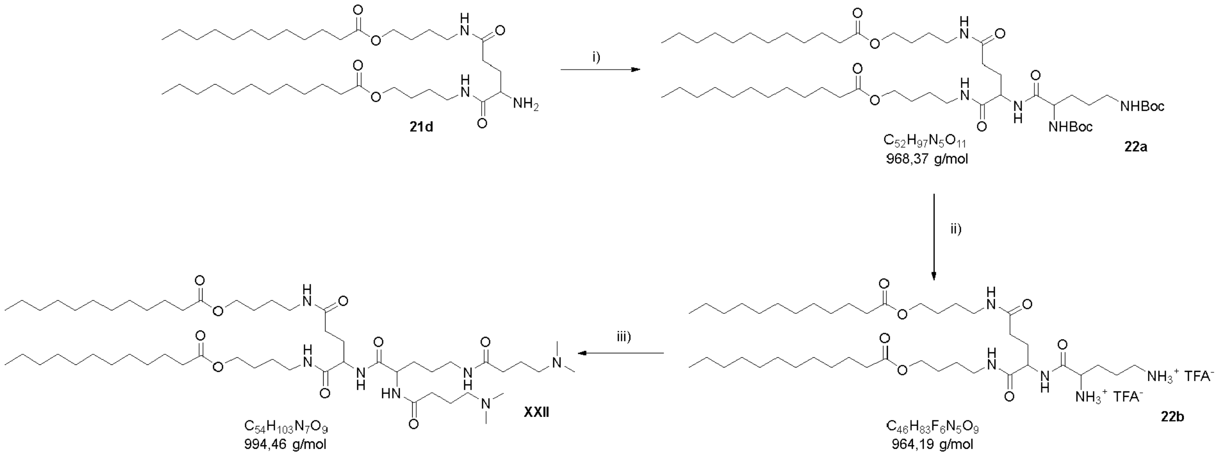

## [Figure 26]

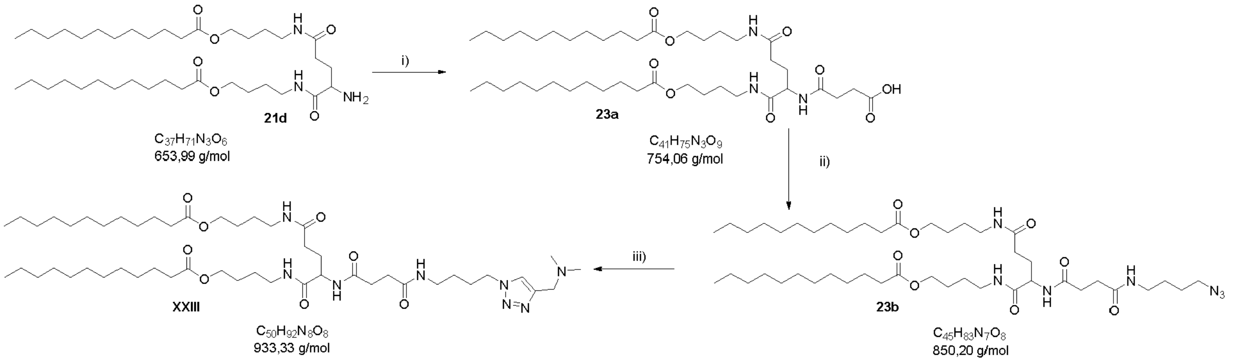

21d
$C_{37}H_{71}N_3O_6$
653,99 g/mol
23a
$C_{41}H_{75}N_3O_9$
754,06 g/mol
23b
$C_{45}H_{83}N_7O_8$
850,20 g/mol
XXIII
$C_{50}H_{92}N_8O_8$
933,33 g/mol

## [Figure 27]

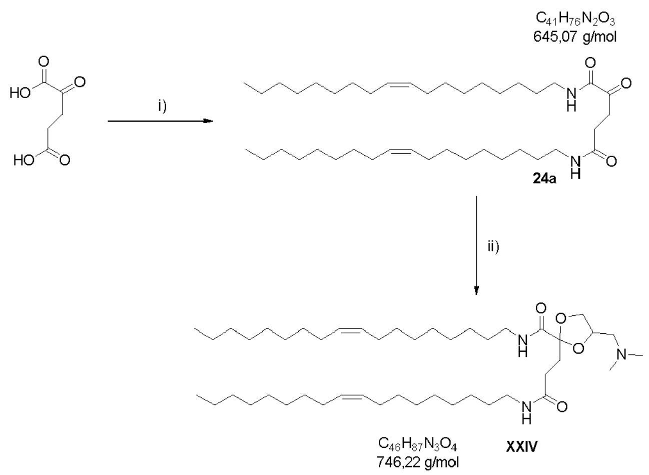

$C_{41}H_{76}N_2O_3$
645,07 g/mol
24a
$C_{46}H_{87}N_3O_4$
746,22 g/mol
XXIV

## [Figure 28]

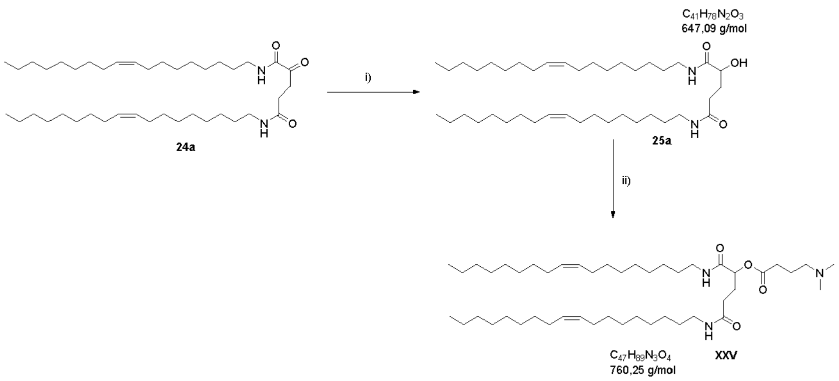

24a
$C_{41}H_{78}N_2O_3$
647,09 g/mol
25a
$C_{47}H_{89}N_3O_4$
760,25 g/mol
XXV

## [Figure 29]

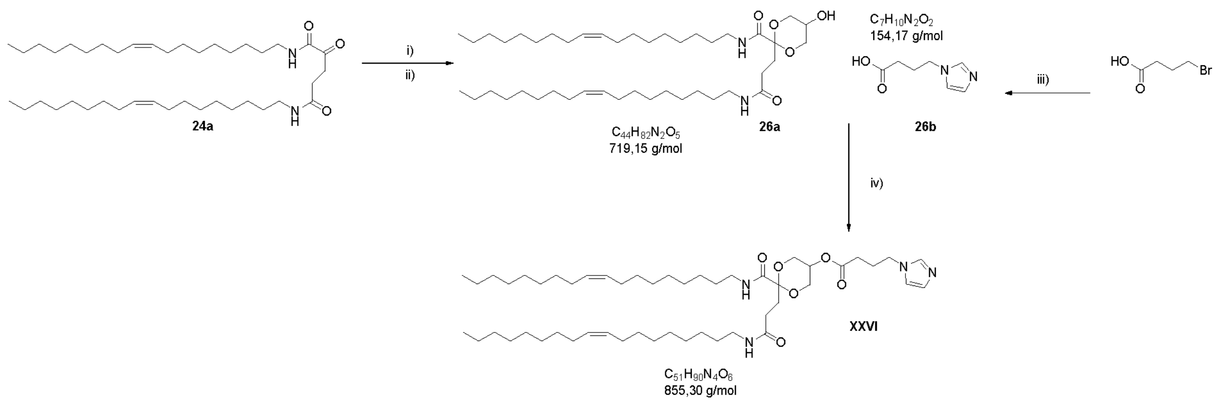


## [Figure 30]

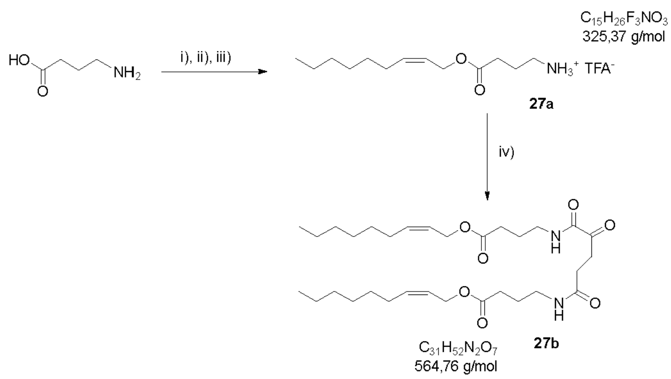


## [Figure 31]

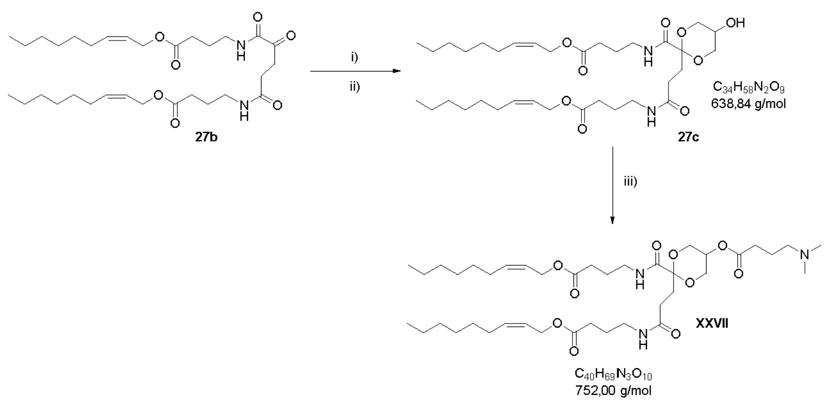

## [Figure 32]

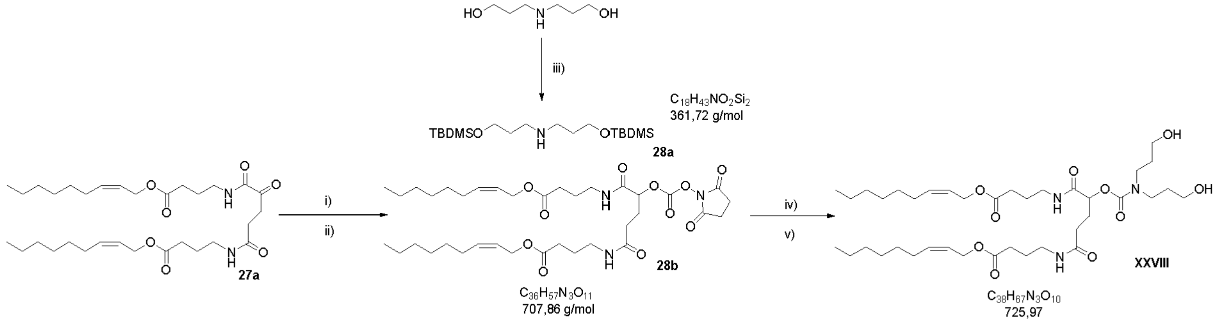


## [Figure 33]

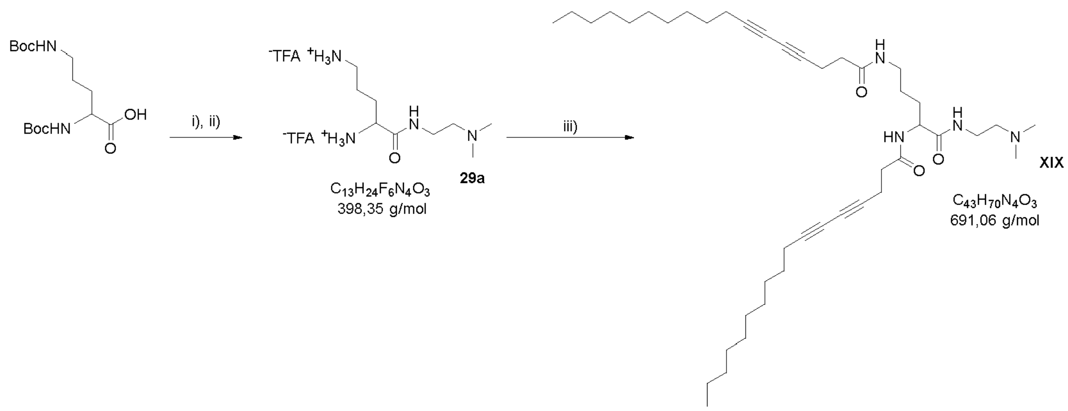


## [Figure 34]

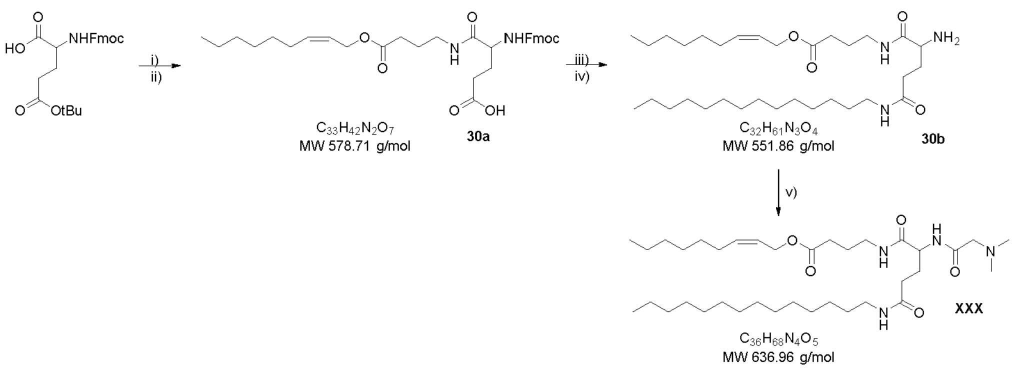

***[Figure 35]***

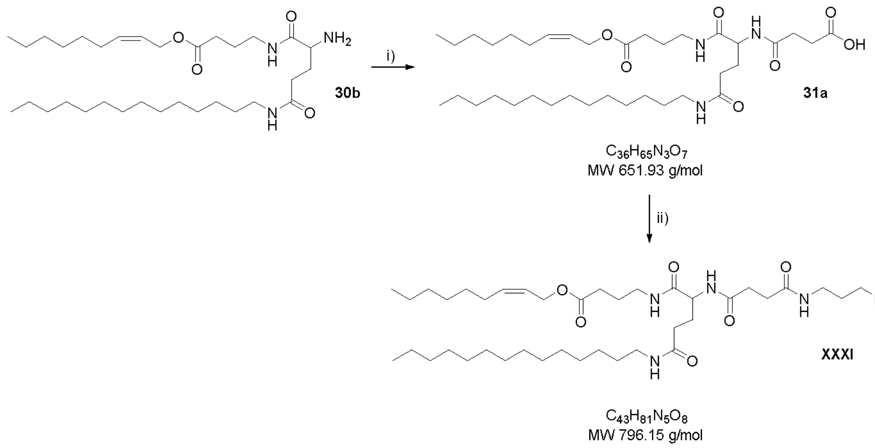


**[Figure 36]**

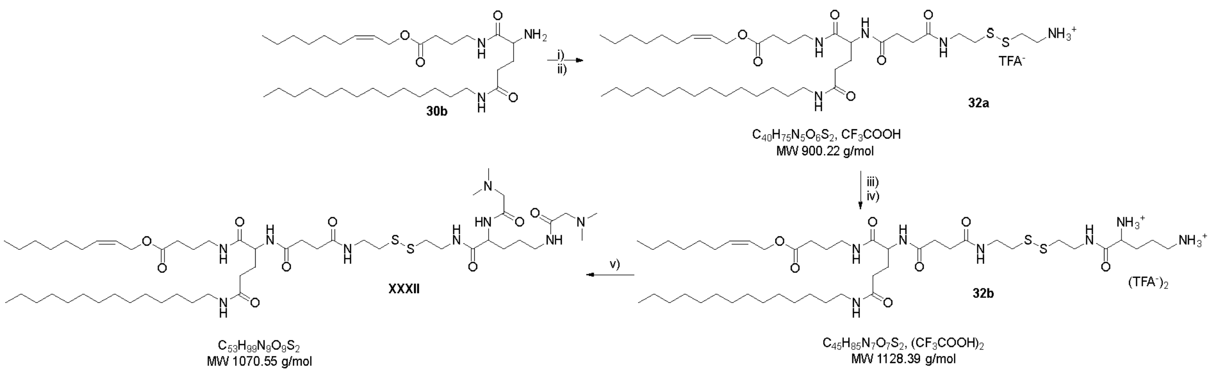


**[Figure 37]**

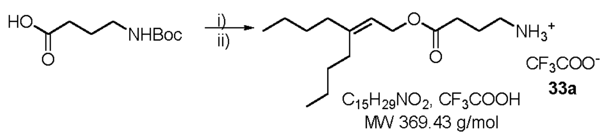

**[Figure 38]**

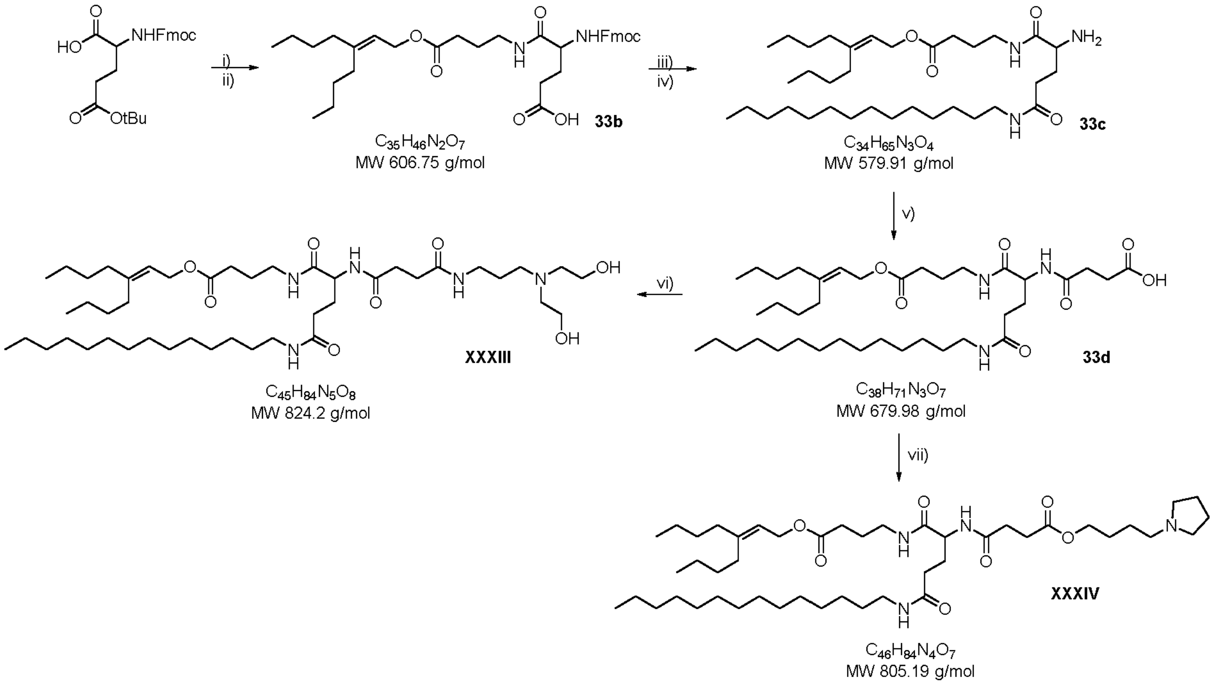


**[Figure 39]**

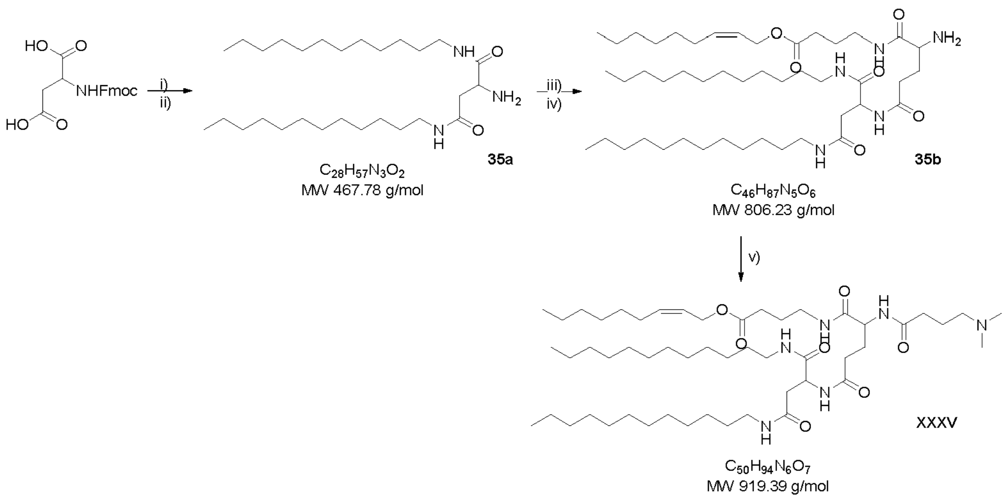

## [Figure 40]

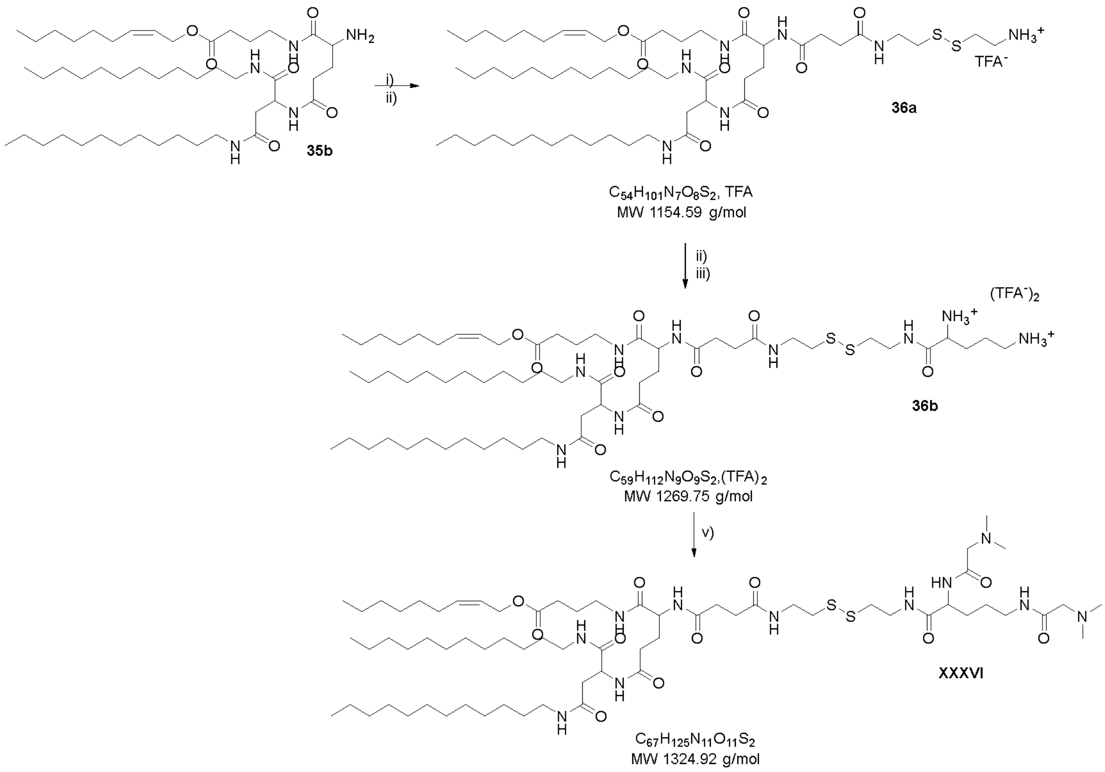


35b

36a

$C_{54}H_{101}N_7O_8S_2$, TFA
MW 1154.59 g/mol

36b

$C_{59}H_{112}N_9O_9S_2$,(TFA)$_2$
MW 1269.75 g/mol

XXXVI

$C_{67}H_{125}N_{11}O_{11}S_2$
MW 1324.92 g/mol

## [Figure 41]

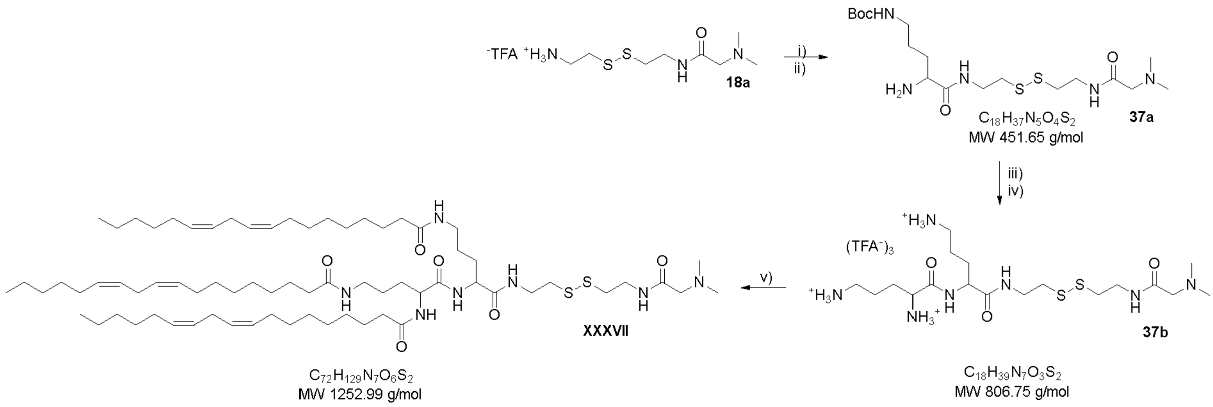


18a

37a

$C_{18}H_{37}N_5O_4S_2$
MW 451.65 g/mol

37b

$C_{18}H_{39}N_7O_3S_2$
MW 806.75 g/mol

XXXVII

$C_{72}H_{129}N_7O_6S_2$
MW 1252.99 g/mol

## [Figure 42]

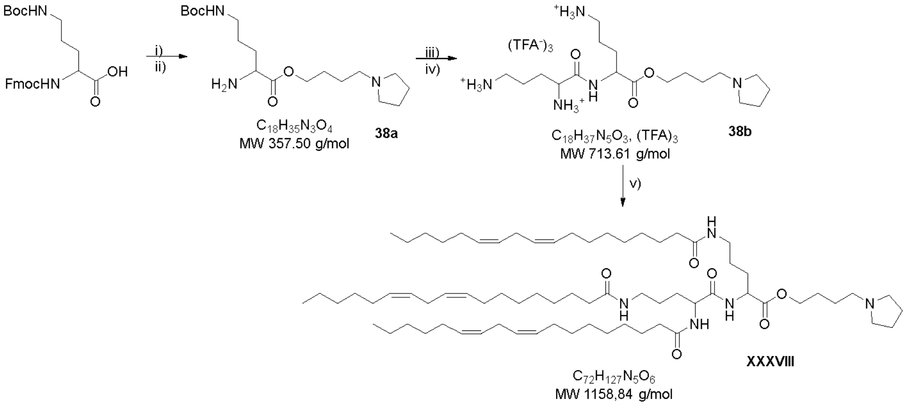


## [Figure 43]

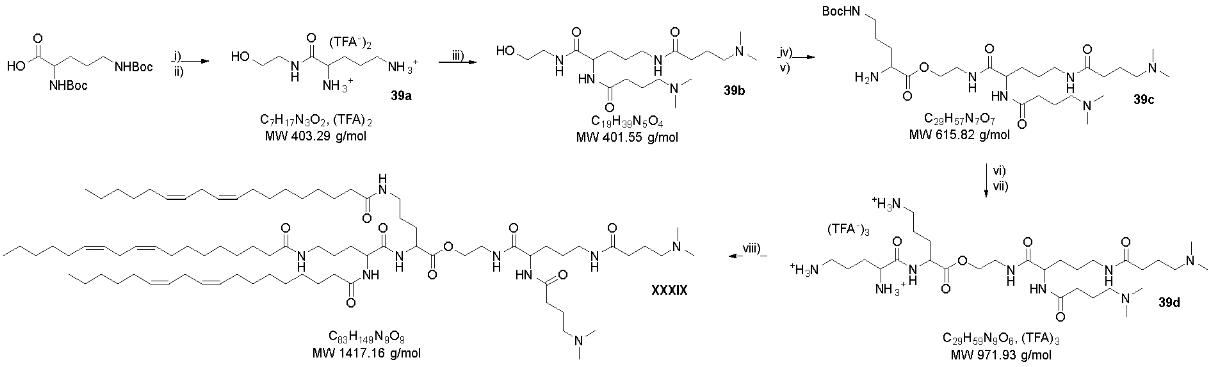


## [Figure 44]

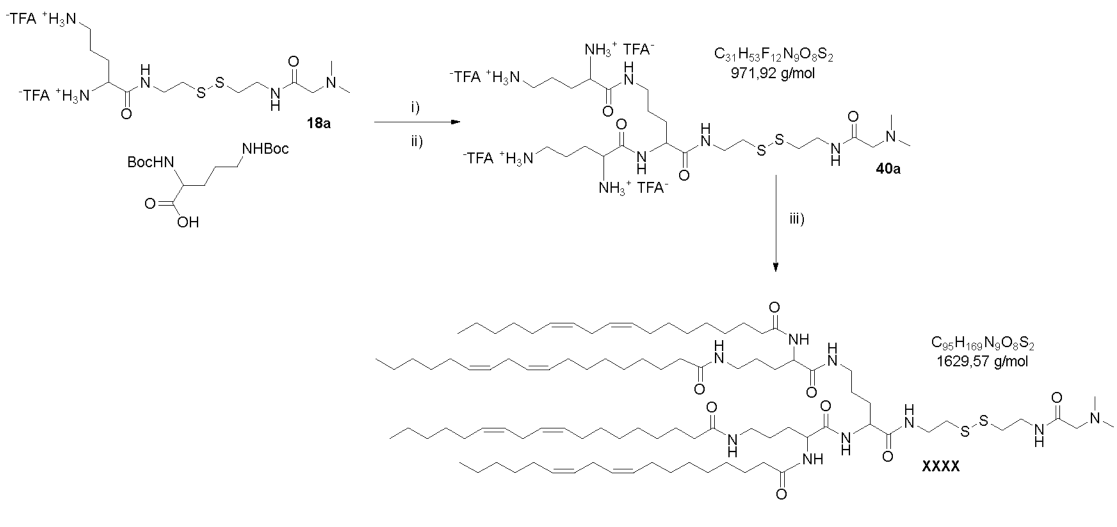

## [Figure 45]

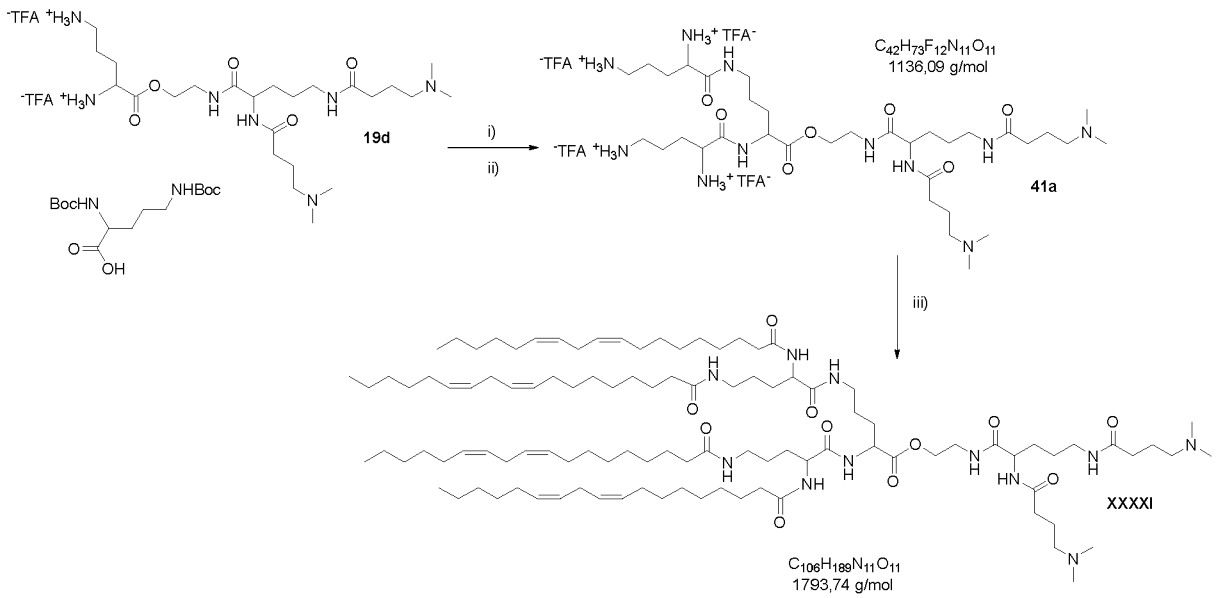


## [Figure 46]

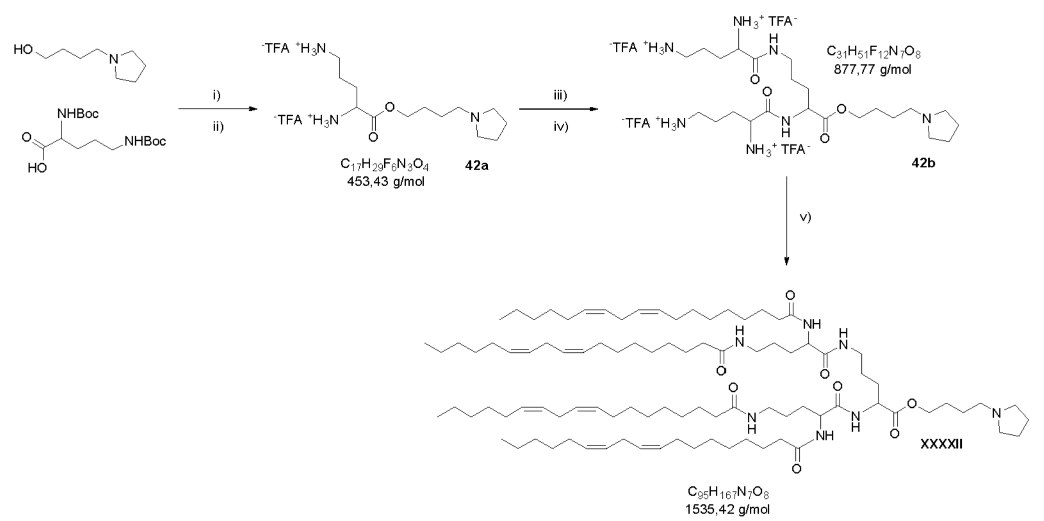


## [Figure 47]

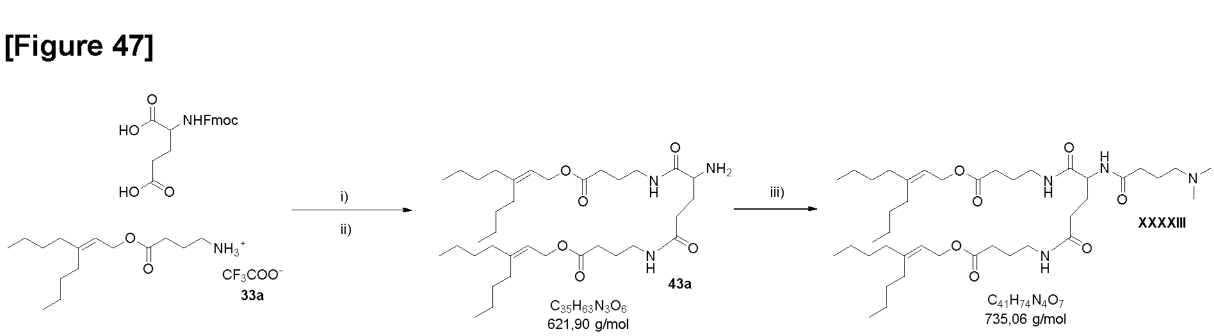

[Figure 48A]

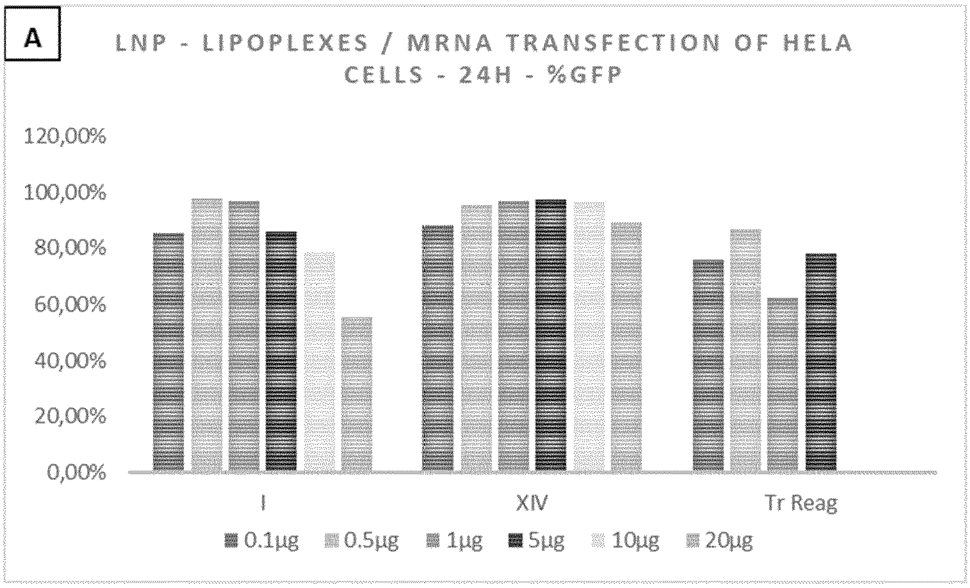


[Figure 48B]

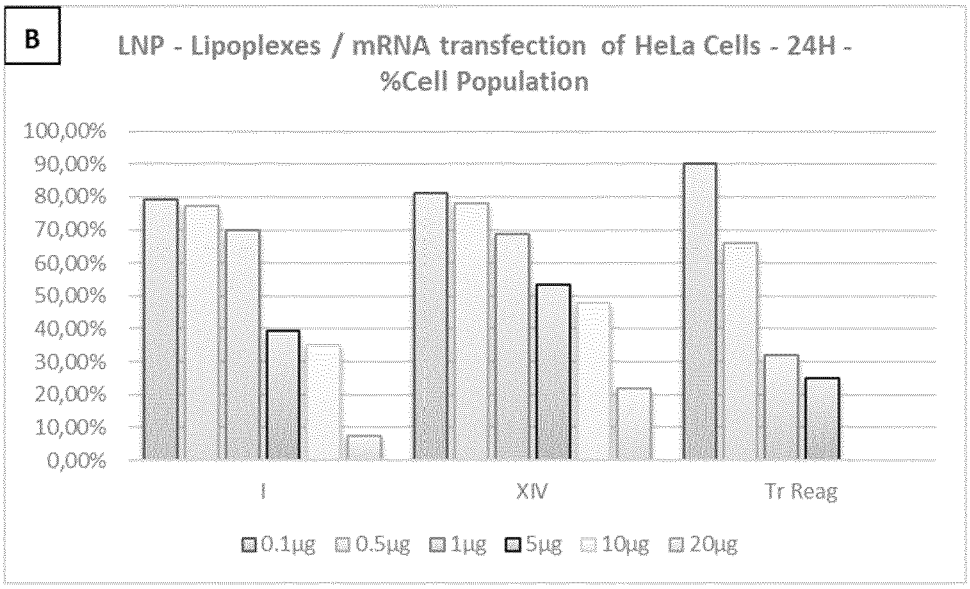

[Figure 49A]

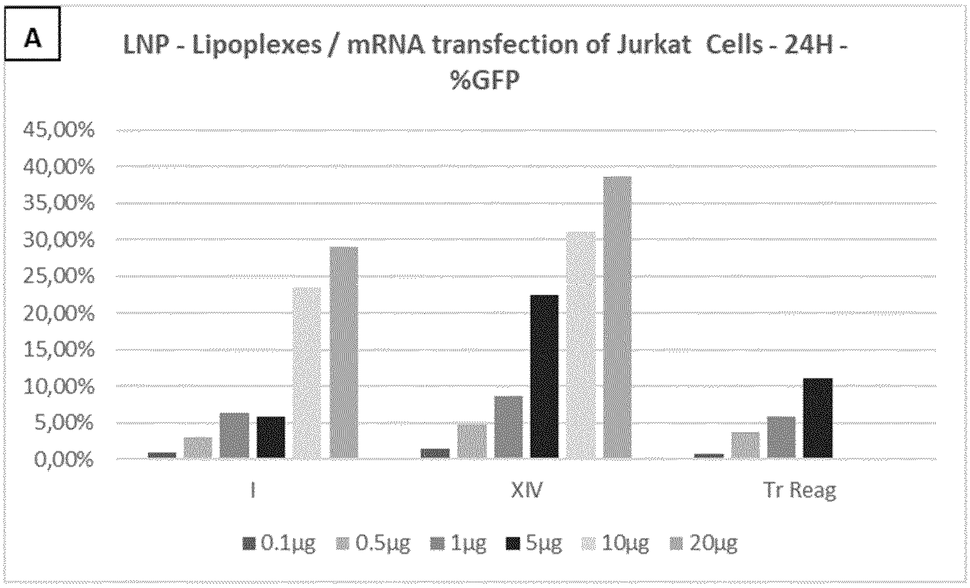


[Figure 49B]

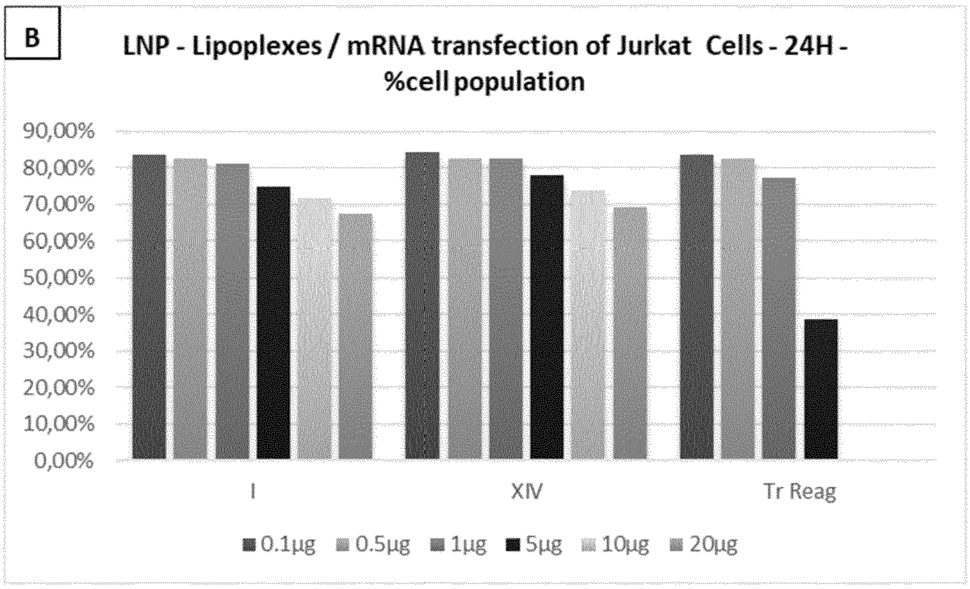

[Figure 50A]

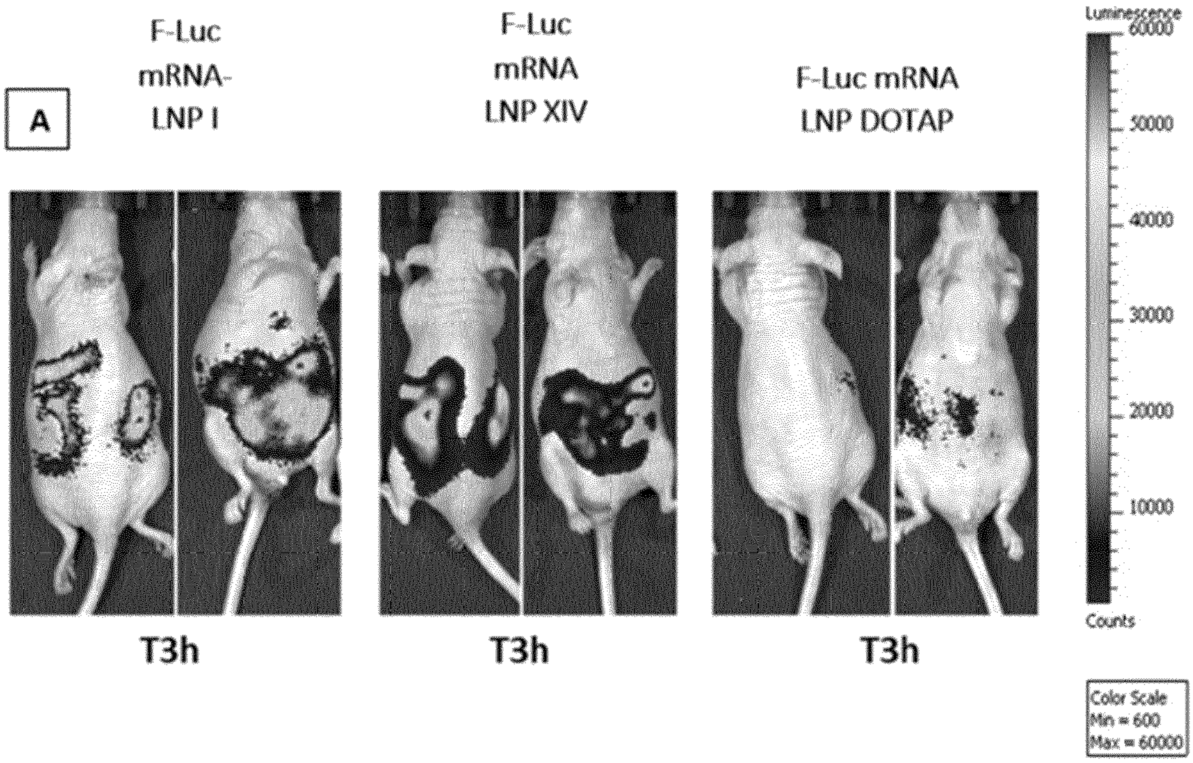


[Figure 50B]

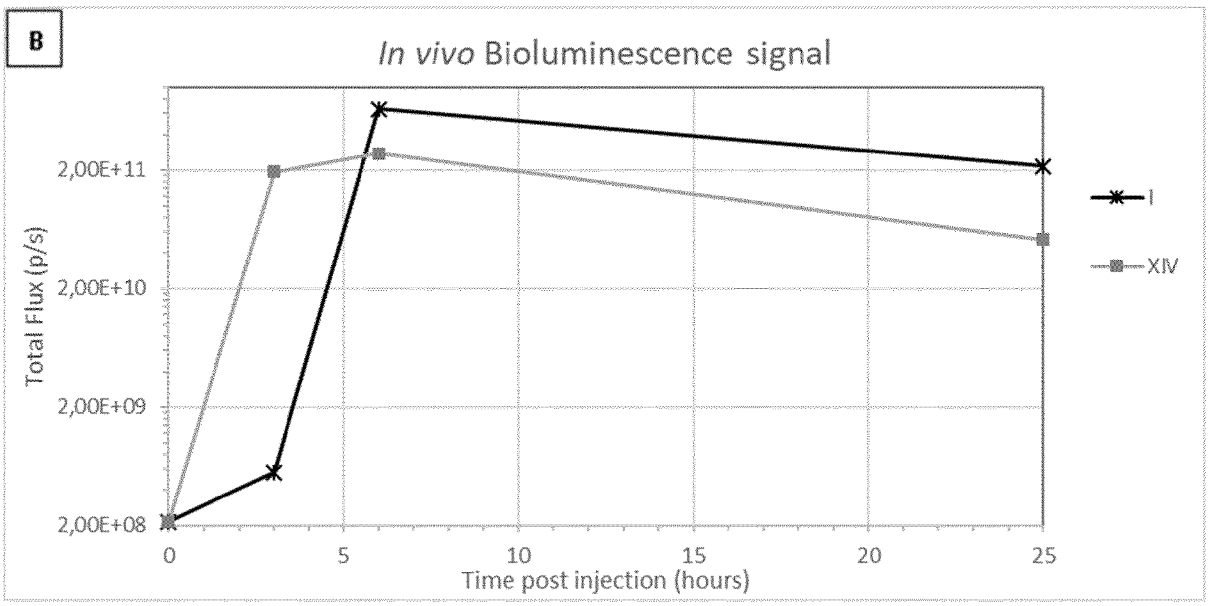

*[Figure 51A,B,C et D]*

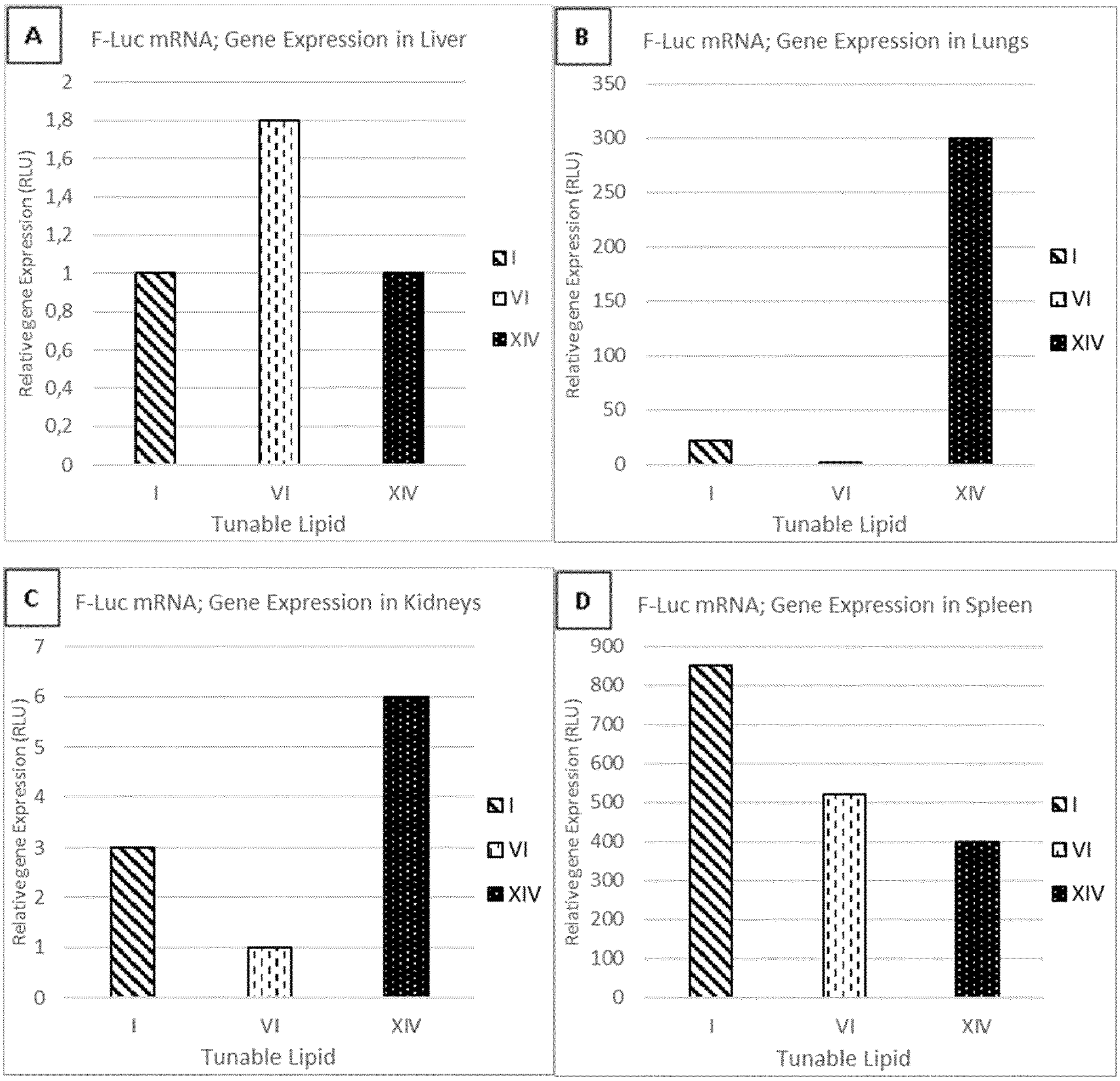

*[Figure 52A]*

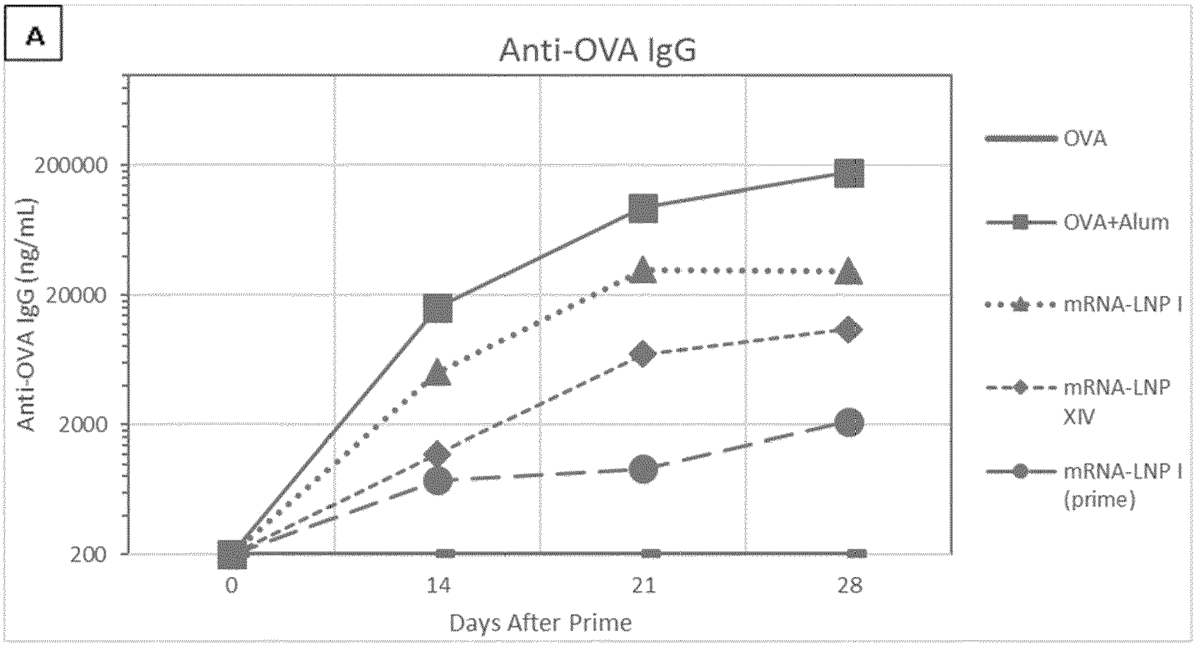


*[Figure 52B]*

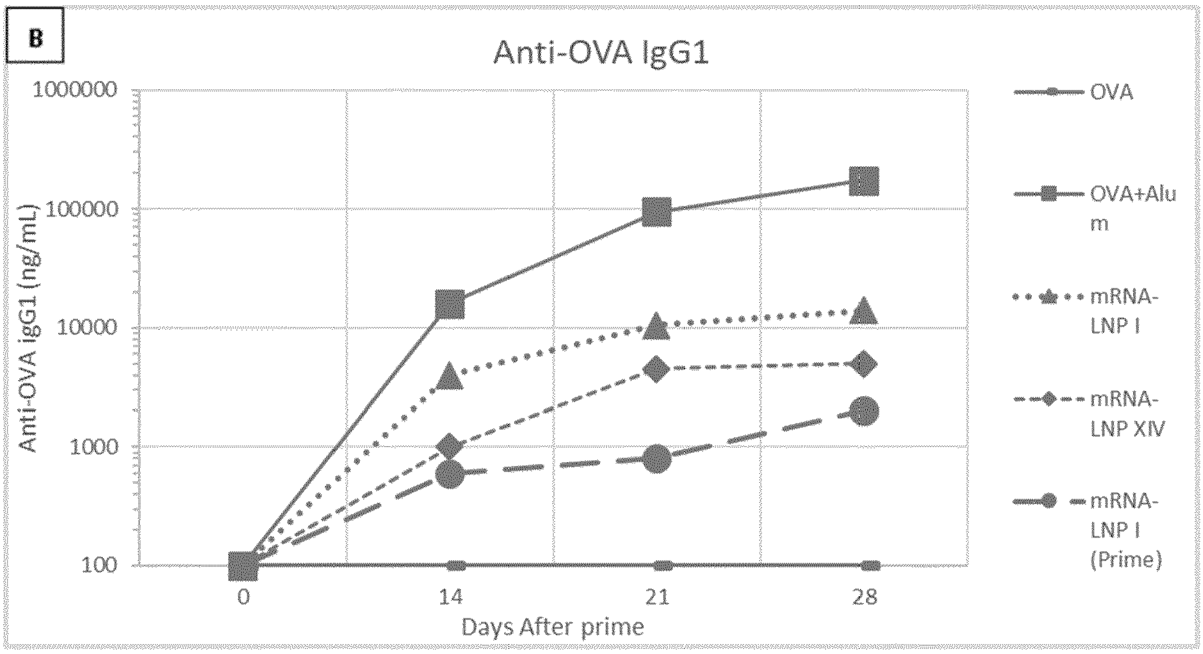

*[Figure 52C]*

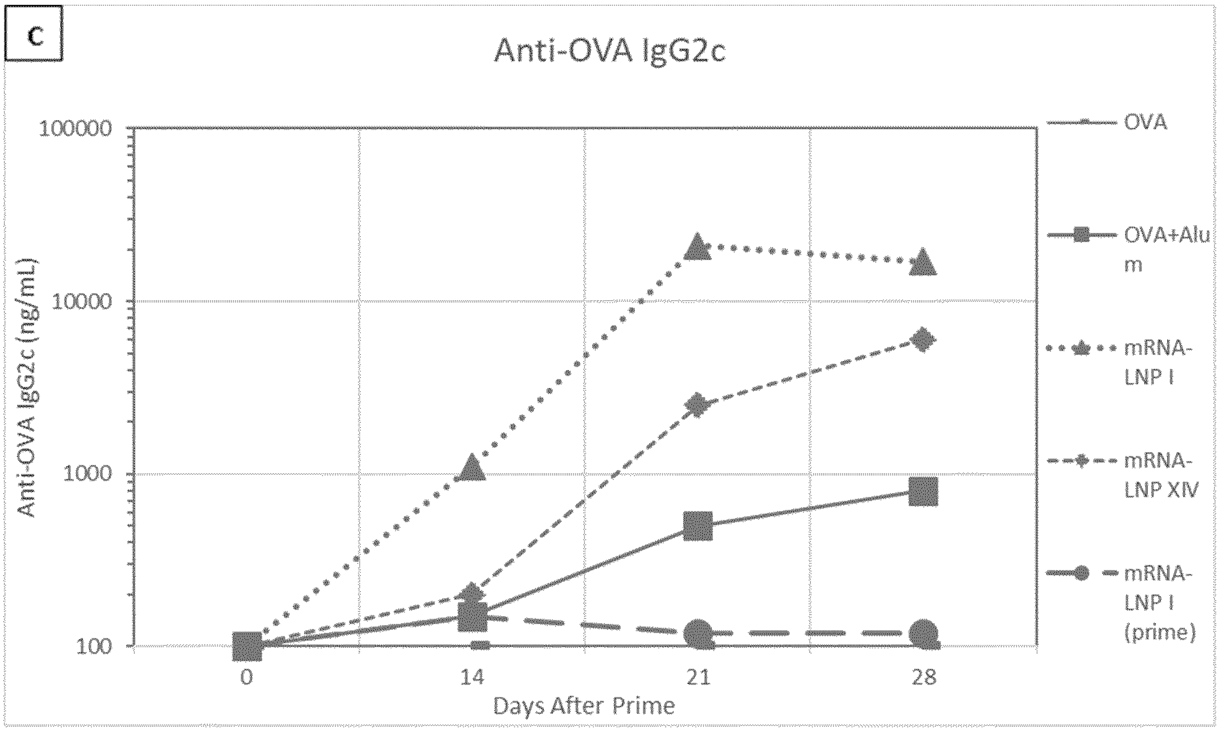


*[Figure 53A]*

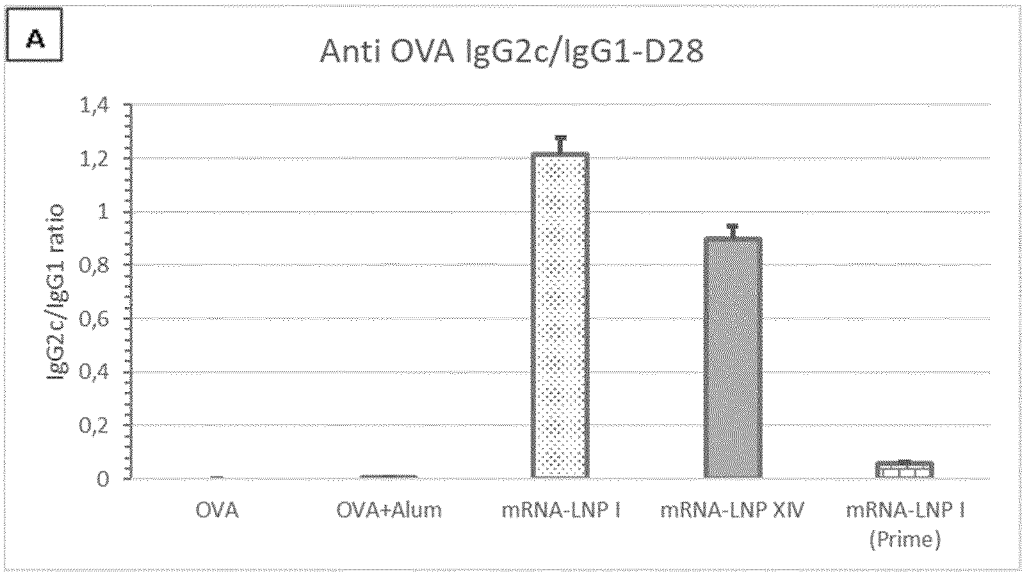

*[Figure 53B]*

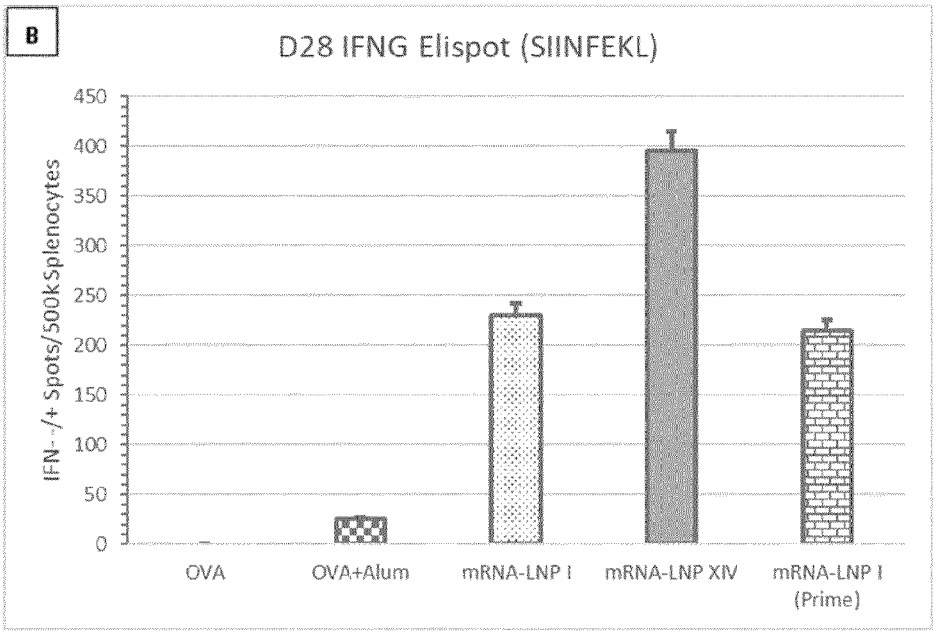


*[Figure 54]*

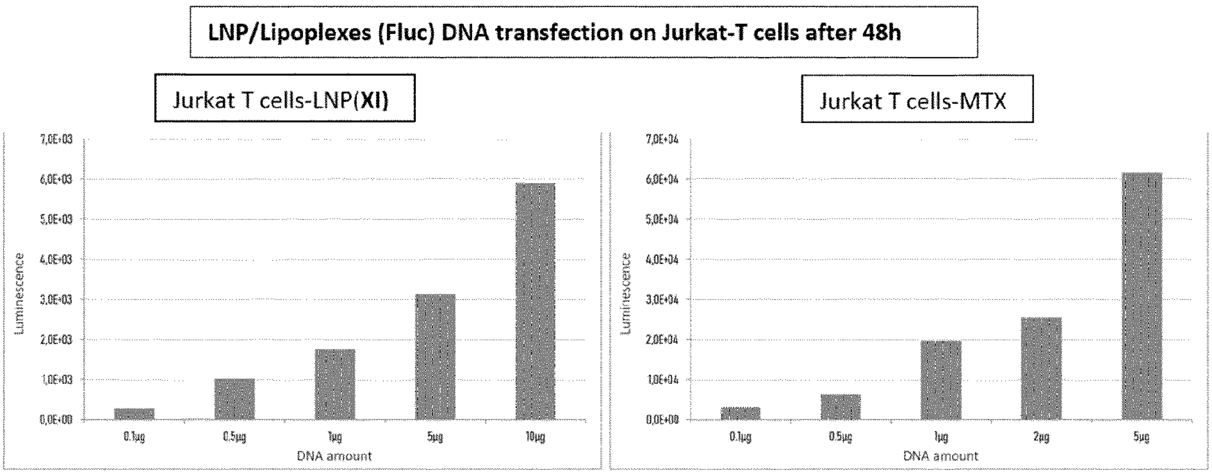

*[Figure 55]*

**SiRNA-LNPs Cellular uptake after 4 hours,**

**Cell line: HEK293**

LNP(**XI**)-DiO                LNP(**XI**)-Cy5-NTsiRNA

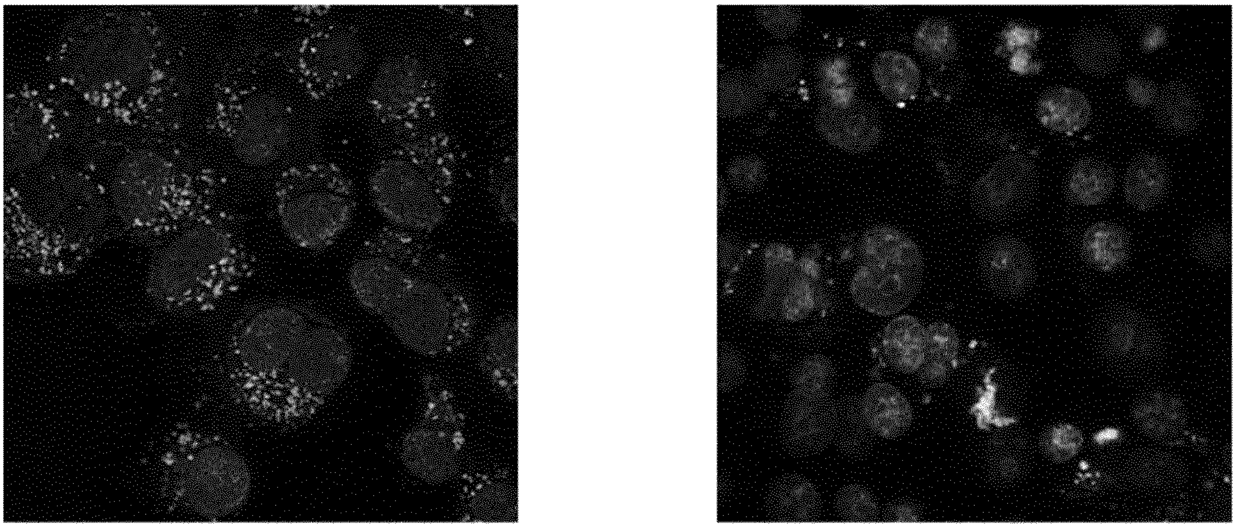

Europäisches Patentamt
European Patent Office
Office européen des brevets

# EUROPEAN SEARCH REPORT

Application Number
EP 23 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 285 772 B1 (OZ BIOSCIENCES SAS [FR]) 13 July 2022 (2022-07-13)<br>* page 40; claim 7; compound I.11 *<br>* claims 2,8-24 *<br>----- | 1-15 | INV.<br>C07C233/49<br>C07C237/12<br>C07C237/22<br>C07C271/16<br>C07D233/64<br>C07K5/00<br>C07D295/13<br>A61K47/54 |
| X | HOU X ET AL: "Lipid nanoparticles for mRNA delivery",<br>NATURE REVIEWS. MATERIALS,<br>vol. 6, no. 12,<br>10 August 2021 (2021-08-10), pages 1078-1094, XP093022718,<br>* abstract *<br>* page 1083; compound DOSPA *<br>----- | 1-15 | |
| X | WO 2022/013443 A1 (SANOFI PASTEUR [FR]) 20 January 2022 (2022-01-20)<br>* page 29; compounds III,V *<br>* page 30; compounds IV, VI-X *<br>* page 31; compounds XI-XIV *<br>* claims 18-30 *<br>----- | 1-15 | |
| X | WO 2021/155274 A1 (MODERNATX INC [US]; SMITH MIKE [US] ET AL.)<br>5 August 2021 (2021-08-05)<br>* page 97; compound 1 *<br>* claim 1 *<br>----- | 1-15 | TECHNICAL FIELDS SEARCHED (IPC)<br>C07C<br>C07D<br>C07K<br>A61K |
| X | EP 2 998 289 B1 (NITTO DENKO CORP [JP]) 7 August 2019 (2019-08-07)<br>* page 12; compound DSPE *<br>* page 79; claim 8 *<br>----- | 1-15 | |
| X | US 2013/123485 A1 (PARK MYUNG-OK [KR] ET AL) 16 May 2013 (2013-05-16)<br>* claim 6 *<br>-----<br>-/-- | 1-15 | |

The present search report has been drawn up for all claims

1

| Place of search | Date of completion of the search | Examiner |
|---|---|---|
| The Hague | 20 December 2023 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DHUMAL DINESH MOTILAL ET AL: "Development and evaluation of amphiphilic heterolipid based pH-sensitive nanomicelles of doxorubicin", JOURNAL OF DRUG DELIVERY SCIENCE AND TECHNOLOGY, vol. 68, 1 February 2022 (2022-02-01), page 103079, XP093113843, FR ISSN: 1773-2247, DOI: 10.1016/j.jddst.2021.103079 Retrieved from the Internet: URL:https://sdfestaticassets-eu-west-1.sciencedirectassets.com/shared-assets/67/images/1px.png?fr=cpcnjs> * abstract * * Figure 1, Series II * ----- | 1-15 | |
| X | CN 114 539 083 A (CHINESE PHARMACOLOGY UNIV) 27 May 2022 (2022-05-27) * abstract * * claim 1 * * compounds LA1-LA12; OA1-OA12; TA2-NH2-HCL * ----- -/-- | 1-15 | TECHNICAL FIELDS SEARCHED (IPC) |

The present search report has been drawn up for all claims

| Place of search | Date of completion of the search | Examiner |
|---|---|---|
| The Hague | 20 December 2023 | Seitner, Irmgard |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 23 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DHUMAL DINESH M. ET AL: "Experimentally Validated QSAR Model for Surface p K a Prediction of Heterolipids Having Potential as Delivery Materials for Nucleic Acid Therapeutics", ACS OMEGA, vol. 5, no. 49, 30 November 2020 (2020-11-30), pages 32023-32031, XP093113856, US ISSN: 2470-1343, DOI: 10.1021/acsomega.0c04931 Retrieved from the Internet: URL:http://pubs.acs.org/doi/pdf/10.1021/acsomega.0c04931> * abstract * * Figure 1; Series II * * scheme 2 * ----- | 1-15 | |
| X | CN 111 087 317 B (UNIV CHINA PHARMA) 14 April 2023 (2023-04-14) * abstract * * claim 1 * * paragraphs [0009], [0086], [0101] * ----- | 1-15 | TECHNICAL FIELDS SEARCHED (IPC) |
| X | CN 109 503 411 A (UNIV CHINA PHARMA) 22 March 2019 (2019-03-22) * abstract * * claim 1 * * compounds A1-A21 * ----- -/-- | 1-15 | |

The present search report has been drawn up for all claims

1

| Place of search | Date of completion of the search | Examiner |
|---|---|---|
| The Hague | 20 December 2023 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GANBOLD TSOGZOLMAA ET AL: "-peptide-based lipid nanoparticles", RSC ADVANCES, vol. 7, no. 15, 1 January 2017 (2017-01-01), pages 8823-8831, XP093113858, GB ISSN: 2046-2069, DOI: 10.1039/C6RA25862J * abstract * * scheme 1 * ----- | 1-15 | |
| X | WO 2016/210190 A1 (NITTO DENKO CORP [JP]; YING WENBIN [US]) 29 December 2016 (2016-12-29) * claims 15,30-40 * ----- | 1-15 | |
| X | NAKASHIMA TAKUYA ET AL: "Controlled self-assembly of amphiphiles in ionic liquids and the formation of ionogels by molecular tuning of cohesive energies", POLYMER JOURNAL, vol. 44, no. 6, 25 April 2012 (2012-04-25), pages 665-671, XP093113935, London ISSN: 0032-3896, DOI: 10.1038/pj.2012.73 * abstract * * figure 1 * ----- | 1-15 | TECHNICAL FIELDS SEARCHED (IPC) |
| X | CN 106 188 223 A (UNIV INNER MONGOLIA) 7 December 2016 (2016-12-07) * abstract * * claims 1-6 * ----- | 1-15 | |

The present search report has been drawn up for all claims

1

| Place of search | Date of completion of the search | Examiner |
|---|---|---|
| The Hague | 20 December 2023 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 30 5999

This annex lists the patent family members relating to the patent documents cited in the above-mentioned European search report. The members are as contained in the European Patent Office EDP file on
The European Patent Office is in no way liable for these particulars which are merely given for the purpose of information.

20-12-2023

| Patent document cited in search report | Publication date | Patent family member(s) | Publication date |
|---|---|---|---|
| EP 2285772 B1 | 13-07-2022 | CA 2708988 A1 | 03-09-2009 |
| | | EP 2285772 A2 | 23-02-2011 |
| | | FR 2925491 A1 | 26-06-2009 |
| | | JP 2011509074 A | 24-03-2011 |
| | | US 2012015865 A1 | 19-01-2012 |
| | | WO 2009106713 A2 | 03-09-2009 |
| WO 2022013443 A1 | 20-01-2022 | AU 2021308443 A1 | 16-03-2023 |
| | | BR 112023000855 A2 | 07-03-2023 |
| | | CA 3189385 A1 | 20-01-2022 |
| | | CN 116134020 A | 16-05-2023 |
| | | EP 4182302 A1 | 24-05-2023 |
| | | IL 299791 A | 01-03-2023 |
| | | JP 2023533864 A | 04-08-2023 |
| | | KR 20230041744 A | 24-03-2023 |
| | | US 2023295097 A1 | 21-09-2023 |
| | | WO 2022013443 A1 | 20-01-2022 |
| WO 2021155274 A1 | 05-08-2021 | AU 2021212262 A1 | 22-09-2022 |
| | | BR 112022014970 A2 | 20-09-2022 |
| | | CA 3169669 A1 | 05-08-2021 |
| | | CN 116133652 A | 16-05-2023 |
| | | EP 4096644 A1 | 07-12-2022 |
| | | IL 294866 A | 01-09-2022 |
| | | JP 2023513043 A | 30-03-2023 |
| | | KR 20220133957 A | 05-10-2022 |
| | | TW 202139976 A | 01-11-2021 |
| | | US 2023285297 A1 | 14-09-2023 |
| | | WO 2021155274 A1 | 05-08-2021 |
| EP 2998289 B1 | 07-08-2019 | AU 2012267467 A1 | 16-01-2014 |
| | | AU 2017203075 A1 | 01-06-2017 |
| | | CA 2837101 A1 | 13-12-2012 |
| | | CA 3094645 A1 | 13-12-2012 |
| | | CN 103857654 A | 11-06-2014 |
| | | CN 107043334 A | 15-08-2017 |
| | | CN 107082747 A | 22-08-2017 |
| | | CY 1117433 T1 | 26-04-2017 |
| | | CY 1123807 T1 | 29-10-2021 |
| | | DK 2718261 T3 | 29-03-2016 |
| | | DK 2998289 T3 | 16-09-2019 |
| | | EP 2718261 A2 | 16-04-2014 |
| | | EP 2998289 A1 | 23-03-2016 |
| | | ES 2570184 T3 | 17-05-2016 |
| | | ES 2743540 T3 | 19-02-2020 |
| | | HR P20191463 T1 | 15-11-2019 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 30 5999

This annex lists the patent family members relating to the patent documents cited in the above-mentioned European search report. The members are as contained in the European Patent Office EDP file on
The European Patent Office is in no way liable for these particulars which are merely given for the purpose of information.

20-12-2023

| Patent document cited in search report | Publication date | Patent family member(s) | Publication date |
|---|---|---|---|
| | | HU E045821 T2 | 28-01-2020 |
| | | JP 5873553 B2 | 01-03-2016 |
| | | JP 6232083 B2 | 15-11-2017 |
| | | JP 6590888 B2 | 16-10-2019 |
| | | JP 2014529328 A | 06-11-2014 |
| | | JP 2016153477 A | 25-08-2016 |
| | | JP 2018065804 A | 26-04-2018 |
| | | KR 20140033492 A | 18-03-2014 |
| | | KR 20180134424 A | 18-12-2018 |
| | | KR 20180134425 A | 18-12-2018 |
| | | LT 2998289 T | 25-10-2019 |
| | | PL 2718261 T3 | 31-08-2016 |
| | | PL 2998289 T3 | 31-01-2020 |
| | | PT 2998289 T | 19-09-2019 |
| | | RU 2013158456 A | 20-07-2015 |
| | | RU 2017135548 A | 08-02-2019 |
| | | SI 2998289 T1 | 29-11-2019 |
| | | TW 201313256 A | 01-04-2013 |
| | | TW 201711706 A | 01-04-2017 |
| | | WO 2012170952 A2 | 13-12-2012 |
| US 2013123485 A1 | 16-05-2013 | KR 20120009532 A | 02-02-2012 |
| | | US 2013123485 A1 | 16-05-2013 |
| | | WO 2012011693 A2 | 26-01-2012 |
| CN 114539083 A | 27-05-2022 | NONE | |
| CN 111087317 B | 14-04-2023 | NONE | |
| CN 109503411 A | 22-03-2019 | NONE | |
| WO 2016210190 A1 | 29-12-2016 | AU 2016281685 A1 | 18-01-2018 |
| | | CA 2990668 A1 | 29-12-2016 |
| | | CN 107708739 A | 16-02-2018 |
| | | EP 3313449 A1 | 02-05-2018 |
| | | EP 3686184 A2 | 29-07-2020 |
| | | ES 2828717 T3 | 27-05-2021 |
| | | HK 1253031 A1 | 06-06-2019 |
| | | JP 6480025 B2 | 06-03-2019 |
| | | JP 2018524330 A | 30-08-2018 |
| | | KR 20180021115 A | 28-02-2018 |
| | | PT 3313449 T | 30-10-2020 |
| | | RU 2018102536 A | 24-07-2019 |
| | | TW 201713614 A | 16-04-2017 |
| | | US 2016376229 A1 | 29-12-2016 |
| | | US 2019127318 A1 | 02-05-2019 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 23 30 5999

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2023

| Patent document cited in search report | Publication date | Patent family member(s) | Publication date |
|---|---|---|---|
| | | US 2022242820 A1 | 04-08-2022 |
| | | WO 2016210190 A1 | 29-12-2016 |
| CN 106188223 A | 07-12-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009106713 A3

### Non-patent literature cited in the description


- **HAN et al.** *Nature Communications*, 2021, vol. 12, 7233
- **HOU et al.** *Nature reviews materials*, 2021, vol. 6, 1078-1094
- **T.W. GREENE**. Greene's Protective Groups in Organic Synthesis. Wiley-Interscience, 1999
- **FRESHNEY, R.I.** Culture of Animal Cells: A Manual of Basic Technique. John Wiley & Sons, Inc., 2005